(19) Europäisches Patentamt
European Patent Office
Office européen des brevets

(11) EP 3 748 004 A1

(12) **EUROPEAN PATENT APPLICATION**

(43) Date of publication:
**09.12.2020 Bulletin 2020/50**

(21) Application number: **20172019.0**

(22) Date of filing: **01.04.2016**

(51) Int Cl.:
***C12N 15/113*** *(2010.01)* ***C12N 15/10*** *(2006.01)*
***C12N 9/22*** *(2006.01)* ***C12N 9/52*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2015 US 201562141833 P**
**18.03.2016 US 201662310479 P**

(62) Document number(s) of the earlier application(s) in accordance with Art. 76 EPC:
**16717520.7 / 3 277 816**

(71) Applicant: **Editas Medicine, Inc.**
**Cambridge, MA 02142 (US)**

(72) Inventors:
- **HSU, Patrick, David**
  **La Jolla, California 92037 (US)**
- **MAEDER, Morgan, Lee**
  **Jamaica Plain, Massachusetts 02130 (US)**
- **ODONNELL, Penrose**
  **Yarmouth, Maine 04096 (US)**
- **TYCKO, Joshua, C.**
  **Redwood city, California 94063 (US)**
- **HUSTON, Nicholas, C.**
  **Lawrence, New Jersey 08648 (US)**

(74) Representative: **Barker Brettell LLP**
**100 Hagley Road**
**Edgbaston**
**Birmingham B16 8QQ (GB)**

Remarks:
- The complete document including Reference Tables and the Sequence Listing can be downloaded from the EPO website
- This application was filed on 29-04-2020 as a divisional application to the application mentioned under INID code 62.
- Claims filed after the date of receipt of the divisional application (Rule 68(4) EPC).

(54) **CRISPR/CAS-RELATED METHODS AND COMPOSITIONS FOR TREATING DUCHENNE MUSCULAR DYSTROPHY AND BECKER MUSCULAR DYSTROPHY**

(57) CRISPR/CAS-related compositions and methods for treatment of DMD, BMD, or DCM type 3B are described.

**Fig. 1A**

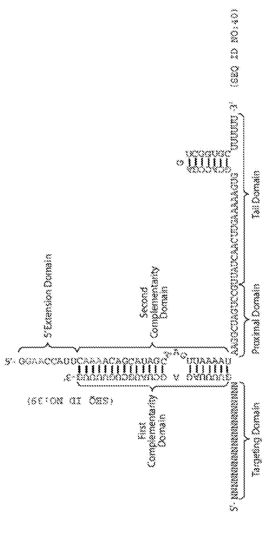


EP 3 748 004 A1

## Description

## PRIORITY CLAIM

This application claims priority to United States Provisional Application No. 62/141,833, filed April 1, 2015, and United States Provisional Application No. 62/310,479, filed March 18, 2016, the contents of which are hereby incorporated by reference in their entirety herein.

## SEQUENCE LISTING

The present specification makes reference to a Sequence Listing (submitted electronically as a .txt file named "084177.0121_ST25" on April 1, 2016). The .txt file was generated on March 30, 2016 and is 157,009,229 bytes in size. The entire contents of the Sequence Listing are hereby incorporated by reference.

## FIELD OF INVENTION

The invention relates to CRISPR/CAS-related methods, compositions and genome-editing systems for editing of a target nucleic acid sequence, e.g., editing a *DMD* gene (e.g., human *DMD* gene), and applications thereof in connection with Duchenne Muscular Dystrophy (DMD), Becker Muscular Dystrophy (BMD) and dilated cardiomyopathy (DCM).

## BACKGROUND

Duchenne muscular dystrophy (DMD) is a severe, progressive inherited muscular dystrophy. Becker muscular dystrophy (BMD) is a moderately severe, progressive inherited muscular dystrophy. Dilated cardiomyopathy (DCM) type 3B (also called DMD-associated dilated cardiomyopathy or DCM3B) is a severe, progressive disease of cardiac muscle.

DMD, BMD and DCM type 3B result from mutations in the *DMD* gene. The *DMD* gene is located on the X chromosome at locus Xp21. The *DMD* gene is 2.2 megabases in length and comprises 79 exons (Roberts et al., Genomics 1993: 16:536-8). DMD gene encodes a dystrophin protein, which is over 3500 amino acids in length. Dystrophin is an essential structural protein expressed in muscle cells that connects the cytoskeleton to the extracellular matrix (Ervasti et al., Journal of Cell Biology 1993; 122(4): 809-823). In subjects with DMD, BMD and DCM type 3B, mutations in *DMD* gene lead to a lack of dystrophin expression that causes necrosis and eventual fibrosis of muscle tissue.

DMD is one of the most common fatal genetic disorders diagnosed in childhood. It affects 1 in 3500 boys worldwide (Drousiotou et al., Genetic Testing 1998; 2:55-60; Emery, Neuromuscular Disorders 1991; 1:19-29; Bradley et al., Seminars in Neonatology 1998; 3:27-34; Emery. "Duchenne Muscular Dystrophy" 2nd Ed. New York: Oxford University Press; 1988.). Annually, about 20,000 infants are diagnosed with the disease worldwide. BMD affects 3-6 in every 100,000 births. DMD, BMD and DCM type 3B mainly affect boys; female carriers are rarely affected.

There are a variety of mutations in the *DMD* gene that cause DMD, BMD and DCM type 3B. 70% of subjects with DMD or BMD have mutations in the region of exons 44-55, representing a mutational hotspot (Kunkel et al., Nature 1986; 322:73-75). This region corresponds to the rod domain of dystrophin. Nearly 30% of subjects with DMD or BMD have mutations in the region of exons 2-20 (Wapennar et al., Genomics 1998; 2:10-18). About 60% of the mutations in *DMD* gene are large deletions and 5% of the mutations are duplications (Bennett Ibid). Approximately 35% of the mutations are point mutations causing premature termination codons due to nonsense or frame-shift mutations. Mutations may also be aberrant splice acceptor sites or aberrant splice donor sites (White et al., American Journal of Human Genetics 2002; 71:365-374).

Subjects with DMD develop muscle weakness between ages 2 and 5 and lose the ability to walk in their teens, requiring the use of a wheelchair (Boland et al., Pediatric Neurology 1996; 14(1):7-12). Death most commonly occurs in the third decade due to dilated cardiomyopathy or respiratory failure (Sussman et al., American Academy of Orthopedic Surgery 2002; 10(2): 138-151). Subjects with BMD have a later onset of muscle weakness (or may have no muscle weakness). BMD causes death in most subjects in the mid-40s due to dilated cardiomyopathy.

Dilated cardiomyopathy affects subjects with DMD, BMD and DCM type 3B. Males present with dilated cardiomyopathy between age 20 and 40; females present later in life. After presentation, cardiomyopathy-related heart failure leads to rapid progression to death in males and death after approximately 10 years in females (Beggs, Circulation 1997; 95:2344-2347).

Currently, no treatments fully prevent the progression of disease and eventual death in DMD, BMD and DCM type 3B. Steroids slightly slow the progression of disease and time to loss of ambulation (Manzur et al., Cochrane Database of Systemic Reviews 2008; (1):CD003725.). Ventilatory support also provides some prolongation of life as smooth muscles of the diaphragm fail (Manzur et al., Archives of Diseases of Childhood 2008; 93(11):986-990; Villanova

et al., American Journal of Physical Medicine and Rehabilitation 2014; 93(7):595-599). ACE inhibitors and beta-blockers can be administered to treat dilated cardiomyopathy, and may offer slight benefit in slowing the progression to heart failure (Colan, Circulation 2005; 112:2756-2758. Duboc et al., American Heart Journal 2007; 154:596-602).

Modern gene therapy approaches have faced significant barriers due to the size of the *DMD* gene; delivery of such a large gene has not proven feasible. Oligonucleotide-based exon skipping therapies are in development and may delay progression of disease. However, there are barriers to the long-term success of these approaches. These therapies are likely to require repeat administration annually or every several years to ensure continued presence of the exon skipping machinery (oligonucleotides and/or proteins) in muscle cells. The administration of exon skipping therapies or gene therapies requires multiple (e.g., once every 1-5 year basis) intramuscular (IM) injections of viral vectors to access muscle cells. All of these consequences of repeat administration threaten to compromise the efficacy of the therapy. Hence, there is a need for improved treatment of DMD, BMD and cardiomyopathy type 3B.

## SUMMARY OF THE INVENTION

The methods, genome-editing systems, and compositions discussed herein provide for treating or delaying the onset or progression of diseases of skeletal, cardiac and smooth muscle, e.g. disorders that affect skeletal muscle cells, cardiomyocytes, cardiac muscle cells and smooth muscle cells.

The methods , genome-editing systems, and compositions discussed herein provide for treating or delaying the onset or progression of Duchenne muscular dystrophy (DMD), Becker muscular dystrophy (BMD), dilated cardiomyopathy (DCM) type 3B, or a symptom associated thereof (e.g., dilated cardiomyopathy, e.g., DMD-associated dilated cardiomyopathy). DMD, BMD and DCM type 3B can be caused by a mutation in the *DMD* gene, e.g., a deletion, a duplication, a nonsense, a frameshift, or a splice site donor or splice site acceptor mutation in the *DMD* gene. The *DMD* gene is also known as: *BMD; CMD3B; MRX85; DXS142; DXS164; DXS206; DXS230; DXS239; DXS268; DXS269; DXS270; or DXS272.*

In certain embodiments, methods, genome-editing systems, and compositions discussed herein provide for altering a DMD target position in the *DMD* gene. In certain embodiments, the methods, genome-editing systems, and compositions described herein introduce one or more breaks near the site of the DMD target position in at least one allele of the *DMD* gene. Altering the DMD target position refers to: (1) break-induced deletion (e.g., NHEJ-mediated deletion) of genomic sequence including the DMD target position; or (2) break-induced introduction of an indel (e.g., NHEJ-mediated introduction of an indel) in close proximity to or including the DMD target position. Either approach can give rise to a dystrophin sequence that has correct reading frame.

In certain embodiments disclosed herein, the *DMD* gene can be altered by gene editing, e.g., using CRISPR-Cas9 mediated methods as described herein. Methods and compositions discussed herein provide for altering a DMD target position in the *DMD* gene. The alteration of the *DMD* gene can be mediated by any mechanism. Exemplary mechanisms that can be associated with the alteration of the *DMD* gene include, but are not limited to, non-homologous end joining (e.g., classical or alternative), microhomology-mediated end joining (MMEJ), SDSA (synthesis dependent strand annealing) or single strand annealing or single strand invasion.

In certain embodiments, a single strand break is introduced (e.g., positioned by one gRNA molecule) at or in close proximity to a DMD target position in the *DMD* gene. In certain embodiments, a single gRNA molecule (e.g., with a Cas9 nickase) is used to create a single strand break at or in close proximity to the DMD target position, e.g., the gRNA is configured such that the single strand break is positioned either upstream (e.g., within 500, 400, 300, 200, 100, 50, 25, or 10 bp upstream) or downstream (e.g., within 500, 400, 300, 200, 100, 50, 25, or 10 bp downstream) of the DMD target position. In certain embodiments, the break is positioned to avoid unwanted target chromosome elements, such as repeat elements, e.g., an Alu repeat.

In certain embodiments, a double strand break is introduced (e.g., positioned by one gRNA molecule) at or in close proximity to a DMD target position in the *DMD* gene. In certain embodiments, a single gRNA molecule (e.g., with a Cas9 nuclease other than a Cas9 nickase) is used to create a double strand break at or in close proximity to the DMD target position, e.g., the gRNA molecule is configured such that the double strand break is positioned either upstream (e.g., within 500, 400, 300, 200, 100, 50, 25, or 10 bp upstream) or downstream of (e.g., within 500, 400, 300, 200, 100, 50, 25, or 10 bp downstream) of a DMD target position. In certain embodiments, the break is positioned to avoid unwanted target chromosome elements, such as repeat elements, e.g., an Alu repeat.

In certain embodiments, two single strand breaks are introduced (e.g., positioned by two gRNA molecules) at or in close proximity to a DMD target position in the *DMD* gene. In certain embodiments, two gRNA molecules (e.g., with one or two Cas9 nickcases) are used to create two single strand breaks at or in close proximity to the DMD target position, e.g., the gRNAs molecules are configured such that both of the single strand breaks are positioned upstream (e.g., within 500, 400, 300, 200, 100, 50, 25, or 10 bp upstream) or downstream (e.g., within 500, 400, 300, 200, 100, 50, 25, or 10 bp downstream) of the DMD target position. In certain embodiments, two gRNA molecules (e.g., with two Cas9 nickcases) are used to create two single strand breaks at or in close proximity to the DMD target position, e.g.,

the gRNAs molecules are configured such that one single strand break is positioned upstream (e.g., within 500, 400, 300, 200, 100, 50, 25, or 10 bp upstream) and a second single strand break is positioned downstream (e.g., within 500, 400, 300, 200, 100, 50, 25, or 10 bp downstream) of the DMD target position. In certain embodiments, the breaks are positioned to avoid unwanted target chromosome elements, such as repeat elements, e.g., an Alu repeat.

In certain embodiments, two double strand breaks are introduced (e.g., positioned by two gRNA molecules) at or in close proximity to a DMD target position in the *DMD* gene. In certain embodiments, two gRNA molecules (e.g., with one or two Cas9 nucleases that are not Cas9 nickases) are used to create two double strand breaks to flank a DMD target position, e.g., the gRNA molecules are configured such that one double strand break is positioned upstream (e.g., within 500, 400, 300, 200, 100, 50, 25, or 10 bp upstream) and a second double strand break is positioned downstream (e.g., within 500, 400, 300, 200, 100, 50, 25, or 10 bp downstream) of the DMD target position. In certain embodiments, the breaks are positioned to avoid unwanted target chromosome elements, such as repeat elements, e.g., an Alu repeat.

In certain embodiments, one double strand break and two single strand breaks are introduced (e.g., positioned by three gRNA molecules) at or in close proximity to a DMD target position in the *DMD* gene. In certain embodiments, three gRNA molecules (e.g., with a Cas9 nuclease other than a Cas9 nickase and one or two Cas9 nickases) to create one double strand break and two single strand breaks to flank a DMD target position, e.g., the gRNA molecules are configured such that the double strand break is positioned upstream or downstream of (e.g., within 500, 400, 300, 200, 100, 50, 25, or 10 bp upstream or downstream) of the DMD target position, and the two single strand breaks are positioned at the opposite site, e.g., downstream or upstream (within 200 bp downstream or upstream), of the DMD target position. In certain embodiments, the breaks are positioned to avoid unwanted target chromosome elements, such as repeat elements, e.g., an Alu repeat.

In certain embodiments, four single strand breaks are introduced (e.g., positioned by four gRNA molecules) at or in close proximity to a DMD target position in the *DMD* gene. In certain embodiments, four gRNA molecule (e.g., with one or more Cas9 nickases are used to create four single strand breaks to flank a DMD target position in the *DMD* gene, e.g., the gRNA molecules are configured such that a first and second single strand breaks are positioned upstream (e.g., within 500, 400, 300, 200, 100, 50, 25, or 10 bp upstream) of the DMD target position, and a third and a fourth single stranded breaks are positioned downstream (e.g., within 500, 400, 300, 200, 100, 50, 25, or 10 bp downstream) of the DMD target position. In certain embodiments, the breaks are positioned to avoid unwanted target chromosome elements, such as repeat elements, e.g., an Alu repeat.

In certain embodiments, two or more (e.g., three or four) gRNA molecules are used with one Cas9 molecule. In certain embodiments, when two or more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

When two or more gRNAs are used to position two or more cleavage events, e.g., double strand or single strand breaks, in a target nucleic acid, it is contemplated that the two or more cleavage events may be made by the same or different Cas9 proteins. For example, when two gRNAs are used to position two double strand breaks, a single Cas9 nuclease may be used to create both double strand breaks. When two or more gRNAs are used to position two or more single stranded breaks (single strand breaks), a single Cas9 nickase may be used to create the two or more single strand breaks. When two or more gRNAs are used to position at least one double strand break and at least one single strand break, two Cas9 proteins may be used, e.g., one Cas9 nuclease and one Cas9 nickase. It is contemplated that when two or more Cas9 proteins are used that the two or more Cas9 proteins may be delivered sequentially to control specificity of a double strand versus a single strand break at the desired position in the target nucleic acid.

In certain embodiments, the targeting domain of the first gRNA molecule and the targeting domain of the second gRNA molecule hybridize to the target domain through complementary base pairing to opposite strands of the target nucleic acid molecule. In certain embodiments, the gRNA molecule and the second gRNA molecule are configured such that the PAMs are oriented outward.

In certain embodiments, the targeting domain of a gRNA molecule is configured to avoid unwanted target chromosome elements, such as repeat elements, e.g., an Alu repeat, or the endogenous *DMD* splice sites, in the target domain. The gRNA molecule may be a first, second, third and/or fourth gRNA molecule.

In certain embodiments, the targeting domain of a gRNA molecule is configured to position a cleavage event sufficiently far from a preselected nucleotide, e.g., the nucleotide of a coding region, such that the nucleotide is not altered. In certain embodiments, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In certain embodiments, two or more gRNAs (e.g., a pair of gRNAs) are used to position breaks, e.g., two single stranded breaks or two double stranded breaks, or a combination of single strand and double strand breaks, e.g., to create one or more indels or deletions, in the *DMD* gene sequence, wherein the targeting domains of each gRNAs can

comprise nucleotide sequences set forth in SEQ ID NOs:206-826366.

The presently disclosed subject matter provides for a gRNA molecule, e.g., an isolated or non-naturally occurring gRNA molecule, comprising a targeting domain which is complementary with a target domain from the *DMD* gene.

In certain embodiments, the targeting domain of the gRNA molecule is configured to target an exon of the *DMD* gene. In certain embodiments, the targeting domain of the gRNA molecule is configured to target exon 51 of the *DMD* gene. In certain embodiments, the targeting domain of the gRNA molecule is configured to target an intron of the *DMD* gene. In certain embodiments, the targeting domain of the gRNA molecule is configured to target intron 50 or intron 51 of the *DMD* gene. In certain embodiments, the targeting domain comprises a sequence that is identical to, or differs by no more than 1, no more than 2, no more than 3, no more than 4 or no more than 5 nucleotides from a nucleotide sequence selected from SEQ ID NOs: 206-826366. In certain embodiments, the targeting domain comprises or consists of a nucleotide sequence selected from SEQ ID NOs: 206-826366. In certain embodiments, the gRNA is a unimodular gRNA molecule. In certain embodiments, the gRNA is a chimeric gRNA molecule.

In certain embodiments, the targeting domain is 16 or more nucleotides in length. In certain embodiments, the targeting domain is 17 nucleotides in length. In certain embodiments, the targeting domain is 18 nucleotides in length. In certain embodiments, the targeting domain is 19 nucleotides in length. In certain embodiments, the targeting domain is 20 nucleotides in length. In certain embodiments, the targeting domain is 21 nucleotides in length. In certain embodiments, the targeting domain is 22 nucleotides in length. In certain embodiments, the targeting domain is 23 nucleotides in length. In certain embodiments, the targeting domain is 24 nucleotides in length. In certain embodiments, the targeting domain is 25 nucleotides in length. In certain embodiments, the targeting domain is 26 nucleotides in length.

In certain embodiments, the gRNA comprises from 5' to 3': a targeting domain (comprising a "core domain", and optionally a "secondary domain"); a first complementarity domain; a linking domain; a second complementarity domain; a proximal domain; and a tail domain. In certain embodiments, the proximal domain and tail domain are taken together as a single domain.

In certain embodiments, the gRNA comprises a linking domain of no more than 25 nucleotides in length; a proximal and tail domain, that taken together, are at least 20, at least 30, or at least 40 nucleotides in length; and a targeting domain of 16, 17, 18, 19, 20, 21, 22, 23, 24, 25 or 26 nucleotides in length.

A cleavage event, e.g., a double strand or single strand break, is generated by a Cas9 molecule. In certain embodiments, the Cas9 molecule forms a double strand break or a single strand break in a target nucleic acid (e.g., a nickase molecule). In certain embodiments, the Cas9 molecule catalyzes a double strand break.

In certain embodiments, the Cas9 molecule comprises HNH-like domain cleavage activity but has no, or no significant, N-terminal RuvC-like domain cleavage activity. In certain embodiments, the Cas9 molecule is an HNH-like domain nickase, e.g., the Cas9 molecule comprises a mutation at D10, e.g., D10A. In certain embodiments, the Cas9 molecule comprises N-terminal RuvC-like domain cleavage activity but has no, or no significant, HNH-like domain cleavage activity. In certain embodiments, the Cas9 molecule is an N-terminal RuvC-like domain nickase, e.g., the Cas9 molecule comprises a mutation at H840, e.g., H840A. In certain embodiments, the Cas9 molecule is an N-terminal RuvC-like domain nickase, e.g., the Cas9 molecule comprises a mutation at N863, e.g., the N863A mutation.

In certain embodiments, a single strand break is formed in the strand of the target nucleic acid to which the targeting domain of said gRNA is complementary. In certain embodiments, a single strand break is formed in the strand of the target nucleic acid other than the strand to which the targeting domain of said gRNA is complementary.

The presently disclosed subject matter also provides for a nucleic acid composition, e.g., an isolated or non-naturally occurring nucleic acid composition, e.g., DNA, that comprises (a) a first nucleotide sequence that encodes a first gRNA molecule comprising a targeting domain that is complementary with a target domain in *DMD* gene as disclosed herein.

In certain embodiments, the nucleic acid composition further comprises (b) a second nucleotide sequence that encodes a Cas9 molecule as described above. In certain embodiments, a nucleic acid composition further comprises (c) a third nucleotide sequence that encodes a second gRNA molecule comprising a targeting domain that is complementary to a second target domain of the *DMD* gene. The second gRNA molecule can be any one of the gRNAs described above. In certain embodiments, the targeting domain of said second gRNA comprises or consists of a sequence that is identical to, or differs by no more than 1, no more than 2, no more than 3, no more than 4 or no more than 5 nucleotides from a nucleotide sequence selected from SEQ ID NOs: 206-826366. In certain embodiments, the first gRNA molecule and the second gRNA molecule are selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;

(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;

(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

In certain embodiments, the first and second gRNA molecules are selected from the pairs of gRNA molecules listed in Table 15, i.e., selected from the group consisting of:

(a) a first gRNA molecule comprising a targeting domain that comprises a nucleotide sequence set forth in SEQ ID NO: 2048, and a second gRNA molecule comprising a targeting domain that comprises a nucleotide sequence selected from SEQ ID NOs: 1977, 18458, 481, 9997, 1499, 2121, 8709, 21570, 16467, and 23958;
(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(c) a first gRNA molecule comprising a targeting domain that comprises a nucleotide sequence set forth in SEQ ID NO: 18720, SEQ ID NO: 22349 or SEQ ID NO: 10092, and a second gRNA molecule comprising a targeting domain that comprises a nucleotide sequence set forth in SEQ ID NO: 4709 or SEQ ID NO: 20303;
(d) a first gRNA molecule comprising a targeting domain that comprises a nucleotide sequence set forth in SEQ ID NO: 3536, and a second gRNA molecule comprising a targeting domain that comprises a nucleotide sequence selected from SEQ ID NOs: 5869, 20145, 18752, 20870, 9530, 18062, 4134, and 23958;
(e) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 9765;
(f) a first gRNA molecule comprising a targeting domain that comprises a nucleotide sequence set forth in SEQ ID NO: 18752, and a second gRNA molecule comprising a targeting domain that comprises a nucleotide sequence selected from SEQ ID NOs: 17253, 14607, 16967, 7419, 1744, and 8569;
(g) a first gRNA molecule comprising a targeting domain that comprises a nucleotide sequence set forth in SEQ ID NO: 19187, and a second gRNA molecule comprising a targeting domain that comprises a nucleotide sequence selected from SEQ ID NOs: 14695, 7252, 17253, 7419, 1744, 8569, and 9358;
(h) a first gRNA molecule comprising a targeting domain that comprises a nucleotide sequence set forth in SEQ ID NO: 20145, and a second gRNA molecule comprising a targeting domain that comprises a nucleotide sequence selected from SEQ ID NOs: 5614, 20764, 14607, 8569, and 7252;

(i) a first gRNA molecule comprising a targeting domain that comprises a nucleotide sequence set forth in SEQ ID NO: 1499, and a second gRNA molecule comprising a targeting domain that comprises a nucleotide sequence set forth in SEQ ID NO: 14296;

(i) a first gRNA molecule comprising a targeting domain that comprises a nucleotide sequence set forth in SEQ ID NO: 4134, and a second gRNA molecule comprising a targeting domain that comprises a nucleotide sequence selected from SEQ ID NOs: 20924, 4072, and 8569;

(k) a first gRNA molecule comprising a targeting domain that comprises a nucleotide sequence set forth in SEQ ID NO: 6209, and a second gRNA molecule comprising a targeting domain that comprises a nucleotide sequence selected from SEQ ID NOs: 14035, 13527, 21873, 14298, 7419, and 20924;

(l) a first gRNA molecule comprising a targeting domain that comprises a nucleotide sequence set forth in SEQ ID NO: 9346, and a second gRNA molecule comprising a targeting domain that comprises a nucleotide sequence selected from SEQ ID NOs: 7419, 20764, and 2769;

(m) a first gRNA molecule comprising a targeting domain that comprises a nucleotide sequence set forth in SEQ ID NO: 7649, and a second gRNA molecule comprising a targeting domain that comprises a nucleotide sequence selected from SEQ ID NOs: 16967, 1484, 20924, 4072, and 9496;

(n) a first gRNA molecule comprising a targeting domain that comprises a nucleotide sequence set forth in SEQ ID NO: 5869, and a second gRNA molecule comprising a targeting domain that comprises a nucleotide sequence selected from SEQ ID NOs: 8569, 13527, 1484, 5614, 4072, and 14695;

(o) a first gRNA molecule comprising a targeting domain that comprises a nucleotide sequence set forth in SEQ ID NO: 4165, and a second gRNA molecule comprising a targeting domain that comprises a nucleotide sequence selected from SEQ ID NOs: 7252, 1484, and 9279;

(p) a first gRNA molecule comprising a targeting domain that comprises a nucleotide sequence set forth in SEQ ID NO: 15622, and a second gRNA molecule comprising a targeting domain that comprises a nucleotide sequence set forth in SEQ ID NO: 17253;

(q) a first gRNA molecule comprising a targeting domain that comprises a nucleotide sequence set forth in SEQ ID NO: 18062, and a second gRNA molecule comprising a targeting domain that comprises a nucleotide sequence set forth in SEQ ID NO: 2769 or SEQ ID NO: 14035;

(r) a first gRNA molecule comprising a targeting domain that comprises a nucleotide sequence set forth in SEQ ID NO: 21131, and a second gRNA molecule comprising a targeting domain that comprises a nucleotide sequence set forth in SEQ ID NO: 13527 or SEQ ID NO: 13285;

(s) a first gRNA molecule comprising a targeting domain that comprises a nucleotide sequence set forth in SEQ ID NO: 20870, and a second gRNA molecule comprising a targeting domain that comprises a nucleotide sequence set forth in SEQ ID NO: 16967 or SEQ ID NO: 7252;

(t) a first gRNA molecule comprising a targeting domain that comprises a nucleotide sequence set forth in SEQ ID NO: 16235, and a second gRNA molecule comprising a targeting domain that comprises a nucleotide sequence set forth in SEQ ID NO: 10140;

(u) a first gRNA molecule comprising a targeting domain that comprises a nucleotide sequence set forth in SEQ ID NO: 15271, and a second gRNA molecule comprising a targeting domain that comprises a nucleotide sequence set forth in SEQ ID NO: 13518;

(v) a first gRNA molecule comprising a targeting domain that comprises a nucleotide sequence set forth in SEQ ID NO: 11675, and a second gRNA molecule comprising a targeting domain that comprises a nucleotide sequence selected from SEQ ID NOs: 2769, 1484, and 4072; and

(w) a first gRNA molecule comprising a targeting domain that comprises a nucleotide sequence set forth in SEQ ID NO: 23958, and a second gRNA molecule comprising a targeting domain that comprises a nucleotide sequence set forth in SEQ ID NO: 14035 or SEQ ID NO: 9496.

In certain embodiments, the nucleic acid composition further comprises a nucleotide sequence that encodes a third gRNA molecule comprising a targeting domain that is complementary to a third target domain of the *DMD* gene. In certain embodiments, the nucleic acid composition further comprises a nucleotide sequence that encodes a fourth gRNA molecule comprising a targeting domain that is complementary to a fourth target domain of the *DMD* gene. The targeting domains of the third and the fourth gRNA molecules can comprise nucleotide sequences set forth in SEQ ID NOs: 206-826366.

In certain embodiments, the second gRNA molecule provides a cleavage event, e.g., a double strand break or a single strand break, sufficiently close to a DMD target position to allow alteration, e.g., alteration associated with NHEJ, of the a DMD target position, either alone or in combination with the break positioned by the first gRNA molecule.

In certain embodiments, (a) and (b) are present on one nucleic acid molecule, e.g., one vector, e.g., one viral vector, e.g., one adeno-associated virus (AAV) vector. In certain embodiments, the nucleic acid molecule is an AAV vector. Exemplary AAV vectors that may be used in any of the described compositions and methods include an AAV2

vector, a modified AAV2 vector, an AAV3 vector, a modified AAV3 vector, an AAV6 vector, a modified AAV6 vector, an AAV8 vector and an AAV9 vector.

In certain embodiments, (a) is present on a first nucleic acid molecule, e.g. a first vector, e.g., a first viral vector, e.g., a first AAV vector; and (b) is present on a second nucleic acid molecule, e.g., a second vector, e.g., a second vector, e.g., a second AAV vector. The first and second nucleic acid molecules may be AAV vectors.

In certain embodiments, (a) and (c) are present on one nucleic acid molecule, e.g., one vector, e.g., one viral vector, e.g., one AAV vector. In certain embodiments, the nucleic acid molecule is an AAV vector. In certain embodiments, (a) and (c) are present on different vectors. For example, (a) is present on a first nucleic acid molecule, e.g. a first vector, e.g., a first viral vector, e.g., a first AAV vector; and (c) is present on a second nucleic acid molecule, e.g., a second vector, e.g., a second vector, e.g., a second AAV vector. In certain embodiments, the first and second nucleic acid molecules are AAV vectors.

In certain embodiments, (a), (b), and (c) are present on one nucleic acid molecule, e.g., one vector, e.g., one viral vector, e.g., one AAV vector. In certain embodiments, the nucleic acid molecule is an AAV vector. In certain embodiments, one of (a), (b), and (c) is present on a first nucleic acid molecule, e.g., a first vector, e.g., a first viral vector, e.g., a first AAV vector; and a second and third of (a), (b), and (c) is present on a second nucleic acid molecule, e.g., a second vector, e.g., a second vector, e.g., a second AAV vector. The first and second nucleic acid molecule can be AAV vectors.

In certain embodiments, (a) is present on a first nucleic acid molecule, e.g., a first vector, e.g., a first viral vector, a first AAV vector; and (b) and (c) are present on a second nucleic acid molecule, e.g., a second vector, e.g., a second vector, e.g., a second AAV vector.

In certain embodiments, (b) is present on a first nucleic acid molecule, e.g., a first vector, e.g., a first viral vector, e.g., a first AAV vector; and (a) and (c) are present on a second nucleic acid molecule, e.g., a second vector, e.g., a second vector, e.g., a second AAV vector.

In certain embodiments, (c) is present on a first nucleic acid molecule, e.g., a first vector, e.g., a first viral vector, e.g., a first AAV vector; and (b) and (a) are present on a second nucleic acid molecule, e.g., a second vector, e.g., a second vector, e.g., a second AAV vector.

In certain embodiments, the first and second nucleic acid molecules are AAV vectors.

In certain embodiments, (a), (b) and (c) are present together in a genome-editing system. In certain embodiments, each of (a), (b) and (c) are present on different nucleic acid molecules, e.g., different vectors, e.g., different viral vectors, e.g., different AAV vector. For example, (a) is present on a first nucleic acid molecule, (b) is present on a second nucleic acid molecule, and (c) is present on a third nucleic acid molecule. The first, second and third nucleic acid molecule can be AAV vectors.

In certain embodiments, when a third and/or fourth gRNA molecules are present, each of (a), (b), (c), the third and/or fourth gRNAs can be present on one nucleic acid molecule, e.g., one vector, e.g., one viral vector, e.g., an AAV vector. In certain embodiments, the nucleic acid molecule is an AAV vector. In certain embodiments, each of (a), (b), (c), the third and/or fourth gRNAs are present on the different nucleic acid molecules, e.g., different vectors, e.g., the different viral vectors, e.g., different AAV vectors. In certain embodiments, each of (a), (b), (c), the third and/or fourth gRNAs are present on more than one nucleic acid molecule, but fewer than five nucleic acid molecules, e.g., AAV vectors.

The nucleic acids composition described herein may comprise a promoter operably linked to the first nucleotide sequence that encodes the first gRNA molecule, e.g., a promoter described herein. The nucleic acid composition may further comprise a second promoter operably linked to the third nucleotide sequence that encodes the second gRNA molecule, e.g., a promoter described herein. In certain embodiment, the promoter and second promoter differ from one another. In certain embodiments, the promoter and second promoter are the same.

The nucleic acids composition described herein may further comprise a promoter operably linked to the second nucleotide sequence that encodes the Cas9 molecule, e.g., a promoter described herein.

In certain embodiments, disclosed herein is a composition comprising (a) a gRNA molecule comprising a targeting domain that is complementary with a target domain in the *DMD* gene, as described herein. In certain embodiments, the targeting domain comprises a nucleotide sequence selected from SEQ ID NOs: 206-826366. In certain embodiments, the composition further comprises (b) a Cas9 molecule, e.g., a Cas9 molecule as described herein. In certain embodiments, the composition further comprises (c) a second gRNA molecule, e.g., a second gRNA molecule as described herein.

The presently disclosed subject matter provides for a composition comprising (a) first gRNA molecule comprising a first targeting domain that is complementary with a target domain of the *DMD* gene, wherein the first targeting domain is 19 to 24 nucleotides in length; (b) a second gRNA molecule comprising a second targeting domain that is complementary with a target domain of the *DMD* gene, wherein the second targeting domain is 19 to 24 nucleotides in length; and (c) at least one Cas9 molecule that recognizes a PAM sequence set forth in NNGRRT (SEQ ID NO: 204) or NNGRRV (SEQ ID NO:205), wherein the composition is adapted for use in a genome-editing system to form first and second double strand breaks in first and second introns flanking exon 51 of the human *DMD* gene, respectively, thereby deleting a

segment of the *DMD* gene comprising exon 51.

The presently disclosed subject matter also provides for a composition comprising at least one of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

In certain embodiments, the composition further comprises at least one Cas9 molecule. In certain embodiments, the at least one Cas9 molecule is an *S. aureus* Cas9 molecule. In certain embodiments, the at least one Cas9 molecule is a mutant *S. aureus* Cas9 molecule. In certain embodiments, the at least one Cas9 molecule recognizes a Protospacer Adjacent Motif (PAM) sequence set forth in NNGRRT (SEQ ID NO: 204) or NNGRRV (SEQ ID NO:205).

The presently disclosed subject matter further provides for a composition comprising one gRNA molecule comprising a targeting domain that is complementary with a target domain of the *DMD* gene, wherein the targeting domain comprises a nucleotide sequence selected from SEQ ID NOS: 206-826366. In certain embodiments, the composition comprises one, two, three, or four gRNA molecules. In certain embodiments, the composition further comprises a Cas9 molecule. In certain embodiments, the first and second gRNA molecules are selected from the gRNA pairs listed in Table 15, i.e., selected from the group consisting of:

(a) a first gRNA molecule comprising a targeting domain that comprises a nucleotide sequence set forth in SEQ ID NO: 2048, and a second gRNA molecule comprising a targeting domain that comprises a nucleotide sequence selected from SEQ ID NOs: 1977, 18458, 481, 9997, 1499, 2121, 8709, 21570, 16467, and 23958;
(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(c) a first gRNA molecule comprising a targeting domain that comprises a nucleotide sequence set forth in SEQ ID

NO: 18720, SEQ ID NO: 22349 or SEQ ID NO: 10092, and a second gRNA molecule comprising a targeting domain that comprises a nucleotide sequence set forth in SEQ ID NO: 4709 or SEQ ID NO: 20303;

(d) a first gRNA molecule comprising a targeting domain that comprises a nucleotide sequence set forth in SEQ ID NO: 3536, and a second gRNA molecule comprising a targeting domain that comprises a nucleotide sequence selected from SEQ ID NOs: 5869, 20145, 18752, 20870, 9530, 18062, 4134, and 23958;

(e) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 9765;

(f) a first gRNA molecule comprising a targeting domain that comprises a nucleotide sequence set forth in SEQ ID NO: 18752, and a second gRNA molecule comprising a targeting domain that comprises a nucleotide sequence selected from SEQ ID NOs: 17253, 14607, 16967, 7419, 1744, and 8569;

(g) a first gRNA molecule comprising a targeting domain that comprises a nucleotide sequence set forth in SEQ ID NO: 19187, and a second gRNA molecule comprising a targeting domain that comprises a nucleotide sequence selected from SEQ ID NOs: 14695, 7252, 17253, 7419, 1744, 8569, and 9358;

(h) a first gRNA molecule comprising a targeting domain that comprises a nucleotide sequence set forth in SEQ ID NO: 20145, and a second gRNA molecule comprising a targeting domain that comprises a nucleotide sequence selected from SEQ ID NOs: 5614, 20764, 14607, 8569, and 7252;

(i) a first gRNA molecule comprising a targeting domain that comprises a nucleotide sequence set forth in SEQ ID NO: 1499, and a second gRNA molecule comprising a targeting domain that comprises a nucleotide sequence set forth in SEQ ID NO: 14296;

(i) a first gRNA molecule comprising a targeting domain that comprises a nucleotide sequence set forth in SEQ ID NO: 4134, and a second gRNA molecule comprising a targeting domain that comprises a nucleotide sequence selected from SEQ ID NOs: 20924, 4072, and 8569;

(k) a first gRNA molecule comprising a targeting domain that comprises a nucleotide sequence set forth in SEQ ID NO: 6209, and a second gRNA molecule comprising a targeting domain that comprises a nucleotide sequence selected from SEQ ID NOs: 14035, 13527, 21873, 14298, 7419, and 20924;

(l) a first gRNA molecule comprising a targeting domain that comprises a nucleotide sequence set forth in SEQ ID NO: 9346, and a second gRNA molecule comprising a targeting domain that comprises a nucleotide sequence selected from SEQ ID NOs: 7419, 20764, and 2769;

(m) a first gRNA molecule comprising a targeting domain that comprises a nucleotide sequence set forth in SEQ ID NO: 7649, and a second gRNA molecule comprising a targeting domain that comprises a nucleotide sequence selected from SEQ ID NOs: 16967, 1484, 20924, 4072, and 9496;

(n) a first gRNA molecule comprising a targeting domain that comprises a nucleotide sequence set forth in SEQ ID NO: 5869, and a second gRNA molecule comprising a targeting domain that comprises a nucleotide sequence selected from SEQ ID NOs: 8569, 13527, 1484, 5614, 4072, and 14695;

(o) a first gRNA molecule comprising a targeting domain that comprises a nucleotide sequence set forth in SEQ ID NO: 4165, and a second gRNA molecule comprising a targeting domain that comprises a nucleotide sequence selected from SEQ ID NOs: 7252, 1484, and 9279;

(p) a first gRNA molecule comprising a targeting domain that comprises a nucleotide sequence set forth in SEQ ID NO: 15622, and a second gRNA molecule comprising a targeting domain that comprises a nucleotide sequence set forth in SEQ ID NO: 17253;

(q) a first gRNA molecule comprising a targeting domain that comprises a nucleotide sequence set forth in SEQ ID NO: 18062, and a second gRNA molecule comprising a targeting domain that comprises a nucleotide sequence set forth in SEQ ID NO: 2769 or SEQ ID NO: 14035;

(r) a first gRNA molecule comprising a targeting domain that comprises a nucleotide sequence set forth in SEQ ID NO: 21131, and a second gRNA molecule comprising a targeting domain that comprises a nucleotide sequence set forth in SEQ ID NO: 13527 or SEQ ID NO: 13285;

(s) a first gRNA molecule comprising a targeting domain that comprises a nucleotide sequence set forth in SEQ ID NO: 20870, and a second gRNA molecule comprising a targeting domain that comprises a nucleotide sequence set forth in SEQ ID NO: 16967 or SEQ ID NO: 7252;

(t) a first gRNA molecule comprising a targeting domain that comprises a nucleotide sequence set forth in SEQ ID NO: 16235, and a second gRNA molecule comprising a targeting domain that comprises a nucleotide sequence set forth in SEQ ID NO: 10140;

(u) a first gRNA molecule comprising a targeting domain that comprises a nucleotide sequence set forth in SEQ ID NO: 15271, and a second gRNA molecule comprising a targeting domain that comprises a nucleotide sequence set forth in SEQ ID NO: 13518;

(v) a first gRNA molecule comprising a targeting domain that comprises a nucleotide sequence set forth in SEQ ID NO: 11675, and a second gRNA molecule comprising a targeting domain that comprises a nucleotide sequence

selected from SEQ ID NOs: 2769, 1484, and 4072; and

(w) a first gRNA molecule comprising a targeting domain that comprises a nucleotide sequence set forth in SEQ ID NO: 23958, and a second gRNA molecule comprising a targeting domain that comprises a nucleotide sequence set forth in SEQ ID NO: 14035 or SEQ ID NO: 9496.

In certain embodiments, the first and second gRNA molecules are selected from the group consisting of:

(a) a first gRNA molecule comprising a targeting domain that comprises a nucleotide sequence set forth in SEQ ID NO: 692257, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(b) a first gRNA molecule comprising a targeting domain that comprises a nucleotide sequence set forth in SEQ ID NO: 692258, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(c) a first gRNA molecule comprising a targeting domain that comprises a nucleotide sequence set forth in SEQ ID NO: 692260, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(d) a first gRNA molecule comprising a targeting domain that comprises a nucleotide sequence set forth in SEQ ID NO: 692253, and a second gRNA molecule comprising a targeting domain that comprises a nucleotide sequence set forth in SEQ ID NO: 692270, SEQ ID NO: 692271, or SEQ ID NO: 692272;

(e) a first gRNA molecule comprising a targeting domain that comprises a nucleotide sequence set forth in SEQ ID NO: 692254, and a second gRNA molecule comprising a targeting domain that comprises a nucleotide sequence set forth in SEQ ID NO: 692270, SEQ ID NO: 692271, or SEQ ID NO: 692272; and

(f) a first gRNA molecule comprising a targeting domain that comprises a nucleotide sequence set forth in SEQ ID NO: 692256, and a second gRNA molecule comprising a targeting domain that comprises a nucleotide sequence set forth in SEQ ID NO: 692270, SEQ ID NO: 692271, or SEQ ID NO: 692272.

Furthermore, the presently disclosed subject matter provides for a genome-editing system, comprising:

a first and a second gRNA, each gRNA having a targeting domain of 19 to 24 nucleotides in length and at least one Cas9 molecule that recognizes a PAM of either NNGRRT (SEQ ID NO:204) or NNGRRV (SEQ ID NO:205), wherein the genome-editing system is configured to form a first and a second double strand break in a first and a second intron flanking exon 51 of the human *DMD* gene, respectively, thereby deleting a segment of the *DMD* gene comprising exon 51.

Also provided is a genome-editing system comprising: a gRNA pair selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;

(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;

(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;

(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;

(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;

(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;

(g) a first gRNA molecule comprising a targeting domain that comprises a nucleotide sequence set forth in SEQ ID

NO: 5869, and a second gRNA molecule comprising a targeting domain that comprises a nucleotide sequence set forth in SEQ ID NO: 3536;

(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;

(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;

(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;

(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and

(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

In certain embodiments, the genome-editing system further comprises at least one one Cas9 molecule, e.g., an *S aureus* Cas9 molecule.

In certain embodiments, the first gRNA molecule, the at least one Cas9 molecule and the second gRNA molecule are present in the system as naked DNA. In certain embodiments, the first gRNA molecule, the at least one Cas9 molecule and the second gRNA molecule are present in the system in a nanoparticle. In certain embodiments, the first gRNA molecule, the at least one Cas9 molecule and the second gRNA molecule are present in the system in a cationic liposome. In certain embodiments, the first gRNA molecule, the at least one Cas9 molecule and the second gRNA molecule are present in the system in a biological non-viral delivery vehicle selected from the group consisting of: attenuated bacteria; engineered bacteriophages; mammalian virus-like particles; and biological liposomes. In certain embodiments, the nanoparticle, cationic liposome, or biological non-viral delivery vehicle comprises a targeting modification capable of increasing target cell uptake of the system.

In certain embodiments, the segment of the DMD gene deleted by the above-described compositions, or the above-described genome editing systems has a length of about 800-900, about 1500-2600, about 5200-5500, about 20,000-30,000, about 35,000-45,000, or about 60,000-72,000 base pairs. In certain embodiments, the segment has a length selected from the group consisting of 806 base pairs, 867 base pairs, 1,557 base pairs, 2,527 base pairs, 5,305 base pairs, 5,415 base pairs, 20,768 base pairs, 27,398 base pairs, 36,342 base pairs, 44,269 base pairs, 60,894 base pairs, and 71,832 base pairs.

Furthermore, the presently disclosed subject matter provides for use of the above-described compositions or the above-described genome editing systems in a medicament, e.g., a medicament for treating Duchenne muscular dystrophy, Becker muscular dystrophy (BMD), or Dilated Cardiomyopathy (DCM) Type 3B, or a medicament for modifying a *DMD* gene.

Additionally, the presently disclosed subject matter provides for a method of modifying a *DMD* gene of a cell, comprising administering to the cell the above-described composition or the above-described genome editing system. In certain embodiments, the modification comprises restoration of correct reading frame of the *DMD* gene. In certain embodiments, the cell comprises one or more mutation in the *DMD* gene. In certain embodiments, the one or more mutation is selected from the group consisting of a premature stop codon, disrupted reading frame, an aberrant splice acceptor site, and an aberrant splice donor site. In certain embodiments, the target domain is upstream or downstream of the one or more mutation. In certain embodiments, the modification comprises deletion of a premature stop codon, restoration of correct reading frame, modulation of splicing by disruption of a splice acceptor site or disruption of a splice donor sequence, or a combination thereof. In certain embodiments, the modification comprises deletion of exons 45-55 of the *DMD* gene. In certain embodiments, the modification comprises deletion of exon 51 of the *DMD* gene. In certain embodiments, the cell is from a subject suffering from Duchenne muscular dystrophy, BMD, or Dilated Cardiomyopathy (DCM) Type 3B. In certain embodiments, the cell is from a subject suffering from Duchenne muscular dystrophy.

Furthermore, presently disclosed subject matter provides for a method of treating Duchenne muscular dystrophy, BMD, or DCM Type 3B in a subject, comprising administering to the subject the above-described composition, or the above-described genome editing system.

Additionally, the presently disclosed subject matter provides for a vector comprising a polynucleotide encoding a first gRNA molecule and a second gRNA molecule, wherein the first gRNA molecule and the second gRNA molecule are selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

In certain embodiments, the vector is a viral vector. In certain embodiments, the vector is an adeno-associated virus (AAV) vector.

The presently disclosed subject matter further provides for a cell comprising the above-described composition, the above-described genome-editing system, or the above-described vector.

The presently disclosed subject matter further provides for a method of treating Duchenne muscular dystrophy in a subject, comprising administering to the subject an effective amount of the above-described composition.

In certain embodiments, the method further comprises contacting the cell with a third and/or fourth gRNA molecules. In certain embodiments, the method comprises contacting a cell from a subject suffering from or likely to develop DMD, BMD, or DCM type 3B. The cell may be from a subject having one or more mutation in the *DMD* gene. In certain embodiments, the cell being contacted in the disclosed method is a photoreceptor cell. The contacting is performed *ex vivo* and the contacted cell is returned to the subject's body after the contacting step. In certain embodiments, the contacting step is performed *in vivo.*

In certain embodiments, the method comprises acquiring knowledge of the presence of a DMD target position in said cell, prior to the contacting step. Acquiring knowledge of the presence of a DMD target position in the cell may be by sequencing the *DMD* gene, or a portion of the *DMD* gene.

In certain embodiments, the contacting step comprises contacting the cell with a nucleic acid composition, e.g., a vector, e.g., an AAV vector, that encodes at least one of the first gRNA, Cas9 and second gRNA as defined above. In certain embodiments, the contacting step comprises delivering to the cell a Cas9 molecule of and a nucleic acid which encodes the first gRNA, and optionally, the second gRNA, and further optionally, a third gRNA and/or fourth gRNA. In certain embodiments, contacting comprises contacting the cell with a nucleic acid, e.g., a vector, e.g., an AAV vector, e.g., an AAV2 vector, a modified AAV2 vector, an AAV3 vector, a modified AAV3 vector, an AAV6 vector, a modified

AAV6 vector, an AAV8 vector or an AAV9 vector. In certain embodiments, contacting comprises delivering to said cell said Cas9 molecule, as a protein or an mRNA, and a nucleic acid which encodes and the first and the second gRNA molecules. In certain embodiments, contacting comprises delivering to said cell the Cas9 molecule as a protein or an mRNA, the first gRNA molecule as an RNA, and optionally the second gRNA molecule as an RNA. In certain embodiments, contacting comprises delivering to said cell the first gRNA molecule as an RNA, optionally the second gRNA molecule as an RNA, and a nucleic acid that encodes the Cas9 molecule. In certain embodiments, the first gRNA molecule, the Cas 9 molecule, and the second gRNA molecule are present together in a genome-editing system.

In certain embodiments, disclosed herein is a method of treating, or preventing a subject suffering from developing, DMD, BMD, or DCM type 3B, e.g., by altering the structure, e.g., sequence, of a target nucleic acid of the subject, comprising contacting the subject (or a cell from the subject) with: (a) a gRNA that targets the *DMD* gene, e.g., a gRNA disclosed herein; (b) a Cas9 molecule, e.g., a Cas9 molecule disclosed herein; and optionally, (c) a second gRNA that targets the *DMD* gene, e.g., a second gRNA disclosed herein, and further optionally, a third gRNA, and still further optionally, a fourth gRNA that target the *DMD* gene, e.g., a third and fourth gRNA disclosed herein. In certain embodiments, the contacting comprises contacting with (a) and (b). In certain embodiments, the contacting comprises contacting with (a), (b), and (c). In certain embodiments, the contacting comprises contacting with (a), (b), (c) and the third gRNA. In certain embodiments, contacting comprises contacting with (a), (b), (c), the third and the fourth gRNAs. The targeting domains of (a), (c), the third and/or fourth gRNAs can comprise a nucleotide sequence selected from SEQ ID NOs: 206-826366, or comprises a nucleotide sequence that differs by no more than 1, 2, 3, 4, or 5 nucleotides from the nucleotides sequences set forth in SEQ ID NOs: 206-826366.

In certain embodiments, said subject is suffering from, or likely to develop DMD, BMD, or DCM type 3B. In certain embodiments, said subject has one or more mutation at a DMD target position. In certain embodiments, the method comprises acquiring knowledge of the presence of one or more mutation at a DMD target position in said subject. In certain embodiments, the method comprises acquiring knowledge of the presence of one or more mutation a DMD target position in said subject by sequencing the *DMD* gene or a portion of the *DMD* gene.

In certain embodiments, the method comprises altering the DMD target position in the *DMD* gene.

In certain embodiments, a cell of said subject is contacted *ex vivo* with (a), (b) and optionally (c). In certain embodiments, said cell is returned to the subject's body. In certain embodiments, the method comprises the treatment comprises introducing a cell into said subject's body, wherein said cell subject is contacted *ex vivo* with (a), (b) and optionally (c). In certain embodiments, the method comprises said contacting is performed *in vivo.*

In certain embodiments, the method comprises injection directly into one or more muscle of the subject, e.g., skeletal muscles. In certain embodiments, the method comprises injection directly into cardiac muscle or smooth muscle of the subject. In certain embodiments, contacting comprises contacting the subject with a nucleic acid composition, e.g., a vector, e.g., an AAV vector, described herein, e.g., a nucleic acid that encodes at least one of (a), (b), and optionally (c).

In certain embodiments, the contacting comprises delivering to said subject said Cas9 molecule of (b), as a protein or mRNA, and a nucleic acid composition that encodes (a) and optionally (c). In certain embodiments, the contacting comprises delivering to said subject said Cas9 molecule of (b), as a protein or mRNA, said gRNA of (a), as an RNA, and optionally said second gRNA of (c), as an RNA. In certain embodiments, the contacting comprises delivering to said subject said gRNA of (a), as an RNA, optionally said second gRNA of (c), as an RNA, and a nucleic acid composition that encodes the Cas9 molecule of (b).

The presently disclosed subject matter further provides for a reaction mixture comprising a, gRNA, a nucleic acid composition, or a composition described herein, and a cell, e.g., a cell from a subject having, or likely to develop DMD, BMD, or DCM type 3B, or a subject having one or more mutation at a DMD target position.

The presently disclosed subject matter further provides for a kit comprising, (a) gRNA molecule described herein, or nucleic acid composition that encodes said gRNA, and one or more of the following: (b) a Cas9 molecule, e.g., a Cas9 molecule described herein, or a nucleic acid composition or mRNA that encodes the Cas9; (c) a second gRNA molecule, e.g., a second gRNA molecule described herein or a nucleic acid composition that encodes (c). In certain embodiments, the kit further comprises a third gRNA molecule or a nucleic acid composition that encodes thereof, and/or a fourth gRNA molecule or a nucleic acid composition that encodes thereof.

In certain embodiments, the kit comprises a nucleic acid composition, e.g., an AAV vector, that encodes one or more of (a), (b), (c), the third and/or fourth gRNAs.

The presently disclosed subject matter further provides for a gRNA molecule, e.g., a gRNA molecule described herein, for use in treating, or delaying the onset or progression of, DMD, BMD, or DCM type 3B in a subject, e.g., in accordance with a method of treating, or delaying the onset or progression of, DMD, BMD, or DCM type 3B as described herein. In certain embodiments, the gRNA molecule in used in combination with a Cas9 molecule, e.g., a Cas9 molecule described herein. Additionally or alternatively, in certain embodiments, the gRNA molecule is used in combination with a second, third and/or fourth gRNA molecule, e.g., a second, third and/or fourth gRNA molecule described herein.

The presently disclosed subject matter further provides for use of a gRNA molecule, e.g., a gRNA molecule

described herein, in the manufacture of a medicament for treating, or delaying the onset or progression of, DMD, BMD, or DCM type 3B in a subject, e.g., in accordance with a method of treating, or delaying the onset or progression of, DMD, BMD, or DCM type 3B as described herein. In certain embodiments, the medicament comprises a Cas9 molecule, e.g., a Cas9 molecule described herein. Additionally or alternatively, in certain embodiments, the medicament comprises a second, third and/or fourth gRNA molecule, e.g., a second, third and/or fourth gRNA molecule described herein.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In addition, the materials, methods, and examples are illustrative only and not intended to be limiting.

Headings, including numeric and alphabetical headings and subheadings, are for organization and presentation and are not intended to be limiting.

Other features and advantages of the invention will be apparent from the detailed description, drawings, and from the claims.

Aspects or embodiments of the invention may also be provided according to the following paragraphs:

1. A genome-editing system, comprising:

    a first and a second gRNA, each gRNA having a targeting domain of 19 to 24 nucleotides in length and at least one Cas9 molecule that recognizes a Protospacer Adjacent Motif (PAM) of either NNGRRT (SEQ ID NO:204) or NNGRRV (SEQ ID NO:205),
    wherein the genome-editing system is configured to form a first and a second double strand break in a first and a second intron flanking exon 51 of the human *DMD* gene, respectively, thereby deleting a segment of the *DMD* gene comprising exon 51.

2. The genome-editing system of paragraph 1, wherein the segment has a length of about 800-900, about 1500-2600, about 5200-5500, about 20,000-30,000, about 35,000-45,000, or about 60,000-72,000 base pairs.
3. The genome-editing system of paragraph 2, wherein the segment has a length selected from the group consisting of 806 base pairs, 867 base pairs, 1,557 base pairs, 2,527 base pairs, 5,305 base pairs, 5,415 base pairs, 20,768 base pairs, 27,398 base pairs, 36,342 base pairs, 44,269 base pairs, 60,894 base pairs, and 71,832 base pairs.
4. The genome-editing system of any one of paragraphs 1-3, wherein the at least one Cas9 molecule is an *S. aureus* Cas9 molecule.
5. The genome-editing system of any one of paragraphs 1-4, wherein the first gRNA molecule, the at least one Cas9 molecule and the second gRNA molecule are present in the system in a nanoparticle.
6. The genome-editing system of any one of paragraphs 1-4, wherein the first gRNA molecule, the at least one Cas9 molecule and the second gRNA molecule are present in the system in a cationic liposome.
7. The genome-editing system of any one of paragraphs 1-4, wherein the first gRNA molecule, the at least one Cas9 molecule and the second gRNA molecule are present in the system in a biological non-viral delivery vehicle selected from the group consisting of: attenuated bacteria; engineered bacteriophages; mammalian virus-like particles; and biological liposomes.
8. The genome-editing system of any one of paragraphs 5-7, wherein the nanoparticle, cationic liposome, or biological non-viral delivery vehicle comprises a targeting modification capable of increasing target cell uptake of the system.
9. The genome-editing system of any one of paragraphs 1-8 for use in a medicament.
10. The genome-editing system of any one of paragraphs 1-8, for use in the treatment of Duchenne Muscular Dystrophy.
11. A composition comprising:

    (a) first gRNA molecule comprising a first targeting domain that is complementary with a target domain of the *DMD* gene, wherein the first targeting domain is 19 to 24 nucleotides in length;
    (b) a second gRNA molecule comprising a second targeting domain that is complementary with a target domain of the *DMD* gene, wherein the second targeting domain is 19 to 24 nucleotides in length; and
    (c) at least one Cas9 molecule that recognizes a PAM sequence set forth in NNGRRT (SEQ ID NO: 204) or NNGRRV (SEQ ID NO:205),

wherein the composition is adapted for use in a genome-editing system to form first and second double strand breaks in first and second introns flanking Exon 51 of the human *DMD* gene, respectively, thereby deleting a segment of the *DMD* gene including Exon 51.

12. The composition of paragraph 11, wherein the segment has a length of about 800-900, about 1500-2600, about 5200-5500, about 20,000-30,000, about 35,000-45,000, or about 60,000-72,000 base pairs.
13. The composition of paragraph 12, wherein the segment has a length selected from the group consisting of 806 base pairs, 867 base pairs, 1,557 base pairs, 2,527 base pairs, 5,305 base pairs, 5,415 base pairs, 20,768 base pairs, 27,398 base pairs, 36,342 base pairs, 44,269 base pairs, 60,894 base pairs, and 71,832 base pairs.
14. The composition of any one of paragraphs 11-13, wherein the at least one Cas9 molecule is an *S. aureus* Cas9 molecule.
15. The composition of paragraph 14, wherein the at least one Cas9 molecule is a mutant *S. aureus* Cas9 molecule.
16. The composition of any one of paragraphs 11-15 for use in a medicament.
17. The composition of any one of paragraphs 11-15, for use in the treatment of Duchenne Muscular Dystrophy.
18. A composition comprising at least one of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

19. The composition of paragraph 18, further comprising at least one Cas9 molecule.
20. The composition of paragraph 19, wherein the at least one Cas9 molecule is an *S. aureus* Cas9 molecule.
21. The composition of paragraph 20, wherein the at least one Cas9 molecule is a mutant *S. aureus* Cas9 molecule.
22. The composition of any one of paragraphs 19-21, wherein the at least one Cas9 molecule recognizes a PAM sequence set forth in NNGRRT (SEQ ID NO: 204) or NNGRRV (SEQ ID NO:205).
23. A genome-editing system comprising a gRNA pair selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence

set forth in SEQ ID NO: 2048;

(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;

(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;

(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;

(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;

(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;

(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;

(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;

(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;

(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;

(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and

(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

24. The genome-editing system of paragraph 23, comprising at least one Cas9 molecule.

25. The genome-editing system of paragraph 24, wherein the at least one Cas9 molecule is an *S. Aureus* Cas9 molecule.

26. The genome-editing system of any one of paragraphs 23-25, wherein the first gRNA molecule, the at least one Cas9 molecule and the second gRNA molecule are present in the system in a nanoparticle.

27. The genome-editing system of any one of paragraphs 23-25, wherein the first gRNA molecule, the at least one Cas9 molecule and the second gRNA molecule are present in the system in a cationic liposome.

28. The genome-editing system of any one of paragraphs 23-25, wherein the first gRNA molecule, the at least one Cas9 molecule and the second gRNA molecule are present in the system in a biological non-viral delivery vehicle selected from the group consisting of: attenuated bacteria; engineered bacteriophages; mammalian virus-like particles; and biological liposomes.

29. The genome-editing system of any one of paragraphs 26-28, wherein the nanoparticle, cationic liposome, or biological non-viral delivery vehicle comprises a targeting modification capable of increasing target cell uptake of the system.

30. The genome-editing system of any one of paragraphs 23-28, for use in modifying a *DMD* gene in a cell.

31. The genome-editing system of paragraph 30, wherein the cell is from a subject suffering from Duchenne muscular dystrophy.

32. A vector comprising a polynucleotide encoding a first gRNA molecule and a second gRNA molecule, wherein the first gRNA molecule and the second gRNA molecule are selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;

(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

33. The vector of paragraph 32, wherein the vector is a viral vector.
34. The vector of paragraph 33, wherein the vector is an adeno-associated virus (AAV) vector.
35. A method of modifying a *DMD* gene in a cell, comprising administering to the cell one of:

(i) a genome-editing system comprising a polynucleotide encoding a first gRNA molecule, a polynucleotide encoding a Cas9 molecule, and a polynucleotide encoding a second gRNA molecule; or
(ii) a genome-editing system comprising a first gRNA molecule, a Cas9 molecule, and a second gRNA molecule,

wherein the first gRNA molecule and the second gRNA molecule are selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence

set forth in SEQ ID NO: 2048;

(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;

(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;

(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;

(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;

(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;

(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and

(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

36. The method of paragraph 35, wherein the modification comprises deletion of exon 51 of the *DMD* gene.

37. The method of paragraph 35 or 36, wherein the modification comprises restoration of correct reading frame of the *DMD* gene.

38. The method of any one of paragraphs 35-37, wherein the cell is from a subject suffering from Duchenne muscular dystrophy.

39. A method of treating Duchenne muscular dystrophy in a subject, comprising administering to the subject one of:

(i) a genome-editing system comprising a polynucleotide encoding a first gRNA molecule, a polynucleotide encoding a Cas9 molecule, and a polynucleotide encoding a second gRNA molecule; or

(ii) a genome-editing system comprising a first gRNA molecule, at least one Cas9 molecule, and a second gRNA molecule,

wherein the first gRNA molecule and the second gRNA molecule are selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;

(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;

(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;

(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;

(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;

(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;

(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;

(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

40. A composition comprising one gRNA molecule comprising a targeting domain that is complementary with a target domain of the *DMD* gene, wherein the targeting domain comprises a nucleotide sequence selected from SEQ ID NOS: 206-826366.
41. The composition of paragraph 40, comprising one, two, three, or four gRNA molecules.
42. The composition of paragraph 41, comprising two gRNA molecules selected from the group consisting of:

(a) a first gRNA molecule comprising a targeting domain that comprises a nucleotide sequence set forth in SEQ ID NO: 2048, and a second gRNA molecule comprising a targeting domain that comprises a nucleotide sequence selected from SEQ ID NOs: 1977, 18458, 481, 9997, 1499, 2121, 8709, 21570, 16467, and 23958;
(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(c) a first gRNA molecule comprising a targeting domain that comprises a nucleotide sequence set forth in SEQ ID NO: 18720, SEQ ID NO: 22349 or SEQ ID NO: 10092, and a second gRNA molecule comprising a targeting domain that comprises a nucleotide sequence set forth in SEQ ID NO: 4709 or SEQ ID NO: 20303;
(d) a first gRNA molecule comprising a targeting domain that comprises a nucleotide sequence set forth in SEQ ID NO: 3536, and a second gRNA molecule comprising a targeting domain that comprises a nucleotide sequence selected from SEQ ID NOs: 5869, 20145, 18752, 20870, 9530, 18062, 4134, and 23958;
(e) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 9765;
(f) a first gRNA molecule comprising a targeting domain that comprises a nucleotide sequence set forth in SEQ ID NO: 18752, and a second gRNA molecule comprising a targeting domain that comprises a nucleotide sequence selected from SEQ ID NOs: 17253, 14607, 16967, 7419, 1744, and 8569;
(g) a first gRNA molecule comprising a targeting domain that comprises a nucleotide sequence set forth in SEQ ID NO: 19187, and a second gRNA molecule comprising a targeting domain that comprises a nucleotide sequence selected from SEQ ID NOs: 14695, 7252, 17253, 7419, 1744, 8569, and 9358;
(h) a first gRNA molecule comprising a targeting domain that comprises a nucleotide sequence set forth in SEQ ID NO: 20145, and a second gRNA molecule comprising a targeting domain that comprises a nucleotide sequence selected from SEQ ID NOs: 5614, 20764, 14607, 8569, and 7252;
(i) a first gRNA molecule comprising a targeting domain that comprises a nucleotide sequence set forth in SEQ ID NO: 1499, and a second gRNA molecule comprising a targeting domain that comprises a nucleotide sequence set forth in SEQ ID NO: 14296;
(i) a first gRNA molecule comprising a targeting domain that comprises a nucleotide sequence set forth in SEQ ID NO: 4134, and a second gRNA molecule comprising a targeting domain that comprises a nucleotide sequence selected from SEQ ID NOs: 20924, 4072, and 8569;
(k) a first gRNA molecule comprising a targeting domain that comprises a nucleotide sequence set forth in SEQ ID NO: 6209, and a second gRNA molecule comprising a targeting domain that comprises a nucleotide sequence selected from SEQ ID NOs: 14035, 13527, 21873, 14298, 7419, and 20924;
(l) a first gRNA molecule comprising a targeting domain that comprises a nucleotide sequence set forth in SEQ ID NO: 9346, and a second gRNA molecule comprising a targeting domain that comprises a nucleotide sequence selected from SEQ ID NOs: 7419, 20764, and 2769;

(m) a first gRNA molecule comprising a targeting domain that comprises a nucleotide sequence set forth in SEQ ID NO: 7649, and a second gRNA molecule comprising a targeting domain that comprises a nucleotide sequence selected from SEQ ID NOs: 16967, 1484, 20924, 4072, and 9496;

(n) a first gRNA molecule comprising a targeting domain that comprises a nucleotide sequence set forth in SEQ ID NO: 5869, and a second gRNA molecule comprising a targeting domain that comprises a nucleotide sequence selected from SEQ ID NOs: 8569, 13527, 1484, 5614, 4072, and 14695;

(o) a first gRNA molecule comprising a targeting domain that comprises a nucleotide sequence set forth in SEQ ID NO: 4165, and a second gRNA molecule comprising a targeting domain that comprises a nucleotide sequence selected from SEQ ID NOs: 7252, 1484, and 9279;

(p) a first gRNA molecule comprising a targeting domain that comprises a nucleotide sequence set forth in SEQ ID NO: 15622, and a second gRNA molecule comprising a targeting domain that comprises a nucleotide sequence set forth in SEQ ID NO: 17253;

(q) a first gRNA molecule comprising a targeting domain that comprises a nucleotide sequence set forth in SEQ ID NO: 18062, and a second gRNA molecule comprising a targeting domain that comprises a nucleotide sequence set forth in SEQ ID NO: 2769 or SEQ ID NO: 14035;

(r) a first gRNA molecule comprising a targeting domain that comprises a nucleotide sequence set forth in SEQ ID NO: 21131, and a second gRNA molecule comprising a targeting domain that comprises a nucleotide sequence set forth in SEQ ID NO: 13527 or SEQ ID NO: 13285;

(s) a first gRNA molecule comprising a targeting domain that comprises a nucleotide sequence set forth in SEQ ID NO: 20870, and a second gRNA molecule comprising a targeting domain that comprises a nucleotide sequence set forth in SEQ ID NO: 16967 or SEQ ID NO: 7252;

(t) a first gRNA molecule comprising a targeting domain that comprises a nucleotide sequence set forth in SEQ ID NO: 16235, and a second gRNA molecule comprising a targeting domain that comprises a nucleotide sequence set forth in SEQ ID NO: 10140;

(u) a first gRNA molecule comprising a targeting domain that comprises a nucleotide sequence set forth in SEQ ID NO: 15271, and a second gRNA molecule comprising a targeting domain that comprises a nucleotide sequence set forth in SEQ ID NO: 13518;

(v) a first gRNA molecule comprising a targeting domain that comprises a nucleotide sequence set forth in SEQ ID NO: 11675, and a second gRNA molecule comprising a targeting domain that comprises a nucleotide sequence selected from SEQ ID NOs: 2769, 1484, and 4072; and

(w) a first gRNA molecule comprising a targeting domain that comprises a nucleotide sequence set forth in SEQ ID NO: 23958, and a second gRNA molecule comprising a targeting domain that comprises a nucleotide sequence set forth in SEQ ID NO: 14035 or SEQ ID NO: 9496.

43. The composition of paragraph 41, comprising two gRNA molecules selected from the group consisting of:

(a) a first gRNA molecule comprising a targeting domain that comprises a nucleotide sequence set forth in SEQ ID NO: 692257, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(b) a first gRNA molecule comprising a targeting domain that comprises a nucleotide sequence set forth in SEQ ID NO: 692258, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(c) a first gRNA molecule comprising a targeting domain that comprises a nucleotide sequence set forth in SEQ ID NO: 692260, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(d) a first gRNA molecule comprising a targeting domain that comprises a nucleotide sequence set forth in SEQ ID NO: 692253, and a second gRNA molecule comprising a targeting domain that comprises a nucleotide sequence set forth in SEQ ID NO: 692270, SEQ ID NO: 692271, or SEQ ID NO: 692272;

(e) a first gRNA molecule comprising a targeting domain that comprises a nucleotide sequence set forth in SEQ ID NO: 692254, and a second gRNA molecule comprising a targeting domain that comprises a nucleotide sequence set forth in SEQ ID NO: 692270, SEQ ID NO: 692271, or SEQ ID NO: 692272; and

(f) a first gRNA molecule comprising a targeting domain that comprises a nucleotide sequence set forth in SEQ ID NO: 692256, and a second gRNA molecule comprising a targeting domain that comprises a nucleotide sequence set forth in SEQ ID NO: 692270, SEQ ID NO: 692271, or SEQ ID NO: 692272.

44. The composition of any one of paragraphs 40-43, further comprising at least one Cas9 molecule.
45. The composition of any one of paragraphs 40-44 for use in modifying a *DMD* gene in a cell.
46. The composition of paragraph 45, wherein the cell is from a subject suffering from Duchenne muscular dystrophy.
47. A method of modifying a *DMD* gene in a subject with one or more mutation in the *DMD* gene, comprising administering to a cell from the subject a composition comprising one gRNA molecule comprising a targeting domain that is complementary with a target domain of the *DMD* gene, wherein the targeting domain comprises a nucleotide sequence selected from SEQ ID NOS: 206-826366, and at least one Cas9 molecule.
48. The method of paragraph 47, wherein the one or more mutation is selected from the group consisting of a premature stop codon, disrupted reading frame, an aberrant splice acceptor site, and an aberrant splice donor site.
49. The method of paragraph 48, wherein the target domain is upstream or downstream of the one or more mutation.
50. The method of any one of paragraphs 47-49, wherein the modification comprises deletion of a premature stop codon, restoration of correct reading frame, modulation of splicing by disruption of a splice acceptor site or disruption of a splice donor sequence, or a combination thereof.
51. The method of any one of paragraphs 47-50, wherein the modification comprises deletion of exon 51.
52. The method of any one of paragraphs 47-51, wherein the subject is suffering from Duchenne muscular dystrophy, Becker muscular dystrophy (BMD), or Dilated Cardiomyopathy (DCM) Type 3B.
53. A method of treating Duchenne muscular dystrophy, BMD, or DCM Type 3B in a subject, comprising administering to the subject a composition comprising one gRNA molecule comprising a targeting domain that is complementary with a target domain of the *DMD* gene, wherein the targeting domain comprises a nucleotide sequence selected from SEQ ID NOS: 206-826366, and at least one Cas9 molecule.
54. A cell comprising the composition of any one of paragraphs 11-22 and 40-44, the genome-editing system of any one of paragraphs 1-8, and 23-29, or the vector of any one of paragraphs 32-34.

## BRIEF DESCRIPTION OF THE DRAWINGS

**Figs. 1A-1I** are representations of several exemplary gRNAs.

**Fig. 1A** depicts a modular gRNA molecule derived in part (or modeled on a sequence in part) from *Streptococcus pyogenes (S. pyogenes)* as a duplexed structure (SEQ ID NOs:39 and 40, respectively, in order of appearance);

**Fig. 1B** depicts a unimolecular gRNA molecule derived in part from *S. pyogenes* as a duplexed structure (SEQ ID NO:41);

**Fig. 1C** depicts a unimolecular gRNA molecule derived in part from *S. pyogenes* as a duplexed structure (SEQ ID NO:42);

**Fig. ID** depicts a unimolecular gRNA molecule derived in part from *S. pyogenes* as a duplexed structure (SEQ ID NO:43);

**Fig. 1E** depicts a unimolecular gRNA molecule derived in part from *S. pyogenes* as a duplexed structure (SEQ ID NO:44);

**Fig. 1F** depicts a modular gRNA molecule derived in part from *Streptococcus thermophilus (S. thermophilus)* as a duplexed structure (SEQ ID NOs:45 and 46, respectively, in order of appearance);

**Fig. 1G** depicts an alignment of modular gRNA molecules of *S. pyogenes* and *S. thermophilus* (SEQ ID NOs:39, 45, 47, and 46, respectively, in order of appearance).

**Figs. 1H-1I** depicts additional exemplary structures of unimolecular gRNA molecules.

**Fig. 1H** shows an exemplary structure of a unimolecular gRNA molecule derived in part from *S. pyogenes* as a duplexed structure (SEQ ID NO:42).

**Fig. 1I** shows an exemplary structure of a unimolecular gRNA molecule derived in part from *S. aureus* as a duplexed structure (SEQ ID NO:38).

**Figs. 2A-2G** depict an alignment of Cas9 sequences (Chylinski 2013). The N-terminal RuvC-like domain is boxed and indicated with a "Y." The other two RuvC-like domains are boxed and indicated with a "B." The HNH-like domain is boxed and indicated by a "G." Sm: *S. mutans* (SEQ ID NO: 1); Sp: *S. pyogenes* (SEQ ID NO:2); St: *S. thermophilus* (SEQ ID NO:4); and Li: *L. innocua* (SEQ ID NO:5). "Motif" (SEQ ID NO:14) is a consensus sequence based on the four sequences. Residues conserved in all four sequences are indicated by single letter amino acid abbreviation; "*" indicates any amino acid found in the corresponding position of any of the four sequences; and "-" indicates absent.

**Figs. 3A-3B** show an alignment of the N-terminal RuvC-like domain from the Cas9 molecules disclosed in Chylinski 2013 (SEQ ID NOs:52-95, 120-123). The last line of **Fig. 3B** identifies 4 highly conserved residues.

**Figs. 4A-4B** show an alignment of the N-terminal RuvC-like domain from the Cas9 molecules disclosed in Chylinski 2013 with sequence outliers removed (SEQ ID NOs:52-123). The last line of **Fig. 4B** identifies 3 highly conserved residues.

**Figs. 5A-5C** show an alignment of the HNH-like domain from the Cas9 molecules disclosed in Chylinski 2013 (SEQ ID NOs: 124-198). The last line of **Fig. 5C** identifies conserved residues.

**Figs. 6A-6B** show an alignment of the HNH-like domain from the Cas9 molecules disclosed in Chylinski 2013 with sequence outliers removed (SEQ ID NOs:124-141, 148, 149, 151-153, 162, 163, 166-174, 177-187, 194-198). The last line of **Fig. 6B** identifies 3 highly conserved residues.

**Fig. 7** illustrates gRNA domain nomenclature using an exemplary gRNA sequence (SEQ ID NO:42).

**Figs. 8A** and **8B** provide schematic representations of the domain organization of *S. pyogenes* Cas9. **Fig. 8A** shows the organization of the Cas9 domains, including amino acid positions, in reference to the two lobes of Cas9 (recognition (REC) and nuclease (NUC) lobes). **Fig. 8B** shows the percent homology of each domain across 83 Cas9 orthologs.

**Fig. 9A-9G** show the results of Sanger sequencing of PCR products amplifying across the targeted deletion region in cells transfected with Cas9 and pairs gRNAs targeting the *DMD* gene. The gRNA pairs used are indicated as well as the targeted deletion region and the predicted deletion size. Sequence results are shown with the wildtype sequence first and deletion sequences below. Dashes indicate deletion of a given base and "..." represents the large missing deletion region. gRNA target sites are boxed and bases that are bold and underlined indicate insertions or an inversion. To the right of the sequence the total number of deleted and/or inserted bases is shown (Δ1740 means a deletion of 1740bp) as well as the number of times that exact sequence was observed (x6 means that that exact sequence was observed 6 times).

**Fig. 10A-10C** depict exemplary exonic target position for modification of *DMD* deletion mutations.

**Fig. 11A-11C** depict exemplary exonic target position for modification of *DMD* duplication mutations.

**Fig. 12A-12B** depict exemplary exonic target position for modification of *DMD* point mutations.

**Fig. 13** depicts am exemplary approach for selection of ectopic (e.g., non-contiguous) exons having the same reading frame.

**Fig. 14** depicts principles of digital droplet PCR ("ddPCR"). ddPCR was used for a sensitive and quantitative assay for DMD Exon 51 deletion without bias for any particular gRNA pair or deletion length. Briefly, Taqman primer-probe sets (like those used in qPCR) are used to identify a genomic region of interest within a partitioned droplet. Each droplet most likely contains 0 or 1 copy of the PCR template, because the reaction is underloaded with gDNA (~3000 copies) compared to the number of droplets (~20,000). After amplification, droplets that contained 1 copy of template will fluoresce. The number of fluorescent droplets is counted by a BioRad droplet reader, and the number of positive droplets compared to total droplets is used to quantify the copy number of template in the original DNA sample. (image from BioRad).

**Fig. 15** depicts the design of DMD Exon 51 Deletion ddPCR assay. The assay multiplexed 2 Taqman probes within each reaction well. For the wild-type allele, both the Exon 51 'deletion' probe and the Exon 59 'control' probe reference should bind and fluoresce in a 1:1 ratio of positive droplets. For any deletion alleles generated by the guide pairs screened, only the Exon 59 probe will fluoresce. The relative depletion of Exon 51 probe signal is used to quantify the deletion rate in the population of cells, according to the following formula: Deletion = 1 - (Exon 51 positive droplets / Exon 59 positive droplets).

**Figs. 16A-16D** depict ddPCR assay validation and screen plate layout. (A) Samples from a female Exon 51 deletion carrier and a male exon 51 deletion patient, and both male and female Exon 51 non-deleted controls were quantified with ddPCR. (B) The two male samples were mixed in varying amounts to create a ladder of DMD deletion rates. Measured deletion rates by ddPCR correlated well with expected deletion rates. (C) The plate layout for paired gRNA screening by plasmid transfection. 30,000 HEK293T cells were plated in each well. Rows A-G were transfected with guide pairs of unknown deletion efficiency. H1-H6 were controls in duplicate: single gRNA, positive control paired gRNAs 1+9, and negative control gRNA plasmid backbone alone. The remaining wells H7-H12 were not transfected, but were used to run an internal standard curve with each plate for ddPCR. (D) An example of the internal standard curve in wells H7-H12, prepared similarly to Fig. 17B. The range was limited to 0 - 30% deletion for resolution in the dynamic range of the gRNA pairs tested in the screen.

**Figs. 17A and 17B** depict Guide Set 1: Pilot study of DMD Exon 51 deletion by plasmid transfection of SaCas9. (A) 8 gRNA pairs, two single gRNAs (5, 12), the gRNA backbone alone (pJT002), and a non-targeting gRNA (pAF013VegF) were transfected into HEK293T cells alongside SaCas9. After 3 days, genomic DNA was extracted and deletion rates were quantified by ddPCR. Biological replicates were plotted separately to confirm consistency (n = 4 technical replicates). (B) The experiment was repeated similarly except for genomic DNA was extracted both 3 and 6 days after transfection.

**Figs. 18A and 18B** depict alternative SaCas9 promoters and codon optimizations. (A) Exon 51 deletion rates were compared with the same guide plasmids transfected and varied SaCas9 plasmids. pJT002 is the guide plasmid backbone alone. Guides 1+9+38+62 is a co-transfection of 4 guides, 25ng each, an equivalent total mass to the other samples. (B) Table of SaCas9 plasmids used in the experiment.

**Fig. 19** depicts effect adding a 5' G to the gRNA on DNA cleavage efficiency. 5 single gRNA targeting DMD Exon

51 were co-transfected with SaCas9 with and without an additional 5' G. For some, this additional 5' G was matched in the genome. The negative control was not transfected with any DNA ('No Tfx').

**Fig. 20** depicts Guide Set 2 - NHP cross reactive gRNA pairs. The number of guide pairs corresponding to individual Z-scores computed for the guide pairs screened in Guide Set 2. The positive control (guides 1+9) and negative controls (Guide backbone only) have been plotted separately. Guide pairs with a Z score greater than or equal to 1.5 were carried forward for a follow-up Guide Hit Validation study.

**Fig. 21** depicts comparison of NHP cross reactive guides with human-only guides. Histogram demonstrating the skewing of NHP cross-reactive guide pairs towards larger deletion sizes. Guide set 3 was designed to capture smaller deletion sizes, therefore requiring human-only gRNAs.

**Fig. 22** depicts guide pairs from Guide Set 3 ranked by deletion efficiency, with positive (guide 1+9) and negative controls (backbone only) highlighted in yellow and red, respectively.

**Fig. 23** depicts a heat map showing results of Guide Set 3, a guide set designed to capture guide pairs that result in smaller deletions.

**Fig. 24** depicts Guide Set 3. 168 pairs of human targeting gRNAs were transfected into HEK293T and assayed by ddPCR for Exon 51 deletion. The Z score was calculated to quantify deviation from the mean for a given guide pair. The histogram reflects single biological samples (2 biological replicates per gRNA pair). Pairs with a Z score > 1.5 were selected as hits for further study.

**Fig. 25** depicts Guide hit validation. Guides from Guide Set 2 or 3 with Z scores passing the threshold of 1.5 were validated in a similar plasmid transfection with four biological replicates. Guide Set 4 was also included, alongside the positive control 1 + 9 and the backbone only negative control.

**Fig. 26** depicts gRNAs pairs in Guide Sets 2 and 3 with passing Z scores (i.e., Z-score > 1.5) alongside the 6 pairs from Guide Set 4.

## DETAILED DESCRIPTION

### 1. Definitions

As used herein, the term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

As used herein, a "genome-editing system" refers to a system that is capable of editing (e.g., modifying or altering) a target gene. In certain embodiments, the target gene is a *DMD* gene. In certain embodiments, the *DMD* gene is a human *DMD* gene. In certain embodiments, the genome editing system comprises a first gRNA molecule or a polynucleotide encoding thereof, and at least one Cas 9 molecule or polynucleotide(s) encoding thereof. In certain embodiments, the genome editing system comprises a second gRNA molecule or a polynucleotide encoding thereof. In certain embodiments, the genome-editing system is implemented in a cell or in an *in vitro* contact. In certain embodiments, the genome-editing system is used in a medicament, e.g., a medicament for modifying a *DMD* gene (e.g., a human *DMD* gene), or a medicament for treating Duchenne muscular dystrophy, Becker muscular dystrophy (BMD), or Dilated Cardiomyopathy (DCM) Type 3B.

"Domain" as used herein, is used to describe segments of a protein or nucleic acid. Unless otherwise indicated, a domain is not required to have any specific functional property. In certain embodiments, calculations of homology or sequence identity between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

"Polypeptide", as used herein, refers to a polymer of amino acids having less than 100 amino acid residues. In certain embodiments, it has less than 50, 20, or 10 amino acid residues.

"Alt-HDR," "alternative homology-directed repair," or "alternative HDR" as used herein refers to the process of repairing DNA damage using a homologous nucleic acid (e.g., an endogenous homologous sequence, e.g., a sister

chromatid, or an exogenous nucleic acid, e.g., a template nucleic acid). Alt-HDR is distinct from canonical HDR in that the process utilizes different pathways from canonical HDR, and can be inhibited by the canonical HDR mediators, RAD51 and BRCA2. Also, alt-HDR uses a single-stranded or nicked homologous nucleic acid for repair of the break.

"Canonical HDR" or "canonical homology-directed repair" as used herein refers to the process of repairing DNA damage using a homologous nucleic acid (e.g., an endogenous homologous sequence, e.g., a sister chromatid, or an exogenous nucleic acid, e.g., a template nucleic acid). Canonical HDR typically acts when there has been significant resection at the double strand break, forming at least one single stranded portion of DNA. In a normal cell, HDR typically involves a series of steps such as recognition of the break, stabilization of the break, resection, stabilization of single stranded DNA, formation of a DNA crossover intermediate, resolution of the crossover intermediate, and ligation. The process requires RAD51 and BRCA2, and the homologous nucleic acid is typically double-stranded.

Unless indicated otherwise, the term "HDR" as used herein encompasses both canonical HDR and alt-HDR.

"Non-homologous end joining" or "NHEJ" as used herein refers to ligation mediated repair and/or non-template mediated repair including canonical NHEJ (cNHEJ), alternative NHEJ (altNHEJ), microhomology-mediated end joining (MMEJ), single-strand annealing (SSA), and synthesis-dependent microhomology-mediated end joining (SD-MMEJ).

"Replacement" or "replaced" as used herein with reference to a modification of a molecule does not require a process limitation but merely indicates that the replacement entity is present.

"Subject" as used herein may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In certain embodiments, the subject is a human. In certain embodiments, the human is an infant, child, young adult, or adult. In certain embodiments, the subject is poultry. "Treat", "treating" and "treatment", as used herein, mean the treatment of a disease in a mammal, e.g., in a human, including (a) inhibiting the disease, i.e., arresting or preventing its development or progression; (b) relieving the disease, i.e., causing regression of the disease state; (c) relieving one or more symptoms of the disease; and (d) curing the disease.

"Prevent," "preventing," and "prevention" as used herein means the prevention of a disease in a mammal, e.g., in a human, including (a) avoiding or precluding the disease; (b) affecting the predisposition toward the disease; (c) preventing or delaying the onset of at least one symptom of the disease. "X" as used herein in the context of an amino acid sequence, refers to any amino acid (e.g., any of the twenty natural amino acids) unless otherwise specified. A "Cas9 molecule" or "Cas9 polypeptide" as used herein refers to a molecule or polypeptide, respectively, that can interact with a gRNA molecule and, in concert with the gRNA molecule, localize to a site comprising a target domain and, in certain embodiments, a PAM sequence. Cas9 molecules and Cas9 polypeptides include both naturally occurring Cas9 molecules and Cas9 polypeptides and engineered, altered, or modified Cas9 molecules or Cas9 polypeptides that differ, e.g., by at least one amino acid residue, from a reference sequence, e.g., the most similar naturally occurring Cas9 molecule.

A "reference molecule" as used herein refers to a molecule to which a modified or candidate molecule is compared. For example, a reference Cas9 molecule refers to a Cas9 molecule to which a modified or candidate Cas9 molecule is compared. Likewise, a reference gRNA refers to a gRNA molecule to which a modified or candidate gRNA molecule is compared. The modified or candidate molecule may be compared to the reference molecule on the basis of sequence (e.g., the modified or candidate molecule may have X% sequence identity or homology with the reference molecule) or activity (e.g., the modified or candidate molecule may have X% of the activity of the reference molecule). For example, where the reference molecule is a Cas9 molecule, a modified or candidate molecule may be characterized as having no more than 10% of the nuclease activity of the reference Cas9 molecule. Examples of reference Cas9 molecules include naturally occurring unmodified Cas9 molecules, e.g., a naturally occurring Cas9 molecule from *S. pyogenes, S. aureus, S. thermophilus,* or *N. meningitidis.* In certain embodiments, the reference Cas9 molecule is the naturally occurring Cas9 molecule having the closest sequence identity or homology with the modified or candidate Cas9 molecule to which it is being compared. In certain embodiments, the reference Cas9 molecule is a parental molecule having a naturally occurring or known sequence on which a mutation has been made to arrive at the modified or candidate Cas9 molecule.

"Target sequence" as used herein refers to a nucleic acid sequence comprising a DMD target position.

"DMD target position" as used herein refers to any of a DMD exonic target position or a DMD intra-exonic target position, as described herein. In certain embodiments, the DMD target position comprises exon 51 of the *DMD* gene (e.g., a human *DMD* gene).

"DMD exonic target position" or "DMD ETP," as used herein, refers to a sequence comprising one or more exons of the *DMD* gene. Deletion of the DMD ETP optimizes a subject's *DMD* sequence, e.g., it increases the activity of the encoded dystrophin protein, or results in an improvement in the disease state of the subject. In certain embodiments, excision of the DMD ETP restores reading frame and removes a premature termination codon (PTC).

A DMD ETP can include one, or a plurality of exons. In certain embodiments, where the DMD ETP comprises a single exon, the exon can be referred to as "X," or "exon X." In certain embodiments, the DMD ETP comprises exon 51 of the *DMD* gene (e.g., human *DMD* gene). In certain embodiments, where the ETP comprises a plurality of exons, the 5' exon can be referred to as "X," or "exon X," and the 3' exon can be referred to as "Y," or "exon Y." If exons X and

Y are not contiguous, the exons between them are included in the DMD ETP. In certain embodiments, the ETP comprises an exon having one or more mutation, including, but not limited to, a premature codon, a point mutation, a frameshift mutation (e.g., disrupted reading frame), a duplicated exon, an aberrant splice acceptor site, and an aberrant splice donor site. In certain embodiments, the DMD ETP flanks a mutation, e.g., a deletion mutation.

In certain embodiments, one or more gRNA molecules are used to position one or more cleavage events in an intron 5' to the DMD ETP, an intron 3' to the DMD ETP, or both.

In certain embodiments, a pair of gRNA molecules (two gRNA molecules) are used to mediate excision of the DMD ETP, wherein one gRNA molecule positions a cleavage event in an intron 5' to the DMD ETP (e.g., the intron adjacent to the 5' of the DMD ETP), and one gRNA molecule positions a cleavage event in an intron 3' to the DMD ETP (e.g., the intron adjacent to the 3' of the *DMD* ETP). In certain embodiments, in the case of the 5' intron, the cleavage site is 5' to a splice donor sequence. In certain embodiments, in the case of the 3' intron, the cleavage site is 3' to a splice acceptor sequence. In certain embodiments, the DMD ETP comprises exon 51 of the *DMD* gene (e.g., human *DMD* gene). In certain embodiments, a pair of gRNA molecules are used to mediate excision of exon 51 of the *DMD* gene (e.g., human *DMD* gene), wherein one gRNA molecule positions a cleavage event in intron 50 of the *DMD* gene, and one gRNA molecule positions a cleavage event in intron 51 of the *DMD* gene.

In certain embodiments, a pair of gRNA molecules (two gRNA molecules) are used to mediate excision of the DMD ETP, wherein one gRNA molecule positions a cleavage event in an intron 5' to the DMD ETP (e.g., the intron adjacent to the 5' of the DMD ETP), and one gRNA molecule positions a cleavage event in the DMD ETP. In certain embodiments, the DMD ETP comprises exon 51 of the *DMD* gene (e.g., human *DMD* gene). In certain embodiments, a pair of gRNA molecules are used to mediate excision of exon 51 of the *DMD* gene (e.g., human DMD gene), wherein one gRNA molecule positions a cleavage event in intron 50 of the *DMD* gene, and one gRNA molecule positions a cleavage event in exon 51 of the *DMD* gene.

In certain embodiments, a pair of gRNA molecules (two gRNA molecules) are used to mediate excision of the DMD ETP, wherein one gRNA molecule positions a cleavage event in the DMD ETP, and one gRNA molecule positions a cleavage event in an intron 3' to the DMD ETP (e.g., the intron adjacent to the 3' of the *DMD* ETP). In certain embodiments, the DMD ETP comprises exon 51 of the *DMD* gene (e.g., human *DMD* gene). In certain embodiments, a pair of gRNA molecules are used to mediate excision of exon 51 of the *DMD* gene (e.g., human DMD gene), wherein one gRNA molecule positions a cleavage event in exon 51 of the *DMD* gene, and one gRNA molecule positions a cleavage event in intron 51 of the *DMD* gene.

In certain embodiments, the first gRNA molecule and the second gRNA molecule are selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence

set forth in SEQ ID NO: 3536;

(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;

(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and

(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

In certain embodiments, the pair of gRNA molecules (two gRNA molecules) are selected from the gRNA pairs listed in Table 15, i.e., selected from the group consisting of:

(a) a first gRNA molecule comprising a targeting domain that comprises a nucleotide sequence set forth in SEQ ID NO: 2048, and a second gRNA molecule comprising a targeting domain that comprises a nucleotide sequence selected from SEQ ID NOs: 1977, 18458, 481, 9997, 1499, 2121, 8709, 21570, 16467, and 23958;

(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;

(c) a first gRNA molecule comprising a targeting domain that comprises a nucleotide sequence set forth in SEQ ID NO: 18720, SEQ ID NO: 22349 or SEQ ID NO: 10092, and a second gRNA molecule comprising a targeting domain that comprises a nucleotide sequence set forth in SEQ ID NO: 4709 or SEQ ID NO: 20303;

(d) a first gRNA molecule comprising a targeting domain that comprises a nucleotide sequence set forth in SEQ ID NO: 3536, and a second gRNA molecule comprising a targeting domain that comprises a nucleotide sequence selected from SEQ ID NOs: 5869, 20145, 18752, 20870, 9530, 18062, 4134, and 23958;

(e) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 9765;

(f) a first gRNA molecule comprising a targeting domain that comprises a nucleotide sequence set forth in SEQ ID NO: 18752, and a second gRNA molecule comprising a targeting domain that comprises a nucleotide sequence selected from SEQ ID NOs: 17253, 14607, 16967, 7419, 1744, and 8569;

(g) a first gRNA molecule comprising a targeting domain that comprises a nucleotide sequence set forth in SEQ ID NO: 19187, and a second gRNA molecule comprising a targeting domain that comprises a nucleotide sequence selected from SEQ ID NOs: 14695, 7252, 17253, 7419, 1744, 8569, and 9358;

(h) a first gRNA molecule comprising a targeting domain that comprises a nucleotide sequence set forth in SEQ ID NO: 20145, and a second gRNA molecule comprising a targeting domain that comprises a nucleotide sequence selected from SEQ ID NOs: 5614, 20764, 14607, 8569, and 7252;

(i) a first gRNA molecule comprising a targeting domain that comprises a nucleotide sequence set forth in SEQ ID NO: 1499, and a second gRNA molecule comprising a targeting domain that comprises a nucleotide sequence set forth in SEQ ID NO: 14296;

(i) a first gRNA molecule comprising a targeting domain that comprises a nucleotide sequence set forth in SEQ ID NO: 4134, and a second gRNA molecule comprising a targeting domain that comprises a nucleotide sequence selected from SEQ ID NOs: 20924, 4072, and 8569;

(k) a first gRNA molecule comprising a targeting domain that comprises a nucleotide sequence set forth in SEQ ID NO: 6209, and a second gRNA molecule comprising a targeting domain that comprises a nucleotide sequence selected from SEQ ID NOs: 14035, 13527, 21873, 14298, 7419, and 20924;

(l) a first gRNA molecule comprising a targeting domain that comprises a nucleotide sequence set forth in SEQ ID NO: 9346, and a second gRNA molecule comprising a targeting domain that comprises a nucleotide sequence selected from SEQ ID NOs: 7419, 20764, and 2769;

(m) a first gRNA molecule comprising a targeting domain that comprises a nucleotide sequence set forth in SEQ ID NO: 7649, and a second gRNA molecule comprising a targeting domain that comprises a nucleotide sequence selected from SEQ ID NOs: 16967, 1484, 20924, 4072, and 9496;

(n) a first gRNA molecule comprising a targeting domain that comprises a nucleotide sequence set forth in SEQ ID NO: 5869, and a second gRNA molecule comprising a targeting domain that comprises a nucleotide sequence selected from SEQ ID NOs: 8569, 13527, 1484, 5614, 4072, and 14695;

(o) a first gRNA molecule comprising a targeting domain that comprises a nucleotide sequence set forth in SEQ ID NO: 4165, and a second gRNA molecule comprising a targeting domain that comprises a nucleotide sequence

selected from SEQ ID NOs: 7252, 1484, and 9279;

(p) a first gRNA molecule comprising a targeting domain that comprises a nucleotide sequence set forth in SEQ ID NO: 15622, and a second gRNA molecule comprising a targeting domain that comprises a nucleotide sequence set forth in SEQ ID NO: 17253;

(q) a first gRNA molecule comprising a targeting domain that comprises a nucleotide sequence set forth in SEQ ID NO: 18062, and a second gRNA molecule comprising a targeting domain that comprises a nucleotide sequence set forth in SEQ ID NO: 2769 or SEQ ID NO: 14035;

(r) a first gRNA molecule comprising a targeting domain that comprises a nucleotide sequence set forth in SEQ ID NO: 21131, and a second gRNA molecule comprising a targeting domain that comprises a nucleotide sequence set forth in SEQ ID NO: 13527 or SEQ ID NO: 13285;

(s) a first gRNA molecule comprising a targeting domain that comprises a nucleotide sequence set forth in SEQ ID NO: 20870, and a second gRNA molecule comprising a targeting domain that comprises a nucleotide sequence set forth in SEQ ID NO: 16967 or SEQ ID NO: 7252;

(t) a first gRNA molecule comprising a targeting domain that comprises a nucleotide sequence set forth in SEQ ID NO: 16235, and a second gRNA molecule comprising a targeting domain that comprises a nucleotide sequence set forth in SEQ ID NO: 10140;

(u) a first gRNA molecule comprising a targeting domain that comprises a nucleotide sequence set forth in SEQ ID NO: 15271, and a second gRNA molecule comprising a targeting domain that comprises a nucleotide sequence set forth in SEQ ID NO: 13518;

(v) a first gRNA molecule comprising a targeting domain that comprises a nucleotide sequence set forth in SEQ ID NO: 11675, and a second gRNA molecule comprising a targeting domain that comprises a nucleotide sequence selected from SEQ ID NOs: 2769, 1484, and 4072; and

(w) a first gRNA molecule comprising a targeting domain that comprises a nucleotide sequence set forth in SEQ ID NO: 23958, and a second gRNA molecule comprising a targeting domain that comprises a nucleotide sequence set forth in SEQ ID NO: 14035 or SEQ ID NO: 9496.

In certain embodiments, the pair of gRNA molecules (two gRNA molecules) are selected from the gRNA pairs listed in Table 7 or Table 8. i.e., from the group consisting of:

(a) a first gRNA molecule comprising a targeting domain that comprises a nucleotide sequence set forth in SEQ ID NO: 692257, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(b) a first gRNA molecule comprising a targeting domain that comprises a nucleotide sequence set forth in SEQ ID NO: 692258, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(c) a first gRNA molecule comprising a targeting domain that comprises a nucleotide sequence set forth in SEQ ID NO: 692260, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(d) a first gRNA molecule comprising a targeting domain that comprises a nucleotide sequence set forth in SEQ ID NO: 692253, and a second gRNA molecule comprising a targeting domain that comprises a nucleotide sequence set forth in SEQ ID NO: 692270, SEQ ID NO: 692271, or SEQ ID NO: 692272;

(e) a first gRNA molecule comprising a targeting domain that comprises a nucleotide sequence set forth in SEQ ID NO: 692254, and a second gRNA molecule comprising a targeting domain that comprises a nucleotide sequence set forth in SEQ ID NO: 692270, SEQ ID NO: 692271, or SEQ ID NO: 692272; and

(f) a first gRNA molecule comprising a targeting domain that comprises a nucleotide sequence set forth in SEQ ID NO: 692256, and a second gRNA molecule comprising a targeting domain that comprises a nucleotide sequence set forth in SEQ ID NO: 692270, SEQ ID NO: 692271, or SEQ ID NO: 692272.

Excision of a DMD ETP can be used to modify or alter a *DMD* sequence having a deletion mutation. In certain embodiments, the DMD target sequence comprises a deletion of one or more dystrophin exons, e.g., resulting in ectopic exons, having different reading frames from one another, being placed adjacent to one another, giving rise to a PTC, typically in the first exon following the deletion. Excision of the DMD ETP results in restoration of the correct reading frame and removal of the PTC. **Fig. 13** can be used to select ectopic (e.g., non-contiguous) exons having the same reading frame. In certain embodiments, the DMD ETP is configured so as to remove the minimal number of exons from the *DMD* sequence to restore the correct reading frame. In certain embodiments, the DMD ETP is configured so as to

remove one or more specific exons from the *DMD* sequence such that the correct reading frame is restored and one or more preselected function domains are restored.

In certain embodiments, the DMD ETP is contiguous with the subject's deletion mutation delPQ, e.g., 3' to the deletion mutation. Excision of the DMD ETP results in the exon just 5' to the deletion being contiguous with a remaining exon 3' to the deletion having the same reading frame as the exon just 5' to the deletion. In certain embodiments, the exon 3' to the deletion is the first remaining exon 3' to the deletion having the desired reading frame. See **Fig. 10A.** In **Fig. 10A,** the deleted exons of the subject's *DMD* gene are indicated by a curly bracket. The DMD ETP includes exons X and Y, adjacent to or contiguous with the deletion. Excision of X and Y places ectopic exons X-1 and Y+1 (which have the same reading frame) contiguous with one another, restoring the correct reading frame.

In certain embodiments, the DMD ETP is contiguous with the subject's deletion mutation, e.g., 5' to the deletion mutation. Excision of the DMD ETP results in the exon just 3' to the deletion being contiguous with a remaining exon 5' to the deletion having the same reading frame as the exon just 3' to the deletion. In certain embodiments, the exon 5' to the deletion is the first remaining exon 5' to the deletion having the desired reading frame. See **Fig. 10B.** In **Fig. 10B,** the deleted exons of the subject's *DMD* gene are indicated by a curly bracket. The DMD ETP includes exons X and Y, adjacent to or contiguous with the deletion. Excision of X and Y places ectopic exons X-1 and Y+1 (which have the same reading frame) contiguous with one another, restoring the correct reading frame.

In certain embodiments, the DMD ETP flanks the subject's deletion mutation and includes an exon 5' to the deletion mutation and an exon 3' to the deletion mutation. Excision of the DMD ETP results in a remaining exon 5' to the deletion being contiguous with a remaining exon 3' to the deletion having the same reading frame as the remaining exon 5' to the deletion. In certain embodiments, the exon 5' to the deletion is the first remaining exon 5' to the deletion having the desired reading frame. In certain embodiments, the exon 3' to the deletion is the first remaining exon 3' to the deletion having the desired reading frame. In certain embodiments, the exon 5' to the deletion is the first remaining exon 5' to the deletion having the desired reading frame and the exon 3' to the deletion is the first remaining exon 3' to the deletion having the desired reading frame. See **Fig. 10C.** In **Fig. 10C,** the deleted exons of the subject's *DMD* gene are indicated by a curly bracket. The DMD ETP includes exons X and Y, adjacent to or contiguous with the deletion. Excision of X and Y places ectopic exons X-1 and Y+1 (which have the same reading frame) contiguous with one another, restoring the correct reading frame.

In certain embodiments, e.g., where the deletion mutation comprises a single exon, the exon can be referred to as "P," or "exon P," and the deletion mutation can be denoted delP. In certain embodiments, e.g., where the deletion mutation comprises a plurality of exons, the 5' exon can be referred to as "P," or "exon P," and the 3' exon can be referred to as "Q," or "exon Q," and the deletion mutation can be denoted delPQ. If exons P and Q are not contiguous, the exons between them are included in the deletion mutation.

Excision of a DMD ETP can be used to modify or alter a *DMD* sequence having a duplication mutation. In certain embodiments, the DMD target sequence comprises a duplication of one or more *DMD* exons, e.g., resulting in ectopic exons, having different reading frames from one another, being placed adjacent to one another, giving rise to a PTC, typically in the first duplicated exon. Excision of the DMD ETP results in restoration of the correct reading frame and removal of the PTC. **Fig. 13** can be used to select ectopic (e.g., non-contiguous) exons having the same reading frame. In certain embodiments, the DMD ETP is configured so as to remove the minimal number of exons from the *DMD* sequence to restore the correct reading frame. In certain embodiments, the DMD ETP is configured so as to remove one or more specific exons from the *DMD* sequence such that the correct reading frame is restored and one or more preselected function domains are restored. In certain embodiments, the DMD ETP includes the subject's duplication mutation and an exon contiguous with the duplication, e.g., 3' to the duplication. Excision of the DMD ETP results in the exon just 5' to the duplication being contiguous with a remaining exon 3' to the duplication having the same reading frame as the exon just 5' to the duplication. In certain embodiments, the exon 3' to the duplication is the first remaining exon 3' to the duplication having the desired reading frame. See **Fig. 11A.** In **Fig. 11A,** the duplicated exons of the subject's *DMD* gene are indicated by a curly bracket. The DMD ETP includes exons X and Y, adjacent to or contiguous with the duplication. Excision of X, Y and the duplicated exons places ectopic exons X-1 and Y+1 (which have the same reading frame) contiguous with one another, restoring the correct reading frame.

In certain embodiments, the DMD ETP includes the subject's duplication mutation and an exon contiguous with the duplication, e.g., 5' of the duplication. Excision of the DMD ETP results in the exon just 3' of the duplication being contiguous with a remaining exon 5' to the duplication having the same reading frame as the exon just 3' to the duplication. In certain embodiments, the exon 5' of the duplication is the first remaining exon 5' of the duplication having the desired reading frame. See **Fig. 11B**. In **Fig. 11B**, the duplicated exons of the subject's *DMD* gene are indicated by a curly bracket. The DMD ETP includes exons X and Y, adjacent to or contiguous with the duplication. Excision of X, Y and the duplicated exons places ectopic exons X-1 and Y+1 (which have the same reading frame) contiguous with one another, restoring the correct reading frame.

In certain embodiments, the DMD ETP flanks the subject's duplication mutation and includes an exon 5' to the duplication and an exon 3' to the duplication. Excision of the DMD ETP results in a remaining exon 5' to the duplication

being contiguous with a remaining exon 3' to the duplication having the same reading frame as the remaining exon 5' to the duplication. In certain embodiments, the exon 5' to the duplication is the first remaining exon 5' to the duplication having the desired reading frame. In certain embodiments, the exon 3' to the duplication is the first remaining exon 3' to the duplication having the desired reading frame. In certain embodiments, the exon 5' to the duplication is the first remaining exon 5' to the duplication having the desired reading frame and the exon 3' to the duplication is the first remaining exon 3' to the duplication having the desired reading frame. See **Fig. 11C.** In **Fig. 11C,** the duplicated exons of the subject's *DMD* gene are indicated by a curly bracket. The DMD ETP includes exons X and Y, adjacent to or contiguous with the duplication. Excision of X, Y and the duplicated exons places ectopic exons X-1 and Y+1 (which have the same reading frame) contiguous with one another, restoring the correct reading frame.

Excision of a DMD ETP can be used to modify or alter a *DMD* sequence having a point mutation, e.g., a missense or nonsense mutation, or an indel mutation. In certain embodiments, the DMD ETP comprises an exon that has a missense mutation. In certain embodiments, the DMD ETP comprises an exon that has a nonsense mutation. In certain embodiments, the DMD ETP comprises an exon that has an indel mutation.

In certain embodiments, if excision of that exon results in now-contiguous ectopic exons (e.g., exons just 5' and 3' of the exon having the mutation that are now contiguous with one another) that have the correct reading frame, the DMD ETP can include only the exon having the mutation. See **Fig. 12A.** In **Fig. 12A,** the exon having the point mutation is indicated by a curly bracket. The DMD ETP includes exon X. Excision of X places ectopic exons X-1 and X+1 (which have the same reading frame) contiguous with one another, maintaining the correct reading frame.

In certain embodiments, if excision of that exon does not result in ectopic exons (e.g., exons just 5' and 3' of the exon having the mutation) that have the correct reading frame, additional exons, 3', 5' or both, can be included in the DMD ETP, such that after excision of the DMD ETP, the *DMD* gene sequence is in the correct reading frame. See **Fig. 12B.** In **Fig. 12B,** the exon having the point mutation is indicated by a curly bracket. The DMD ETP includes exons X and Y. X is the exon that has the point mutation or indel mutation. Excision of X and Y places ectopic exons X-1 and Y+1 (which have the same reading frame) contiguous with one another, maintaining or restoring the correct reading frame.

"DMD intra-exonic target position" or "DMD ITP," as used herein, refers to any of a DMD intra-exonic point or indel target position, a DMD intra-exonic frameshift target position, or a DMD intra-exonic duplication target position, as described herein.

"DMD intra-exonic point or indel target position" or "DMD IPTP," as used here, refers to a position in the *DMD* gene, within an exon, e.g., a point mutation, e.g., a missense or nonsense mutation, an indel mutation, a mutational hotspot, a sequence which, if frameshifted, would give rise to a premature termination codon. Alteration or modification of a DMD IPTP optimizes a subject's *DMD* sequence, e.g., it increases the activity of the encoded dystrophin protein, or results in an improvement in the disease state of the subject. In certain embodiments, the exon is exon 51 of the *DMD* gene (e.g., human *DMD* gene).

In certain embodiments, a gRNA molecule that positions a cleavage event at or in close proximity to the DMD IPTP, e.g., the point mutation, e.g., in or near a mutant codon, is used to alter or modify the DMD IPTP. In certain embodiments, the NHEJ mediated repair or resolution of the cleavage event results in alteration or modification of the DMD IPTP, e.g., the point mutation, e.g., the missense or nonsense mutation, or the indel mutation, e.g., by removal of the point mutation, e.g., the missense or nonsense mutation, or the indel mutation.

In certain embodiments, the DMD IPTP comprises a sequence which, if frameshifted, would give rise to a premature stop codon. The DMD IPTP can be excised by two cleavage events, which flank the sequence which, if frameshifted, would give rise to a premature stop codon.

In certain embodiments, a first gRNA molecule positions a cleavage event at or in close proximity to a first premature termination codon, e.g., 5' of the first premature termination codon, and a second gRNA molecule positions a cleavage event at or in close proximity to a second premature termination codon, e.g., 3' of the second premature termination codon. In certain embodiments, the first and second premature termination codons are in the same exon. In certain embodiments, the first and second premature termination codons are in exon 51 of human DMD gene. In certain embodiments, the first premature termination codon is the 5'-most termination codon in the exon. In certain embodiments, the second premature termination codon is the 3'-most termination codon in the exon. In certain embodiments, the first premature termination codon is the 5'-most termination codon, and the second premature termination codon is the 3'-most termination codon, in the same exon. In certain embodiments, NHEJ mediated deletion with two or more gRNAs of a sequence containing a mutational hotspot or a premature termination codon will excise the region containing the mutational hotspot or the premature termination codon, restoring the correct reading frame.

"DMD intra-exonic frame-shift target position" or "DMD IFTP," as used herein, refers to a position in the *DMD* gene, within an exon, at which alteration or modification, e.g., insertion or deletion of one or two bases (or a multiple of 1 or 2 bases) restores correct reading frame and negates the effect of a frame shifting event. In certain embodiments, the frameshifting event is a deletion or duplication event that has created a frameshift in the *DMD* gene. Alteration or modification of a DMD IFTP optimizes a subject's *DMD* sequence, e.g., it increases the activity of the encoded dystrophin protein, or results in an improvement in the disease state of the subject. In certain embodiments, the exon is exon 51

of the *DMD* gene (e.g., human *DMD* gene).

In certain embodiments, a gRNA molecule that positions a cleavage event upstream or downstream of the frame shifting event is used to alter or modify the DMD IFTP. In certain embodiments, a gRNA molecule that positions a cleavage event upstream of a premature termination codon is used to alter or modify the DMD IFTP. In certain embodiments, the NHEJ mediated repair or resolution of the cleavage event results in restoring correct reading frame in at least some *DMD* sequences. In certain embodiments, two gRNA molecules are used to alter or modify the DMD IFTP, wherein one gRNA positions a cleavage event upstream of the frame shifting event, and one gRNA molecule positions a cleavage event downstream of the frame shifting event. In certain embodiment, the DMD IFTP is within exon 51 of the *DMD* gene (e.g., human *DMD* gene). In certain embodiments, two gRNA molecules are used to alter or modify a DMD IFTP within exon 51 of the *DMD* gene (e.g., human *DMD* gene), wherein one gRNA positions a cleavage event upstream of the frame shifting event, and one gRNA molecule positions a cleavage event downstream of the frame shifting event. In certain embodiments, one gRNA molecule targets intron 50 of the *DMD* gene, and one gRNA molecule targets intron 51 of the *DMD* gene. In certain embodiments, both two gRNA molecules target exon 51 of the *DMD* gene. In certain embodiments, one gRNA molecule targets intron 50 of the *DMD* gene, and one gRNA molecule targets exon 51 of the *DMD* gene. In certain embodiments, one gRNA molecule targets exon 51 of the *DMD* gene, and one gRNA molecule targets intron 51 of the *DMD* gene. In certain embodiments, the first gRNA molecule and the second gRNA molecule are selected from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

In certain embodiments, the pair of gRNA molecules (two gRNA molecules) are selected from the gRNA pairs listed in Table 15, i.e., the group consisting of :

(a) a first gRNA molecule comprising a targeting domain that comprises a nucleotide sequence set forth in SEQ ID NO: 2048, and a second gRNA molecule comprising a targeting domain that comprises a nucleotide sequence selected from SEQ ID NOs: 1977, 18458, 481, 9997, 1499, 2121, 8709, 21570, 16467, and 23958;
(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(c) a first gRNA molecule comprising a targeting domain that comprises a nucleotide sequence set forth in SEQ ID NO: 18720, SEQ ID NO: 22349 or SEQ ID NO: 10092, and a second gRNA molecule comprising a targeting domain that comprises a nucleotide sequence set forth in SEQ ID NO: 4709 or SEQ ID NO: 20303;
(d) a first gRNA molecule comprising a targeting domain that comprises a nucleotide sequence set forth in SEQ ID NO: 3536, and a second gRNA molecule comprising a targeting domain that comprises a nucleotide sequence selected from SEQ ID NOs: 5869, 20145, 18752, 20870, 9530, 18062, 4134, and 23958;
(e) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 9765;
(f) a first gRNA molecule comprising a targeting domain that comprises a nucleotide sequence set forth in SEQ ID NO: 18752, and a second gRNA molecule comprising a targeting domain that comprises a nucleotide sequence selected from SEQ ID NOs: 17253, 14607, 16967, 7419, 1744, and 8569;
(g) a first gRNA molecule comprising a targeting domain that comprises a nucleotide sequence set forth in SEQ ID NO: 19187, and a second gRNA molecule comprising a targeting domain that comprises a nucleotide sequence selected from SEQ ID NOs: 14695, 7252, 17253, 7419, 1744, 8569, and 9358;
(h) a first gRNA molecule comprising a targeting domain that comprises a nucleotide sequence set forth in SEQ ID NO: 20145, and a second gRNA molecule comprising a targeting domain that comprises a nucleotide sequence selected from SEQ ID NOs: 5614, 20764, 14607, 8569, and 7252;
(i) a first gRNA molecule comprising a targeting domain that comprises a nucleotide sequence set forth in SEQ ID NO: 1499, and a second gRNA molecule comprising a targeting domain that comprises a nucleotide sequence set forth in SEQ ID NO: 14296;
(i) a first gRNA molecule comprising a targeting domain that comprises a nucleotide sequence set forth in SEQ ID NO: 4134, and a second gRNA molecule comprising a targeting domain that comprises a nucleotide sequence selected from SEQ ID NOs: 20924, 4072, and 8569;
(k) a first gRNA molecule comprising a targeting domain that comprises a nucleotide sequence set forth in SEQ ID NO: 6209, and a second gRNA molecule comprising a targeting domain that comprises a nucleotide sequence selected from SEQ ID NOs: 14035, 13527, 21873, 14298, 7419, and 20924;
(l) a first gRNA molecule comprising a targeting domain that comprises a nucleotide sequence set forth in SEQ ID NO: 9346, and a second gRNA molecule comprising a targeting domain that comprises a nucleotide sequence selected from SEQ ID NOs: 7419, 20764, and 2769;
(m) a first gRNA molecule comprising a targeting domain that comprises a nucleotide sequence set forth in SEQ ID NO: 7649, and a second gRNA molecule comprising a targeting domain that comprises a nucleotide sequence selected from SEQ ID NOs: 16967, 1484, 20924, 4072, and 9496;
(n) a first gRNA molecule comprising a targeting domain that comprises a nucleotide sequence set forth in SEQ ID NO: 5869, and a second gRNA molecule comprising a targeting domain that comprises a nucleotide sequence selected from SEQ ID NOs: 8569, 13527, 1484, 5614, 4072, and 14695;
(o) a first gRNA molecule comprising a targeting domain that comprises a nucleotide sequence set forth in SEQ ID NO: 4165, and a second gRNA molecule comprising a targeting domain that comprises a nucleotide sequence selected from SEQ ID NOs: 7252, 1484, and 9279;
(p) a first gRNA molecule comprising a targeting domain that comprises a nucleotide sequence set forth in SEQ ID NO: 15622, and a second gRNA molecule comprising a targeting domain that comprises a nucleotide sequence set forth in SEQ ID NO: 17253;
(q) a first gRNA molecule comprising a targeting domain that comprises a nucleotide sequence set forth in SEQ ID NO: 18062, and a second gRNA molecule comprising a targeting domain that comprises a nucleotide sequence set forth in SEQ ID NO: 2769 or SEQ ID NO: 14035;
(r) a first gRNA molecule comprising a targeting domain that comprises a nucleotide sequence set forth in SEQ ID NO: 21131, and a second gRNA molecule comprising a targeting domain that comprises a nucleotide sequence set forth in SEQ ID NO: 13527 or SEQ ID NO: 13285;
(s) a first gRNA molecule comprising a targeting domain that comprises a nucleotide sequence set forth in SEQ ID NO: 20870, and a second gRNA molecule comprising a targeting domain that comprises a nucleotide sequence set forth in SEQ ID NO: 16967 or SEQ ID NO: 7252;
(t) a first gRNA molecule comprising a targeting domain that comprises a nucleotide sequence set forth in SEQ ID

NO: 16235, and a second gRNA molecule comprising a targeting domain that comprises a nucleotide sequence set forth in SEQ ID NO: 10140;

(u) a first gRNA molecule comprising a targeting domain that comprises a nucleotide sequence set forth in SEQ ID NO: 15271, and a second gRNA molecule comprising a targeting domain that comprises a nucleotide sequence set forth in SEQ ID NO: 13518;

(v) a first gRNA molecule comprising a targeting domain that comprises a nucleotide sequence set forth in SEQ ID NO: 11675, and a second gRNA molecule comprising a targeting domain that comprises a nucleotide sequence selected from SEQ ID NOs: 2769, 1484, and 4072; and

(w) a first gRNA molecule comprising a targeting domain that comprises a nucleotide sequence set forth in SEQ ID NO: 23958, and a second gRNA molecule comprising a targeting domain that comprises a nucleotide sequence set forth in SEQ ID NO: 14035 or SEQ ID NO: 9496.

In certain embodiments, the pair of gRNA molecules (two gRNA molecules) are selected from the gRNA pairs listed in Table 7 or Table 8, i.e., the group consisting of:

(a) a first gRNA molecule comprising a targeting domain that comprises a nucleotide sequence set forth in SEQ ID NO: 692257, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(b) a first gRNA molecule comprising a targeting domain that comprises a nucleotide sequence set forth in SEQ ID NO: 692258, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(c) a first gRNA molecule comprising a targeting domain that comprises a nucleotide sequence set forth in SEQ ID NO: 692260, and a second gRNA molecule comprising a targeting domain that comprises a nucleotide sequence set forth in SEQ ID NO: 692261, SEQ ID NO: 692262, SEQ ID NO: 692263, SEQ ID NO: 692264, SEQ ID NO: 692270, SEQ ID NO: 692271, or SEQ ID NO: 692272;

(d) a first gRNA molecule comprising a targeting domain that comprises a nucleotide sequence set forth in SEQ ID NO: 692253, and a second gRNA molecule comprising a targeting domain that comprises a nucleotide sequence set forth in SEQ ID NO: 692270, SEQ ID NO: 692271, or SEQ ID NO: 692272;

(e) a first gRNA molecule comprising a targeting domain that comprises a nucleotide sequence set forth in SEQ ID NO: 692254, and a second gRNA molecule comprising a targeting domain that comprises a nucleotide sequence set forth in SEQ ID NO: 692270, SEQ ID NO: 692271, or SEQ ID NO: 692272; and

(f) a first gRNA molecule comprising a targeting domain that comprises a nucleotide sequence set forth in SEQ ID NO: 692256, and a second gRNA molecule comprising a targeting domain that comprises a nucleotide sequence set forth in SEQ ID NO: 692270, SEQ ID NO: 692271, or SEQ ID NO: 692272.

"DMD intra-exonic duplication target position" or "DMD IDTP," as used herein, refers to a region, within duplicated exons. Deletion of the DMD IDTP optimizes a subject's *DMD* sequence, e.g., it increases the activity of the encoded dystrophin protein, or results in an improvement in the disease state of the subject. In certain embodiments, excision of the DMD IDTP restores reading frame and removes a premature termination codon (PTC). In certain embodiments, excision of the DMD IDTP restores the RNA profile of dystrophin and improves the phenotype of DMD

In certain embodiments, one or more gRNA molecules are used to position one or more cleavage events in a 5' duplicated exon, a 3' duplicated exon, or both. In certain embodiments, a single gRNA molecule is used to position a cleavage event in a 5' duplicated exon and a cleavage event in a 3' duplicated exon. In certain embodiments, a pair of gRNA molecules, one which positions a cleavage event in a 5' duplicated exon, and one which positions a cleavage event in a 3' duplicated exon, are used to mediate excision of the DMD IDTP. If the 5' exon and the 3' exon are not contiguous, the additional exon(s) between them can be included in the DMD IDTP.

## 2. Methods of Altering *DMD* Gene

The *DMD* gene encodes the protein dystrophin, an integral structural protein of muscle cells. Dystrophin is part of the dystrophin-associated glycoprotein complex (DCG) that connects the actin cytoskeleton to the extracellular matrix. Dystrophin plays an essential role in anchoring the DCG and facilitating muscle contraction. Mutations in the *DMD* gene have been shown to cause DMD, BMD, or DCM3B.

The DMD gene can produce different transcripts encoding various dystrophin isoforms, i.e. proteins of varying lengths comprising different segments of the basic dystrophin sequence. The dystrophin isoforms are encoded by different mRNAs, which can be generated by use of different and unique promoters, alternative splicing, or use of different

polyA-addition signals.

In certain embodiments, the presently disclosed subject matter provides for a one-time treatment via a gene editing approach, which could be effective over a long time period (when compared with oligonucleotide therapies in development). Further, the one-time treatment would reduce inflammation and the requirement of multiple, repeat IM injections into already fragile muscle tissue (which is under inflammatory strain). In all, a one-time treatment for DMD, BMD and DCM type 3B would be superior to methods currently in development.

Methods described herein can, in certain embodiments, alter or modify the *DMD* gene in subjects with dystrophin mutations. Dystrophin mutations occur throughout the gene and include, but are not limited to, deletion, duplication, point (missense or nonsense), splice site (e.g., an aberrant splice acceptor site, or an aberrant splice donor site), and indel mutations, a premature stop codon, and disrupted reading frame. These mutations may all lead to premature truncation of the dystrophin protein. Premature truncation of the dystrophin protein generally leads to a severe DMD phenotype. In general, deletions that lead to out-of-frame reading of the dystrophin protein cause premature truncation of the *DMD* gene which can cause DMD in subjects. Mutations that cause deletions within the *DMD* gene which do not lead to out-of-frame reading of the dystrophin protein generally do not cause premature truncation. Subjects with in-frame deletion mutations generally have a milder phenotype, described as BMD.

Disclosed herein are compositions, genome-editing systems and methods for altering a DMD target position in the *DMD* gene. In certain embodiments, the compositions, genome-editing systems and methods introduce one or more breaks at or near a DMD target position in at least one allele of the *DMD* gene. In certain embodiments, the one or more breaks are repaired or resolved by NHEJ. During repair or resolution of the one or more breaks, a sequence can be inserted or deleted, which resulting in, e.g., removal of one or more exons (e.g., deletion of exon 51 (exon 51 skipping), deletion of exon 44-55, or deletion of any exon 2-20) leading to exon skipping and restoration of a functional or partially functional dystrophin protein; deletion of an insertion mutation that has caused early truncation of dystrophin or a mutant protein to restore the reading frame); deletion of a premature stop codon (e.g., in the case of a nonsense mutation); or deletion of a missense mutation and restoration of the reading frame. The mutations addressed by the methods described herein may be, but are not limited to, deletion, duplication, point (e.g., missense or nonsense), splice site (e.g., an aberrant splice acceptor site, or an aberrant splice donor site), and indel mutations, a premature stop codon, and disrupted reading frame.

There are many different dystrophin mutations that lead to common pathways of altered dystrophin expression. Subjects often have discrete, individual mutations that lead to common exonic deletions within the *DMD* gene. Many different mutations (often at different intronic locations) lead to the same deletion or duplication mutation, with the same location of truncation. Many subjects with different indel or point mutations may have premature truncation of the dystrophin protein at a common site within an exon or within a common exon. The methods described herein can target subsets of patients who have different mutations but can be treated with a common guide RNA(s).

Subjects can be genotyped via any of the following exemplary methods: multiplex polymerase chain reaction (PCR), Southern blot analysis, dosimetric PCR-based methods, multiplex amplifiable probe hybridization (MAPH), multiplex ligation-dependent probe amplification (MLPA), and multiplex single-strand conformational polymorphism analysis (SSCP), denaturing high-performance liquid phase chromatography (dHPLC), denaturing gradient gel-electrophoresis (DGGE), and single-condition amplification/internal primer (SCAIP) sequencing. RNA profiling may also be used to determine the dystrophin protein transcript in a subject. After analysis of an individual subject's mutation, a beneficial approach out of those described herein can be chosen. For example, a beneficial approach can be that which preserves the greatest amount of the dystrophin transcript, or the most important exons of the dystrophin transcript. Examples of certain mutations and associated approaches are detailed herein.

In certain embodiments, the compositions, genome-editing systems and methods disclosed herein restore the reading frame of the *DMD* gene by removing one or more exons to restore the reading frame of the *DMD* gene. In certain embodiments, the compositions, genome-editing systems and methods restore the reading frame of the *DMD* gene by removing a mutational hotspot. In certain embodiments, the compositions, genome-editing systems and methods restore the reading frame of the *DMD* gene by removing a segment of the *DMD* gene to avoid the read-through of a point or indel mutation. In certain embodiments, the compositions, genome-editing systems and methods restore the reading frame of the *DMD* gene by removing a segment of the *DMD* gene that is contiguous to a deletion mutation, e.g., a segment of the DMD gene is removed 5' of a deletion mutation to restore the reading frame, e.g., a segment of the DMD gene is removed 3' of a deletion mutation to restore the reading frame. In certain embodiments, the compositions, genome-editing systems and methods restore the reading frame of the *DMD* gene by removing a segment of the *DMD* gene to excise common point or indel mutations. In certain embodiments, the compositions, genome-editing systems and methods restore the reading frame of the *DMD* gene by removing a segment of the *DMD* gene to restore the reading frame of the *DMD* gene 5' to a point mutation or indel mutation. In certain embodiments, the compositions, genome-editing systems and methods described herein lead to production of a mini-dystrophin or a dystrophin that is no longer prematurely truncated in any subject with a mutation that causes premature truncation of the *DMD* gene. In certain embodiments, the compositions, genome-editing systems and methods restore the reading frame of the *DMD* gene by

removing an out-of-frame deletion or duplication mutation. In certain embodiments, in a subject with an in-frame duplication mutation, e.g., a duplication mutation that leads to a DMD phenotype due to post-transcriptional-translational mechanisms, the compositions, genome-editing systems and methods described herein remove a region of the DMD gene containing a duplication mutation and can lead to production of a mini-dystrophin or a dystrophin.

A mini-dystrophin is a dystrophin protein missing some internal segment of the protein. A truncated dystrophin protein is a dystrophin protein missing some length of the 3' end of the protein. For example, in a subject with the deletion mutation del45-50, the subject produces a truncated dystrophin that is truncated in the coding region of exon 51. The truncated dystrophin protein contains the amino acids encoded by exons 1-44 plus a segment of exon 51 that is 5' of the premature termination codon (PTC). In certain embodiments, a mini-dystrophin is produced in such a subject. For example, in a subject with del45-50 mutation, exonic deletion of exon 51 with intronic cleavage can result in a corrected mini-dystrophin transcript containing the amino acids encoded by exons 1-44 and 52-79. In certain embodiments, in a subject with a deletion mutation that involves exons X-50, e.g., in a subject with a del45-50 mutation, a del47-50 mutation, a del48-50 mutation, a del49-50 mutation, a del50 mutation, exonic deletion of exon 51 with intronic cleavage can result in a corrected mini-dystrophin transcript containing the amino acids encoded by exons 1-(X-1) and 52-79, e.g., in a subject with a del45-50 mutation, the corrected mini-dystrophin will include exons 1-44 and 52-79, e.g., in a subject with a del47-50 mutation, the corrected mini-dystrophin will include exons 1-46 and 52-79, e.g., in a subject with a del48-50 mutation, the corrected mini-dystrophin will include exons 1-47 and 52-79, e.g., in a subject with a del49-50 mutation, the corrected mini-dystrophin will include exons 1-48 and 52-79, e.g., in a subject with a del50 mutation the corrected mini-dystrophin will include exons 1-49 and 52-79. In certain embodiments, in a subject with a deletion mutation that involves exons 52-Y, e.g., in a subject with a del52 mutation, a del52-63 mutation, exonic deletion of exon 51 with intronic cleavage can result in a corrected mini-dystrophin transcript containing the amino acids encoded by exons 1-50 and (Y+1)-79, e.g., in a subject with a del52 mutation, the corrected mini-dystrophin will include exons 1-50 and 53-79, e.g., in a subject with a del52-63 mutation, the corrected mini-dystrophin will include exons 1-50 and 64-79.

In certain embodiments, the compositions, genome-editing systems and methods produce a functional or partially functional dystrophin protein, e.g., restore reading frame in a subject with an out-of-frame deletion or duplication mutation, and produce or generate an intact dystrophin or a mini-dystrophin. In certain embodiments, the compositions, genome-editing systems and methods restore or improve the dystrophin RNA profile in a subject with an in-frame deletion or duplication mutation, and produce or generate an intact dystrophin or a mini-dystrophin. In certain embodiments, the compositions, genome-editing systems and methods produce or generate a mini-dystrophin in a subject with a point or indel mutation.

In certain embodiments, the mini-dystrophin lacks exons X through Y, in a subject with delP-Q, or lacking exon X in a subject with a point or indel mutation in exon X. In certain embodiments, this mini-dystrophin is more functional than a truncated dystrophin, e.g., that commonly found in a DMD patient. In certain embodiments, this mini-dystrophin is associated with a milder phenotype, e.g., that of BMD. In certain embodiments, the compositions, genome-editing systems and methods described herein can ameliorate the phenotype of subjects with DMD who have a mutation described herein, e.g., a deletion, missense nonsense, indel or duplication mutation. In certain embodiments, the subject is converted to a BMD phenotype or, to a phenotype that is milder than BMD, or to having no symptoms.

Exemplary targeting approaches are described as follows.

### *2.1 Excision of exons 45-55 and with intronic cleavage events*

In certain embodiments, excision of exons 45-55 with intronic cleavage is expected to ameliorate the phenotype of up to 60% of DMD subjects (Aartsma-Rus et al., Human Mutation 2009; 30:293-299). For example, subjects found to have a mutation described as exon 44 deletion or del44 encounter a premature stop codon, due to an out-of-frame deletion, within exon 45 at position p.2113. In certain embodiments, excision of exons 45-55 will restore the reading frame of *DMD* in a subject with del44 mutation. Treated subjects will produce a mini-dystrophin missing only exons 44 through 55, rather than missing exons 44-79.

In certain embodiments, a subject with a deletion, e.g., del45-50, del47-50, del48-50, del49-50, del50, del52, del46-47, del46-48, del46-49, del46-51, del46-53, del46-55, del45, del45-54, del45-52, del46-52, del47-52, del48-52, del49-52, del50-52, del52, del47-54, del47-56, del51, del51-53, del51-55, or del53-55, can be treated with excision of exons 45 through exon 55. In certain embodiments, a subject with a duplication mutation, e.g., dup45, dup49-50, dup44-49, dup45-55, dup46-55, or dup53-54, can be treated with excision of exons 45 through exon 55. In certain embodiments, a subject with a point mutation, e.g., point mutation in exon 45, 46, 47, 48, 49, 50, 51, 52, 53, 54, or 55, can be treated with excision of exons 45 through exon 55. In certain embodiments, a subject with an indel mutation, e.g., indel mutation in exon 45 46, 47, 48, 49, 50, 51, 52, 53, 54, or 55, can be treated with excision of exons 45 through exon 55. In certain embodiments, a subject with a deletion mutation, e.g., any deletion mutation del(P-Q) such that P=1-54 and Q=44-55 and such that the deletion mutation puts *DMD* out of frame, can be treated with excision of exons 45 through exon 55. In certain embodiments, a subject with a duplication mutation, e.g., any duplication mutation dup(P-Q) such that P=44-55 and Q=45-55 and such that the duplication mutation puts *DMD* out of frame would restore *DMD* reading frame, can be treated with excision of exons 45 through exon 55. The subjects would be expected to have an improvement in their disease severity by the approaches described herein.

In certain embodiments, two presently disclosed gRNA molecules are used to delete exons 45-55 of the *DMD* gene (e.g., human *DMD* gene). In certain embodiments, the two gRNA molecules are selected from the group consisting of:

(a) a first gRNA molecule comprising a targeting domain that comprises a nucleotide sequence set forth in SEQ ID NO: 692253, and a second gRNA molecule comprising a targeting domain that comprises a nucleotide sequence set forth in SEQ ID NO: 692270, SEQ ID NO: 692271, or SEQ ID NO: 692272;
(b) a first gRNA molecule comprising a targeting domain that comprises a nucleotide sequence set forth in SEQ ID NO: 692254, and a second gRNA molecule comprising a targeting domain that comprises a nucleotide sequence set forth in SEQ ID NO: 692270, SEQ ID NO: 692271, or SEQ ID NO: 692272; and
(c) a first gRNA molecule comprising a targeting domain that comprises a nucleotide sequence set forth in SEQ ID NO: 692256, and a second gRNA molecule comprising a targeting domain that comprises a nucleotide sequence set forth in SEQ ID NO: 692270, SEQ ID NO: 692271, or SEQ ID NO: 692272..

### *2.2 Excision of exon 45 with intronic cleavage events*

In certain embodiments, excision of exon 45 with intronic cleavage is expected to ameliorate the phenotype of up to 11% of DMD subjects and up to 13% of DMD subjects with deletion mutations (Flanigan et al., Human Mutation 2009; 30:1657-1666. Aartsma-Rus et al., Human Mutation 2009; 30:293-299. DMD Mutation Database. Bladen et al., Human Mutation 2015; 36(2)). In certain embodiments, subjects found to have a mutation at exon 44, e.g., a deletion of exon 44, e.g. del44, encounter a premature stop codon, due to an out-of-frame deletion, within exon 45 at position p.2113.

In certain embodiments, a subject with a deletion mutation, e.g., del12-44, del18-44, del46-47, del46-48, del46-49, del46-51, del46-53, del46-55, can be treated with excision of exon 45 with intronic cleavage. In certain embodiments, excision of exon 45 causes the production of a min-dystrophin transcript without exon 45. In certain embodiments, a subject with a deletion mutation that would have the *DMD* reading frame restored by excision of exon 45 can be treated with excision of exon 45. In certain embodiments, a subject with a duplication mutation that would have the *DMD* reading frame restored by excision of exon 45 can be treated with excision of exon 45. In certain embodiments, a subject with a point mutation in exon 45 can be treated with cas9-mediated excision of exon 45. In certain embodiments, a subject with an indel mutation in exon 45 can be treated with excision of exon 45. The subjects would be expected to have an improvement in their disease severity by the approaches described herein.

### *2.3 Excision of exon 51 with intronic cleavage events*

In certain embodiments, Cas9-mediated excision of exon 51 with intronic cleavage ameliorates the phenotype of up to 17% of DMD subjects, and up to 21% of DMD subjects with deletion mutations (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation, del45-50, del47-50, del48-50, del49-50, del50, del52, or del 52-63, is treated with excision of exon 51 of the *DMD* gene (e.g., human *DMD* gene). In certain embodiments, excision of exon 51 causes the production of a min-dystrophin transcript without exon 51. In certain embodiments, exon 51 excision causes the production of a mini-dystrophin, e.g., in a subject with a del45-50 mutation, the corrected mini-dystrophin will include exons 1-44 and 52-79, e.g., in a subject with a del47-50 mutation, the corrected mini-dystrophin will include exons 1-46 and 52-79, e.g., in a subject with a del48-50 mutation, the corrected mini-dystrophin will include exons 1-47 and 52-79, e.g., in a subject with a del49-50 mutation, the corrected mini-dystrophin will include exons 1-48 and 52-79, e.g., in a subject with a del50 mutation the corrected mini-dystrophin will include exons 1-49 and 52-79, e.g., in a subject with a del52 mutation, the corrected mini-dystrophin will include exons 1-50 and 53-79, e.g., in a subject with a del52-63 mutation, the corrected mini-dystrophin will include exons 1-50 and 64-79.

In certain embodiments, a subject with a deletion mutation that would have the *DMD* reading frame restored by excision of exon 51 can be treated with excision of exon 51. In certain embodiments, a subject with a duplication mutation that would have the *DMD* reading frame restored by excision of exon 51 can be treated with excision of exon 51. In certain embodiments, a subject with an indel mutation in exon 51 can be treated with excision of exon 51. The subjects can have an improvement in their disease severity by the approaches described herein.

In certain embodiments, two presently disclosed gRNA molecules (i.e., a first gRNA molecule and a second gRNA molecule) are used to delete exon 51 of the *DMD* gene (e.g., human *DMD* gene) (referred to as "exon 51 skipping" or "excision of exon 51"). In certain embodiments, the first gRNA molecule and the second gRNA molecule are selected

from the group consisting of:

(a) a first gRNA molecule comprising a targeting domain that comprises a nucleotide sequence set forth in SEQ ID NO: 1977, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(c) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(e) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(f) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(g) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(h) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(i) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(j) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(k) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
(l) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

In certain embodiments, the two gRNA molecules are selected from the gRNA pairs listed in Table 15, i.e., those selected from the group consisting of:

(a) a first gRNA molecule comprising a targeting domain that comprises a nucleotide sequence set forth in SEQ ID NO: 2048, and a second gRNA molecule comprising a targeting domain that comprises a nucleotide sequence selected from SEQ ID NOs: 1977, 18458, 481, 9997, 1499, 2121, 8709, 21570, 16467, and 23958;
(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(c) a first gRNA molecule comprising a targeting domain that comprises a nucleotide sequence set forth in SEQ ID NO: 18720, SEQ ID NO: 22349 or SEQ ID NO: 10092, and a second gRNA molecule comprising a targeting domain that comprises a nucleotide sequence set forth in SEQ ID NO: 4709 or SEQ ID NO: 20303;
(d) a first gRNA molecule comprising a targeting domain that comprises a nucleotide sequence set forth in SEQ ID NO: 3536, and a second gRNA molecule comprising a targeting domain that comprises a nucleotide sequence selected from SEQ ID NOs: 5869, 20145, 18752, 20870, 9530, 18062, 4134, and 23958;
(e) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 9765;
(f) a first gRNA molecule comprising a targeting domain that comprises a nucleotide sequence set forth in SEQ ID

NO: 18752, and a second gRNA molecule comprising a targeting domain that comprises a nucleotide sequence selected from SEQ ID NOs: 17253, 14607, 16967, 7419, 1744, and 8569;

(g) a first gRNA molecule comprising a targeting domain that comprises a nucleotide sequence set forth in SEQ ID NO: 19187, and a second gRNA molecule comprising a targeting domain that comprises a nucleotide sequence selected from SEQ ID NOs: 14695, 7252, 17253, 7419, 1744, 8569, and 9358;

(h) a first gRNA molecule comprising a targeting domain that comprises a nucleotide sequence set forth in SEQ ID NO: 20145, and a second gRNA molecule comprising a targeting domain that comprises a nucleotide sequence selected from SEQ ID NOs: 5614, 20764, 14607, 8569, and 7252;

(i) a first gRNA molecule comprising a targeting domain that comprises a nucleotide sequence set forth in SEQ ID NO: 1499, and a second gRNA molecule comprising a targeting domain that comprises a nucleotide sequence set forth in SEQ ID NO: 14296;

(i) a first gRNA molecule comprising a targeting domain that comprises a nucleotide sequence set forth in SEQ ID NO: 4134, and a second gRNA molecule comprising a targeting domain that comprises a nucleotide sequence selected from SEQ ID NOs: 20924, 4072, and 8569;

(k) a first gRNA molecule comprising a targeting domain that comprises a nucleotide sequence set forth in SEQ ID NO: 6209, and a second gRNA molecule comprising a targeting domain that comprises a nucleotide sequence selected from SEQ ID NOs: 14035, 13527, 21873, 14298, 7419, and 20924;

(l) a first gRNA molecule comprising a targeting domain that comprises a nucleotide sequence set forth in SEQ ID NO: 9346, and a second gRNA molecule comprising a targeting domain that comprises a nucleotide sequence selected from SEQ ID NOs: 7419, 20764, and 2769;

(m) a first gRNA molecule comprising a targeting domain that comprises a nucleotide sequence set forth in SEQ ID NO: 7649, and a second gRNA molecule comprising a targeting domain that comprises a nucleotide sequence selected from SEQ ID NOs: 16967, 1484, 20924, 4072, and 9496;

(n) a first gRNA molecule comprising a targeting domain that comprises a nucleotide sequence set forth in SEQ ID NO: 5869, and a second gRNA molecule comprising a targeting domain that comprises a nucleotide sequence selected from SEQ ID NOs: 8569, 13527, 1484, 5614, 4072, and 14695;

(o) a first gRNA molecule comprising a targeting domain that comprises a nucleotide sequence set forth in SEQ ID NO: 4165, and a second gRNA molecule comprising a targeting domain that comprises a nucleotide sequence selected from SEQ ID NOs: 7252, 1484, and 9279;

(p) a first gRNA molecule comprising a targeting domain that comprises a nucleotide sequence set forth in SEQ ID NO: 15622, and a second gRNA molecule comprising a targeting domain that comprises a nucleotide sequence set forth in SEQ ID NO: 17253;

(q) a first gRNA molecule comprising a targeting domain that comprises a nucleotide sequence set forth in SEQ ID NO: 18062, and a second gRNA molecule comprising a targeting domain that comprises a nucleotide sequence set forth in SEQ ID NO: 2769 or SEQ ID NO: 14035;

(r) a first gRNA molecule comprising a targeting domain that comprises a nucleotide sequence set forth in SEQ ID NO: 21131, and a second gRNA molecule comprising a targeting domain that comprises a nucleotide sequence set forth in SEQ ID NO: 13527 or SEQ ID NO: 13285;

(s) a first gRNA molecule comprising a targeting domain that comprises a nucleotide sequence set forth in SEQ ID NO: 20870, and a second gRNA molecule comprising a targeting domain that comprises a nucleotide sequence set forth in SEQ ID NO: 16967 or SEQ ID NO: 7252;

(t) a first gRNA molecule comprising a targeting domain that comprises a nucleotide sequence set forth in SEQ ID NO: 16235, and a second gRNA molecule comprising a targeting domain that comprises a nucleotide sequence set forth in SEQ ID NO: 10140;

(u) a first gRNA molecule comprising a targeting domain that comprises a nucleotide sequence set forth in SEQ ID NO: 15271, and a second gRNA molecule comprising a targeting domain that comprises a nucleotide sequence set forth in SEQ ID NO: 13518;

(v) a first gRNA molecule comprising a targeting domain that comprises a nucleotide sequence set forth in SEQ ID NO: 11675, and a second gRNA molecule comprising a targeting domain that comprises a nucleotide sequence selected from SEQ ID NOs: 2769, 1484, and 4072; and

(w) a first gRNA molecule comprising a targeting domain that comprises a nucleotide sequence set forth in SEQ ID NO: 23958, and a second gRNA molecule comprising a targeting domain that comprises a nucleotide sequence set forth in SEQ ID NO: 14035 or SEQ ID NO: 9496.

In certain embodiments, the two gRNA molecules are selected from thethe group consisting of:

(a) a first gRNA molecule comprising a targeting domain that comprises a nucleotide sequence set forth in SEQ ID NO: 692257, and a second gRNA molecule comprising a targeting domain that comprises a nucleotide sequence

set forth in SEQ ID NO: 692261, SEQ ID NO: 692262, SEQ ID NO: 692263, or SEQ ID NO: 692264;
(b) a first gRNA molecule comprising a targeting domain that comprises a nucleotide sequence set forth in SEQ ID NO: 692258, and a second gRNA molecule comprising a targeting domain that comprises a nucleotide sequence set forth in SEQ ID NO: 692261, SEQ ID NO: 692262, SEQ ID NO: 692263, or SEQ ID NO: 692264; and
(c) a first gRNA molecule comprising a targeting domain that comprises a nucleotide sequence set forth in SEQ ID NO: 692260, and a second gRNA molecule comprising a targeting domain that comprises a nucleotide sequence set forth in SEQ ID NO: 692261, SEQ ID NO: 692262, SEQ ID NO: 692263, or SEQ ID NO: 692264.

In certain embodiments, a *S. pyogenes* Cas 9 molecule mediates the exon 51 skipping or excision of exon 51. In certain embodiments, a *S. aureus* Cas 9 molecule mediates the exon 51 skipping or excision of exon 51.

### *2.4 Excision of exon 53 with intronic cleavage events*

In certain embodiments, Cas9 mediated excision of exon 53 with intronic cleavage is expected to ameliorate the phenotype of up to 10% of DMD subjects, and up to 15% of DMD subjects with deletion mutations (Flanigan et al., Human Mutation 2009; 30:1657-1666. Bladen et al., Human Mutation 2015; 36(2)).

In certain embodiments, a subject with a deletion mutation, e.g., del10-52, del45-52, del46-52, del47-52, del48-52, del49-52, del50-52, or del52, can be treated with excision of exon 53 with intronic cleavage. In certain embodiments, excision of exon 53 causes the production of a min-dystrophin transcript without exon 53. In certain embodiments, exon 53 excision causes the production of a mini-dystrophin, e.g., in a subject with a del10-52, del45-52, del46-52, del47-52, del48-52, del49-52, del50-52, or del52 mutation, the corrected mini-dystrophin will include, respectively, exons 1-9 and 54-79, exons 1-44 and 54-79, exons 1-45 and 54-79, exons 1-46 and 54-79, exons 1-47 and 54-79, exons 1-48 and 54-79, exons 1-49 and 54-79, exons 1-51 and 54-79. In certain embodiments, any subject with a deletion mutation that would have the *DMD* reading frame restored by excision of exon 53 can be treated with excision of exon 53. In certain embodiments, a subject with a duplication mutation that would have the *DMD* reading frame restored by excision of exon 53 can be treated with excision of exon 53. In certain embodiments, a subject with a point mutation in exon 53 can be treated with excision of exon 53. In certain embodiments, a subject an indel mutation in exon 53 can be treated with excision of exon 53. The subjects would be expected to have an improvement in their disease severity by the approach described herein.

### *2.5 Excision of exon 44 with intronic cleavage events*

In certain embodiments, excision of exon 44 with intronic cleavage is expected to ameliorate the phenotype of up to 7.8% of DMD subjects (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation can be treated with excision of exon 44 with intronic cleavage, e.g., del14-43, del19-43, del30-43, del35-43, del36-43, del40-43, del42-43, del45, or del45-54. In certain embodiments, excision of exon 44 causes the production of a min-dystrophin transcript without exon 44. In certain embodiments, exon 44 excision causes the production of a mini-dystrophin, e.g., in a subject with a del14-43, del19-43, del30-43, del35-43, del36-43, del40-43, del42-43, del45, or del45-54 mutation, the corrected mini-dystrophin will include, respectively, exons 1-13 and 45-79, exons 1-18 and 45-79, exons 1-29 and 45-79, exons 1-34 and 45-79, exons 1-35 and 45-79, exons 1-39 and 45-79, exons 1-41 and 45-79, exons 1-43 and 46-79, exons 1-43 and 55-79. In certain embodiments, any subject with a deletion mutation that would have the *DMD* reading frame restored by excision of exon 44 can be treated with excision of exon 44. In certain embodiments, a subject with a duplication mutation that would have the *DMD* reading frame restored by excision of exon 44 can be treated with excision of exon 44. In certain embodiments, a subject with a point mutation in exon 44 can be treated with excision of exon 44. In certain embodiments, a subject with an indel mutation in exon 44 can be treated with excision of exon 44. The subjects would be expected to have an improvement in their disease severity by the approach described herein.

### *2.6 Excision of exon 46 with intronic cleavage events*

In certain embodiments, excision of exon 46 with intronic cleavage is expected to ameliorate the phenotype of up to 5.6% of DMD subjects (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation, e.g., del21-45, del45, del47-54, or del47-56, can be treated with excision of exon 46 with intronic cleavage. In certain embodiments, excision of exon 46 causes the production of a min-dystrophin transcript without exon 46. In certain embodiments, exon 46 excision causes the production of a mini-dystrophin, e.g., in a subject with a del21-45, del45, del47-54, or del47-56 mutation, the corrected mini-dystrophin

will include, respectively, exons 1-20 and 47-79, exons 1-44 and 47-79, exons 1-45 and 55-79, exons 1-45 and 57-79. In certain embodiments, a subject with a deletion mutation that would have the *DMD* reading frame restored by excision of exon 46 can be treated with excision of exon 46. In certain embodiments, a subject with a duplication mutation that would have the *DMD* reading frame restored by excision of exon 46 can be treated with excision of exon 46. In certain embodiments, a subject with a point mutation in exon 46 can be treated with excision of exon 46. In certain embodiments, a subject an indel mutation in exon 46 can be treated with excision of exon 46. The subjects would be expected to have an improvement in their disease severity by the approach described herein.

### *2.7 Excision of exon 50 with intronic cleavage events*

In certain embodiments, excision of exon 50 with intronic cleavage is expected to ameliorate the phenotype of up to 5.2% of DMD subjects (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation, e.g., del51, del51-53, or del51-55, can be treated with excision of exon 50. In certain embodiments, excision of exon 50 causes the production of a min-dystrophin transcript without exon 50. In certain embodiments, exon 50 excision causes the production of a mini-dystrophin, e.g., in a subject with a del51, del51-53, or del51-55 mutation, the corrected mini-dystrophin will include, respectively, exons 1-49 and 52-79, exons 1-49 and 54-79, exons 1-49 and 56-79. In certain embodiments, a subject with a deletion mutation that would have the *DMD* reading frame restored by excision of exon 50 can be treated with excision of exon 50. In certain embodiments, a subject with a duplication mutation that would have the *DMD* reading frame restored by excision of exon 50 can be treated with excision of exon 50. In certain embodiments, a subject with a point mutation in exon 50 can be treated with excision of exon 50. In certain embodiments, a subject with an indel mutation in exon 50 can be treated with excision of exon 50. The subjects would be expected to have an improvement in their disease severity by the approach described herein.

### *2.8 Excision of exon 52 with intronic cleavage events*

In certain embodiments, Cas9 mediated excision of exon 52 with intronic cleavage is expected to ameliorate the phenotype of up to 5% of DMD subjects (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation, e.g., del53-55, can be treated with excision of exon 52. In certain embodiments, excision of exon 52 causes the production of a min-dystrophin transcript without exon 52. In certain embodiments, exon 52 excision causes the production of a mini-dystrophin, e.g., in a subject with a del53-55 mutation, the corrected mini-dystrophin will include exons 1-51 and 56-79. In certain embodiments, a subject with a deletion mutation that would have the *DMD* reading frame restored by excision of exon 52 can be treated with excision of exon 52. In certain embodiments, a subject with a duplication mutation, e.g., dup52, that would have the *DMD* reading frame restored by excision of exon 52 can be treated with excision of exon 52. In certain embodiments, a subject with a point mutation in exon 52 can be treated with excision of exon 52. In certain embodiments, a subject an indel mutation in exon 52 can be treated with excision of exon 52. The subjects would be expected to have an improvement in their disease severity by the approach described herein.

### *2.9 Excision of exon 55 with intronic cleavage events*

In certain embodiments, excision of exon 55 with intronic cleavage is expected to ameliorate the phenotype of up to 4% of DMD subjects (Flanigan et al., Human Mutation 2009; 30:1657-1666. Aartsma-Rus et al., Human Mutation 2009; 30:293-299. Bladen et al., Human Mutation 2015; 36(2))

In certain embodiments, a subject with a deletion mutation, e.g., del54, can be treated with excision of exon 55. In certain embodiments, excision of exon 55 causes the production of a min-dystrophin transcript without exon 55. In certain embodiments, exon 55 excision causes the production of a mini-dystrophin, e.g., in a subject with a del54 mutation, the corrected mini-dystrophin will include exons 1-53 and 56-79. In certain embodiments, a subject with a deletion mutation that would have the *DMD* reading frame restored by excision of exon 55 can be treated with excision of exon 55. In certain embodiments, a subject with a duplication mutation that would have the *DMD* reading frame restored by excision of exon 55 can be treated with excision of exon 55. In certain embodiments, a subject with a point mutation in exon 55 can be treated with excision of exon 55. In certain embodiments, a subject an indel mutation in exon 55 can be treated with excision of exon 55. The subjects would be expected to have an improvement in their disease severity by the approach described herein.

### 2.10 *Excision of exon 8 with intronic cleavage events*

In certain embodiments, Cas9 mediated excision of exon 8 with intronic cleavage is expected to ameliorate the phenotype of up to 5.2% of DMD subjects (Flanigan et al., Human Mutation 2009; 30:1657-1666. Aartsma-Rus et al., Human Mutation 2009; 30:293-299. Bladen et al., Human Mutation 2015; 36(2))

In certain embodiments, a subject with a deletion mutation, e.g., del3-7, del4-7, del5-7, or del6-7, can be treated with excision of exon 8. In certain embodiments, excision of exon 8 causes the production of a min-dystrophin transcript without exon 8. In certain embodiments, exon 8 excision causes the production of a mini-dystrophin, e.g., in a subject with a del3-7, del4-7, del5-7, or del6-7 mutation, the corrected mini-dystrophin will include, respectively, exons 1-2 and 9-79, exons 1-3 and 9-79, exons 1-4 and 9-79, exons 1-5 and 9-79. In certain embodiments, a subject with a deletion mutation that would have the *DMD* reading frame restored by excision of exon 8 can be treated with excision of exon 8. In certain embodiments, a subject with a duplication mutation that would have the *DMD* reading frame restored by excision of exon 8 can be treated with excision of exon 8. In certain embodiments, a subject with a point mutation in exon 8 can be treated with excision of exon 8. In certain embodiments, a subject an indel mutation in exon 8 can be treated with excision of exon 8. The subjects would be expected to have an improvement in their disease severity by the approach described herein.

### 2.11 *Excision of exons 69-70 with intronic cleavage events*

In certain embodiments, Cas9 mediated excision of exons 69-70 with intronic cleavage is expected to ameliorate the phenotype of up to 6% of DMD subjects with nonsense or in/del mutations (Based on frequency of exon 70 nonsense mutations from Duchenne Muscular Dystrophy database).

In certain embodiments, a subject with a deletion mutation, e.g., del71-75, can be treated with excision of exons 69-70. In certain embodiments, excision of exons 69-70 causes the production of a min-dystrophin transcript without exons 69-70. In certain embodiments, excision of exons 69-70 causes the production of a mini-dystrophin, e.g., in a subject with a del71-75 mutation, the corrected mini-dystrophin will include exons 1-68 and 76-79. In certain embodiments, a subject with a deletion mutation, that would have the *DMD* reading frame restored by excision of exons 69-70 can be treated with excision of exons 69-70. In certain embodiments, a subject with a duplication mutation that would have the *DMD* reading frame restored by excision of exons 69-70 can be treated with excision of exons 69-70. In certain embodiments, a subject with a point mutation in exon 69 or exon 70, e.g., nonsense mutation c.10171C>T, nonsense mutation c.10141C>T, or nonsense mutation c.10108C>T, can be treated with excision of exons 69-70 with intronic cleavage. In certain embodiments, a subject with an indel mutation, e.g., an indel mutation in exon 69 or 70, can be treated with excision of exon 8. The subjects would be expected to have an improvement in their disease severity by the approach described herein.

### *2.12 Excision of exons 1-6 with intronic cleavage events*

In certain embodiments, Cas9 mediated excision of exons 1-6 with intronic cleavage is expected to ameliorate the phenotype of approximately 9% of DMD subjects who have nonsense or in/del mutations (based on frequency of nonsense mutations in exons 1-6 from Duchenne Muscular Dystrophy database).

In certain embodiments, a subject with a deletion mutation that would have the *DMD* reading frame restored by excision of exons 1-6 can be treated with excision of exons 1-6. In certain embodiments, excision of exons 1-6 causes the production of a min-dystrophin transcript without exons 1-6. In certain embodiments, a subject with an in-frame duplication mutation in any of exons 1, 2, 3, 4, 5 and/or 6 can be treated with excision of exons 1-6. In certain embodiments, an in-frame duplication mutation in exons 1, 2, 3, 4, 5 and/or 6 can cause post-transcriptional-translational modifications leading to a DMD phenotype. In certain embodiments, a subject with an out-of-frame duplication mutation, e.g., dup1, dup2, dup3, dup3-4, or dup3-6, that would have the *DMD* reading frame restored by excision of exons 1-6, can be treated with excision of exons 1-6. In certain embodiments, a subject with an in-frame duplication mutation, e.g., dup1, dup2, dup3, dup3-4, or dup3-6, that would have an improved or restored dystrophin RNA profile by excision of exons 1-6, can be treated with excision of exons 1-6.

In certain embodiments, a subject with a point mutation in exon 1, 2, 3, 4, 5, or 6 can be treated with excision of exons 1-6. In certain embodiments, a subject with an indel mutation in exon 1, 2, 3, 4, 5, or 6 can be treated with excision of exons 1-6. In certain embodiments, a subject with a nonsense mutation, e.g., nonsense mutation c.9G>A, nonsense mutation c.355C>T, nonsense mutation c.440C>G, nonsense mutation c.429G>A, nonsense mutation c.433C>T, or nonsense mutation c.457C>T, can be treated with excision of exons 1-6. The subjects would be expected to have an improvement in their disease severity by the approach described herein.

### *2.13 Excision of exons 6-8 with intronic cleavage events*

In certain embodiments, Cas9 mediated excision of exons 6-8 with intronic cleavage is expected to ameliorate the phenotype of approximately 6% of DMD subjects who have nonsense or in/del mutations (based on frequency of nonsense mutations in exons 6-8 from Duchenne Muscular Dystrophy database).

In certain embodiments, a subject with a deletion mutation that would have the *DMD* reading frame restored by excision of exons 6-8 can be treated with excision of exons 6-8. In certain embodiments, excision of exons 6-8 causes the production of a min-dystrophin transcript without exons 6-8. In certain embodiments, a subject with a duplication mutation that would have the *DMD* reading frame restored by excision of exons 6-8 can be treated with excision of exons 6-8. In certain embodiments, a subject with a point mutation in exon 6, 7, or 8, can be treated with excision of exons 6-8. In certain embodiments, a subject with a nonsense mutation, e.g., nonsense mutation c.440C>G, nonsense mutation c.429G>A, nonsense mutation c.433C>T, nonsense mutation c.457C>T, nonsense mutation c.568C>T, nonsense mutation c.565C>T, nonsense mutation c.583C>T, nonsense mutation c.583C>T, nonsense mutation c.799C>T, nonsense mutation c.709C>T, nonsense mutation c.745C>T, or nonsense mutation c.903C>G, can be treated with excision of exons 6-8. In certain embodiments, a subject with an indel mutation, e.g., an indel mutation in exon 6, 7, or 8, can be treated with excision of exons 6-8. Prior to treatment, these subjects produce a truncated dystrophin containing only exons 1-5, 1-6, 1-7, 1-8 or 1-9. After treatment, subjects with a point or indel mutation in exons 6-8 will produce a mini-dystrophin lacking exons 6-8 but containing exons 1-5 and 9-79. The subjects would be expected to have an improvement in their disease severity by the approach described herein.

### *2.14 Excision of exon 14 with intronic cleavage events*

In certain embodiments, excision of exon 14 with intronic cleavage is expected to ameliorate the phenotype of approximately 3% of DMD subjects who have point or indel mutations.

In certain embodiments, a subject with a deletion mutation, that would have the *DMD* reading frame restored by excision of exon 14, can be treated with excision of exon 14. In certain embodiments, excision of exon 14 causes the production of a min-dystrophin transcript without exon 14. In certain embodiments, a subject with a duplication mutation, that would have the *DMD* reading frame restored by excision of exon 14, can be treated with excision of exon 14. In certain embodiments, a subject with a point mutation in exon 14, e.g., nonsense mutation c.1663C>T, nonsense mutation c.1619G>A, nonsense mutation c.1702C>T, or nonsense mutation c.1702C>T, can be treated with excision of exon 14. In certain embodiments, a subject with an indel mutation in exon 14 can be treated with excision of exon 14. Prior to treatment, subjects with an indel or point mutation in the *DMD* gene produce a truncated *DMD* transcript containing exons 1-14, with only some segment of exon 14. In any subjects with these mutations, excision of exon 14 will restore the reading frame *of DMD.* After treatment, subjects with a point mutation or indel mutation in exon 14 will produce a mini-dystrophin lacking exon 14 but containing exons 1-13 and 15-79. The subjects would be expected to have an improvement in their disease severity by the approach described herein.

### *2.15 Excision of exons 20 -21 with intronic cleavage events*

In certain embodiments, excision of exons 20-21 is expected to ameliorate the phenotype of approximately 5% of DMD subjects who have nonsense or indel mutations.

In certain embodiments, a subject with a deletion mutation that would have the *DMD* reading frame restored by excision of exons 20-21 can be treated with excision of exons 20-21. In certain embodiments, excision of exons 20-21 causes the production of a min-dystrophin transcript without exons 20-21. In certain embodiments, a subject with a duplication mutation that would have the *DMD* reading frame restored by excision of exons 20-21 can be treated with excision of exons 20-21. In certain embodiments, a subject with a point mutation in exon 20 or exon 21 can be treated with excision of exons 20-21. In certain embodiments, a subject with nonsense mutation c.2435G>A, nonsense mutation c.2521C>T, nonsense mutation c.2485C>T, nonsense mutation c.2419C>T, nonsense mutation c.2479G>T, nonsense mutation c.2404A>T, nonsense mutation c.2416G>T, nonsense mutation c.2440G>T, nonsense mutation c.2611A>T, nonsense mutation c.2582C>G, nonsense mutation c.2758C>T, or nonsense mutation c.2788A>T, can be treated with excision of exons 20-21. In certain embodiments, a subject with an indel mutation in exon 20 or exon 21, can be treated with excision of exons 20-21. Prior to treatment, subjects with an indel or point mutation in exon 20 or 21 of the *DMD* gene produce a truncated dystrophin containing exons 1-20 or 1-21, respectively, with only some segment of exon 20 or exon 21, respectively. After treatment, subjects with a point or indel mutation in exons 20-21 will produce a mini-dystrophin lacking exons 20-21 but containing exons 1-19 and 22-79. In any subjects with these mutations, excision of exons 20-21 will restore the reading frame *of DMD.* The subjects would be expected to have an improvement in their disease severity by the approach described herein.

### *2.16 Intra-exonic targeting exon 45*

In certain embodiments, modification of exon 45, e.g., 5' of p.2113 in exon 45, e.g., by NHEJ, to restore reading frame will ameliorate the phenotype of up to 11% of DMD subjects and up to 13% of DMD subjects with deletion mutations (Flanigan et al., Human Mutation 2009; 30:1657-1666. Aartsma-Rus et al., Human Mutation 2009; 30:293-299. DMD Mutation Database. Bladen et al., Human Mutation 2015; 36(2)).

In certain embodiments, subjects found to have a mutation at exon 44, e.g., a deletion of exon 44, e.g. del44, encounter a premature stop codon, due to an out-of-frame deletion, within exon 45 at position p.2113. In certain embodiments, modification of exon 45, e.g., by NHEJ, will restore the reading frame *of DMD.* Subjects will produce a mini-dystrophin missing only exons 44 and 45, rather than missing exons 44-79. In certain embodiments, a subject with a deletion mutation, e.g., del12-44, del18-44, del46-47, del46-48, del46-49, del46-51, del46-53, or del46-55, can be treated with modification of exon 45, e.g., 5' of p.2113. In certain embodiments, a subject with any mutation, e.g., a duplication mutation that would have the *DMD* reading frame restored by modification of exon 45 can be treated with modification of exon 45, e.g., 5' of p.2113. In certain embodiments, a subject with a deletion mutation, e.g., del12-44, del18-44, del46-47, del46-48, del46-49, del46-51, del46-53, or del46-55, can be treated with NHEJ mediated modification of exon 45, e.g., 5' of p.2113 and 3' of p.2509. In certain embodiments, a subject with a point mutation, e.g. a point mutation in exon 45, can be treated with modification of exon 45, e.g., 5' of mutation. In certain embodiments, a subject with an indel mutation, e.g. an indel mutation in exon 45, can be treated with modification of exon 45, e.g, 5' of mutation. The subjects would be expected to have an improvement in their disease severity by the approach described herein.

### *2.17 Intra-exonic targeting exon 51*

In certain embodiments, modification of exon 51, e.g., by NHEJ, to restore reading frame will ameliorate the phenotype of up to 17% of DMD subjects, and up to 21% of DMD subjects with deletion mutations. (Flanigan et al., Human Mutation 2009; 30:1657-1666. Aartsma-Rus et al., Human Mutation 2009; 30:293-299. Bladen et al., Human Mutation 2015; 36(2).)

In certain embodiments, a subject with a deletion mutation, e.g., del45-50, del47-50, del48-50, del49-50, del50, del52, or del 52-63, can be treated with modification of exon 51, e.g., 5' of p.2445. In certain embodiments, modification of exon 51 will cause the production of a mini-dystrophin missing only exon 51, exons 45-51, exons 46-51, exons 47-51, exons 48-51, exons 49-51, exons 50-51, exons 51-52, or exons 51-63, rather than missing exons 45-79, exons 46-51, exons 47-79, exons 48-79, exons 49-79, exons 50-79, exons 51-79, orexons 52-79, respectively. In certain embodiments, a subject with any mutation, e.g., a duplication mutation that would have the *DMD* reading frame restored by modification of exon 51 can be treated with modification of exon 51, e.g., 5' of p.2445. In certain embodiments, a subject with a point mutation, e.g. a point mutation in exon 51, can be treated with modification of exon 51, e.g., 5' of point mutation. In certain embodiments, a subject with an indel mutation, e.g. an indel mutation in exon 51, can be treated with modification of exon 51, e.g., 5' of indel mutation. The subjects would be expected to have an improvement in their disease severity by the approach described herein.

### *2.18 Intra-exonic targeting exon 53*

In certain embodiments, modification exon 53, e.g. 5' of premature termination codon in exon 53, e.g. 5' of p.2554 *of DMD,* e.g., by NHEJ, to restore reading frame will ameliorate the phenotype of up to 10% of DMD subjects, and up to 15% of DMD subjects with deletion mutations. (Flanigan et al., Human Mutation 2009; 30:1657-1666. Bladen et al., Human Mutation 2015; 36(2)).

In certain embodiments, a subject with a deletion mutation, e.g., del10-52, del45-52, del46-52, del47-52, del48-52, del49-52, del50-52, or del52, can be treated with modification of exon 53, e.g. 5' of p.2554. In certain embodiments, a subject with any mutation, e.g., a duplication mutation that would have the *DMD* reading frame restored by modification of exon 53 can be treated with modification of exon 53, e.g. 5' of p.2554. In certain embodiments, a subject with a point mutation, e.g. a point mutation in exon 53 can be treated with modification of exon 53, e.g., 5' of point mutation. In certain embodiments, a subject with an indel mutation, e.g. an indel mutation in exon 53, can be treated with modification of exon 53, e.g., 5' of indel mutation. The subjects would be expected to have an improvement in their disease severity by the approach described herein.

### *2.19 Intra-exonic targeting exon 44*

In certain embodiments, modification of exon 44, e.g. 5' of premature termination codon in exon 44, e.g. 5' of p.2099 *of DMD,* e.g., by NHEJ, to restore reading frame will ameliorate the phenotype of up to 7.8% of DMD subjects. (Flanigan et al., Human Mutation 2009; 30:1657-1666. Aartsma-Rus et al., Human Mutation 2009; 30:293-299. Bladen

et al., Human Mutation 2015; 36(2).)

In certain embodiments, any subject with a deletion mutation, e.g., del14-43, del19-43, del30-43, del35-43, del36-43, del40-43, del42-43, del45, or del45-54, can be treated with modification of exon 44, e.g., 5' of p.2099. In certain embodiments, any subject with any mutation, e.g., a duplication mutation that would have the *DMD* reading frame restored by modification of exon 44, can be treated with modification of exon 44, e.g., 5' of the premature termination codon. In certain embodiments, a subject with a point mutation, e.g. a point mutation in exon 44 can be treated with modification of exon 44, e.g., 5' of point mutation. In certain embodiments, a subject with an indel mutation, e.g. an indel mutation in exon 44, can be treated with modification of exon 44, e.g., 5' of the indel mutation, e.g., 5' of indel mutation. The subjects would be expected to have an improvement in their disease severity by the approach described herein.

### *2.20 Intra-exonic targeting exon 46*

In certain embodiments, modification of exon 46, e.g. 5' of premature termination codon in exon 46, e.g., by NHEJ, is expected to ameliorate the phenotype of up to 5.6% of DMD subjects (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation, e.g., del21-45, del45, del47-54, or del47-56, can be treated with modification of exon 46, e.g., 5' of p.2206. In certain embodiments, a subject with any mutation, e.g., a duplication mutation that would have the *DMD* reading frame restored by modification of exon 46, can be treated with modification of exon 46, 5' of the premature termination codon, e.g., 5' of p.2206. In certain embodiments, a subject with a point mutation, e.g. a point mutation in exon 46, can be treated with modification of exon 46, e.g., 5' of point mutation. In certain embodiments, a subject with an indel mutation, e.g., an indel mutation in exon 46, can be treated with modification of exon 46, e.g., 5' of indel mutation. The subjects would be expected to have an improvement in their disease severity by the approach described herein.

### *2.21 Intra-exonic targeting exon 50*

In certain embodiments, modification of exon 50, e.g. 5' of premature termination codon in exon 50, e.g., by NHEJ, is expected to ameliorate the phenotype of up to 5.2% of DMD subjects (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation, e.g., del51, e.g., del51-53, e.g., del51-55, e.g., del53-55, e.g., del54, can be treated with modification of exon 50, IFTP at exon 50, e.g., 5' of p.2403. In certain embodiments, a subject with any mutation, e.g., a duplication mutation that would have the *DMD* reading frame restored by modification of exon 50, can be treated with modification of exon 50, e.g., 5' of the premature termination codon, e.g., 5' of p.2403. In certain embodiments, a subject with a point mutation, e.g. a point mutation in exon 50, can be treated with modification of exon 50. In certain embodiments, a subject with an indel mutation, e.g. an indel mutation in exon 50, can be treated with modification of exon 50, e.g, 5' of the indel mutation, e.g., at exon 50 5' of indel mutation. The subjects would be expected to have an improvement in their disease severity by the approach described herein.

### *2.22 Intra-exonic targeting exon 52*

In certain embodiments, modification of exon 52, e.g. 5' of premature termination codon in exon 52, e.g., by NHEJ, is expected to ameliorate the phenotype of up to 5% of DMD subjects. (Flanigan et al., Human Mutation 2009; 30:1657-1666. Aartsma-Rus et al., Human Mutation 2009; 30:293-299. Bladen et al., Human Mutation 2015; 36(2))

In certain embodiments, a subject with a deletion mutation, e.g., del53-55, can be treated with modification of exon 52, e.g., 5' of p.2537. In certain embodiments, a subject with any mutation, e.g., a duplication mutation that would have the *DMD* reading frame restored by modification of exon 52, can be treated with modification of exon 52, e.g., 5' of the premature termination codon, e.g., 5' of p.2537. In certain embodiments, a subject with a point mutation, e.g. a point mutation in exon 52, can be treated with modification of exon 52. In certain embodiments, a subject with an indel mutation, e.g. an indel mutation in exon 52, can be treated with modification of exon 52, e.g, 5' of the indel mutation, e.g., at exon 50 5' of indel mutation. The subjects would be expected to have an improvement in their disease severity by the approach described herein.

### *2.23 Intra-exonic targeting exon 55*

In certain embodiments, modification of exon 55, e.g. 5' of premature termination codon in exon 55, e.g., by NHEJ, is expected to ameliorate the phenotype of up to 4% of DMD subjects (Flanigan et al., Human Mutation 2009; 30:1657-1666. Aartsma-Rus et al., Human Mutation 2009; 30:293-299. Bladen et al., Human Mutation 2015; 36(2))

In certain embodiments, a subject with a deletion mutation, e.g., del54, can be treated with modification of exon

55, e.g., 5' of p.2677. In certain embodiments, a subject with any mutation, e.g., a duplication mutation that would have the *DMD* reading frame restored by modification of exon 55, can be treated with modification of exon 55, e.g., 5' of the premature termination codon, e.g., 5' of p.2677. In certain embodiments, a subject with a point mutation, e.g. a point mutation in exon 55, can be treated with modification of exon 55. In certain embodiments, a subject with an indel mutation, e.g. an indel mutation in exon 55, can be treated with modification of exon 55, e.g., 5' of the indel mutation. The subjects would be expected to have an improvement in their disease severity by the approach described herein.

***2.24 Intra-exonic targeting exon 8***

In certain embodiments, modification of exon 8, e.g. 5' of premature termination codon in exon 8, e.g., by NHEJ, is expected to ameliorate the phenotype of up to 5.2% of DMD subjects (Flanigan et al., Human Mutation 2009; 30:1657-1666. Aartsma-Rus et al., Human Mutation 2009; 30:293-299. Bladen et al., Human Mutation 2015; 36(2)).

In certain embodiments, a subject with a deletion mutation, e.g., del3-7, del4-7, del5-7, or del6-7, can be treated with modification of exon 8, e.g., 5' of p.229. In certain embodiments, a subject with any mutation, e.g., a duplication mutation that would have the *DMD* reading frame restored by modification of exon 8, can be treated with modification of exon 8, e.g., 5' of the premature termination codon, e.g., 5' of p.229. In certain embodiments, a subject with a point mutation, e.g. a point mutation in exon 8, can be treated with modification of exon 8. In certain embodiments, a subject with an indel mutation, e.g. an indel mutation in exon 8, can be treated with modification of exon 8, e.g., at exon 8 5' of indel mutation. The subjects would be expected to have an improvement in their disease severity by the approach described herein.

***2.25 Intra-exonic targeting exon 70***

In certain embodiments, modification of exon 70, e.g., 5' of premature termination codon in exon 70, e.g., by NHEJ, is expected to ameliorate the phenotype of up to 6% of DMD subjects with nonsense or in/del mutations. (Based on frequency of exon 70 nonsense mutations from Duchenne Muscular Dystrophy database)

In certain embodiments, any subject with a deletion mutation, e.g., del71-75, can be treated with modification of exon 70. In certain embodiments, any subject with any mutation, e.g., a duplication mutation that would have the *DMD* reading frame restored by modification of exon 70, can be treated with modification of exon 70, e.g., 5' of the premature termination codon. In certain embodiments, any subject with a point mutation, e.g. a point mutation in exon 70, e.g., nonsense mutation c.10171C>T, e.g., nonsense mutation c.10141C>T, e.g., nonsense mutation c.10108C>T, can be treated with modification of exon 70. In certain embodiments, any subject with an indel mutation, e.g., and indel mutation in exon 70, can be treated with modification of exon 70, e.g, 5' of the indel mutation. In subjects with these mutations, modification of exon 70 will restore the reading frame *of DMD.* After treatment, these subjects will produce a dystrophin with modifications in exon 70 but containing exons 1-68 and 71-79. The subjects would be expected to have an improvement in their disease severity by the approach described herein.

***2.26 Intra-exonic targeting exon 1***

In certain embodiments, modification of exon 1 e.g., 5' of premature termination codon in exon 1, e.g., by NHEJ, is expected to ameliorate the phenotype of approximately 3% of DMD subjects who have nonsense or in/del mutations.

(Based on frequency of nonsense mutations in exon 1 from Duchenne Muscular Dystrophy database)

In certain embodiments, any subject with any mutation, e.g., a deletion mutation that would have the *DMD* reading frame restored by modification of exon 1, can be treated with modification of exon 1, e.g., 5' of the premature termination codon. In certain embodiments, any subject with a point mutation, e.g. a point mutation in exon 1, can be treated with modification of exon 1, e.g, at the position of point mutation. In certain embodiments, a subject with a nonsense mutation, can be treated with modification of exon 1, e.g, at the position of nonsense mutation. In certain embodiments, a subject with nonsense mutation c.9G>A, can be treated with modification of exon 1, e.g, at the position of nonsense mutation, e.g., at c.9. In certain embodiments, a subject with an indel mutation, e.g., an indel mutation in exon 1, can be treated with modification of exon 1, e.g, at the position of indel mutation. The subjects described herein would be expected to have an improvement in their disease severity by the approach described herein.

***2.27 Intra-exonic targeting exon 6***

In certain embodiments, modification of exon 6 e.g., 5' of premature termination codon in exon 6, e.g., by NHEJ, is expected to ameliorate the phenotype of approximately 3% of DMD subjects who have nonsense or in/del mutations.

(Based on frequency of nonsense mutations in exon 1 from Duchenne Muscular Dystrophy database)

In certain embodiments, any subject with any mutation, e.g., a deletion mutation that would have the *DMD* reading frame restored by modification of exon 6, can be treated with modification of exon 1, e.g., 5' of the premature termination codon. In certain embodiments, any subject with any mutation, e.g., a duplication mutation, that would have the *DMD* reading frame restored by modification of exon 6, can be treated with modification of exon 6, e.g., 5' of the premature termination codon. In certain embodiments, any subject with a point mutation, e.g. a point mutation in exon 6, can be treated with modification of exon 6, e.g, at the position of point mutation. In certain embodiments, a subject with a nonsense mutation can be treated with modification of exon 6, e.g, at the position of nonsense mutation. In certain embodiments, a subject with nonsense mutation c.355C>T can be treated with modification of exon 6, e.g, at the position of nonsense mutation, e.g., at c.355 in *DMD.* In certain embodiments, a subject with nonsense mutation c.440C>G can be treated with modification of exon 6, e.g, at the position of nonsense mutation, e.g., at c.440 in *DMD.* In certain embodiments, a subject with nonsense mutation c.429G>A can be treated with modification of exon 6, e.g, at the position of nonsense mutation, e.g., at c. c.429G>A in *DMD.* In certain embodiments, a subject with nonsense mutation c.433C>T can be treated with modification of exon 6, e.g, at the position of nonsense mutation, e.g., at c.433 in *DMD.* In certain embodiments, a subject with nonsense mutation c.457C>T can be treated with modification of exon 6, e.g, at the position of nonsense mutation, e.g., at c.457 in *DMD.* In certain embodiments, a subject with an indel mutation, e.g., an indel mutation in exon 6, can be treated with modification of exon 6, e.g, at the position of indel mutation in *DMD.* The subjects described herein would be expected to have an improvement in their disease severity by the approach described herein.

### *2.28 Intra-exonic targeting exon 14*

In certain embodiments, modification of exon 14, e.g. 5' of premature termination codon in exon 14, e.g., by NHEJ, is expected to ameliorate the phenotype of approximately 3% of DMD subjects who have nonsense or indel mutations. (Based on frequency of exon 14 nonsense mutations from Duchenne Muscular Dystrophy database)

In certain embodiments, any subject with any mutation, e.g., a deletion mutation that would have the *DMD* reading frame restored by modification of exon 14, can be treated with modification of exons 14. In certain embodiments, any subject with any mutation, e.g., a duplication mutation that would have the *DMD* reading frame restored by modification of exon 14, can be treated with modification of exon 14, e.g., 5' of the premature termination codon. In certain embodiments, any subject with a point mutation, e.g. a point mutation in exon 14, e.g., nonsense mutation c.1663C>T, e.g., nonsense mutation c.1619G>A, e.g., nonsense mutation c.1702C>T, e.g., nonsense mutation c.1615C>T, can be treated with modification of exon 14. In certain embodiments, a subject with an indel mutation, e.g. an indel mutation in exon 14, can be treated with modification of exon 14, e.g, 5' of the indel mutation. The subjects would be expected to have an improvement in their disease severity by the approach described herein.

### *2.29 Intra-exonic targeting exon 20*

In certain embodiments, modification of exon 20, e.g., 5' of premature termination codon in exon 21, e.g., by NHEJ, is expected to ameliorate the phenotype of approximately 5% of DMD subjects who have nonsense or in/del mutations.

(Based on frequency of exon 20 nonsense mutations from Duchenne Muscular Dystrophy database)

In certain embodiments, any subject with any mutation, e.g., a deletion mutation that would have the *DMD* reading frame restored by modification of exon 20, can be treated with modification of exon 20. In certain embodiments, any subject with any mutation, e.g., a duplication mutation that would have the *DMD* reading frame restored by modification of exon 20, can be treated with modification of exons 20, e.g., 5' of the premature termination codon. In certain embodiments, any subject with a point mutation, e.g. a point mutation in exon 20, can be treated with modification of exon 20. In certain embodiments, a subject with nonsense mutation c.2521C>T can be treated with modification of exon 20, e.g, at the position of nonsense mutation, e.g., at c.2521. In certain embodiments, a subject with nonsense mutation c.2485C>T can be treated with modification of exon 20, e.g, at the position of nonsense mutation, e.g., at c.2485. In certain embodiments, a subject with nonsense mutation c.2419C>T can be treated with modification of exon 20, e.g, at the position of nonsense mutation, e.g., at c.2419. In certain embodiments, a subject with c.2479G>T nonsense mutation can be treated with modification of exon 20, e.g, at the position of nonsense mutation, e.g., at c.2479. certain embodiments, a subject with nonsense mutation c.2404A>T can be treated with modification of exon 20, e.g, at the position of nonsense mutation, e.g., at c.2404. In certain embodiments, a subject with nonsense mutation c.2416G>T can be treated with modification of exon 20, e.g, at the position of nonsense mutation, e.g., at c.2416. In certain embodiments, a subject with nonsense mutation c.2440G>T can be treated with modification of exon 20, e.g, at the position of nonsense mutation,

e.g., at c.2440. In certain embodiments, a subject with nonsense mutation c.2611A>T can be treated with modification of exon 20, e.g, at the position of nonsense mutation, e.g., at c.2611. In certain embodiments, a subject with nonsense mutation c.2582C>G can be treated with modification of exon 20, e.g, at the position of nonsense mutation, e.g., at c.2582. In certain embodiments, a subject with an indel mutation, e.g., an indel mutation in exon 20, can be treated with modification of exon 20, e.g, 5' of the indel mutation, e.g., 5' of indel mutation. The subjects would be expected to have an improvement in their disease severity by the approach described herein.

***2.30 Additional Exemplary Approaches***

In certain embodiments, any subject with any mutation, e.g., a duplication mutation that would have the *DMD* reading frame restored by single-gRNA mediated NHEJ excision of an exon, can be treated with modification of that exon. In certain embodiments, any subject with dup2 can be treated with a single gRNA mediated NHEJ excision of one of two exon 2's in *DMD.* In certain embodiments, any subject with dup3-7 can be treated with a single gRNA mediated NHEJ excision of one of two exon 3-7 transcripts in *DMD.*

In certain embodiments, any subject with a point mutation, e.g. a point mutation in exon 34, can be treated with modification of exon 34. In certain embodiments, a subject with nonsense mutation c.4693C>T can be treated with modification of exon 34, e.g, at the position of nonsense mutation, e.g., at c.4693 in *DMD.* In certain embodiments, a subject with nonsense mutation c.4729C>T can be treated with modification of exon 34, e.g, at the position of nonsense mutation, e.g., at c.4729 in *DMD.* In certain embodiments, a subject with nonsense mutation c.4793C>A can be treated with modification of exon 34, e.g, at the position of nonsense mutation, e.g., at c.4793 in *DMD.* In certain embodiments, a subject with nonsense mutation c.4697T>A can be treated with modification of exon 34, e.g, at the position of nonsense mutation, e.g., at c.4697T in *DMD.*

In certain embodiments, any subject with a point mutation, e.g. a point mutation in exon 38, can be treated with modification of exon 38. In certain embodiments, a subject with nonsense mutation c.5371C>T can be treated with modification of exon 38, e.g, at the position of nonsense mutation, e.g., at c.5371 in *DMD.* In certain embodiments, a subject with nonsense mutation c.5404C>T can be treated with modification of exon 38, e.g, at the position of nonsense mutation, e.g., at c.5404 in *DMD.* In certain embodiments, a subject with nonsense mutation c.5398G>T can be treated with modification of exon 38, e.g, at the position of nonsense mutation, e.g., at c.5398 in *DMD.* In certain embodiments, a subject with nonsense mutation c.5344G>T can be treated with modification of exon 38, e.g, at the position of nonsense mutation, e.g., at c.5344 in *DMD.*

In certain embodiments, any subject with a point mutation, e.g. a point mutation in exon 41, can be treated with modification of exon 41. In certain embodiments, a subject with nonsense mutation c.5902A>T can be treated with modification of exon 41, e.g, at the position of nonsense mutation, e.g., at c.5902 in *DMD.* In certain embodiments, a subject with nonsense mutation c.5758C>T can be treated with modification of exon 41, e.g, at the position of nonsense mutation, e.g., at c.5758 in *DMD.* In certain embodiments, a subject with nonsense mutation c.5917C> can be treated with modification of exon 41, e.g, at the position of nonsense mutation, e.g., at c.5917 in *DMD.* In certain embodiments, a subject with nonsense mutation c.5758C>T can be treated with modification of exon 41, e.g, at the position of nonsense mutation, e.g., at c.5758 in *DMD.* In certain embodiments, a subject with nonsense mutation c.5800G>T can be treated with modification of exon 41, e.g, at the position of nonsense mutation, e.g., at c.5800 *in DMD.* In certain embodiments, a subject with nonsense mutation c.5899C>T can be treated with modification of exon 41, e.g, at the position of nonsense mutation, e.g., at c.5899 in *DMD.* In certain embodiments, a subject with nonsense mutation c.5752G>T can be treated with modification of exon 41, e.g, at the position of nonsense mutation, e.g., at c.5752 in *DMD.* In certain embodiments, a subject with nonsense mutation c.5867G>A can be treated with modification of exon 41, e.g, at the position of nonsense mutation, e.g., at c.5867 in *DMD.*

In certain embodiments, any subject with a point mutation, e.g. a point mutation in exon 59, can be treated with modification of exon 59. In certain embodiments, a subject with nonsense mutation c. 8775G>A can be treated with modification of exon 59, e.g, at the position of nonsense mutation, e.g., at c.8775 in *DMD.* In certain embodiments, a subject with nonsense mutation c.8713C>T can be treated with modification of exon 59, e.g, at the position of nonsense mutation, e.g., at c.8713 in *DMD.* In certain embodiments, a subject with nonsense mutation c.8680G>T can be treated with modification of exon 59, e.g, at the position of nonsense mutation, e.g., at c.8680 *in DMD.* In certain embodiments, a subject with nonsense mutation c.8872G>T can be treated with modification of exon 59, e.g, at the position of nonsense mutation, e.g., at c.8872 in *DMD.*

In certain embodiments, any subject with a point mutation, e.g. a point mutation in exon 47, can be treated with modification of exon 47, e.g, at site of point mutation. In certain embodiments, any subject with a point mutation, e.g. a point mutation in exon 48, can be treated with modification of exon 48, e.g, at site of point mutation. In certain embodiments, any subject with a point mutation, e.g. a point mutation in exon 49, can be treated with modification of exon 49, e.g, at site of point mutation. In certain embodiments, any subject with a point mutation, e.g. a point mutation in exon 54, can be treated with modification of exon 54, e.g, at site of point mutation.

In certain embodiments, any subject with any mutation, e.g., a deletion mutation that would have the *DMD* reading frame restored by modification of exon 47, can be treated with modification of exon 47, IFTP at exon 47 5' of premature termination codon, e.g., 5' of p.2257. In certain embodiments, any subject with any mutation, e.g., a deletion mutation that would have the *DMD* reading frame restored by modification of exon 48, can be treated with modification of exon 48, IFTP at exon 48 5' of premature termination codon, e.g., 5' of p.2316. In certain embodiments, any subject with any mutation, e.g., a deletion mutation that would have the *DMD* reading frame restored by modification of exon 49, can be treated with modification of exon 49, IFTP at exon 49 5' of premature termination codon, e.g., 5' of p. 2368. In certain embodiments, any subject with any mutation, e.g., a deletion mutation that would have the *DMD* reading frame restored by modification of exon 54, can be treated with modification of exon 54, IFTP at exon 54 5' of premature termination codon, e.g., 5' of p.2637.

**3. Methods to Treat/Prevent DMD and BMD and Dilated Cardiomyopathy (DCM) Type 3B**

Described herein are the compositions, genome-editing systems and methods for treating or preventing, e.g., delaying the onset or progression, of Duchenne muscular dystrophy (DMD), Becker muscular dystrophy (BMD), dilated cardiomyopathy (DCM) type 3B, or a symptom associated thereof (e.g., dialted cardiomyopathy, e.g., DMD-associated dilated cardiomyopathy). DMD, BMD and DCM type 3B can be caused by a mutation in the *DMD* gene, including, e.g., a duplication, a deletion, or a nonsense or frameshift mutation in the *DMD* gene. The disclosed compositions, genome-editing systems and methods alter the *DMD* gene by genome editing, e.g., using a guide RNA (gRNA) targeting a DMD target position and a Cas9 molecule.

In certain embodiments, restoring the *DMD* reading frame in myocytes, including skeletal, smooth and cardiac myocytes, or myoblasts, will generate mini-dystrophins or dystrophins with a slightly altered transcript. Mini-dystrophins or slightly altered dystrophins can be more functional than truncated dystrophins. In certain embodiments, the disease does not progress or has delayed progression compared to a subject who has not received the therapy. In certain embodiments, correction of dystrophin in myocytes or myoblasts will prevent the progression of skeletal, smooth and cardiac muscle disease in subjects with DMD and BMD. In certain embodiments, the disease is cured, does not progress or has delayed progression compared to a subject who has not received the therapy.

In certain embodiments, the compositions, genome-editing systems and methods described herein are used to treat a subject having DMD, BMD or DMD-associated cardiomyopathy. In certain embodiments, the compositions, genome-editing systems and methods described herein are used to prevent, or delay the onset or progression of, DMD, BMD or DMD-associated cardiomyopathy.

In certain embodiments, the treatment is initiated prior to onset of the disease. In certain embodiments, the treatment is initiated after onset of the disease. In certain embodiments, the treatment is initiated prior to onset of muscle weakness or the appearance of declining muscle function. In certain embodiments, the treatment is initiated at onset of muscle weakness or the appearance of declining muscle function. In certain embodiments, the treatment is initiated after onset of muscle weakness or the appearance of declining muscle function.

In certain embodiments, the treatment is initiated *in utero,* after birth, or prior to the age of 1, 2, 3, 4, 5, 6, 7, 8, 9, 10, 15, 20, 25, 30, 35, 40, or 50.

In certain embodiments, the treatment is initiated in a subject prior to the onset of elevated serum creatinine phosphokinase (CK) concentration. In certain embodiments, the treatment is initiated in a subject at the onset of elevated serum creatinine phosphokinase (CK) concentration. In certain embodiments, the treatment is initiated in a subject after the onset of elevated serum creatinine phosphokinase (CK) concentration.

In certain embodiments, the treatment is initiated in a subject prior to muscle a biopsy showing a change associated with DMD, BMD, and/or dilated cardiomyopathy. In certain embodiments, the treatment is initiated in a subject when a muscle biopsy shows a change associated with DMD, BMD, and/or dilated cardiomyopathy. In certain embodiments, the treatment is initiated in a subject after a muscle biopsy showing a change associate with DMD, BMD, and/or dilated cardiomyopathy. Exemplary changes associated with DMD, BMD, or dilated cardiomyopathy, as detected by muscle biopsy, include, but are not limited to, nonspecific dystrophic changes, e.g., variation in fiber size, foci of necrosis and regeneration, hyalinization, and, deposition of fat and connective tissue.

In certain embodiments, the treatment is initiated in a subject prior to an immunoassay showing a change associated with DMD, BMD, and/or dilated cardiomyopathy. In certain embodiments, the treatment is initiated in a subject when an immunoassay shows a change associated with DMD, BMD, and/or dilated cardiomyopathy. In certain embodiments, the treatment is initiated in a subject after an immunoassay showing a change associated with DMD, BMD, and/or dilated cardiomyopathy. Exemplary immunoassays include, but are not limited to, Western blot, immunostaining (e.g., immunohistochemistry), fluorescent immunoassay, and enzyme-linked immuno assay (ELISA). Exemplary changes associated with DMD, BMD, and/or dilated cardiomyopathy, as determined by an immunoassay, include, but are not limited to, reduced dystrophin quantity (e.g., on western blot), abnormal or undetectable dystrophin molecular weight (e.g., on western blot), absent or reduced intensity or patchy staining or mosaic pattern or dystrophin-negative fibers

(e.g., on immunohistochemistry).

In certain embodiments, the treatment is initiated in a subject prior to, when, or after an electromyography (EMG) showing an activity associated with DMD, BMD, and/or dilated cardiomyopathy. Exemplary activities associated with or consistent with DMD, BMD, and/or dilated cardiomyopathy, include, but are not limited to, short-duration, low-amplitude, polyphasic, rapidly recruited motor unit potentials on EMG or no potentials on EMG. In certain embodiments, the treatment is initiated in a subject prior to, at, or after the onset of dilated cardiomyopathy, with or without presence of skeletal muscle disease. In certain embodiments, the treatment is initiated in a subject prior to, at, or after the onset of respiratory compromise or respiratory failure.

A subject's muscle weakness can be evaluated, e.g., prior to the treatment, during the treatment, or after the treatment, e.g., to monitor the progress of the treatment. In certain embodiments, the subject's muscle function is evaluated prior to treatment, e.g., to determine the need for treatment. In certain embodiments, the subject's muscle function is evaluated after treatment has been initiated, e.g., to access the effectiveness of the treatment. Muscle function can be evaluated, e.g., by one or more of: physical examination (noting scoliosis, muscle deformities, including contractures or heels and legs and/or abnormal fat and connective tissues in calf muscles), wheelchair dependency, six-minute walk test (6MWT), presence of dilated cardiomyopathy, echocardiography, cardiac function, pulmonary function tests (including forced vital capacity, forced expiratory volume, peak expiratory flow rate, maximal inspiratory pressure, maximal inspiratory pressure), CPK blood test, electromyography (EMG) nerve testing, muscle biopsy and quantification of dystrophin on western blot or immunohistochemistry.

In certain embodiments, a subject's muscle function may be assessed by measuring the subject's mobility, e.g., the subject's ability to perform activities of daily living, including ambulation, unassisted breathing.

In certain embodiments, the treatment is initiated in a subject who has tested positive for a mutation in the *DMD* gene, e.g., a mutation described herein, e.g., prior to disease onset, in the earliest stages of disease, or at time of disease onset. In certain embodiments, the mutation is tested by sequencing.

In certain embodiments, a subject has a family member that has been diagnosed with DMD, BMD, and/or dilated cardiomyopathy. For example, and the subject demonstrates a symptom or sign of the disease or has been found to have a mutation in the *DMD* gene. In certain embodiments, the subject has a family member that has been diagnosed with DMD, BMD, and/or dilated cardiomyopathy.

In certain embodiments, a cell (e.g., a skeletal muscle cell, a smooth muscle cell, a cardiac muscle cell, or a cardiomyocyte) from a subject suffering from or likely to develop DMD, BMD, and/or dilated cardiomyopathy is treated *ex vivo*. In certain embodiments, the cell is removed from the subject, altered as described herein, and introduced into, e.g., returned to, the subject.

In certain embodiments, a cell (e.g., a skeletal muscle cell, a smooth muscle cell, a cardiac muscle cell, or a cardiomyocyte) is altered to correct a mutation in the *DMD* gene, e.g., a mutation described herein, is introduced into the subject.

In certain embodiments, a population of cells (e.g., a population of muscle cells, e.g., a population of skeletal muscle cells, smooth muscle cells, cardiac muscle cells, cardiomyocytes, or a combination thereof) from a subject may be contacted ex *vivo* to alter a mutation in the *DMD* gene, e.g., a mutation described herein. In certain embodiments, such cells are introduced to the subject's body to treat or delay the onset or progression of DMD, BMD, DCM type 3B, or a symptom associated therefore (e.g., dilated cardiomyopathy, e.g., DMD-associated dilated cardiomyopathy).

In certain embodiments, the method described herein comprises delivery of gRNA or other components described herein, e.g., a Cas9 molecule, by one or more AAV vectors.

### 4. Guide RNA (gRNA) molecules

A gRNA molecule, as that term is used herein, refers to a nucleic acid that promotes the specific targeting or homing of a gRNA molecule/Cas9 molecule complex to a target nucleic acid. gRNA molecules can be unimolecular (having a single RNA molecule) (e.g., chimeric), or modular (comprising more than one, and typically two, separate RNA molecules). The gRNA molecules provided herein comprise a targeting domain comprising, consisting of, or consisting essentially of a nucleic acid sequence fully or partially complementary to a target domain. In certain embodiments, the gRNA molecule further comprises one or more additional domains, including for example a first complementarity domain, a linking domain, a second complementarity domain, a proximal domain, a tail domain, and a 5' extension domain. Each of these domains is discussed in detail below. In certain embodiments, one or more of the domains in the gRNA molecule comprises an amino acid sequence identical to or sharing sequence homology with a naturally occurring sequence, e.g., from *S. pyogenes, S. aureus,* or *S. thermophilus.*

Several exemplary gRNA structures are provided in **Figs. 1A-1I**. With regard to the three-dimensional form, or intra- or inter-strand interactions of an active form of a gRNA, regions of high complementarity are sometimes shown as duplexes in **Figs. 1A-1I** and other depictions provided herein. **Fig. 7** illustrates gRNA domain nomenclature using the gRNA sequence of SEQ ID NO:42, which contains one hairpin loop in the tracrRNA-derived region. In certain

embodiments, a gRNA may contain more than one (e.g., two, three, or more) hairpin loops in this region (see, e.g.,

**Figs. 1H-1I).**

In certain embodiments, a unimolecular, or chimeric, gRNA comprises, preferably from 5' to 3':

a targeting domain complementary to a target domain in a *DMD* gene (e.g., human *DMD* gene), e.g., a targeting domain comprising a nucleotide sequence selected from SEQ ID NOs: 206-826366;
a first complementarity domain;
a linking domain;
a second complementarity domain (which is complementary to the first complementarity domain);
a proximal domain; and
optionally, a tail domain.

In certain embodiments, a modular gRNA comprises:

a first strand comprising, preferably from 5' to 3':

a targeting domain complementary to a target domain in a *DMD* gene (e.g., human *DMD* gene), e.g., a targeting domain comprising a nucleotide sequence selected from SEQ ID NOs: 206-826366; and
a first complementarity domain; and

a second strand, comprising, preferably from 5' to 3':

optionally, a 5' extension domain;
a second complementarity domain;
a proximal domain; and
optionally, a tail domain.

### *4.1 Targeting domain*

The targeting domain (sometimes referred to alternatively as the guide sequence) comprises, consists of, or consists essentially of a nucleic acid sequence that is complementary or partially complementary to a target nucleic acid sequence in a *DMD* gene (e.g., human *DMD* gene). The nucleic acid sequence in a *DMD* gene to which all or a portion of the targeting domain is complementary or partially complementary is referred to herein as the target domain.

Methods for selecting targeting domains are known in the art (see, e.g., Fu 2014; Sternberg 2014). Examples of suitable targeting domains for use in the methods, compositions, and kits described herein comprise nucleotide sequences set forth in SEQ ID NOs: 206-826366.

The strand of the target nucleic acid comprising the target domain is referred to herein as the complementary strand because it is complementary to the targeting domain sequence. Since the targeting domain is part of a gRNA molecule, it comprises the base uracil (U) rather than thymine (T); conversely, any DNA molecule encoding the gRNA molecule will comprise thymine rather than uracil. In a targeting domain/target domain pair, the uracil bases in the targeting domain will pair with the adenine bases in the target domain. In certain embodiments, the degree of complementarity between the targeting domain and target domain is sufficient to allow targeting of a Cas9 molecule to the target nucleic acid.

In certain embodiments, the targeting domain comprises a core domain and an optional secondary domain. In certain of these embodiments, the core domain is located 3' to the secondary domain, and in certain of these embodiments the core domain is located at or near the 3' end of the targeting domain. In certain of these embodiments, the core domain consists of or consists essentially of about 8 to about 13 nucleotides at the 3' end of the targeting domain. In certain embodiments, only the core domain is complementary or partially complementary to the corresponding portion of the target domain, and in certain of these embodiments the core domain is fully complementary to the corresponding portion of the target domain. In certain embodiments, the secondary domain is also complementary or partially complementary to a portion of the target domain. In certain embodiments, the core domain is complementary or partially complementary to a core domain target in the target domain, while the secondary domain is complementary or partially complementary to a secondary domain target in the target domain. In certain embodiments, the core domain and secondary domain have the same degree of complementarity with their respective corresponding portions of the target domain. In certain embodiments, the degree of complementarity between the core domain and its target and the degree of complementarity between the secondary domain and its target may differ. In certain of these embodiments, the core

domain may have a higher degree of complementarity for its target than the secondary domain, whereas in other embodiments the secondary domain may have a higher degree of complementarity than the core domain.

In certain embodiments, the targeting domain and/or the core domain within the targeting domain is 3 to 100, 5 to 100, 10 to 100, or 20 to 100 nucleotides in length, and in certain of these embodiments the targeting domain or core domain is 3 to 15, 3 to 20, 5 to 20, 10 to 20, 15 to 20, 5 to 50, 10 to 50, or 20 to 50 nucleotides in length. In certain embodiments, the targeting domain and/or the core domain within the targeting domain is 6, 7, 8, 9, 10, 11, 12, 13, 14, 15, 16, 17, 18, 19, 20, 21, 22, 23, 24, 25, or 26 nucleotides in length. In certain embodiments, the targeting domain and/or the core domain within the targeting domain is 6 +/-2, 7+/-2, 8+/-2, 9+/-2, 10+/-2, 10+/-4, 10 +/-5, 11÷/-2, 12+/-2, 13+/-2, 14+/-2, 15+/-2, or 16+-2, 20+/-5, 30+/-5, 40+/-5, 50+/-5, 60+/-5, 70+/-5, 80+/-5, 90+/-5, or 100+/-5 nucleotides in length.

In certain embodiments wherein the targeting domain includes a core domain, the core domain is 3 to 20 nucleotides in length, and in certain of these embodiments the core domain 5 to 15 or 8 to 13 nucleotides in length. In certain embodiments wherein the targeting domain includes a secondary domain, the secondary domain is 0, 1, 2, 3, 4, 5, 6, 7, 8, 9, 10, 11, 12, 13, 14 or 15 nucleotides in length. In certain embodiments wherein the targeting domain comprises a core domain that is 8 to 13 nucleotides in length, the targeting domain is 26, 25, 24, 23, 22, 21, 20, 19, 18, 17, or 16 nucleotides in length, and the secondary domain is 13 to 18, 12 to 17, 11 to 16, 10 to 15, 9 to 14, 8 to 13, 7 to 12, 6 to 11, 5 to 10, 4 to 9, or 3 to 8 nucleotides in length, respectively.

In certain embodiments, the targeting domain is fully complementary to the target domain. Likewise, where the targeting domain comprises a core domain and/or a secondary domain, in certain embodiments one or both of the core domain and the secondary domain are fully complementary to the corresponding portions of the target domain. In certain embodiments, the targeting domain is partially complementary to the target domain, and in certain of these embodiments where the targeting domain comprises a core domain and/or a secondary domain, one or both of the core domain and the secondary domain are partially complementary to the corresponding portions of the target domain. In certain of these embodiments, the nucleic acid sequence of the targeting domain, or the core domain or targeting domain within the targeting domain, is at least about 80%, about 85%, about 90%, or about 95% complementary to the target domain or to the corresponding portion of the target domain. In certain embodiments, the targeting domain and/or the core or secondary domains within the targeting domain include one or more nucleotides that are not complementary with the target domain or a portion thereof, and in certain of these embodiments the targeting domain and/or the core or secondary domains within the targeting domain include 1, 2, 3, 4, 5, 6, 7, or 8 nucleotides that are not complementary with the target domain. In certain embodiments, the core domain includes 1, 2, 3, 4, or 5 nucleotides that are not complementary with the corresponding portion of the target domain. In certain embodiments wherein the targeting domain includes one or more nucleotides that are not complementary with the target domain, one or more of said non-complementary nucleotides are located within five nucleotides of the 5' or 3' end of the targeting domain. In certain of these embodiments, the targeting domain includes 1, 2, 3, 4, or 5 nucleotides within five nucleotides of its 5' end, 3' end, or both its 5' and 3' ends that are not complementary to the target domain. In certain embodiments wherein the targeting domain includes two or more nucleotides that are not complementary to the target domain, two or more of said non-complementary nucleotides are adjacent to one another, and in certain of these embodiments the two or more consecutive non-complementary nucleotides are located within five nucleotides of the 5' or 3' end of the targeting domain. In certain embodiments, the two or more consecutive non-complementary nucleotides are both located more than five nucleotides from the 5' and 3' ends of the targeting domain.

In certain embodiments, the targeting domain, core domain, and/or secondary domain do not comprise any modifications. In certain embodiments, the targeting domain, core domain, and/or secondary domain, or one or more nucleotides therein, have a modification, including but not limited to the modifications set forth below. In certain embodiments, one or more nucleotides of the targeting domain, core domain, and/or secondary domain may comprise a 2' modification (e.g., a modification at the 2' position on ribose), e.g., a 2-acetylation, e.g., a 2' methylation. In certain embodiments, the backbone of the targeting domain can be modified with a phosphorothioate. In certain embodiments, modifications to one or more nucleotides of the targeting domain, core domain, and/or secondary domain render the targeting domain and/or the gRNA comprising the targeting domain less susceptible to degradation or more bio-compatible, e.g., less immunogenic. In certain embodiments, the targeting domain and/or the core or secondary domains include 1, 2, 3, 4, 5, 6, 7, or 8 or more modifications, and in certain of these embodiments the targeting domain and/or core or secondary domains include 1, 2, 3, or 4 modifications within five nucleotides of their respective 5' ends and/or 1, 2, 3, or 4 modifications within five nucleotides of their respective 3' ends. In certain embodiments, the targeting domain and/or the core or secondary domains comprise modifications at two or more consecutive nucleotides.

In certain embodiments wherein the targeting domain includes core and secondary domains, the core and secondary domains contain the same number of modifications. In certain of these embodiments, both domains are free of modifications. In other embodiments, the core domain includes more modifications than the secondary domain, or vice versa.

In certain embodiments, modifications to one or more nucleotides in the targeting domain, including in the core

or secondary domains, are selected to not interfere with targeting efficacy, which can be evaluated by testing a candidate modification using a system as set forth below. gRNAs having a candidate targeting domain having a selected length, sequence, degree of complementarity, or degree of modification can be evaluated using a system as set forth below. The candidate targeting domain can be placed, either alone or with one or more other candidate changes in a gRNA molecule/Cas9 molecule system known to be functional with a selected target, and evaluated.

In certain embodiments, all of the modified nucleotides are complementary to and capable of hybridizing to corresponding nucleotides present in the target domain. In certain embodiments, 1, 2, 3, 4, 5, 6, 7 or 8 or more modified nucleotides are not complementary to or capable of hybridizing to corresponding nucleotides present in the target domain.

### *4.2 First and second complementarity domains*

The first and second complementarity (sometimes referred to alternatively as the crRNA-derived hairpin sequence and tracrRNA-derived hairpin sequences, respectively) domains are fully or partially complementary to one another. In certain embodiments, the degree of complementarity is sufficient for the two domains to form a duplexed region under at least some physiological conditions. In certain embodiments, the degree of complementarity between the first and second complementarity domains, together with other properties of the gRNA, is sufficient to allow targeting of a Cas9 molecule to a target nucleic acid. Examples of first and second complementary domains are set forth in **Figs. 1A-1G.**

In certain embodiments (see, e.g., **Figs. 1A-1B**) the first and/or second complementarity domain includes one or more nucleotides that lack complementarity with the corresponding complementarity domain. In certain embodiments, the first and/or second complementarity domain includes 1, 2, 3, 4, 5, or 6 nucleotides that do not complement with the corresponding complementarity domain. For example, the second complementarity domain may contain 1, 2, 3, 4, 5, or 6 nucleotides that do not pair with corresponding nucleotides in the first complementarity domain. In certain embodiments, the nucleotides on the first or second complementarity domain that do not complement with the corresponding complementarity domain loop out from the duplex formed between the first and second complementarity domains. In certain of these embodiments, the unpaired loop-out is located on the second complementarity domain, and in certain of these embodiments the unpaired region begins 1, 2, 3, 4, 5, or 6 nucleotides from the 5' end of the second complementarity domain.

In certain embodiments, the first complementarity domain is 5 to 30, 5 to 25, 7 to 25, 5 to 24, 5 to 23, 7 to 22, 5 to 22, 5 to 21, 5 to 20, 7 to 18, 7 to 15, 9 to 16, or 10 to 14 nucleotides in length, and in certain of these embodiments the first complementarity domain is 5, 6, 7, 8, 9, 10, 11, 12, 13, 14, 15, 16, 17, 18, 19, 20, 21, 22, 23, 24, or 25 nucleotides in length. In certain embodiments, the second complementarity domain is 5 to 27, 7 to 27, 7 to 25, 5 to 24, 5 to 23, 5 to 22, 5 to 21, 7 to 20, 5 to 20, 7 to 18, 7 to 17, 9 to 16, or 10 to 14 nucleotides in length, and in certain of these embodiments the second complementarity domain is 5, 6, 7, 8, 9, 10, 11, 12, 13, 14, 15, 16, 17, 18, 19, 20, 21, 22, 23, 24, 25, or 26 nucleotides in length. In certain embodiments, the first and second complementarity domains are each independently 6+/-2, 7+/-2, 8+/-2, 9+/-2, 10+/-2, 11+/-2, 12+/-2, 13+/-2, 14+/-2, 15+/-2, 16+/-2, 17+/-2, 18+/-2, 19+/-2, or 20+/-2, 21+/-2, 22+/-2, 23+/-2, or 24+/-2 nucleotides in length. In certain embodiments, the second complementarity domain is longer than the first complementarity domain, e.g., 2, 3, 4, 5, or 6 nucleotides longer.

In certain embodiments, the first and/or second complementarity domains each independently comprise three subdomains, which, in the 5' to 3' direction are: a 5' subdomain, a central subdomain, and a 3' subdomain. In certain embodiments, the 5' subdomain and 3' subdomain of the first complementarity domain are fully or partially complementary to the 3' subdomain and 5' subdomain, respectively, of the second complementarity domain.

In certain embodiments, the 5' subdomain of the first complementarity domain is 4 to 9 nucleotides in length, and in certain of these embodiments the 5' domain is 4, 5, 6, 7, 8, or 9 nucleotides in length. In certain embodiments, the 5' subdomain of the second complementarity domain is 3 to 25, 4 to 22, 4 to 18, or 4 to 10 nucleotides in length, and in certain of these embodiments the 5' domain is 3, 4, 5, 6, 7, 8, 9, 10, 11, 12, 13, 14, 15, 16, 17, 18, 19, 20, 21, 22, 23, 24, or 25 nucleotides in length. In certain embodiments, the central subdomain of the first complementarity domain is 1, 2, or 3 nucleotides in length. In certain embodiments, the central subdomain of the second complementarity domain is 1, 2, 3, 4, or 5 nucleotides in length. In certain embodiments, the 3' subdomain of the first complementarity domain is 3 to 25, 4 to 22, 4 to 18, or 4 to 10 nucleotides in length, and in certain of these embodiments the 3' subdomain is 3, 4, 5, 6, 7, 8, 9, 10, 11, 12, 13, 14, 15, 16, 17, 18, 19, 20, 21, 22, 23, 24, or 25 nucleotides in length. In certain embodiments, the 3' subdomain of the second complementarity domain is 4 to 9, e.g., 4, 5, 6, 7, 8 or 9 nucleotides in length.

The first and/or second complementarity domains can share homology with, or be derived from, naturally occurring or reference first and/or second complementarity domain. In certain of these embodiments, the first and/or second complementarity domains have at least about 50%, about 60%, about 70%, about 80%, about 85%, about 90%, or about 95% homology with, or differ by no more than 1, 2, 3, 4, 5, or 6 nucleotides from, the naturally occurring or reference first and/or second complementarity domain. In certain of these embodiments, the first and/or second complementarity domains may have at least about 50%, about 60%, about 70%, about 80%, about 85%, about 90%, or

about 95% homology with homology with a first and/or second complementarity domain from *S. pyogenes* or *S. aureus.*

In certain embodiments, the first and/or second complementarity domains do not comprise any modifications. In other embodiments, the first and/or second complementarity domains or one or more nucleotides therein have a modification, including but not limited to a modification set forth below. In certain embodiments, one or more nucleotides of the first and/or second complementarity domain may comprise a 2' modification (e.g., a modification at the 2' position on ribose), e.g., a 2-acetylation, e.g., a 2' methylation. In certain embodiments, the backbone of the targeting domain can be modified with a phosphorothioate. In certain embodiments, modifications to one or more nucleotides of the first and/or second complementarity domain render the first and/or second complementarity domain and/or the gRNA comprising the first and/or second complementarity less susceptible to degradation or more bio-compatible, e.g., less immunogenic. In certain embodiments, the first and/or second complementarity domains each independently include 1, 2, 3, 4, 5, 6, 7, or 8 or more modifications, and in certain of these embodiments the first and/or second complementarity domains each independently include 1, 2, 3, or 4 modifications within five nucleotides of their respective 5' ends, 3' ends, or both their 5' and 3' ends. In certain embodiments, the first and/or second complementarity domains each independently contain no modifications within five nucleotides of their respective 5' ends, 3' ends, or both their 5' and 3' ends. In certain embodiments, one or both of the first and second complementarity domains comprise modifications at two or more consecutive nucleotides.

In certain embodiments, modifications to one or more nucleotides in the first and/or second complementarity domains are selected to not interfere with targeting efficacy, which can be evaluated by testing a candidate modification in a system as set forth below. gRNAs having a candidate first or second complementarity domain having a selected length, sequence, degree of complementarity, or degree of modification can be evaluated in a system as set forth below. The candidate complementarity domain can be placed, either alone or with one or more other candidate changes in a gRNA molecule/Cas9 molecule system known to be functional with a selected target, and evaluated.

In certain embodiments, the duplexed region formed by the first and second complementarity domains is, for example, 6, 7, 8, 9, 10, 11, 12, 13, 14, 15, 16, 17, 18, 19, 20, 21, or 22 bp in length, excluding any looped out or unpaired nucleotides.

In certain embodiments, the first and second complementarity domains, when duplexed, comprise 11 paired nucleotides (see, for e.g., gRNA of SEQ ID NO:48). In certain embodiments, the first and second complementarity domains, when duplexed, comprise 15 paired nucleotides (see, e.g., gRNA of SEQ ID NO:50). In certain embodiments, the first and second complementarity domains, when duplexed, comprise 16 paired nucleotides (see, e.g., gRNA of SEQ ID NO:51). In certain embodiments, the first and second complementarity domains, when duplexed, comprise 21 paired nucleotides (see, e.g., gRNA of SEQ ID NO:29).

In certain embodiments, one or more nucleotides are exchanged between the first and second complementarity domains to remove poly-U tracts. For example, nucleotides 23 and 48 or nucleotides 26 and 45 of the gRNA of SEQ ID NO:48 may be exchanged to generate the gRNA of SEQ ID NOs:49 or 31, respectively. Similarly, nucleotides 23 and 39 of the gRNA of SEQ ID NO:29 may be exchanged with nucleotides 50 and 68 to generate the gRNA of SEQ ID NO:30.

### *4.3 Linking domain*

The linking domain is disposed between and serves to link the first and second complementarity domains in a unimolecular or chimeric gRNA. **Figs. 1B-1E** provide examples of linking domains. In certain embodiments, part of the linking domain is from a crRNA-derived region, and another part is from a tracrRNA-derived region.

In certain embodiments, the linking domain links the first and second complementarity domains covalently. In certain of these embodiments, the linking domain consists of or comprises a covalent bond. In other embodiments, the linking domain links the first and second complementarity domains non-covalently. In certain embodiments, the linking domain is ten or fewer nucleotides in length, e.g., 1, 2, 3, 4, 5, 6, 7, 8, 9, or 10 nucleotides. In other embodiments, the linking domain is greater than 10 nucleotides in length, e.g., 11, 12, 13, 14, 15, 16, 17, 18, 19, 20, 21, 22, 23, 24, or 25 or more nucleotides. In certain embodiments, the linking domain is 2 to 50, 2 to 40, 2 to 30, 2 to 20, 2 to 10, 2 to 5, 10 to 100, 10 to 90, 10 to 80, 10 to 70, 10 to 60, 10 to 50, 10 to 40, 10 to 30, 10 to 20, 10 to 15, 20 to 100, 20 to 90, 20 to 80, 20 to 70, 20 to 60, 20 to 50, 20 to 40, 20 to 30, or 20 to 25 nucleotides in length. In certain embodiments, the linking domain is 10 +/-5, 20+/-5, 20+/-10, 30+/-5, 30+/-10, 40+/-5, 40+/-10, 50+/-5, 50+/-10, 60+/-5, 60+/-10, 70+/-5, 70+/-10, 80+/-5, 80+/-10, 90+/-5, 90+/-10, 100+/-5, or 100+/-10 nucleotides in length.

In certain embodiments, the linking domain shares homology with, or is derived from, a naturally occurring sequence, e.g., the sequence of a tracrRNA that is 5' to the second complementarity domain. In certain embodiments, the linking domain has at least about 50%, about 60%, about 70%, about 80%, about 90%, or about 95% homology with or differs by no more than 1, 2, 3, 4, 5, or 6 nucleotides from a linking domain disclosed herein, e.g., the linking domains of **Figs. 1B-1E.**

In certain embodiments, the linking domain does not comprise any modifications. In other embodiments, the linking domain or one or more nucleotides therein have a modification, including but not limited to the modifications set

forth below. In certain embodiments, one or more nucleotides of the linking domain may comprise a 2' modification (e.g., a modification at the 2' position on ribose), e.g., a 2-acetylation, e.g., a 2' methylation. In certain embodiments, the backbone of the linking domain can be modified with a phosphorothioate. In certain embodiments, modifications to one or more nucleotides of the linking domain render the linking domain and/or the gRNA comprising the linking domain less susceptible to degradation or more bio-compatible, e.g., less immunogenic. In certain embodiments, the linking domain includes 1, 2, 3, 4, 5, 6, 7, or 8 or more modifications, and in certain of these embodiments the linking domain includes 1, 2, 3, or 4 modifications within five nucleotides of its 5' and/or 3' end. In certain embodiments, the linking domain comprises modifications at two or more consecutive nucleotides.

In certain embodiments, modifications to one or more nucleotides in the linking domain are selected to not interfere with targeting efficacy, which can be evaluated by testing a candidate modification in a system as set forth below. gRNAs having a candidate linking domain having a selected length, sequence, degree of complementarity, or degree of modification can be evaluated in a system as set forth below. The candidate linking domain can be placed, either alone or with one or more other candidate changes in a gRNA molecule/Cas9 molecule system known to be functional with a selected target, and evaluated.

In certain embodiments, the linking domain comprises a duplexed region, typically adjacent to or within 1, 2, or 3 nucleotides of the 3' end of the first complementarity domain and/or the 5' end of the second complementarity domain. In certain of these embodiments, the duplexed region of the linking region is 10+/-5, 15+/-5, 20+/-5, 20+/-10, or 30+/-5 bp in length. In certain embodiments, the duplexed region of the linking domain is 1, 2, 3, 4, 5, 6, 7, 8, 9, 10, 11, 12, 13, 14, or 15 bp in length. In certain embodiments, the sequences forming the duplexed region of the linking domain are fully complementarity. In other embodiments, one or both of the sequences forming the duplexed region contain one or more nucleotides (e.g., 1, 2, 3, 4, 5, 6, 7, or 8 nucleotides) that are not complementary with the other duplex sequence.

#### *4.4 5' extension domain*

In certain embodiments, a modular gRNA as disclosed herein comprises a 5' extension domain, i.e., one or more additional nucleotides 5' to the second complementarity domain (see, e.g., **Fig. 1A**). In certain embodiments, the 5' extension domain is 2 to 10 or more, 2 to 9, 2 to 8, 2 to 7, 2 to 6, 2 to 5, or 2 to 4 nucleotides in length, and in certain of these embodiments the 5' extension domain is 2, 3, 4, 5, 6, 7, 8, 9, or 10 or more nucleotides in length.

In certain embodiments, the 5' extension domain nucleotides do not comprise modifications, e.g., modifications of the type provided below. However, in certain embodiments, the 5' extension domain comprises one or more modifications, e.g., modifications that it render it less susceptible to degradation or more bio-compatible, e.g., less immunogenic. By way of example, the backbone of the 5' extension domain can be modified with a phosphorothioate, or other modification(s) as set forth below. In certain embodiments, a nucleotide of the 5' extension domain can comprise a 2' modification (e.g., a modification at the 2' position on ribose), e.g., a 2-acetylation, e.g., a 2' methylation, or other modification(s) as set forth below.

In certain embodiments, the 5' extension domain can comprise as many as 1, 2, 3, 4, 5, 6, 7, or 8 modifications. In certain embodiments, the 5' extension domain comprises as many as 1, 2, 3, or 4 modifications within 5 nucleotides of its 5' end, e.g., in a modular gRNA molecule. In certain embodiments, the 5' extension domain comprises as many as 1, 2, 3, or 4 modifications within 5 nucleotides of its 3' end, e.g., in a modular gRNA molecule.

In certain embodiments, the 5' extension domain comprises modifications at two consecutive nucleotides, e.g., two consecutive nucleotides that are within 5 nucleotides of the 5' end of the 5' extension domain, within 5 nucleotides of the 3' end of the 5' extension domain, or more than 5 nucleotides away from one or both ends of the 5' extension domain. In certain embodiments, no two consecutive nucleotides are modified within 5 nucleotides of the 5' end of the 5' extension domain, within 5 nucleotides of the 3' end of the 5' extension domain, or within a region that is more than 5 nucleotides away from one or both ends of the 5' extension domain. In certain embodiments, no nucleotide is modified within 5 nucleotides of the 5' end of the 5' extension domain, within 5 nucleotides of the 3' end of the 5' extension domain, or within a region that is more than 5 nucleotides away from one or both ends of the 5' extension domain.

Modifications in the 5' extension domain can be selected so as to not interfere with gRNA molecule efficacy, which can be evaluated by testing a candidate modification in a system as set forth below. gRNAs having a candidate 5' extension domain having a selected length, sequence, degree of complementarity, or degree of modification, can be evaluated in a system as set forth below. The candidate 5' extension domain can be placed, either alone, or with one or more other candidate changes in a gRNA molecule/Cas9 molecule system known to be functional with a selected target and evaluated.

In certain embodiments, the 5' extension domain has at least about 60%, about 70%, about 80%, about 85%, about 90%, or about 95% homology with, or differs by no more than 1, 2, 3, 4, 5, or 6 nucleotides from, a reference 5' extension domain, e.g., a naturally occurring, e.g., an *S. pyogenes, S. aureus,* or *S. thermophilus,* 5' extension domain, or a 5' extension domain described herein, e.g., from **Figs. 1A-1G.**

### *4.5 Proximal domain*

**Figs. 1A-1G** provide examples of proximal domains.

In certain embodiments, the proximal domain is 5 to 20 or more nucleotides in length, e.g., 5, 6, 7, 8, 9, 10, 11, 12, 13, 14, 15, 16, 17, 18, 19, 20, 21, 22, 23, 24, 25, or 26 nucleotides in length. In certain of these embodiments, the proximal domain is 6+/-2, 7+/-2, 8+/-2, 9+/-2, 10+/-2, 11+/-2, 12+/-2, 13+/-2, 14+/-2, 14+/-2, 16+/-2, 17+/-2, 18+/-2, 19+/-2, or 20+/-2 nucleotides in length. In certain embodiments, the proximal domain is 5 to 20, 7, to 18, 9 to 16, or 10 to 14 nucleotides in length.

In certain embodiments, the proximal domain can share homology with or be derived from a naturally occurring proximal domain. In certain of these embodiments, the proximal domain has at least about 50%, about 60%, about 70%, about 80%, about 85%, about 90%, or about 95% homology with or differs by no more than 1, 2, 3, 4, 5, or 6 nucleotides from a proximal domain disclosed herein, e.g., an *S. pyogenes, S. aureus,* or *S. thermophilus* proximal domain, including those set forth in **Figs. 1A-1G.**

In certain embodiments, the proximal domain does not comprise any modifications. In other embodiments, the proximal domain or one or more nucleotides therein have a modification, including but not limited to the modifications set forth in herein. In certain embodiments, one or more nucleotides of the proximal domain may comprise a 2' modification (e.g., a modification at the 2' position on ribose), e.g., a 2-acetylation, e.g., a 2' methylation. In certain embodiments, the backbone of the proximal domain can be modified with a phosphorothioate. In certain embodiments, modifications to one or more nucleotides of the proximal domain render the proximal domain and/or the gRNA comprising the proximal domain less susceptible to degradation or more bio-compatible, e.g., less immunogenic. In certain embodiments, the proximal domain includes 1, 2, 3, 4, 5, 6, 7, or 8 or more modifications, and in certain of these embodiments the proximal domain includes 1, 2, 3, or 4 modifications within five nucleotides of its 5' and/or 3' end. In certain embodiments, the proximal domain comprises modifications at two or more consecutive nucleotides.

In certain embodiments, modifications to one or more nucleotides in the proximal domain are selected to not interfere with targeting efficacy, which can be evaluated by testing a candidate modification in a system as set forth below. gRNAs having a candidate proximal domain having a selected length, sequence, degree of complementarity, or degree of modification can be evaluated in a system as set forth below. The candidate proximal domain can be placed, either alone or with one or more other candidate changes in a gRNA molecule/Cas9 molecule system known to be functional with a selected target, and evaluated.

### *4.6 Tail domain*

A broad spectrum of tail domains are suitable for use in the gRNA molecules disclosed herein. **Figs. 1A and 1C-1G** provide examples of such tail domains.

In certain embodiments, the tail domain is absent. In other embodiments, the tail domain is 1 to 100 or more nucleotides in length, e.g., 1, 2, 3, 4, 5, 6, 7, 8, 9, 10, 20, 30, 40, 50, 60, 70, 80, 90, or 100 nucleotides in length. In certain embodiments, the tail domain is 1 to 5, 1 to 10, 1 to 15, 1 to 20, 1 to 50, 10 to 100, 20 to 100, 10 to 90, 20 to 90, 10 to 80, 20 to 80, 10 to 70, 20 to 70, 10 to 60, 20 to 60, 10 to 50, 20 to 50, 10 to 40, 20 to 40, 10 to 30, 20 to 30, 20 to 25, 10 to 20, or 10 to 15 nucleotides in length. In certain embodiments, the tail domain is 5 +/-5, 10 +/-5, 20+/-10, 20+/-5, 25+/-10, 30+/-10, 30+/-5, 40+/-10, 40+/-5, 50+/-10, 50+/-5, 60+/-10, 60+/-5, 70+/-10, 70+/-5, 80+/-10, 80+/-5, 90+/-10, 90+/-5, 100+/-10, or 100+/-5 nucleotides in length,

In certain embodiments, the tail domain can share homology with or be derived from a naturally occurring tail domain or the 5' end of a naturally occurring tail domain. In certain of these embodiments, the proximal domain has at least about 50%, about 60%, about 70%, about 80%, about 85%, about 90%, or about 95% homology with or differs by no more than 1, 2, 3, 4, 5, or 6 nucleotides from a naturally occurring tail domain disclosed herein, e.g., an *S. pyogenes, S. aureus,* or *S. thermophilus* tail domain, including those set forth in **Figs. 1A and 1C-1G.**

In certain embodiments, the tail domain includes sequences that are complementary to each other and which, under at least some physiological conditions, form a duplexed region. In certain of these embodiments, the tail domain comprises a tail duplex domain which can form a tail duplexed region. In certain embodiments, the tail duplexed region is 3, 4, 5, 6, 7, 8, 9, 10, 11, or 12 bp in length. In certain embodiments, the tail domain comprises a single stranded domain 3' to the tail duplex domain that does not form a duplex. In certain of these embodiments, the single stranded domain is 3 to 10 nucleotides in length, e.g., 3, 4, 5, 6, 7, 8, 9, 10, or 4 to 6 nucleotides in length.

In certain embodiments, the tail domain does not comprise any modifications. In other embodiments, the tail domain or one or more nucleotides therein have a modification, including but not limited to the modifications set forth herein. In certain embodiments, one or more nucleotides of the tail domain may comprise a 2' modification (e.g., a modification at the 2' position on ribose), e.g., a 2-acetylation, e.g., a 2' methylation. In certain embodiments, the backbone of the tail domain can be modified with a phosphorothioate. In certain embodiments, modifications to one or more nucleotides of the tail domain render the tail domain and/or the gRNA comprising the tail domain less susceptible to

degradation or more bio-compatible, e.g., less immunogenic. In certain embodiments, the tail domain includes 1, 2, 3, 4, 5, 6, 7, or 8 or more modifications, and in certain of these embodiments the tail domain includes 1, 2, 3, or 4 modifications within five nucleotides of its 5' and/or 3' end. In certain embodiments, the tail domain comprises modifications at two or more consecutive nucleotides.

In certain embodiments, modifications to one or more nucleotides in the tail domain are selected to not interfere with targeting efficacy, which can be evaluated by testing a candidate modification as set forth below. gRNAs having a candidate tail domain having a selected length, sequence, degree of complementarity, or degree of modification can be evaluated using a system as set forth below. The candidate tail domain can be placed, either alone or with one or more other candidate changes in a gRNA molecule/Cas9 molecule system known to be functional with a selected target, and evaluated.

In certain embodiments, the tail domain includes nucleotides at the 3' end that are related to the method of *in vitro* or *in vivo* transcription. When a T7 promoter is used for *in vitro* transcription of the gRNA, these nucleotides may be any nucleotides present before the 3' end of the DNA template. When a U6 promoter is used for *in vivo* transcription, these nucleotides may be the sequence UUUUUU. When an H1 promoter is used for transcription, these nucleotides may be the sequence UUUU When alternate pol-III promoters are used, these nucleotides may be various numbers of uracil bases depending on, e.g., the termination signal of the pol-III promoter, or they may include alternate bases.

In certain embodiments, the proximal and tail domain taken together comprise, consist of, or consist essentially of the sequence set forth in SEQ ID NOs:32, 33, 34, 35, 36, or 37.

### *4.7 Exemplary unimolecular/chimeric gRNAs*

In certain embodiments, a gRNA as disclosed herein has the structure: 5' [targeting domain]-[first complementarity domain]-[linking domain]-[second complementarity domain]-[proximal domain]-[tail domain]-3', wherein:

the targeting domain comprises a core domain and optionally a secondary domain, and is 10 to 50 nucleotides in length;

the first complementarity domain is 5 to 25 nucleotides in length and, in certain embodiments has at least about 50%, about 60%, about 70%, about 80%, about 85%, about 90%, or about 95% homology with a reference first complementarity domain disclosed herein;

the linking domain is 1 to 5 nucleotides in length;

the second complementarity domain is 5 to 27 nucleotides in length and, in certain embodiments has at least about 50%, about 60%, about 70%, about 80%, about 85%, about 90%, or about 95% homology with a reference second complementarity domain disclosed herein;

the proximal domain is 5 to 20 nucleotides in length and, in certain embodiments has at least about 50%, about 60%, about 70%, about 80%, about 85%, about 90%, or about 95% homology with a reference proximal domain disclosed herein; and

the tail domain is absent or a nucleotide sequence is 1 to 50 nucleotides in length and, in certain embodiments has at least about 50%, about 60%, about 70%, about 80%, about 85%, about 90%, or about 95% homology with a reference tail domain disclosed herein.

In certain embodiments, a unimolecular gRNA as disclosed herein comprises, preferably from 5' to 3':

a targeting domain, e.g., comprising 10-50 nucleotides;
a first complementarity domain, e.g., comprising 15, 16, 17, 18, 19, 20, 21, 22, 23, 24, 25, or 26 nucleotides;
a linking domain;
a second complementarity domain;
a proximal domain; and
a tail domain,

wherein,

(a) the proximal and tail domain, when taken together, comprise at least 15, 18, 20, 25, 30, 31, 35, 40, 45, 49, 50, or 53 nucleotides;

(b) there are at least 15, 18, 20, 25, 30, 31, 35, 40, 45, 49, 50, or 53 nucleotides 3' to the last nucleotide of the second complementarity domain; or

(c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the sequence from (a), (b), and/or (c) has at least about 50%, about 60%, about 70%, about 75%, about 60%, about 70%, about 80%, about 85%, about 90%, about 95%, or about 99% homology with the corresponding sequence of a naturally occurring gRNA, or with a gRNA described herein.

In certain embodiments, the proximal and tail domain, when taken together, comprise at least 15, 18, 20, 25, 30, 31, 35, 40, 45, 49, 50, or 53 nucleotides.

In certain embodiments, there are at least 15, 18, 20, 25, 30, 31, 35, 40, 45, 49, 50, or 53 nucleotides 3' to the last nucleotide of the second complementarity domain.

In certain embodiments, there are at least 16, 19, 21, 26, 31, 32, 36, 41, 46, 50, 51, or 54 nucleotides 3' to the last nucleotide of the second complementarity domain that are complementary to the corresponding nucleotides of the first complementarity domain.

In certain embodiments, the targeting domain consists of, consists essentially of, or comprises 16, 17, 18, 19, 20, 21, 22, 23, 24, 25, or 26 nucleotides (e.g., 16, 17, 18, 19, 20, 21, 22, 23, 24, 25, or 26 consecutive nucleotides) complementary or partially complementary to the target domain or a portion thereof, e.g., the targeting domain is 16, 17, 18, 19, 20, 21, 22, 23, 24, 25, or 26 nucleotides in length. In certain of these embodiments, the targeting domain is complementary to the target domain over the entire length of the targeting domain, the entire length of the target domain, or both.

In certain embodiments, a unimolecular or chimeric gRNA molecule disclosed herein (comprising a targeting domain, a first complementary domain, a linking domain, a second complementary domain, a proximal domain and, optionally, a tail domain) comprises the amino acid sequence set forth in SEQ ID NO:42, wherein the targeting domain is listed as 20 N's (residues 1-20) but may range in length from 16 to 26 nucleotides, and wherein the final six residues (residues 97-102) represent a termination signal for the U6 promoter buy may be absent or fewer in number. In certain embodiments, the unimolecular, or chimeric, gRNA molecule is a *S. pyogenes* gRNA molecule.

In certain embodiments, a unimolecular or chimeric gRNA molecule disclosed herein (comprising a targeting domain, a first complementary domain, a linking domain, a second complementary domain, a proximal domain and, optionally, a tail domain) comprises the amino acid sequence set forth in SEQ ID NO:38, wherein the targeting domain is listed as 20 Ns (residues 1-20) but may range in length from 16 to 26 nucleotides, and wherein the final six residues (residues 97-102) represent a termination signal for the U6 promoter but may be absent or fewer in number. In certain embodiments, the unimolecular or chimeric gRNA molecule is an *S. aureus* gRNA molecule.

The sequences and structures of exemplary chimeric gRNAs are also shown in **Figs. 1H-1I**

### *4.8 Exemplary modular gRNAs*

In certain embodiments, a modular gRNA disclosed herein comprises:

a first strand comprising, preferably from 5' to 3';

a targeting domain, e.g., comprising 15, 16, 17, 18, 19, 20, 21, 22, 23, 24, 25, or 26 nucleotides;
a first complementarity domain; and

a second strand, comprising, preferably from 5' to 3':

optionally a 5' extension domain;
a second complementarity domain;
a proximal domain; and
a tail domain,

wherein:

(a) the proximal and tail domain, when taken together, comprise at least 15, 18, 20, 25, 30, 31, 35, 40, 45, 49, 50, or 53 nucleotides;
(b) there are at least 15, 18, 20, 25, 30, 31, 35, 40, 45, 49, 50, or 53 nucleotides 3' to the last nucleotide of the second complementarity domain; or
(c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the sequence from (a), (b), or (c), has at least about 50%, about 60%, about 70%, about 80%, about 85%, about 90%, about 95%, or about 99% homology with the corresponding sequence of a naturally

occurring gRNA, or with a gRNA described herein.

In certain embodiments, the proximal and tail domain, when taken together, comprise at least 15, 18, 20, 25, 30, 31, 35, 40, 45, 49, 50, or 53 nucleotides.

In certain embodiments, there are at least 15, 18, 20, 25, 30, 31, 35, 40, 45, 49, 50, or 53 nucleotides 3' to the last nucleotide of the second complementarity domain.

In certain embodiments, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 16, 17, 18, 19, 20, 21, 22, 23, 24, 25, or 26 nucleotides (e.g., 16, 17, 18, 19, 20, 21, 22, 23, 24, 25, or 26 consecutive nucleotides) having complementarity with the target domain, e.g., the targeting domain is 16, 17, 18, 19, 20, 21, 22, 23, 24, 25, or 26 nucleotides in length.

In certain embodiments, the targeting domain consists of, consists essentially of, or comprises 16, 17, 18, 19, 20, 21, 22, 23, 24, 25, or 26 nucleotides (e.g., 16, 17, 18, 19, 20, 21, 22, 23, 24, 25, or 26 consecutive nucleotides) complementary to the target domain or a portion thereof. In certain of these embodiments, the targeting domain is complementary to the target domain over the entire length of the targeting domain, the entire length of the target domain, or both.

In certain embodiments, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In certain embodiments, the targeting domain has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In certain embodiments, the targeting domain has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 24 nucleotides (e.g., 24 consecutive nucleotides) having complementarity with the target domain, e.g., the targeting domain is 24 nucleotides in length; and

there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

### *4.9 gRNA delivery*

In certain embodiments of the methods provided herein, the methods comprise delivery of one or more (e.g., two, three, or four) gRNA molecules as described herein. In certain of these embodiments, the gRNA molecules are delivered by intravenous injection, intramuscular injection, subcutaneous injection, or inhalation. In certain embodiments, the gRNA molecules are delivered with a Cas9 molecule in a genome-editing system.

## 5. Methods for Designing gRNAs

Methods for selecting, designing, and validating targeting domains for use in the gRNAs described herein are provided. Exemplary targeting domains for incorporation into gRNAs are also provided herein.

Methods for selection and validation of target sequences as well as off-target analyses have been described previously (see, e.g., Mali 2013; Hsu 2013; Fu 2014; Heigwer 2014; Bae 2014; Xiao 2014). For example, a software tool can be used to optimize the choice of potential targeting domains corresponding to a user's target sequence, e.g., to minimize total off-target activity across the genome. Off-target activity may be other than cleavage. For each possible targeting domain choice using *S. pyogenes* Cas9, the tool can identify all off-target sequences (preceding either NAG or NGG PAMs) across the genome that contain up to certain number (e.g., 1, 2, 3, 4, 5, 6, 7, 8, 9, or 10) of mismatched base-pairs. The cleavage efficiency at each off-target sequence can be predicted, e.g., using an experimentally-derived weighting scheme. Each possible targeting domain is then ranked according to its total predicted off-target cleavage; the top-ranked targeting domains represent those that are likely to have the greatest on-target cleavage and the least off-target cleavage. Other functions, e.g., automated reagent design for CRISPR construction, primer design for the on-target Surveyor assay, and primer design for high-throughput detection and quantification of off-target cleavage via next-gen sequencing, can also be included in the tool. Candidate targeting domains and gRNAs comprising those targeting domains can be functionally evaluated using methods known in the art and/or as set forth herein.

As a non-limiting example, targeting domains for use in gRNAs for use with *S. pyogenes* and *S. aureus* Cas9s were identified using a DNA sequence searching algorithm. 17-mer and 20-mer targeting domains were designed for *S. pyogenes* targets, while 18-mer, 19-mer, 20-mer, 21-mer, 22-mer, 23-mer, and 24-mer targeting domains were

designed for *S. aureus* targets. gRNA design was carried out using custom gRNA design software based on the public tool cas-offinder (Bae 2014). This software scores guides after calculating their genome-wide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential target sites adjacent to PAM sequences, the software also identifies all PAM adjacent sequences that differ by 1, 2, 3, or more than 3 nucleotides from the selected target sites. Genomic DNA sequences for each gene (e.g., *DMD* gene) were obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, targeting domain were ranked into tiers based on their distance to the target site, their orthogonality, and presence of a 5' G (based on identification of close matches in the human genome containing a relevant PAM, e.g., an NGG PAM for *S. pyogenes,* or an NNGRRT (SEQ ID NO:204) or NNGRRV (SEQ ID NO:205) PAM for *S. aureus*). Orthogonality refers to the number of sequences in the human genome that contain a minimum number of mismatches to the target sequence. A "high level of orthogonality" or "good orthogonality" may, for example, refer to 20-mer targeting domain that have no identical sequences in the human genome besides the intended target, nor any sequences that contain one or two mismatches in the target sequence. Targeting domains with good orthogonality are selected to minimize off-target DNA cleavage.

Targeting domains were identified for both single-gRNA nuclease cleavage and for a dual-gRNA paired "nickase" strategy. Criteria for selecting targeting domains and the determination of which targeting domains can be used for the dual-gRNA paired "nickase" strategy is based on two considerations:

(1) Targeting domain pairs should be oriented on the DNA such that PAMs are facing out and cutting with the D10A Cas9 nickase will result in 5' overhangs; and

(2) An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the target site of one targeting domain.

### *5.1 Targeting Domains For Use In Altering A DMD Target Position*

Targeting domains for use in gRNAs for altering a DMD target position in conjunction with the methods disclosed herein were identified and ranked into 4 tiers for *S. pyogenes* and 5 tiers for *S. aureus.*

For *S. pyogenes,* tier 1 targeting domains were selected based on (1) the target site, e.g., an intron (e.g., intron 44, 45, 46, 47, 48, 49, 50, 51, 52, 53, 54, or 55), (2) a high level of orthogonality, and (3) the presence of 5' G. Tier 2 targeting domains were selected based on (1) the target site, e.g., an intron (e.g., intron 44, 45, 46, 47, 48, 49, 50, 51, 52, 53, 54, or 55), and (2) a high level of orthogonality. Tier 3 targeting domains were selected based on (1) the target site, e.g., an intron (e.g., intron 44, 45, 46, 47, 48, 49, 50, 51, 52, 53, 54, or 55) site and (2) the presence of 5' G. Tier 4 targeting domains were selected based on the target site, e.g., an intron (e.g., intron 44, 45, 46, 47, 48, 49, 50, 51, 52, 53, 54, or 55).

For *S. aureus,* tier 1 targeting domains were selected based on (1) the target site, e.g., an intron (e.g., intron 44, 45, 46, 47, 48, 49, 50, 51, 52, 53, 54, or 55), (2) a high level of orthogonality, (3) the presence of 5' G, and (4) PAM having the sequence NNGRRT (SEQ ID NO:204). Tier 2 targeting domains were selected based on (1) the target site, e.g., an intron (e.g., intron 44, 45, 46, 47, 48, 49, 50, 51, 52, 53, 54, or 55), (2) a high level of orthogonality, and (3) PAM having the sequence NNGRRT (SEQ ID NO:204). Tier 3 targeting domains were selected based on (1) the target site, e.g., an intron (e.g., intron 44, 45, 46, 47, 48, 49, 50, 51, 52, 53, 54, or 55), (2) the presence of 5' G, and (3) PAM having the sequence NNGRRT (SEQ ID NO:204). Tier 4 targeting domains were selected based on (1) the target site, e.g., an intron (e.g., intron 44, 45, 46, 47, 48, 49, 50, 51, 52, 53, 54, or 55), and (2) PAM having the sequence NNGRRT (SEQ ID NO:204). Tier 5 targeting domains were selected based on (1) the target site, e.g., an intron (e.g., intron 44, 45, 46, 47, 48, 49, 50, 51, 52, 53, 54, or 55),, and (2) PAM having the sequence NNGRRV (SEQ ID NO:205).

Note that tiers are non-inclusive (each targeting domain is listed only once for the strategy). In certain instances, no targeting domain was identified based on the criteria of the particular tier. The identified targeting domains are summarized below in **Table 1.**

**Table 1. Amino acid sequences of *S. pyogenes* and *S. aureus* targeting domains**

| | ***S. aureus*** | ***S. pyogenes*** |
|---|---|---|
| **Tier 1** | SEQ ID NOs: 206-23994 | SEQ ID NOs: 692253-694972 |

(continued)

| | *S. aureus* | *S. pyogenes* |
|---|---|---|
| **Tier 2** | SEQ ID NOs: 23995-127213 | SEQ ID NOs: 694973-703214 |
| **Tier 3** | SEQ ID NOs: 127214-134855 | SEQ ID NOs: 703215-727252 |
| **Tier 4** | SEQ ID NOs: 134856-167390 | SEQ ID NOs: 727253-826366 |
| **Tier 5** | SEQ ID NOs: 167391-692252 | Not applicable |

In certain embodiments, two or more (e.g., three or four) gRNA molecules are used with one Cas9 molecule. In certain embodiments, when two or more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

Any of the targeting domains in the tables described herein can be used with a Cas9 molecule that generates a single strand break (e.g., *S. pyogenes* or *S. aureus* Cas9 nickase) or with a Cas9 molecule that generates a double strand break (e.g., *S. pyogenes* or *S. aureus* Cas9 nuclease).

In certain embodiments, two gRNAs are used with two Cas9 molecules, the two Cas9 molecules are from different species. Both Cas9 species can be used to generate a single or double-strand break, as desired.

Any upstream gRNA described herein can be paired with any downstream gRNA described herein. When an upstream gRNA designed for use with one species of Cas9 is paired with a downstream gRNA designed for use from a different species of Cas9, both Cas9 species are used to generate a single or double-strand break, as desired.

### *5.2 Deletion Size*

The presently disclosed genome-editing systems and compositions can generate deletions in the *DMD* gene, e.g., the human *DMD* gene. In certain embodiments, the genome-editing system is configured to form two double stand breaks (a first double strand break and a second double strand break) in two introns (a first intron and a second intron) flanking a target position of the *DMD* gene, thereby deleting a segment of the *DMD* gene comprising the DMD target position. The DMD target position can be a DMD exonic target position or a DMD intra-exonic target position. In certain embodiments, the DMD target position is a DMD exonic target position. Deletion of the DMD exonic target position can optimize the DMD sequence of a subject suffering from Duchenne muscular dystrophy, Becker muscular dystrophy (BMD), or Dilated Cardiomyopathy (DCM) Type 3B, e.g., it can increase the function or activity of the encoded dystrophin protein, or results in an improvement in the disease state of the subject. In certain embodiments, excision of the DMD exonic target position restores reading frame. The DMD exonic target position can comprise one or more exons of the *DMD* gene. In certain embodiments, the DMD target position comprises exon 51 of the *DMD* gene (e.g., a human *DMD* gene). In certain embodiments, the DMD target position comprises exons 45-55 of the *DMD* gene (e.g., a human *DMD* gene). In certain embodiments, the DMD target position comprises exons 51-55 of the *DMD* gene (e.g., a human *DMD* gene).

The deletion efficiency of the presently disclosed genome-editing systems and compositions can be related to the deletion size, i.e., the size of the segment deleted by the systems and compositions. In certain embodiments, the length or size of specific deletions is determined by the distance between the PAM sequences in the gene being targeted (e.g., a *DMD* gene). In certain embodiments, a specific deletion of a segment of the *DMD* gene, which is defined in terms of its length and a sequence it comprises (e.g., exon 51), is the result of breaks made adjacent to specific PAM sequences within the target gene (e.g., a *DMD* gene).

In certain embodiments, the deletion size is about 800-72,000 base pairs (bp), e.g., about 800-900, about 900-1000, about 1200-1400, about 1500-2600, about 2600-2700, about 3000-3300, about 5200-5500, about 20,000-30,000, about 35,000-45,000, or about 60,000-72,000. In certain embodiments, the deletion size is about 800-900, about 1500-2600, about 5200-5500, about 20,000-30,000, about 35,000-45,000, or about 60,000-72,000 bp. In certain embodiments, the deletion size is 806 base pairs, 867 base pairs, 1,557 base pairs, 2,527 base pairs, 5,305 base pairs, 5,415 base pairs, 20,768 base pairs, 27,398 base pairs, 36,342 base pairs, 44,269 base pairs, 60,894 base pairs, and 71,832 base pairs. In certain embodiments, the deletion size is about 900-1000, about 1200-1400, about 1500-2600, about 2600-2700 bp, or about 3000-3300. In certain embodiments, the deletion size is selected from the group consisting of 972 bp, 1723 bp, 893 bp, 2665 bp, 1326 bp, 2077 bp, 1247 bp, 3019 bp, 1589 bp, 2340 bp, 1852 bp, and 3282 bp. In certain embodiments, the deletion size is larger than about 150 kilobase pairs (kb), e.g., about 300-400 kb. In certain embodiments, the deletion size is about 300-400 kb. In certain embodiments, the deletion size is 341 kb. In certain embodiments, the deletion size is about 100-150 kb. In certain embodiments, the deletion size is 146,500 bp.

## 6. Cas9 Molecules

Cas9 molecules of a variety of species can be used in the methods and compositions described herein. While *S. pyogenes* and *S. aureus* Cas9 molecules are the subject of much of the disclosure herein, Cas9 molecules of, derived from, or based on the Cas9 proteins of other species listed herein can be used as well. These include, for example, Cas9 molecules from *Acidovorax avenae, Actinobacillus pleuropneumoniae, Actinobacillus succinogenes, Actinobacillus suis, Actinomyces sp., cycliphilus denitrificans, Aminomonas paucivorans, Bacillus cereus, Bacillus smithii, Bacillus thuringiensis, Bacteroides sp., Blastopirellula marina, Bradyrhizobium sp., Brevibacillus laterosporus, Campylobacter coli, Campylobacter jejuni, Campylobacter lari, Candidatus Puniceispirillum, Clostridium cellulolyticum, Clostridium perfringens, Corynebacterium accolens, Corynebacterium diphtheria, Corynebacterium matruchotii, Dinoroseobacter shibae, Eubacterium dolichum, gamma proteobacterium, Gluconacetobacter diazotrophicus, Haemophilus parainfluenzae, Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica, Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tistrella mobilis, Treponema sp.,* or *Verminephrobacter eiseniae.*

### *6.1. Cas9 domains*

Crystal structures have been determined for two different naturally occurring bacterial Cas9 molecules (Jinek 2014) and for *S. pyogenes* Cas9 with a guide RNA (e.g., a synthetic fusion of crRNA and tracrRNA) (Nishimasu 2014; Anders 2014).

A naturally occurring Cas9 molecule comprises two lobes: a recognition (REC) lobe and a nuclease (NUC) lobe; each of which further comprise domains described herein. **Figs. 8A-8B** provide a schematic of the organization of important Cas9 domains in the primary structure. The domain nomenclature and the numbering of the amino acid residues encompassed by each domain used throughout this disclosure is as described previously (Nishimasu 2014). The numbering of the amino acid residues is with reference to Cas9 from *S. pyogenes.*

The REC lobe comprises the arginine-rich bridge helix (BH), the REC1 domain, and the REC2 domain. The REC lobe does not share structural similarity with other known proteins, indicating that it is a Cas9-specific functional domain. The BH domain is a long $\alpha$ helix and arginine rich region and comprises amino acids 60-93 of the sequence *of S. pyogenes* Cas9. The REC1 domain is important for recognition of the repeat: anti-repeat duplex, e.g., of a gRNA or a tracrRNA, and is therefore critical for Cas9 activity by recognizing the target sequence. The REC1 domain comprises two REC1 motifs at amino acids 94 to 179 and 308 to 717 of the sequence *of S. pyogenes* Cas9. These two REC1 domains, though separated by the REC2 domain in the linear primary structure, assemble in the tertiary structure to form the REC1 domain. The REC2 domain, or parts thereof, may also play a role in the recognition of the repeat: anti-repeat duplex. The REC2 domain comprises amino acids 180-307 of the sequence *of S. pyogenes* Cas9.

The NUC lobe comprises the RuvC domain, the HNH domain, and the PAM-interacting (PI) domain. The RuvC domain shares structural similarity to retroviral integrase superfamily members and cleaves a single strand, e.g., the non-complementary strand of the target nucleic acid molecule. The RuvC domain is assembled from the three split RuvC motifs (RuvC I, RuvCII, and RuvCIII, which are often commonly referred to in the art as RuvCI domain, or N-terminal RuvC domain, RuvCII domain, and RuvCIII domain) at amino acids 1-59, 718-769, and 909-1098, respectively, of the sequence of *S. pyogenes* Cas9. Similar to the REC1 domain, the three RuvC motifs are linearly separated by other domains in the primary structure, however in the tertiary structure, the three RuvC motifs assemble and form the RuvC domain. The HNH domain shares structural similarity with HNH endonucleases and cleaves a single strand, e.g., the complementary strand of the target nucleic acid molecule. The HNH domain lies between the RuvC II-III motifs and comprises amino acids 775-908 of the sequence of *S. pyogenes* Cas9. The PI domain interacts with the PAM of the target nucleic acid molecule, and comprises amino acids 1099-1368 of the sequence *of S. pyogenes* Cas9.

#### *6.1.1 RuvC-like domain and HNH-like domain*

In certain embodiments, a Cas9 molecule or Cas9 polypeptide comprises an HNH-like domain and a RuvC-like domain, and in certain of these embodiments cleavage activity is dependent on the RuvC-like domain and the HNH-like domain. A Cas9 molecule or Cas9 polypeptide can comprise one or more of a RuvC-like domain and an HNH-like domain. In certain embodiments, a Cas9 molecule or Cas9 polypeptide comprises a RuvC-like domain, e.g., a RuvC-like domain described below, and/or an HNH-like domain, e.g., an HNH-like domain described below.

*RuvC-like domains*

In certain embodiments, a RuvC-like domain cleaves a single strand, e.g., the non-complementary strand of the target nucleic acid molecule. The Cas9 molecule or Cas9 polypeptide can include more than one RuvC-like domain (e.g., one, two, three or more RuvC-like domains). In certain embodiments, a RuvC-like domain is at least 5, 6, 7, 8 amino acids in length but not more than 20, 19, 18, 17, 16 or 15 amino acids in length. In certain embodiments, the Cas9 molecule or Cas9 polypeptide comprises an N-terminal RuvC-like domain of about 10 to 20 amino acids, e.g., about 15 amino acids in length.

*6.1.2 N-terminal RuvC-like domains*

Some naturally occurring Cas9 molecules comprise more than one RuvC-like domain with cleavage being dependent on the N-terminal RuvC-like domain. Accordingly, a Cas9 molecule or Cas9 polypeptide can comprise an N-terminal RuvC-like domain. Exemplary N-terminal RuvC-like domains are described below.

In certain embodiments, a Cas9 molecule or Cas9 polypeptide comprises an N-terminal RuvC-like domain comprising an amino acid sequence of Formula I:

D-$X_1$-G-$X_2$-$X_3$-$X_4$-$X_5$-G-$X_6$-$X_7$-$X_8$-$X_9$ (SEQ ID NO:20), wherein,

$X_1$ is selected from I, V, M, L, and T (e.g., selected from I, V, and L);
$X_2$ is selected from T, I, V, S, N, Y, E, and L (e.g., selected from T, V, and I);
$X_3$ is selected from N, S, G, A, D, T, R, M, and F (e.g., A or N);
$X_4$ is selected from S, Y, N, and F (e.g., S);
$X_5$ is selected from V, I, L, C, T, and F (e.g., selected from V, I and L);
$X_6$ is selected from W, F, V, Y, S, and L (e.g., W);
$X_7$ is selected from A, S, C, V, and G (e.g., selected from A and S);
$X_8$ is selected from V, I, L, A, M, and H (e.g., selected from V, I, M and L); and
$X_9$ is selected from any amino acid or is absent (e.g., selected from T, V, I, L, Δ, F, S, A, Y, M, and R, or, e.g., selected from T, V, I, L, and Δ).
In certain embodiments, the N-terminal RuvC-like domain differs from a sequence of SEQ ID NO:20 by as many as 1 but no more than 2, 3, 4, or 5 residues.

In certain embodiments, the N-terminal RuvC-like domain is cleavage competent. In other embodiments, the N-terminal RuvC-like domain is cleavage incompetent.

In certain embodiments, a Cas9 molecule or Cas9 polypeptide comprises an N-terminal RuvC-like domain comprising an amino acid sequence of Formula II:

D-$X_1$-G-$X_2$-$X_3$-S-$X_5$-G-$X_6$-$X_7$-$X_8$-$X_9$, (SEQ ID NO:21), wherein

$X_1$ is selected from I, V, M, L, and T (e.g., selected from I, V, and L);
$X_2$ is selected from T, I, V, S, N, Y, E, and L (e.g., selected from T, V, and I);
$X_3$ is selected from N, S, G, A, D, T, R, M and F (e.g., A or N);
$X_5$ is selected from V, I, L, C, T, and F (e.g., selected from V, I and L);
$X_6$ is selected from W, F, V, Y, S, and L (e.g., W);
$X_7$ is selected from A, S, C, V, and G (e.g., selected from A and S);
$X_8$ is selected from V, I, L, A, M, and H (e.g., selected from V, I, M and L); and
$X_9$ is selected from any amino acid or is absent (e.g., selected from T, V, I, L, Δ, F, S, A, Y, M, and R or selected from e.g., T, V, I, L, and Δ).

In certain embodiments, the N-terminal RuvC-like domain differs from a sequence of SEQ ID NO:21 by as many as 1 but not more than 2, 3, 4, or 5 residues. In certain embodiments, the N-terminal RuvC-like domain comprises an amino acid sequence of Formula III:

D-I-G-$X_2$-$X_3$-S-V-G-W-A-$X_8$-$X_9$ (SEQ ID NO:22), wherein

$X_2$ is selected from T, I, V, S, N, Y, E, and L (e.g., selected from T, V, and I);
$X_3$ is selected from N, S, G, A, D, T, R, M, and F (e.g., A or N);
$X_8$ is selected from V, I, L, A, M, and H (e.g., selected from V, I, M and L); and
$X_9$ is selected from any amino acid or is absent (e.g., selected from T, V, I, L, Δ, F, S, A, Y, M, and R or selected from e.g., T, V, I, L, and Δ).

In certain embodiments, the N-terminal RuvC-like domain differs from a sequence of SEQ ID NO:22 by as many as 1 but not more than, 2, 3, 4, or 5 residues.

In certain embodiments, the N-terminal RuvC-like domain comprises an amino acid sequence of Formula IV: D-I-G-T-N-S-V-G-W-A-V-X (SEQ ID NO:23), wherein
X is a non-polar alkyl amino acid or a hydroxyl amino acid, e.g., X is selected from V, I, L, and T (e.g., the Cas9 molecule can comprise an N-terminal RuvC-like domain shown in **Figs. 2A-2G** (depicted as Y)).

In certain embodiments, the N-terminal RuvC-like domain differs from a sequence of SEQ ID NO:23 by as many as 1 but not more than, 2, 3, 4, or 5 residues.

In certain embodiments, the N-terminal RuvC-like domain differs from a sequence of an N-terminal RuvC like domain disclosed herein, e.g., in **Figs. 3A-3B**, as many as 1 but no more than 2, 3, 4, or 5 residues. In an embodiment, 1, 2, 3 or all of the highly conserved residues identified in **Figs. 3A-3B** are present.

In certain embodiments, the N-terminal RuvC-like domain differs from a sequence of an N-terminal RuvC-like domain disclosed herein, e.g., in **Figs. 4A-4B**, as many as 1 but no more than 2, 3, 4, or 5 residues. In an embodiment, 1, 2, or all of the highly conserved residues identified in **Figs. 4A-4B** are present.

*6.1.3 Additional RuvC-like domains*

In addition to the N-terminal RuvC-like domain, the Cas9 molecule or Cas9 polypeptide can comprise one or more additional RuvC-like domains. In certain embodiments, the Cas9 molecule or Cas9 polypeptide comprises two additional RuvC-like domains. In certain embodiments, the additional RuvC-like domain is at least 5 amino acids in length and, e.g., less than 15 amino acids in length, e.g., 5 to 10 amino acids in length, e.g., 8 amino acids in length.

An additional RuvC-like domain can comprise an amino acid sequence of Formula V:
I-$X_1$-$X_2$-E-$X_3$-A-R-E (SEQ ID NO: 15) wherein,

$X_1$ is V or H;
$X_2$ is I, L or V (e.g., I or V); and
$X_3$ is M or T.

In certain embodiments, the additional RuvC-like domain comprises an amino acid sequence of Formula VI: I-V-$X_2$-E-M-A-R-E (SEQ ID NO:16), wherein
$X_2$ is I, L or V (e.g., I or V) (e.g., the Cas9 molecule or Cas9 polypeptide can comprise an additional RuvC-like domain shown in **Fig. 2A-2G** (depicted as B)).

An additional RuvC-like domain can comprise an amino acid sequence of Formula VII:
H-H-A-$X_1$-D-A-$X_2$-$X_3$ (SEQ ID NO: 17), wherein

$X_1$ is H or L;
$X_2$ is R or V; and
$X_3$ is E or V.

In certain embodiments, the additional RuvC-like domain comprises the amino acid sequence: H-H-A-H-D-A-Y-L (SEQ ID NO:18).

In certain embodiments, the additional RuvC-like domain differs from a sequence of SEQ ID NOs: 15-18 by as many as 1 but not more than 2, 3, 4, or 5 residues.

In certain embodiments, the sequence flanking the N-terminal RuvC-like domain has the amino acid sequence of Formula VIII:
K-$X_1$'-Y-$X_2$'-$X_3$'-$X_4$'-Z-T-D-$X_9$'-Y (SEQ ID NO:19), wherein

$X_1$' is selected from K and P;
$X_2$' is selected from V, L, I, and F (e.g., V, I and L);
$X_3$' is selected from G, A and S (e.g., G);
$X_4$' is selected from L, I, V, and F (e.g., L);
$X_9$' is selected from D, E, N, and Q; and
Z is an N-terminal RuvC-like domain, e.g., as described above, e.g., having 5 to 20 amino acids.

*6.1.4 HNH-like domains*

In an embodiment, an HNH-like domain cleaves a single stranded complementary domain, e.g., a complementary strand of a double stranded nucleic acid molecule. In certain embodiments, an HNH-like domain is at least 15, 20,

or 25 amino acids in length but not more than 40, 35, or 30 amino acids in length, e.g., 20 to 35 amino acids in length, e.g., 25 to 30 amino acids in length. Exemplary HNH-like domains are described below.

In certain embodiments, a Cas9 molecule or Cas9 polypeptide comprises an HNH-like domain having an amino acid sequence of Formula IX:

$X_1$-$X_2$-$X_3$-H-$X_4$-$X_5$-P-$X_6$-$X_7$-$X_8$-$X^9$-$X^{10}$-$X^{11}$-$X^{12}$-$X^{13}$-$X^{14}$-$X^{15}$-N-$X^{16}$-$X^{17}$-$X^{18}$-$X^{19}$-$X_{20}$-$X_{21}$-$X_{22}$-$X_{23}$-N (SEQ ID NO:25), wherein

$X_1$ is selected from D, E, Q and N (e.g., D and E);
$X^2$ is selected from L, I, R, Q, V, M, and K;
$X_3$ is selected from D and E;
$X_4$ is selected from I, V, T, A, and L (e.g., A, I and V);
$X_5$ is selected from V, Y, I, L, F, and W (e.g., V, I and L);
$X_6$ is selected from Q, H, R, K, Y, I, L, F, and W;
$X_7$ is selected from S, A, D, T, and K (e.g., S and A);
$X_8$ is selected from F, L, V, K, Y, M, I, R, A, E, D, and Q (e.g., F);
$X_9$ is selected from L, R, T, I, V, S, C, Y, K, F, and G;
$X_{10}$ is selected from K, Q, Y, T, F, L, W, M, A, E, G, and S;
$X_{11}$ is selected from D, S, N, R, L, and T (e.g., D);
$X_{12}$ is selected from D, N and S;
$X_{13}$ is selected from S, A, T, G, and R (e.g., S);
$X_{14}$ is selected from I, L, F, S, R, Y, Q, W, D, K, and H (e.g., I, L and F);
$X_{15}$ is selected from D, S, I, N, E, A, H, F, L, Q, M, G, Y, and V;
$X_{16}$ is selected from K, L, R, M, T, and F (e.g., L, R and K);
$X_{17}$ is selected from V, L, I, A and T;
$X_{18}$ is selected from L, I, V, and A (e.g., L and I);
$X_{19}$ is selected from T, V, C, E, S, and A (e.g., T and V);
$X_{20}$ is selected from R, F, T, W, E, L, N, C, K, V, S, Q, I, Y, H, and A;
$X_{21}$ is selected from S, P, R, K, N, A, H, Q, G, and L;
$X_{22}$ is selected from D, G, T, N, S, K, A, I, E, L, Q, R, and Y; and $X_{23}$ is selected from K, V, A, E, Y, I, C, L, S, T, G, K, M, D, and F.

In certain embodiments, a HNH-like domain differs from a sequence of SEQ ID NO:25 by at least one but not more than, 2, 3, 4, or 5 residues.

In certain embodiments, the HNH-like domain is cleavage competent. In certain embodiments, the HNH-like domain is cleavage incompetent.

In certain embodiments, a Cas9 molecule or Cas9 polypeptide comprises an HNH-like domain comprising an amino acid sequence of Formula X:

$$X_1\text{-}X_2\text{-}X_3\text{-H-}X_4\text{-}X_5\text{-P-}X_6\text{-S-}X_8\text{-}X_9\text{-}X_{10}\text{-D-D-S-}X_{14}\text{-}X_{15}\text{-N-K-V-L-}X_{19}\text{-}X_{20}\text{-}X_{21}\text{-}X_{22}\text{-}X_{23}\text{-N (SEQ ID NO:26)},$$

wherein

$X_1$ is selected from D and E;
$X_2$ is selected from L, I, R, Q, V, M, and K;
$X_3$ is selected from D and E;
$X_4$ is selected from I, V, T, A, and L (e.g., A, I and V);
$X_5$ is selected from V, Y, I, L, F, and W (e.g., V, I and L);
$X_6$ is selected from Q, H, R, K, Y, I, L, F, and W;
$X_8$ is selected from F, L, V, K, Y, M, I, R, A, E, D, and Q (e.g., F);
$X_9$ is selected from L, R, T, I, V, S, C, Y, K, F, and G;
$X_{10}$ is selected from K, Q, Y, T, F, L, W, M, A, E, G, and S;
$X_{14}$ is selected from I, L, F, S, R, Y, Q, W, D, K and H (e.g., I, L and F);
$X_{15}$ is selected from D, S, I, N, E, A, H, F, L, Q, M, G, Y, and V;
$X_{19}$ is selected from T, V, C, E, S, and A (e.g., T and V);
$X_{20}$ is selected from R, F, T, W, E, L, N, C, K, V, S, Q, I, Y, H, and A;

$X_{21}$ is selected from S, P, R, K, N, A, H, Q, G, and L;
$X_{22}$ is selected from D, G, T, N, S, K, A, I, E, L, Q, R, and Y; and
$X_{23}$ is selected from K, V, A, E, Y, I, C, L, S, T, G, K, M, D, and F.

In certain embodiment, the HNH-like domain differs from a sequence of SEQ ID NO:26 by 1, 2, 3, 4, or 5 residues.

In certain embodiments, a Cas9 molecule or Cas9 polypeptide comprises an HNH-like domain comprising an amino acid sequence of Formula XI:

$$X_1\text{-V-}X_3\text{-H-I-V-P-}X_6\text{-S-}X_8\text{-}X_9\text{-}X_{10}\text{-D-D-S-}X_{14}\text{-}X_{15}\text{-N-K-V-L-T-}X_{20}\text{-}X_{21}\text{-}X_{22}\text{-}X_{23}\text{-N (SEQ ID NO:27),}$$

wherein

$X_1$ is selected from D and E;
$X_3$ is selected from D and E;
$X_6$ is selected from Q, H, R, K, Y, I, L, and W;
$X_8$ is selected from F, L, V, K, Y, M, I, R, A, E, D, and Q (e.g., F);
$X_9$ is selected from L, R, T, I, V, S, C, Y, K, F, and G;
$X_{10}$ is selected from K, Q, Y, T, F, L, W, M, A, E, G, and S;
$X_{14}$ is selected from I, L, F, S, R, Y, Q, W, D, K, and H (e.g., I, L and F);
$X_{15}$ is selected from D, S, I, N, E, A, H, F, L, Q, M, G, Y, and V;
$X_{20}$ is selected from R, F, T, W, E, L, N, C, K, V, S, Q, I, Y, H, and A;
$X_{21}$ is selected from S, P, R, K, N, A, H, Q, G, and L;
$X_{22}$ is selected from D, G, T, N, S, K, A, I, E, L, Q, R, and Y; and
$X_{23}$ is selected from K, V, A, E, Y, I, C, L, S, T, G, K, M, D, and F.

In certain embodiments, the HNH-like domain differs from a sequence of SEQ ID NO:27 by 1, 2, 3, 4, or 5 residues.

In certain embodiments, a Cas9 molecule or Cas9 polypeptide comprises an HNH-like domain having an amino acid sequence of Formula XII:

$$\text{D-}X_2\text{-D-H-I-}X_5\text{-P-Q-}X_7\text{-F-}X_9\text{-}X_{10}\text{-D-}X_{12}\text{-S-I-D-N-}X_{16}\text{-V-L-}X_{19}\text{-}X_{20}\text{-S-}X_{22}\text{-}X_{23}\text{-N (SEQ ID NO:28),}$$

wherein

$X_2$ is selected from I and V;
$X_5$ is selected from I and V;
$X_7$ is selected from A and S;
$X_9$ is selected from I and L;
$X_{10}$ is selected from K and T;
$X_{12}$ is selected from D and N;
$X_{16}$ is selected from R, K, and L;
$X_{19}$ is selected from T and V;
$X_{20}$ is selected from S, and R;
$X_{22}$ is selected from K, D, and A; and
$X_{23}$ is selected from E, K, G, and N (e.g., the Cas9 molecule or Cas9 polypeptide can comprise an HNH-like domain as described herein).

In certain embodiments, the HNH-like domain differs from a sequence of SEQ ID NO:28 by as many as 1 but no more than 2, 3, 4, or 5 residues.

In certain embodiments, a Cas9 molecule or Cas9 polypeptide comprises the amino acid sequence of Formula XIII:

$$L\text{-}Y\text{-}Y\text{-}L\text{-}Q\text{-}N\text{-}G\text{-}X_1'\text{-}D\text{-}M\text{-}Y\text{-}X_2'\text{-}X_3'\text{-}X_4'\text{-}X_5'\text{-}L\text{-}D\text{-}I\text{-}X_6'\text{-}X_7'\text{-}L\text{-}S\text{-}X_8'\text{-}Y\text{-}Z\text{-}N\text{-}R\text{-}X_9'\text{-}K\text{-}X_{10}'\text{-}D\text{-}X_{11}'\text{-}V\text{-}P \text{ (SEQ ID NO:24)},$$

wherein

$X_1$' is selected from K and R;
$X_2$' is selected from V and T;
$X_3$' is selected from G and D;
$X_4$' is selected from E, Q and D;
$X_5$' is selected from E and D;
$X_6$' is selected from D, N, and H;
$X_7$' is selected from Y, R, and N;
$X_8$' is selected from Q, D, and N;
$X_9$' is selected from G and E;
$X_{10}$' is selected from S and G;
$X_{11}$' is selected from D and N; and
Z is an HNH-like domain, e.g., as described above.

In certain embodiments, the Cas9 molecule or Cas9 polypeptide comprises an amino acid sequence that differs from a sequence of SEQ ID NO:24 by as many as 1 but not more than 2, 3, 4, or 5 residues.

In certain embodiments, the HNH-like domain differs from a sequence of an HNH-like domain disclosed herein, e.g., in **Figs. 5A-5C**, by as many as 1 but not more than 2, 3, 4, or 5 residues. In certain embodiments, 1 or both of the highly conserved residues identified in **Figs. 5A-5C** are present.

In certain embodiments, the HNH -like domain differs from a sequence of an HNH-like domain disclosed herein, e.g., in **Figs. 6A-6B,** by as many as 1 but not more than 2, 3, 4, or 5 residues. In an embodiment, 1, 2, or all 3 of the highly conserved residues identified in **Figs. 6A-6B** are present.

### *6.2 Cas9 Activities*

In certain embodiments, the Cas9 molecule or Cas9 polypeptide is capable of cleaving a target nucleic acid molecule. Typically wild-type Cas9 molecules cleave both strands of a target nucleic acid molecule. Cas9 molecules and Cas9 polypeptides can be engineered to alter nuclease cleavage (or other properties), e.g., to provide a Cas9 molecule or Cas9 polypeptide which is a nickase, or which lacks the ability to cleave target nucleic acid. A Cas9 molecule or Cas9 polypeptide that is capable of cleaving a target nucleic acid molecule is referred to herein as an eaCas9 (an enzymatically active Cas9) molecule or eaCas9 polypeptide.

In certain embodiments, an eaCas9 molecule or eaCas9 polypeptide comprises one or more of the following enzymatic activities:

a nickase activity, i.e., the ability to cleave a single strand, e.g., the non-complementary strand or the complementary strand, of a nucleic acid molecule;
a double stranded nuclease activity, i.e., the ability to cleave both strands of a double stranded nucleic acid and create a double stranded break, which in an embodiment is the presence of two nickase activities;
an endonuclease activity;
an exonuclease activity; and
a helicase activity, i.e., the ability to unwind the helical structure of a double stranded nucleic acid.

In certain embodiments, an enzymatically active Cas9 ("eaCas9") molecule or eaCas9 polypeptide cleaves both DNA strands and results in a double stranded break. In certain embodiments, an eaCas9 molecule or eaCas9 polypeptide cleaves only one strand, e.g., the strand to which the gRNA hybridizes to, or the strand complementary to the strand the gRNA hybridizes with. In certain embodiments, an eaCas9 molecule or eaCas9 polypeptide comprises cleavage activity associated with an HNH domain. In certain embodiments, an eaCas9 molecule or eaCas9 polypeptide comprises cleavage activity associated with a RuvC domain. In certain embodiments, an eaCas9 molecule or eaCas9 polypeptide comprises cleavage activity associated with an HNH domain and cleavage activity associated with a RuvC domain. In certain embodiments, an eaCas9 molecule or eaCas9 polypeptide comprises an active, or cleavage competent, HNH domain and an inactive, or cleavage incompetent, RuvC domain. In certain embodiments, an eaCas9 molecule or eaCas9 polypeptide comprises an inactive, or cleavage incompetent, HNH domain and an active, or cleavage

competent, RuvC domain.

### *6.3 Targeting and PAMs*

A Cas9 molecule or Cas9 polypeptide can interact with a gRNA molecule and, in concert with the gRNA molecule, localizes to a site which comprises a target domain, and in certain embodiments, a PAM sequence.

In certain embodiments, the ability of an eaCas9 molecule or eaCas9 polypeptide to interact with and cleave a target nucleic acid is PAM sequence dependent. A PAM sequence is a sequence in the target nucleic acid. In certain embodiments, cleavage of the target nucleic acid occurs upstream from the PAM sequence. eaCas9 molecules from different bacterial species can recognize different sequence motifs (e.g., PAM sequences). In certain embodiments, an eaCas9 molecule *of S. pyogenes* recognizes the sequence motif NGG and directs cleavage of a target nucleic acid sequence 1 to 10, e.g., 3 to 5, bp upstream from that sequence (see, e.g., Mali 2013). In certain embodiments, an eaCas9 molecule of *S. thermophilus* recognizes the sequence motif NGGNG (SEQ ID NO:199) and/or NNAGAAW (W = A or T) (SEQ ID NO:200) and directs cleavage of a target nucleic acid sequence 1 to 10, e.g., 3 to 5, bp upstream from these sequences (see, e.g., Horvath 2010; Deveau 2008). In certain embodiments, an eaCas9 molecule of *S. mutans* recognizes the sequence motif NGG and/or NAAR (R = A or G) (SEQ ID NO:201) and directs cleavage of a target nucleic acid sequence 1 to 10, e.g., 3 to 5 bp, upstream from this sequence (see, e.g., Deveau 2008). In certain embodiments, an eaCas9 molecule of *S. aureus* recognizes the sequence motif NNGRR (R = A or G) (SEQ ID NO:202) and directs cleavage of a target nucleic acid sequence 1 to 10, e.g., 3 to 5, bp upstream from that sequence. In certain embodiments, an eaCas9 molecule of *S. aureus* recognizes the sequence motif NNGRRN (R = A or G) (SEQ ID NO:203) and directs cleavage of a target nucleic acid sequence 1 to 10, e.g., 3 to 5, bp upstream from that sequence. In certain embodiments, an eaCas9 molecule of *S. aureus* recognizes the sequence motif NNGRRT (R = A or G) (SEQ ID NO:204) and directs cleavage of a target nucleic acid sequence 1 to 10, e.g., 3 to 5, bp upstream from that sequence. In certain embodiments, an eaCas9 molecule of *S. aureus* recognizes the sequence motif NNGRRV (R = A or G) (SEQ ID NO:205) and directs cleavage of a target nucleic acid sequence 1 to 10, e.g., 3 to 5, bp upstream from that sequence. The ability of a Cas9 molecule to recognize a PAM sequence can be determined, e.g., using a transformation assay as described previously (Jinek 2012). In the aforementioned embodiments, N can be any nucleotide residue, e.g., any of A, G, C, or T.

As is discussed herein, Cas9 molecules can be engineered to alter the PAM specificity of the Cas9 molecule.

Exemplary naturally occurring Cas9 molecules have been described previously (see, e.g., Chylinski 2013). Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: *S. aureus, S. pyogenes* (e.g., strain SF370, MGAS10270, MGAS10750, MGAS2096, MGAS315, MGAS5005, MGAS6180, MGAS9429, NZ131 and SSI-1), *S. thermophilus* (e.g., strain LMD-9), *S. pseudoporcinus* (e.g., strain SPIN 20026), *S. mutans* (e.g., strain UA159, NN2025), *S. macacae* (e.g., strain NCTC11558), *S. gallolyticus* (e.g., strain UCN34, ATCC BAA-2069), *S. equines* (e.g., strain ATCC 9812, MGCS 124), *S. dysdalactiae* (e.g., strain GGS 124), *S. bovis* (e.g., strain ATCC 700338), *S. anginosus* (e.g., strain F0211), *S. agalactiae* (e.g., strain NEM316, A909), *Listeria monocytogenes* (e.g., strain F6854), *Listeria innocua* (*L. innocua,* e.g., strain Clip11262), *Enterococcus italicus* (e.g., strain DSM 15952), or *Enterococcus faecium* (e.g., strain 1,231,408).

In certain embodiments, a Cas9 molecule or Cas9 polypeptide comprises an amino acid sequence:

having about 60%, about 65%, about 70%, about 75%, about 80%, about 85%, about 90%, about 95%, about 96%, about 97%, about 98% or about 99% homology with;
differs at no more than, about 2%, about 5%, about 10%, about 15%, about 20%, about 30%, or about 40% of the amino acid residues when compared with;
differs by at least 1, 2, 5, 10 or 20 amino acids, but by no more than 100, 80, 70, 60, 50, 40 or 30 amino acids from; or identical to any Cas9 molecule sequence described herein, or to a naturally occurring Cas9 molecule sequence, e.g., a Cas9 molecule from a species listed herein (e.g., SEQ ID NOs:1, 2, 4-6, or 12) or described in Chylinski 2013. In certain embodiments, the Cas9 molecule or Cas9 polypeptide comprises one or more of the following activities: a nickase activity; a double stranded cleavage activity (e.g., an endonuclease and/or exonuclease activity); a helicase activity; or the ability, together with a gRNA molecule, to localize to a target nucleic acid.

In certain embodiments, a Cas9 molecule or Cas9 polypeptide comprises any of the amino acid sequence of the consensus sequence of **Figs. 2A-2G**, wherein "*" indicates any amino acid found in the corresponding position in the amino acid sequence of a Cas9 molecule of *S. pyogenes, S. thermophilus, S. mutans,* or *L. innocua,* and "-" indicates absent. In certain embodiments, a Cas9 molecule or Cas9 polypeptide differs from the sequence of the consensus sequence disclosed in **Figs. 2A-2G** by at least 1, but no more than 2, 3, 4, 5, 6, 7, 8, 9, or 10 amino acid residues. In certain embodiments, a Cas9 molecule or Cas9 polypeptide comprises the amino acid sequence of SEQ ID NO:2. In other embodiments, a Cas9 molecule or Cas9 polypeptide differs from the sequence of SEQ ID NO:2 by at least 1, but no more than 2, 3, 4, 5, 6, 7, 8, 9, or 10 amino acid residues.

A comparison of the sequence of a number of Cas9 molecules indicate that certain regions are conserved. These are identified below as:

region 1 (residues 1 to 180, or in the case of region 1’residues 120 to 180)
region 2 (residues 360 to 480);
region 3 (residues 660 to 720);
region 4 (residues 817 to 900); and
region 5 (residues 900 to 960).

In certain embodiments, a Cas9 molecule or Cas9 polypeptide comprises regions 1-5, together with sufficient additional Cas9 molecule sequence to provide a biologically active molecule, e.g., a Cas9 molecule having at least one activity described herein. In certain embodiments, each of regions 1-5, independently, have about 50%, about 60%, about 70%, about 80%, about 85%, about 90%, about 95%, about 96%, about 97%, about 98% or about 99% homology with the corresponding residues of a Cas9 molecule or Cas9 polypeptide described herein, e.g., a sequence from **Figs. 2A-2G.**

In certain embodiments, a Cas9 molecule or Cas9 polypeptide comprises an amino acid sequence referred to as region 1:

having about 50%, about 60%, about 70%, about 80%, about 85%, about 90%, about 95%, about 96%, about 97%, about 98% or about 99% homology with amino acids 1-180 (the numbering is according to the motif sequence in **Fig. 2**; 52% of residues in the four Cas9 sequences in **Figs. 2A-2G** are conserved) of the amino acid sequence of Cas9 of *S. pyogenes*;
differs by at least 1, 2, 5, 10 or 20 amino acids but by no more than 90, 80, 70, 60, 50, 40 or 30 amino acids from amino acids 1-180 of the amino acid sequence of Cas9 of *S. pyogenes, S. thermophilus, S. mutans,* or *Listeria innocua*; or
is identical to amino acids 1-180 of the amino acid sequence of Cas9 of *S. pyogenes, S. thermophilus, S. mutans,* or *L. innocua.*

In certain embodiments, a Cas9 molecule or Cas9 polypeptide comprises an amino acid sequence referred to as region 1’:

having about 55%, about 60%, about 65%, about 70%, about 75%, about 80%, about 85%, about 90%, about 95%, about 96%, about 97%, about 98% or about 99% homology with amino acids 120-180 (55% of residues in the four Cas9 sequences in Fig. 2 are conserved) of the amino acid sequence of Cas9 of *S. pyogenes, S. thermophilus, S. mutans* or *L. innocua*;
differs by at least 1, 2, or 5 amino acids but by no more than 35, 30, 25, 20 or 10 amino acids from amino acids 120-180 of the amino acid sequence of Cas9 of *S. pyogenes, S. thermophilus, S. mutans,* or *L. innocua* ; or
is identical to amino acids 120-180 of the amino acid sequence of Cas9 of *S. pyogenes, S. thermophilus, S. mutans,* or *L. innocua.*

In certain embodiments, a Cas9 molecule or Cas9 polypeptide comprises an amino acid sequence referred to as region 2:

having about 50%, about 55%, about 60%, about 65%, about 70%, about 75%, about 80%, about 85%, about 90%, about 95%, about 96%, about 97%, about 98% or about 99% homology with amino acids 360-480 (52% of residues in the four Cas9 sequences in Fig. 2 are conserved) of the amino acid sequence of Cas9 of *S. pyogenes, S. thermophilus, S. mutans,* or *L. innocua*;

differs by at least 1, 2, or 5 amino acids but by no more than 35, 30, 25, 20 or 10 amino acids from amino acids 360-480 of the amino acid sequence of Cas9 of *S. pyogenes, S. thermophilus, S. mutans,* or *L. innocua*; or

is identical to amino acids 360-480 of the amino acid sequence of Cas9 of *S. pyogenes, S. thermophilus, S. mutans,* or *L. innocua*.

In certain embodiments, a Cas9 molecule or Cas9 polypeptide comprises an amino acid sequence referred to as region 3:

having about 55%, about 60%, about 65%, about 70%, about 75%, about 80%, about 85%, about 90%, about 95%, about 96%, about 97%, about 98%, or about 99% homology with amino acids 660-720 (56% of residues in the four Cas9 sequences in Fig. 2 are conserved) of the amino acid sequence of Cas9 of *S. pyogenes, S. thermophilus, S. mutans* or *L. innocua*;

differs by at least 1, 2, or 5 amino acids but by no more than 35, 30, 25, 20 or 10 amino acids from amino acids 660-720 of the amino acid sequence of Cas9 of *S. pyogenes, S. thermophilus, S. mutans* or *L. innocua*; or

is identical to amino acids 660-720 of the amino acid sequence of Cas9 of *S. pyogenes, S. thermophilus, S. mutans* or *L. innocua*.

In certain embodiments, a Cas9 molecule or Cas9 polypeptide comprises an amino acid sequence referred to as region 4:

having about 50%, about 55%, about 60%, about 65%, about 70%, about 75%, about 80%, about 85%, about 90%, about 95%, about 96%, about 97%, about 98%, or about 99% homology with amino acids 817-900 (55% of residues in the four Cas9 sequences in **Figs. 2A-2G** are conserved) of the amino acid sequence of Cas9 of *S. pyogenes, S. thermophilus, S. mutans,* or *L. innocua*;

differs by at least 1, 2, or 5 amino acids but by no more than 35, 30, 25, 20 or 10 amino acids from amino acids 817-900 of the amino acid sequence of Cas9 of *S. pyogenes, S. thermophilus, S. mutans,* or *L. innocua*; or

is identical to amino acids 817-900 of the amino acid sequence of Cas9 of *S. pyogenes, S. thermophilus, S. mutans,* or *L. innocua.*

In certain embodiments, a Cas9 molecule or Cas9 polypeptide comprises an amino acid sequence referred to as region 5:

having about 50%, about 55%, about 60%, about 65%, about 70%, about 75%, about 80%, about 85%, about 90%, about 95%, about 96%, about 97%, about 98%, or about 99% homology with amino acids 900-960 (60% of residues in the four Cas9 sequences in **Figs. 2A-2G** are conserved) of the amino acid sequence of Cas9 of *S. pyogenes, S. thermophilus, S. mutans,* or *L. innocua*;

differs by at least 1, 2, or 5 amino acids but by no more than 35, 30, 25, 20 or 10 amino acids from amino acids 900-960 of the amino acid sequence of Cas9 of *S. pyogenes, S. thermophilus, S. mutans,* or *L. innocua*; or

is identical to amino acids 900-960 of the amino acid sequence of Cas9 of *S. pyogenes, S. thermophilus, S. mutans,* or *L. innocua*.

### *6.4 Engineered or altered Cas9*

Cas9 molecules and Cas9 polypeptides described herein can possess any of a number of properties, including nuclease activity (e.g., endonuclease and/or exonuclease activity); helicase activity; the ability to associate functionally with a gRNA molecule; and the ability to target (or localize to) a site on a nucleic acid (e.g., PAM recognition and specificity). In certain embodiments, a Cas9 molecule or Cas9 polypeptide can include all or a subset of these properties. In certain embodiments, a Cas9 molecule or Cas9 polypeptide has the ability to interact with a gRNA molecule and, in concert with the gRNA molecule, localize to a site in a nucleic acid. Other activities, e.g., PAM specificity, cleavage activity, or helicase activity can vary more widely in Cas9 molecules and Cas9 polypeptides.

Cas9 molecules include engineered Cas9 molecules and engineered Cas9 polypeptides (engineered, as used

in this context, means merely that the Cas9 molecule or Cas9 polypeptide differs from a reference sequences, and implies no process or origin limitation). An engineered Cas9 molecule or Cas9 polypeptide can comprise altered enzymatic properties, e.g., altered nuclease activity, (as compared with a naturally occurring or other reference Cas9 molecule) or altered helicase activity. As discussed herein, an engineered Cas9 molecule or Cas9 polypeptide can have nickase activity (as opposed to double strand nuclease activity). In certain embodiments, an engineered Cas9 molecule or Cas9 polypeptide can have an alteration that alters its size, e.g., a deletion of amino acid sequence that reduces its size, e.g., without significant effect on one or more, or any Cas9 activity. In certain embodiments, an engineered Cas9 molecule or Cas9 polypeptide can comprise an alteration that affects PAM recognition. In certain embodiments, an engineered Cas9 molecule is altered to recognize a PAM sequence other than that recognized by the endogenous wild-type PI domain. In certain embodiments, a Cas9 molecule or Cas9 polypeptide can differ in sequence from a naturally occurring Cas9 molecule but not have significant alteration in one or more Cas9 activities.

Cas9 molecules or Cas9 polypeptides with desired properties can be made in a number of ways, e.g., by alteration of a parental, e.g., naturally occurring, Cas9 molecules or Cas9 polypeptides, to provide an altered Cas9 molecule or Cas9 polypeptide having a desired property. For example, one or more mutations or differences relative to a parental Cas9 molecule, e.g., a naturally occurring or engineered Cas9 molecule, can be introduced. Such mutations and differences comprise: substitutions (e.g., conservative substitutions or substitutions of non-essential amino acids); insertions; or deletions. In certain embodiments, a Cas9 molecule or Cas9 polypeptide can comprises one or more mutations or differences, e.g., at least 1, 2, 3, 4, 5, 10, 15, 20, 30, 40 or 50 mutations but less than 200, 100, or 80 mutations relative to a reference, e.g., a parental, Cas9 molecule.

In certain embodiments, a mutation or mutations do not have a substantial effect on a Cas9 activity, e.g. a Cas9 activity described herein. In certain embodiments, a mutation or mutations have a substantial effect on a Cas9 activity, e.g. a Cas9 activity described herein.

### *6.5 Modified-cleavage Cas9*

In certain embodiments, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S. pyogenes,* as follows: its ability to modulate, e.g., decreased or increased, cleavage of a double stranded nucleic acid (endonuclease and/or exonuclease activity), e.g., as compared to a naturally occurring Cas9 molecule (e.g., a Cas9 molecule of *S. pyogenes)*; its ability to modulate, e.g., decreased or increased, cleavage of a single strand of a nucleic acid, e.g., a non-complementary strand of a nucleic acid molecule or a complementary strand of a nucleic acid molecule (nickase activity), e.g., as compared to a naturally occurring Cas9 molecule (e.g., a Cas9 molecule of *S. pyogenes)*; or the ability to cleave a nucleic acid molecule, e.g., a double stranded or single stranded nucleic acid molecule, can be eliminated.

In certain embodiments, an eaCas9 molecule or eaCas9 polypeptide comprises one or more of the following activities: cleavage activity associated with an N-terminal RuvC-like domain; cleavage activity associated with an HNH-like domain; cleavage activity associated with an HNH-like domain and cleavage activity associated with an N-terminal RuvC-like domain.

In certain embodiments, an eaCas9 molecule or eaCas9 polypeptide comprises an active, or cleavage competent, HNH-like domain (e.g., an HNH-like domain described herein, e.g., SEQ ID NOs:24-28) and an inactive, or cleavage incompetent, N-terminal RuvC-like domain. An exemplary inactive, or cleavage incompetent N-terminal RuvC-like domain can have a mutation of an aspartic acid in an N-terminal RuvC-like domain, e.g., an aspartic acid at position 9 of the consensus sequence disclosed in **Figs. 2A-2G** or an aspartic acid at position 10 of SEQ ID NO:2, e.g., can be substituted with an alanine. In certain embodiments, the eaCas9 molecule or eaCas9 polypeptide differs from wild-type in the N-terminal RuvC-like domain and does not cleave the target nucleic acid, or cleaves with significantly less efficiency, e.g., less than about 20%, about 10%, about 5%, about 1% or about 0.1 % of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes*, *S. aureus*, or *S. thermophilus.* In certain embodiments, the reference Cas9 molecule is the naturally occurring Cas9 molecule having the closest sequence identity or homology.

In certain embodiments, an eaCas9 molecule or eaCas9 polypeptide comprises an inactive, or cleavage incompetent, HNH domain and an active, or cleavage competent, N-terminal RuvC-like domain (e.g., a RuvC-like domain described herein, e.g., SEQ ID NOs: 15-23). Exemplary inactive, or cleavage incompetent HNH-like domains can have a mutation at one or more of: a histidine in an HNH-like domain, e.g., a histidine shown at position 856 of the consensus sequence disclosed in **Figs. 2A-2G**, e.g., can be substituted with an alanine; and one or more asparagines in an HNH-like domain, e.g., an asparagine shown at position 870 of the consensus sequence disclosed in **Figs. 2A-2G** and/or at position 879 of the consensus sequence disclosed in **Figs. 2A-2G**, e.g., can be substituted with an alanine. In certain

embodiments, the eaCas9 differs from wild-type in the HNH-like domain and does not cleave the target nucleic acid, or cleaves with significantly less efficiency, e.g., less than about 20%, about 10%, about 5%, about 1% or about 0.1% of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes*, *S. aureus*, or *S. thermophilus.* In an embodiment, the reference Cas9 molecule is the naturally occurring Cas9 molecule having the closest sequence identity or homology.

In certain embodiments, exemplary Cas9 activities comprise one or more of PAM specificity, cleavage activity, and helicase activity. A mutation(s) can be present, e.g., in: one or more RuvC domains, e.g., an N-terminal RuvC domain; an HNH domain; a region outside the RuvC domains and the HNH domain. In certain embodiments, a mutation(s) is present in a RuvC domain. In certain embodiments, a mutation(s) is present in an HNH domain. In certain embodiments, mutations are present in both a RuvC domain and an HNH domain.

Exemplary mutations that may be made in the RuvC domain or HNH domain with reference to the *S. pyogenes* Cas9 sequence include: D10A, E762A, H840A, N854A, N863A and/or D986A. Exemplary mutations that may be made in the RuvC domain with reference to the *S. aureus* Cas9 sequence include N580A (see, e.g., SEQ ID NO:11).

Whether or not a particular sequence, e.g., a substitution, may affect one or more activity, such as targeting activity, cleavage activity, etc., can be evaluated or predicted, e.g., by evaluating whether the mutation is conservative. In certain embodiments, a "non-essential" amino acid residue, as used in the context of a Cas9 molecule, is a residue that can be altered from the wild-type sequence of a Cas9 molecule, e.g., a naturally occurring Cas9 molecule, e.g., an eaCas9 molecule, without abolishing or more preferably, without substantially altering a Cas9 activity (e.g., cleavage activity), whereas changing an "essential" amino acid residue results in a substantial loss of activity (e.g., cleavage activity).

In certain embodiments, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule can differ from naturally occurring Cas9 molecules, e.g., a Cas9 molecule of *S aureus* or *S. pyogenes,* as follows: its ability to modulate, e.g., decreased or increased, cleavage of a double stranded break (endonuclease and/or exonuclease activity), e.g., as compared to a naturally occurring Cas9 molecule (e.g., a Cas9 molecule of *S aureus* or *S. pyogenes)*; its ability to modulate, e.g., decreased or increased, cleavage of a single strand of a nucleic acid, e.g., a non-complimentary strand of a nucleic acid molecule or a complementary strand of a nucleic acid molecule (nickase activity), e.g., as compared to a naturally occurring Cas9 molecule (e.g., a Cas9 molecule of *S aureus* or *S. pyogenes*); or the ability to cleave a nucleic acid molecule, e.g., a double stranded or single stranded nucleic acid molecule, can be eliminated. In certain embodiments, the nickase is *S. aureus* Cas9-derived nickase comprising the sequence of SEQ ID NO:10 (D10A) or SEQ ID NO:11 (N580A) (Friedland 2015).

In certain embodiments, the altered Cas9 molecule is an eaCas9 molecule comprising one or more of the following activities: cleavage activity associated with a RuvC domain; cleavage activity associated with an HNH domain; cleavage activity associated with an HNH domain and cleavage activity associated with a RuvC domain.

In certain embodiments, the altered Cas9 molecule or Cas9 polypeptide comprises a sequence in which:

the sequence corresponding to the fixed sequence of the consensus sequence disclosed in **Figs. 2A-2G** differs at no more than about 1%, about 2%, about 3%, about 4%, about 5%, about 10%, about 15%, or about 20% of the fixed residues in the consensus sequence disclosed in **Figs. 2A-2G**; and

the sequence corresponding to the residues identified by "*" in the consensus sequence disclosed in **Figs. 2A-2G** differs at no more than about 1%, about 2%, about 3%, about 4%, about 5%, about 10%, about 15%, about 20%, about 25%, about 30%, about 35%, or about 40% of the "*" residues from the corresponding sequence of naturally occurring Cas9 molecule, e.g., an *S. pyogenes*, *S. thermophilus*, *S. mutans*, or *L. innocua* Cas9 molecule.

In certain embodiments, the altered Cas9 molecule or Cas9 polypeptide is an eaCas9 molecule or eaCas9 polypeptide comprising the amino acid sequence of *S. pyogenes* Cas9 disclosed in **Figs. 2A-2G** with one or more amino acids that differ from the sequence *of S. pyogenes* (e.g., substitutions) at one or more residues (e.g., 2, 3, 5, 10, 15, 20, 30, 50, 70, 80, 90, 100, or 200 amino acid residues) represented by an "*" in the consensus sequence disclosed in **Figs. 2A-2G.**

In certain embodiments, the altered Cas9 molecule or Cas9 polypeptide is an eaCas9 molecule or eaCas9 polypeptide comprising the amino acid sequence of *S. thermophilus* Cas9 disclosed in **Figs. 2A-2G** with one or more amino acids that differ from the sequence of *S. thermophilus* (e.g., substitutions) at one or more residues (e.g., 2, 3, 5, 10, 15, 20, 30, 50, 70, 80, 90, 100, or 200 amino acid residues) represented by an "*" in the consensus sequence disclosed in **Figs. 2A-2G.**

In certain embodiments, the altered Cas9 molecule or Cas9 polypeptide is an eaCas9 molecule or eaCas9 polypeptide comprising the amino acid sequence of *S. mutans* Cas9 disclosed in **Figs. 2A-2G** with one or more amino acids that differ from the sequence of *S. mutans* (e.g., substitutions) at one or more residues (e.g., 2, 3, 5, 10, 15, 20,

30, 50, 70, 80, 90, 100, or 200 amino acid residues) represented by an "*" in the consensus sequence disclosed in **Figs. 2A-2G.**

In certain embodiments, the altered Cas9 molecule or Cas9 polypeptide is an eaCas9 molecule or eaCas9 polypeptide comprising the amino acid sequence of *L. innocua* Cas9 disclosed in **Figs. 2A-2G** with one or more amino acids that differ from the sequence of *L. innocua* (e.g., substitutions) at one or more residues (e.g., 2, 3, 5, 10, 15, 20, 30, 50, 70, 80, 90, 100, or 200 amino acid residues) represented by an "*" in the consensus sequence disclosed in **Figs. 2A-2G.**

In certain embodiments, the altered Cas9 molecule or Cas9 polypeptide, e.g., an eaCas9 molecule or eaCas9 polypeptide, can be a fusion, e.g., of two of more different Cas9 molecules, e.g., of two or more naturally occurring Cas9 molecules of different species. For example, a fragment of a naturally occurring Cas9 molecule of one species can be fused to a fragment of a Cas9 molecule of a second species. As an example, a fragment of a Cas9 molecule *of S. pyogenes* comprising an N-terminal RuvC-like domain can be fused to a fragment of Cas9 molecule of a species other than *S. pyogenes* (e.g., *S. thermophilus*) comprising an HNH-like domain.

### *6.6 Cas9 with altered or no PAM recognition*

Naturally occurring Cas9 molecules can recognize specific PAM sequences, for example the PAM recognition sequences described above for, e.g., *S. pyogenes*, *S. thermophilus*, *S. mutans*, and *S. aureus.*

In certain embodiments, a Cas9 molecule or Cas9 polypeptide has the same PAM specificities as a naturally occurring Cas9 molecule. In certain embodiments, a Cas9 molecule or Cas9 polypeptide has a PAM specificity not associated with a naturally occurring Cas9 molecule, or a PAM specificity not associated with the naturally occurring Cas9 molecule to which it has the closest sequence homology. For example, a naturally occurring Cas9 molecule can be altered, e.g., to alter PAM recognition, e.g., to alter the PAM sequence that the Cas9 molecule or Cas9 polypeptide recognizes in order to decrease off-target sites and/or improve specificity; or eliminate a PAM recognition requirement. In certain embodiments, a Cas9 molecule or Cas9 polypeptide can be altered, e.g., to increase length of PAM recognition sequence and/or improve Cas9 specificity to high level of identity (e.g., about 98%, about 99% or about 100% match between gRNA and a PAM sequence), e.g., to decrease off-target sites and/or increase specificity. In certain embodiments, the length of the PAM recognition sequence is at least 4, 5, 6, 7, 8, 9, 10 or 15 amino acids in length. In certain embodiments, the Cas9 specificity requires at least about 90%, about 95%, about 96%, about 97%, about 98%, or about 99% homology between the gRNA and the PAM sequence. Cas9 molecules or Cas9 polypeptides that recognize different PAM sequences and/or have reduced off-target activity can be generated using directed evolution. Exemplary methods and systems that can be used for directed evolution of Cas9 molecules are described (see, e.g., Esvelt 2011). Candidate Cas9 molecules can be evaluated, e.g., by methods described below.

### *6.7 Size-optimized Cas9*

Engineered Cas9 molecules and engineered Cas9 polypeptides described herein include a Cas9 molecule or Cas9 polypeptide comprising a deletion that reduces the size of the molecule while still retaining desired Cas9 properties, e.g., essentially native conformation, Cas9 nuclease activity, and/or target nucleic acid molecule recognition. Provided herein are Cas9 molecules or Cas9 polypeptides comprising one or more deletions and optionally one or more linkers, wherein a linker is disposed between the amino acid residues that flank the deletion. Methods for identifying suitable deletions in a reference Cas9 molecule, methods for generating Cas9 molecules with a deletion and a linker, and methods for using such Cas9 molecules will be apparent to one of ordinary skill in the art upon review of this document.

A Cas9 molecule, e.g., a *S. aureus* or *S. pyogenes* Cas9 molecule, having a deletion is smaller, e.g., has reduced number of amino acids, than the corresponding naturally-occurring Cas9 molecule. The smaller size of the Cas9 molecules allows increased flexibility for delivery methods, and thereby increases utility for genome-editing. A Cas9 molecule can comprise one or more deletions that do not substantially affect or decrease the activity of the resultant Cas9 molecules described herein. Activities that are retained in the Cas9 molecules comprising a deletion as described herein include one or more of the following:

a nickase activity, i.e., the ability to cleave a single strand, e.g., the non-complementary strand or the complementary strand, of a nucleic acid molecule; a double stranded nuclease activity, i.e., the ability to cleave both strands of a double stranded nucleic acid and create a double stranded break, which in an embodiment is the presence of two nickase activities;
an endonuclease activity;
an exonuclease activity;
a helicase activity, i.e., the ability to unwind the helical structure of a double stranded nucleic acid;
and recognition activity of a nucleic acid molecule, e.g., a target nucleic acid or a gRNA.

Activity of the Cas9 molecules described herein can be assessed using the activity assays described herein or in the art.

### *6.8 Identifying regions suitable for deletion*

Suitable regions of Cas9 molecules for deletion can be identified by a variety of methods. Naturally-occurring orthologous Cas9 molecules from various bacterial species can be modeled onto the crystal structure of *S. pyogenes* Cas9 (Nishimasu 2014) to examine the level of conservation across the selected Cas9 orthologs with respect to the three-dimensional conformation of the protein. Less conserved or unconserved regions that are spatially located distant from regions involved in Cas9 activity, e.g., interface with the target nucleic acid molecule and/or gRNA, represent regions or domains are candidates for deletion without substantially affecting or decreasing Cas9 activity.

### *6.9 Nucleic acids encoding Cas9 molecules*

Nucleic acids encoding the Cas9 molecules or Cas9 polypeptides, e.g., an eaCas9 molecule or eaCas9 polypeptides are provided herein. Exemplary nucleic acids encoding Cas9 molecules or Cas9 polypeptides have been described previously (see, e.g., Cong 2013; Wang 2013; Mali 2013; Jinek 2012).

In certain embodiments, a nucleic acid encoding a Cas9 molecule or Cas9 polypeptide can be a synthetic nucleic acid sequence. For example, the synthetic nucleic acid molecule can be chemically modified, e.g., as described herein. In certain embodiments, the Cas9 mRNA has one or more (e.g., all of the following properties: it is capped, polyadenylated, substituted with 5-methylcytidine and/or pseudouridine.

Additionally or alternatively, the synthetic nucleic acid sequence can be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein.

Additionally or alternatively, a nucleic acid encoding a Cas9 molecule or Cas9 polypeptide may comprise a nuclear localization sequence (NLS). Nuclear localization sequences are known in the art.

An exemplary codon optimized nucleic acid sequence encoding a Cas9 molecule of *S. pyogenes* is set forth in SEQ ID NO:3. The corresponding amino acid sequence of an *S. pyogenes* Cas9 molecule is set forth in SEQ ID NO:2.

Exemplary codon optimized nucleic acid sequences encoding a Cas9 molecule of *S. aureus* are set forth in SEQ ID NOs:7-9, 826367, and 826368. An amino acid sequence of an *S. aureus* Cas9 molecule is set forth in SEQ ID NO:6.

If any of the above Cas9 sequences are fused with a peptide or polypeptide at the C-terminus, it is understood that the stop codon will be removed.

### *6.10 Other Cas molecules and Cas polypeptides*

Various types of Cas molecules or Cas polypeptides can be used to practice the inventions disclosed herein. In certain embodiments, Cas molecules of Type II Cas systems are used. In certain embodiments, Cas molecules of other Cas systems are used. For example, Type I or Type III Cas molecules may be used. Exemplary Cas molecules (and Cas systems) have been described previously (see, e.g., Haft 2005 and Makarova 2011). Exemplary Cas molecules (and Cas systems) are also shown in

**Table 2.**

| **Table 2: Cas Systems** | | | | | |
|---|---|---|---|---|---|
| **Gene name‡** | **System type or subtype** | **Name from Haft 2005§** | **Structure of encoded protein (PDB accessions)¶** | **Families (and superfamily) of encoded protein#**** | **Representatives** |
| *cas1* | • Type I<br>• Type II<br>• Type III | *cas1* | 3GOD, 3LFX and 2YZS | COG1518 | SERP2463, SPy1047 and *ygbT* |
| *cas2* | • Type I<br>• Type II<br>• Type III | *cas2* | 2IVY, 2I8E and 3EXC | COG1343 and COG3512 | SERP2462, SPy1048, SPy1723 (N-terminal domain) and *ygbF* |
| *cas3'* | • Type I‡‡ | *cas3* | NA | COG1203 | APE1232 and *ygcB* |

(continued)

| Table 2: Cas Systems | | | | | |
|---|---|---|---|---|---|
| **Gene name‡** | **System type or subtype** | **Name from Haft 2005§** | **Structure of encoded protein (PDB accessions)¶** | **Families (and superfamily) of encoded protein#**** | **Representatives** |
| *cas3"* | • Subtype I-A<br>• Subtype I-B | NA | NA | COG2254 | APE1231 and BH0336 |
| *cas4* | • Subtype I-A<br>• Subtype I-B<br>• Subtype I-C<br>• Subtype I-D<br>• Subtype II-B | *cas4* and *csa1* | NA | COG1468 | APE1239 and BH0340 |
| *cas5* | • Subtype I-A<br>• Subtype I-B<br>• Subtype I-C<br>• Subtype I-E | *cas5a*, *cas5d*, *cas5e*, *cas5h*, *cas5p*, *cas5t* and *cmx5* | 3KG4 | COG1688 (RAMP) | APE1234, BH0337, *devS* and *ygcI* |
| *cas6* | • Subtype I-A<br>• Subtype I-B<br>• Subtype I-D<br>• Subtype III-A• Subtype | *cas6* and *cmx6* | 3I4H | COG1583 and COG5551 (RAMP) | PF1131 and slr7014 |
| | III-B | | | | |
| *cas6e* | • Subtype I-E | *cse3* | 1WJ9 | (RAMP) | *ygcH* |
| *cas6f* | • Subtype I-F | *csy4* | 2XLJ | (RAMP) | y1727 |
| *cas7* | • Subtype I-A<br>• Subtype I-B<br>• Subtype I-C<br>• Subtype I-E | *csa2, csd2, cse4, csh2, csp1* and *cst2* | NA | COG1857 and COG3649 (RAMP) | *devR* and *ygcJ* |

(continued)

| Table 2: Cas Systems | | | | | |
|---|---|---|---|---|---|
| **Gene name‡** | **System type or subtype** | **Name from Haft 2005§** | **Structure of encoded protein (PDB accessions)¶** | **Families (and superfamily) of encoded protein#**** | **Representatives** |
| *cas8a1* | • Subtype I-A‡‡ | *cmx1*, *cst1*, *csx8*, *csx13* and CXXC-CXXC | NA | BH0338-like | LA3191§§ and PG2018§§ |
| *cas8a2* | • Subtype I-A‡‡ | *csa4* and *csx9* | NA | PH0918 | AF0070, AF1873, MJ0385, PF0637, PH0918 and SSO1401 |
| *cas8b* | • Subtype I-B‡‡ | *csh1* and TM1802 | NA | BH0338-like | MTH1090 and TM1802 |
| *cas8c* | • Subtype I-C‡‡ | *csd1* and *csp2* | NA | BH0338-like | BH0338 |
| *cas9* | • Type II‡‡ | *csn1* and *csx12* | NA | COG3513 | FTN_0757 and SPy1046 |
| *cas10* | • Type III‡‡ | *cmr2*, *csm1* and *csx11* | NA | COG1353 | MTH326, Rv2823c§§ and TM1794§§ |
| *cas10d* | • Subtype I-D‡‡ | *csc3* | NA | COG1353 | slr7011 |
| *csy1* | • Subtype IF‡‡ | *csy1* | NA | y1724-like | y1724 |
| *csy2* | • Subtype I-F | *csy2* | NA | (RAMP) | y1725 |
| *csy3* | • Subtype I-F | *csy3* | NA | (RAMP) | y1726 |
| *cse1* | • Subtype I-E‡‡ | *cse1* | NA | YgcL-like | *ygcL* |
| *cse2* | • Subtype I-E | *cse2* | 2ZCA | YgcK-like | *ygcK* |
| *csc1* | • Subtype I-D | *csc1* | NA | alr1563-like (RAMP) | alr1563 |
| *csc2* | • Subtype I-D | *csc1* and *csc2* | NA | COG1337 (RAMP) | slr7012 |
| *csa5* | • Subtype I-A | *csa5* | NA | AF1870 | AF1870, MJ0380, PF0643 and SSO1398 |
| *csn2* | • Subtype II-A | *csn2* | NA | SPy1049-like | SPy1049 |
| *csm2* | • Subtype III-A‡‡ | *csm2* | NA | COG1421 | MTH1081 and SERP2460 |
| *csm3* | • Subtype III-A | *csc2* and *csm3* | NA | COG1337 (RAMP) | MTH1080 and SERP2459 |
| *csm4* | • Subtype III-A | *csm4* | NA | COG1567 (RAMP) | MTH1079 and SERP2458 |

(continued)

| Table 2: Cas Systems | | | | | |
|---|---|---|---|---|---|
| **Gene name‡** | **System type or subtype** | **Name from Haft 2005§** | **Structure of encoded protein (PDB accessions)¶** | **Families (and superfamily) of encoded protein#**** | **Representatives** |
| *csm5* | • Subtype III-A | *csm5* | NA | COG1332 (RAMP) | MTH1078 and SERP2457 |
| *csm6* | • Subtype III-A | APE2256 and *csm6* | 2WTE | COG1517 | APE2256 and SSO1445 |
| *cmr1* | • Subtype III-B | *cmr1* | NA | COG1367 (RAMP) | PF1130 |
| *cmr3* | • Subtype III-B | *cmr3* | NA | COG1769 (RAMP) | PF1128 |
| *cmr4* | • Subtype III-B | *cmr4* | NA | COG1336 (RAMP) | PF1126 |
| *cmr5* | • Subtype III-B‡‡ | *cmr5* | 2ZOP and 2OEB | COG3337 | MTH324 and PF1125 |
| *cmr6* | • Subtype III-B | *cmr6* | NA | COG1604 (RAMP) | PF1124 |
| *csb1* | • Subtype I-U | GSU0053 | NA | (RAMP) | Balac_1306 and GSU0053 |
| *csb2* | • Subtype I-U§§ | NA | NA | (RAMP) | Balac_1305 and GSU0054 |
| *csb3* | • Subtype I-U | NA | NA | (RAMP) | Balac_1303§§ |
| *csx17* | • Subtype I-U | NA | NA | NA | Btus_2683 |
| *csx14* | • Subtype I-U | NA | NA | NA | GSU0052 |
| *csx10* | • Subtype I-U | *csx10* | NA | (RAMP) | Caur_2274 |
| *csx16* | • Subtype III-U | VVA1548 | NA | NA | VVA1548 |
| *csaX* | • Subtype III-U | *csaX* | NA | NA | SSO1438 |
| *csx3* | • Subtype III-U | *csx3* | NA | NA | AF1864 |
| *csx1* | • Subtype III-U | *csa3*, *csx1*, *csx2*, DXTHG, NE0113 and TIGR02710 | 1XMX and 2171 | COG1517 and COG4006 | MJ1666, NE0113, PF1127 and TM1812 |
| *csx15* | • Unknown | NA | NA | TTE2665 | TTE2665 |
| *csf1* | • Type U | *csf1* | NA | NA | AFE_1038 |
| *csf2* | • Type U | *csf2* | NA | (RAMP) | AFE_1039 |
| *csf3* | • Type U | *csf3* | NA | (RAMP) | AFE_1040 |
| *csf4* | • Type U | *csf4* | NA | NA | AFE_1037 |

## 7. Functional Analysis of Candidate Molecules

Candidate Cas9 molecules, candidate gRNA molecules, candidate Cas9 molecule/gRNA molecule complexes, can be evaluated by art-known methods or as described herein. For example, exemplary methods for evaluating the endonuclease activity of Cas9 molecule have been described previously (Jinek 2012).

### *7.1 Binding and Cleavage Assay: Testing Cas9 endonuclease activity*

The ability of a Cas9 molecule/gRNA molecule complex to bind to and cleave a target nucleic acid can be evaluated in a plasmid cleavage assay. In this assay, synthetic or *in vitro*-transcribed gRNA molecule is pre-annealed prior to the reaction by heating to 95°C and slowly cooling down to room temperature. Native or restriction digest-linearized plasmid DNA (300 ng (~8 nM)) is incubated for 60 min at 37°C with purified Cas9 protein molecule (50-500 nM) and gRNA (50-500 nM, 1:1) in a Cas9 plasmid cleavage buffer (20 mM HEPES pH 7.5, 150 mM KCl, 0.5 mM DTT, 0.1 mM EDTA) with or without 10 mM $MgCl_2$. The reactions are stopped with 5X DNA loading buffer (30% glycerol, 1.2% SDS, 250 mM EDTA), resolved by a 0.8 or 1% agarose gel electrophoresis and visualized by ethidium bromide staining. The resulting cleavage products indicate whether the Cas9 molecule cleaves both DNA strands, or only one of the two strands. For example, linear DNA products indicate the cleavage of both DNA strands. Nicked open circular products indicate that only one of the two strands is cleaved.

Alternatively, the ability of a Cas9 molecule/gRNA molecule complex to bind to and cleave a target nucleic acid can be evaluated in an oligonucleotide DNA cleavage assay. In this assay, DNA oligonucleotides (10 pmol) are radiolabeled by incubating with 5 units T4 polynucleotide kinase and ~3-6 pmol (~20-40 mCi) [γ-32P]-ATP in IX T4 polynucleotide kinase reaction buffer at 37°C for 30 min, in a 50 μL reaction. After heat inactivation (65°C for 20 min), reactions are purified through a column to remove unincorporated label. Duplex substrates (100 nM) are generated by annealing labeled oligonucleotides with equimolar amounts of unlabeled complementary oligonucleotide at 95°C for 3 min, followed by slow cooling to room temperature. For cleavage assays, gRNA molecules are annealed by heating to 95°C for 30 s, followed by slow cooling to room temperature. Cas9 (500 nM final concentration) is pre-incubated with the annealed gRNA molecules (500 nM) in cleavage assay buffer (20 mM HEPES pH 7.5, 100 mM KCl, 5 mM MgCl2, 1 mM DTT, 5% glycerol) in a total volume of 9 μL. Reactions are initiated by the addition of 1 μL target DNA (10 nM) and incubated for 1 h at 37°C. Reactions are quenched by the addition of 20 μL of loading dye (5 mM EDTA, 0.025% SDS, 5% glycerol in formamide) and heated to 95°C for 5 min. Cleavage products are resolved on 12% denaturing polyacrylamide gels containing 7 M urea and visualized by phosphorimaging. The resulting cleavage products indicate that whether the complementary strand, the non-complementary strand, or both, are cleaved.

One or both of these assays can be used to evaluate the suitability of a candidate gRNA molecule or candidate Cas9 molecule.

### *7.2 Binding Assay: Testing the binding of Cas9 molecule to target DNA*

Exemplary methods for evaluating the binding of Cas9 molecule to target DNA have been described previously , e.g., in Jinek et al., SCIENCE 2012; 337(6096):816-821.

For example, in an electrophoretic mobility shift assay, target DNA duplexes are formed by mixing of each strand (10 nmol) in deionized water, heating to 95°C for 3 min and slow cooling to room temperature. All DNAs are purified on 8% native gels containing IX TBE. DNA bands are visualized by UV shadowing, excised, and eluted by soaking gel pieces in DEPC-treated $H_2O$. Eluted DNA is ethanol precipitated and dissolved in DEPC-treated $H_2O$. DNA samples are 5' end labeled with [γ-32P]-ATP using T4 polynucleotide kinase for 30 min at 37°C. Polynucleotide kinase is heat denatured at 65°C for 20 min, and unincorporated radiolabel is removed using a column. Binding assays are performed in buffer containing 20 mM HEPES pH 7.5, 100 mM KCl, 5 mM $MgCl_2$, 1 mM DTT and 10% glycerol in a total volume of 10 μL. Cas9 protein molecule is programmed with equimolar amounts of pre-annealed gRNA molecule and titrated from 100 pM to 1 μM. Radiolabeled DNA is added to a final concentration of 20 pM. Samples are incubated for 1 h at 37°C and resolved at 4°C on an 8% native polyacrylamide gel containing IX TBE and 5 mM $MgCl_2$. Gels are dried and DNA visualized by phosphorimaging.

### *7.3 Differential Scanning Flourimetry (DSF)*

The thermostability of Cas9-gRNA ribonucleoprotein (RNP) complexes can be measured via DSF. This technique measures the thermostability of a protein, which can increase under favorable conditions such as the addition of a binding RNA molecule, e.g., a gRNA.

The assay is performed using two different protocols, one to test the best stoichiometric ratio of gRNA:Cas9 protein and another to determine the best solution conditions for RNP formation.

To determine the best solution to form RNP complexes, a 2uM solution of Cas9 in water+10x SYPRO Orange® (Life Technologies cat#S-6650) and dispensed into a 384 well plate. An equimolar amount of gRNA diluted in solutions with varied pH and salt is then added. After incubating at room temperature for 10'and brief centrifugation to remove any bubbles,a Bio-Rad CFX384™ Real-Time System C1000 Touch™ Thermal Cycler with the Bio-Rad CFX Manager software is used to run a gradient from 20°C to 90°C with a 1°C increase in temperature every 10 seconds.

The second assay consists of mixing various concentrations of gRNA with 2uM Cas9 in optimal buffer from assay 1 above and incubating at RT for 10' in a 384 well plate. An equal volume of optimal buffer + 10x SYPRO Orange® (Life Technologies cat#S-6650) is added and the plate sealed with Microseal® B adhesive (MSB-1001). Following brief centrifugation to remove any bubbles, a Bio-Rad CFX384™ Real-Time System C1000 Touch™ Thermal Cycler with the Bio-Rad CFX Manager software is used to run a gradient from 20°C to 90°C with a 1° increase in temperature every 10 seconds.

**8. Genome Editing Approaches**

Described herein are compositions, genome-editing systems and methods for targeted alteration (e.g., knockout) of the *DMD* gene, e.g., one or both alleles of the *DMD* gene, e.g., using one or more of the approaches or pathways described herein, e.g., using NHEJ.

***8.1 NHEJ Approaches for Gene Targeting***

In certain embodiments of the methods provided herein, NHEJ-mediated alteration is used to alter a DMD target position. As described herein, nuclease-induced non-homologous end-joining (NHEJ) can be used to target gene-specific knockouts. Nuclease-induced NHEJ can also be used to remove (e.g., delete) sequence insertions in a gene of interest.

In certain embodiments, the genomic alterations associated with the methods described herein rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, at one or both strands, prior to rejoining of the ends. This results in the presence of insertion and/or deletion (indel) mutations in the DNA sequence at the site of the NHEJ repair. Two-thirds of these mutations typically alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in non-critical regions of the protein.

The indel mutations generated by NHEJ are unpredictable in nature; however, at a given break site certain indel sequences are favored and are over represented in the population, likely due to small regions of microhomology. The lengths of deletions can vary widely; they are most commonly in the 1-50 bp range, but can reach greater than 100-200 bp. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs (e.g., motifs less than or equal to 50 nucleotides in length) as long as the generation of a specific final sequence is not required. If a double-strand break is targeted near to a target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence.. In this way, DNA segments as large as several hundred kilobases can be deleted. Both of these approaches can be used to delete specific DNA sequences; however, the error-prone nature of NHEJ may still produce indel mutations at the site of repair.

Both double strand cleaving eaCas9 molecules and single strand, or nickase, eaCas9 molecules can be used in the methods and compositions described herein to generate NHEJ-mediated indels. NHEJ-mediated indels targeted to the early coding region of a gene of interest can be used to knockout (i.e., eliminate expression of) a gene of interest. For example, early coding region of a gene of interest includes sequence immediately following a transcription start site, within a first exon of the coding sequence, or within 500 bp of the transcription start site (e.g., less than 500, 450, 400, 350, 300, 250, 200, 150, 100 or 50 bp).

***8.2 Placement of double strand or single strand breaks relative to the target position***

In certain embodiments, in which a gRNA and Cas9 nuclease generate a double strand break for the purpose

of inducing NHEJ-mediated indels, a gRNA, e.g., a unimolecular (or chimeric) or modular gRNA molecule, is configured to position one double-strand break in close proximity to a nucleotide of the target position. In certain embodiments, the cleavage site is between 0-30 bp away from the target position (e.g., less than 30, 25, 20, 15, 10, 9, 8, 7, 6, 5, 4, 3, 2 or 1 bp from the target position).

In certain embodiments, in which two gRNAs complexing with Cas9 nickases induce two single strand breaks for the purpose of inducing NHEJ-mediated indels, two gRNAs, e.g., independently, unimolecular (or chimeric) or modular gRNA, are configured to position two single-strand breaks to provide for NHEJ repair a nucleotide of the target position. In certain embodiments, the gRNAs are configured to position cuts at the same position, or within a few nucleotides of one another, on different strands, essentially mimicking a double strand break. In certain embodiments, the closer nick is between 0-30 bp away from the target position (e.g., less than 30, 25, 20, 15, 10, 9, 8, 7, 6, 5, 4, 3, 2 or 1 bp from the target position), and the two nicks are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp). In certain embodiments, the gRNAs are configured to place a single strand break on either side of a nucleotide of the target position.

Both double strand cleaving eaCas9 molecules and single strand, or nickase, eaCas9 molecules can be used in the methods and compositions described herein to generate breaks both sides of a target position. Double strand or paired single strand breaks may be generated on both sides of a target position to remove the nucleic acid sequence between the two cuts (e.g., the region between the two breaks in deleted). In one embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In certain embodiments, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20, or 10 bp).

### *8.3 HDR repair, HDR-mediated knock-in, and template nucleic acids*

In certain embodiments of the methods provided herein, HDR-mediated sequence alteration is used to alter the sequence of one or more nucleotides in a *DMD* gene using an exogenously provided template nucleic acid (also referred to herein as a donor construct). In certain embodiments, HDR-mediated alteration of a DMD target position occurs by HDR with an exogenously provided donor template or template nucleic acid. For example, the donor construct or template nucleic acid provides for alteration of a DMD target position. In certain embodiments, a plasmid donor is used as a template for homologous recombination. In certain embodiments, a single stranded donor template is used as a template for alteration of the DMD target position by alternate methods of HDR (e.g., single strand annealing) between the target sequence and the donor template. Donor template-effected alteration of a DMD target position depends on cleavage by a Cas9 molecule. Cleavage by Cas9 can comprise a double strand break or two single strand breaks.

In certain embodiments, HDR-mediated sequence alteration is used to alter the sequence of one or more nucleotides in a *DMD* gene without using an exogenously provided template nucleic acid. In certain embodiments, alteration of a DMD target position occurs by HDR with endogenous genomic donor sequence. For example, the endogenous genomic donor sequence provides for alteration of the DMD target position. In certain embodiments, the endogenous genomic donor sequence is located on the same chromosome as the target sequence. In certain embodiments, the endogenous genomic donor sequence is located on a different chromosome from the target sequence. Alteration of a DMD target position by endogenous genomic donor sequence depends on cleavage by a Cas9 molecule. Cleavage by Cas9 can comprise a double strand break or two single strand breaks.

In certain embodiments of the methods provided herein, HDR-mediated alteration is used to alter a single nucleotide in a *DMD* gene. These embodiments may utilize either one double-strand break or two single-strand breaks. In certain embodiments, a single nucleotide alteration is incorporated using (1) one double-strand break, (2) two single-strand breaks, (3) two double-strand breaks with a break occurring on each side of the target position, (4) one double-strand break and two single strand breaks with the double strand break and two single strand breaks occurring on each side of the target position, (5) four single-strand breaks with a pair of single-strand breaks occurring on each side of the target position, or (6) one single-strand break.

In certain embodiments wherein a single-stranded template nucleic acid is used, the target position can be altered by alternative HDR.

Donor template-effected alteration of a DMD target position depends on cleavage by a Cas9 molecule. Cleavage by Cas9 can comprise a nick, a double-strand break, or two single-strand breaks, e.g., one on each strand of the target nucleic acid. After introduction of the breaks on the target nucleic acid, resection occurs at the break ends resulting in single stranded overhanging DNA regions.

In canonical HDR, a double-stranded donor template is introduced, comprising homologous sequence to the target nucleic acid that will either be directly incorporated into the target nucleic acid or used as a template to change the sequence of the target nucleic acid. After resection at the break, repair can progress by different pathways, e.g., by the double Holliday junction model (or double-strand break repair, DSBR, pathway) or the synthesis-dependent strand annealing (SDSA) pathway. In the double Holliday junction model, strand invasion by the two single stranded overhangs of the target nucleic acid to the homologous sequences in the donor template occurs, resulting in the formation of an intermediate with two Holliday junctions. The junctions migrate as new DNA is synthesized from the ends of the invading strand to fill the gap resulting from the resection. The end of the newly synthesized DNA is ligated to the resected end, and the junctions are resolved, resulting in alteration of the target nucleic acid. Crossover with the donor template may occur upon resolution of the junctions. In the SDSA pathway, only one single stranded overhang invades the donor template and new DNA is synthesized from the end of the invading strand to fill the gap resulting from resection. The newly synthesized DNA then anneals to the remaining single stranded overhang, new DNA is synthesized to fill in the gap, and the strands are ligated to produce the altered DNA duplex.

In alternative HDR, a single strand donor template, e.g., template nucleic acid, is introduced. A nick, single strand break, or double strand break at the target nucleic acid, for altering a desired target position, is mediated by a Cas9 molecule, e.g., described herein, and resection at the break occurs to reveal single stranded overhangs. Incorporation of the sequence of the template nucleic acid to alter a DMD target position typically occurs by the SDSA pathway, as described above.

Additional details on template nucleic acids are provided in Section IV entitled "Template nucleic acids" in International Application PCT/US2014/057905.

In certain embodiments, double strand cleavage is effected by a Cas9 molecule having cleavage activity associated with an HNH-like domain and cleavage activity associated with a RuvC-like domain, e.g., an N-terminal RuvC-like domain, e.g., a wild-type Cas9. Such embodiments require only a single gRNA.

In certain embodiments, one single-strand break, or nick, is effected by a Cas9 molecule having nickase activity, e.g., a Cas9 nickase as described herein. A nicked target nucleic acid can be a substrate for alt-HDR.

In certain embodiments, two single-strand breaks, or nicks, are effected by a Cas9 molecule having nickase activity, e.g., cleavage activity associated with an HNH-like domain or cleavage activity associated with an N-terminal RuvC-like domain. Such embodiments usually require two gRNAs, one for placement of each single-strand break. In certain embodiments, the Cas9 molecule having nickase activity cleaves the strand to which the gRNA hybridizes, but not the strand that is complementary to the strand to which the gRNA hybridizes. In certain embodiments, the Cas9 molecule having nickase activity does not cleave the strand to which the gRNA hybridizes, but rather cleaves the strand that is complementary to the strand to which the gRNA hybridizes.

In certain embodiments, the nickase has HNH activity, e.g., a Cas9 molecule having the RuvC activity inactivated, e.g., a Cas9 molecule having a mutation at D10, e.g., the D10A mutation (see, e.g., SEQ ID NO: 10). D10A inactivates RuvC; therefore, the Cas9 nickase has (only) HNH activity and will cut on the strand to which the gRNA hybridizes (e.g., the complementary strand, which does not have the NGG PAM on it). In certain embodiments, a Cas9 molecule having an H840, e.g., an H840A, mutation can be used as a nickase. H840A inactivates HNH; therefore, the Cas9 nickase has (only) RuvC activity and cuts on the non-complementary strand (e.g., the strand that has the NGG PAM and whose sequence is identical to the gRNA). In certain embodiments, a Cas9 molecule having an N863 mutation, e.g., the N863A mutation, mutation can be used as a nickase. N863A inactivates HNH therefore the Cas9 nickase has (only) RuvC activity and cuts on the non-complementary strand (the strand that has the NGG PAM and whose sequence is identical to the gRNA).

In certain embodiments, in which a nickase and two gRNAs are used to position two single strand nicks, one nick is on the + strand and one nick is on the - strand of the target nucleic acid. The PAMs can be outwardly facing. The gRNAs can be selected such that the gRNAs are separated by, from about 0-50, 0-100, or 0-200 nucleotides. In certain embodiments, there is no overlap between the target sequences that are complementary to the targeting domains of the two gRNAs. In certain embodiments, the gRNAs do not overlap and are separated by as much as 50, 100, or 200 nucleotides. In certain embodiments, the use of two gRNAs can increase specificity, e.g., by decreasing off-target binding (Ran 2013).

In certain embodiments, a single nick can be used to induce HDR, e.g., alt-HDR. It is contemplated herein that a single nick can be used to increase the ratio of HR to NHEJ at a given cleavage site. In certain embodiments, a single strand break is formed in the strand of the target nucleic acid to which the targeting domain of said gRNA is complementary.

In certain embodiments, a single strand break is formed in the strand of the target nucleic acid other than the strand to which the targeting domain of said gRNA is complementary.

### *8.4 Placement of double strand or single strand breaks relative to the target position*

A double strand break or single strand break in one of the strands should be sufficiently close to a DMD target position that an alteration is produced in the desired region, e.g., exon 44, exon 45, exon 46, exon 47, exon 48, exon 49, exon 50, exon 51, exon 52, exon 53, exon 54, or exon 55 of the *DMD* gene. In certain embodiments, the distance is not more than 50, 100, 200, 300, 350 or 400 nucleotides. In certain embodiments, the break should be sufficiently close to target position such that the target position is within the region that is subject to exonuclease-mediated removal during end resection. If the distance between the DMD target position and a break is too great, the sequence desired to be altered may not be included in the end resection and, therefore, may not be altered, as donor sequence, either exogenously provided donor sequence or endogenous genomic donor sequence, in certain embodiments is only used to alter sequence within the end resection region.

In certain embodiments, the methods described herein introduce one or more breaks near a DMD target position. In certain of these embodiments, two or more breaks are introduced that flank a DMD target position. The two or more breaks remove (e.g., delete) a genomic sequence including a DMD target position. All methods described herein result in altering a DMD target position within a *DMD* gene.

In certain embodiments, the gRNA targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, or 200 nucleotides of the region desired to be altered, e.g., a mutation. The break, e.g., a double strand or single strand break, can be positioned upstream or downstream of the region desired to be altered, e.g., a mutation. In certain embodiments, a break is positioned within the region desired to be altered, e.g., within a region defined by at least two mutant nucleotides. In certain embodiments, a break is positioned immediately adjacent to the region desired to be altered, e.g., immediately upstream or downstream of a mutation.

In certain embodiments, a single strand break is accompanied by an additional single strand break, positioned by a second gRNA molecule, as discussed below. For example, the targeting domains bind configured such that a cleavage event, e.g., the two single strand breaks, are positioned within 1, 2, 3, 4, 5, 10, 15, 20, 25, 30, 35, 40, 45, 50, 60, 70, 80, 90, 100, 150, or 200 nucleotides of a target position. In certain embodiments, the first and second gRNA molecules are configured such that, when guiding a Cas9 nickase, a single strand break will be accompanied by an additional single strand break, positioned by a second gRNA, sufficiently close to one another to result in alteration of the desired region. In certain embodiments, the first and second gRNA molecules are configured such that a single strand break positioned by said second gRNA is within 10, 20, 30, 40, or 50 nucleotides of the break positioned by said first gRNA molecule, e.g., when the Cas9 is a nickase. In certain embodiments, the two gRNA molecules are configured to position cuts at the same position, or within a few nucleotides of one another, on different strands, e.g., essentially mimicking a double strand break.

In certain embodiments in which a gRNA (unimolecular (or chimeric) or modular gRNA) and Cas9 nuclease induce a double strand break for the purpose of inducing HDR-mediated sequence alteration, the cleavage site is between 0-200 bp (e.g., 0 to 175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position. In certain embodiments, the cleavage site is between 0-100 bp (e.g., 0 to 75, 0 to 50, 0 to 25, 25 to 100, 25 to 75, 25 to 50, 50 to 100, 50 to 75 or 75 to 100 bp) away from the target position.

In certain embodiments, one can promote HDR by using nickases to generate a break with overhangs. While not wishing to be bound by theory, the single stranded nature of the overhangs can enhance the cell's likelihood of repairing the break by HDR as opposed to, e.g., NHEJ. Specifically, in some embodiments, HDR is promoted by selecting a first gRNA that targets a first nickase to a first target sequence, and a second gRNA that targets a second nickase to a second target sequence which is on the opposite DNA strand from the first target sequence and offset from the first nick.

In certain embodiments, the targeting domain of a gRNA molecule is configured to position a cleavage event sufficiently far from a preselected nucleotide that the nucleotide is not altered. In certain embodiments, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

### *8. 5 Placement of a first break and a second break relative to each other*

In certain embodiments, a double strand break can be accompanied by an additional double strand break, positioned by a second gRNA molecule, as is discussed below.

In certain embodiments, a double strand break can be accompanied by two additional single strand breaks, positioned by a second gRNA molecule and a third gRNA molecule.

In certain embodiments, a first and second single strand breaks can be accompanied by two additional single strand breaks positioned by a third gRNA molecule and a fourth gRNA molecule.

When two or more gRNAs are used to position two or more cleavage events, e.g., double strand or single strand breaks, in a target nucleic acid, it is contemplated that the two or more cleavage events may be made by the same or different Cas9 proteins. For example, when two gRNAs are used to position two double stranded breaks, a single Cas9 nuclease may be used to create both double stranded breaks. When two or more gRNAs are used to position two or more single stranded breaks (nicks), a single Cas9 nickase may be used to create the two or more nicks. When two or more gRNAs are used to position at least one double stranded break and at least one single stranded break, two Cas9 proteins may be used, e.g., one Cas9 nuclease and one Cas9 nickase. In certain embodiments, two or more Cas9 proteins are used, and the two or more Cas9 proteins may be delivered sequentially to control specificity of a double stranded versus a single stranded break at the desired position in the target nucleic acid.

In certain embodiments, the targeting domain of the first gRNA molecule and the targeting domain of the second gRNA molecules are complementary to opposite strands of the target nucleic acid molecule. In certain embodiments, the gRNA molecule and the second gRNA molecule are configured such that the PAMs are oriented outward.

In certain embodiments, two gRNA are selected to direct Cas9-mediated cleavage at two positions that are a preselected distance from each other. In certain embodiments, the two points of cleavage are on opposite strands of the target nucleic acid. In certain embodiments, the two cleavage points form a blunt ended break, and in other embodiments, they are offset so that the DNA ends comprise one or two overhangs (e.g., one or more 5' overhangs and/or one or more 3' overhangs). In certain embodiments, each cleavage event is a nick. In certain embodiments, the nicks are close enough together that they form a break that is recognized by the double stranded break machinery (as opposed to being recognized by, e.g., the SSBr machinery). In certain embodiments, the nicks are far enough apart that they create an overhang that is a substrate for HDR, i.e., the placement of the breaks mimics a DNA substrate that has experienced some resection. For instance, in certain embodiments the nicks are spaced to create an overhang that is a substrate for processive resection. In certain embodiments, the two breaks are spaced within 25-65 nucleotides of each other. The two breaks may be, e.g., about 25, 30, 35, 40, 45, 50, 55, 60, or 65 nucleotides of each other. The two breaks may be, e.g., at least about 25, 30, 35, 40, 45, 50, 55, 60, or 65 nucleotides of each other. The two breaks may be, e.g., at most about 30, 35, 40, 45, 50, 55, 60, or 65 nucleotides of each other. In certain embodiments, the two breaks are about 25-30, 30-35, 35-40, 40-45, 45-50, 50-55, 55-60, or 60-65 nucleotides of each other.

In certain embodiments, the break that mimics a resected break comprises a 3' overhang (e.g., generated by a DSB and a nick, where the nick leaves a 3' overhang), a 5' overhang (e.g., generated by a DSB and a nick, where the nick leaves a 5' overhang), a 3' and a 5' overhang (e.g., generated by three cuts), two 3' overhangs (e.g., generated by two nicks that are offset from each other), or two 5' overhangs (e.g., generated by two nicks that are offset from each other).

In certain embodiments in which two gRNAs (independently, unimolecular (or chimeric) or modular gRNA) complexing with Cas9 nickases induce two single strand breaks for the purpose of inducing HDR-mediated alteration, the closer nick is between 0-200 bp (e.g., 0 to 175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, or 75 to 100 bp) away from the target position and the two nicks will ideally be within 25-65 bp of each other (e.g., 25 to 50, 25 to 45, 25 to 40, 25 to 35, 25 to 30, 30 to 55, 30 to 50, 30 to 45, 30 to 40, 30 to 35, 35 to 55, 35 to 50, 35 to 45, 35 to 40, 40 to 55, 40 to 50, 40 to 45 bp, 45 to 50 bp, 50 to 55 bp, 55 to 60 bp, or 60 to 65 bp) and no more than 100 bp away from each other (e.g., no more than 90, 80, 70, 60, 50, 40, 30, 20, 10, or 5 bp away from each other). In certain embodiments, the cleavage site is between 0-100 bp (e.g., 0 to 75, 0 to 50, 0 to 25, 25 to 100, 25 to 75, 25 to 50, 50 to 100, 50 to 75, or 75 to 100 bp) away from the target position.

In certain embodiments, two gRNAs, e.g., independently, unimolecular (or chimeric) or modular gRNA, are configured to position a double-strand break on both sides of a target position. In certain embodiments, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In certain embodiments, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are, in certain embodiments, within 25-65 bp of each other (e.g., between 25 to 55, 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50, 45 to 50, 35 to 45, 40 to 45 bp, 45 to 50 bp, 50 to 55 bp, 55 to 60 bp, or 60 to 65 bp) and no more than 100 bp away from each other (e.g., no more than 90, 80, 70, 60, 50, 40, 30, or 20 or 10 bp).

When two gRNAs are used to target Cas9 molecules to breaks, different combinations of Cas9 molecules are

envisioned. In certain embodiments, a first gRNA is used to target a first Cas9 molecule to a first target position, and a second gRNA is used to target a second Cas9 molecule to a second target position. In certain embodiments, the first Cas9 molecule creates a nick on the first strand of the target nucleic acid, and the second Cas9 molecule creates a nick on the opposite strand, resulting in a double stranded break (e.g., a blunt ended cut or a cut with overhangs).

Different combinations of nickases can be chosen to target one single stranded break to one strand and a second single stranded break to the opposite strand. When choosing a combination, one can take into account that there are nickases having one active RuvC-like domain, and nickases having one active HNH domain. In certain embodiments, a RuvC-like domain cleaves the non-complementary strand of the target nucleic acid molecule. In certain embodiments, an HNH-like domain cleaves a single stranded complementary domain, e.g., a complementary strand of a double stranded nucleic acid molecule. Generally, if both Cas9 molecules have the same active domain (e.g., both have an active RuvC domain or both have an active HNH domain), one will choose two gRNAs that bind to opposite strands of the target. In more detail, in some embodiments a first gRNA is complementary with a first strand of the target nucleic acid and binds a nickase having an active RuvC-like domain and causes that nickase to cleave the strand that is non-complementary to that first gRNA, i.e., a second strand of the target nucleic acid; and a second gRNA is complementary with a second strand of the target nucleic acid and binds a nickase having an active RuvC-like domain and causes that nickase to cleave the strand that is non-complementary to that second gRNA, i.e., the first strand of the target nucleic acid. Conversely, in some embodiments, a first gRNA is complementary with a first strand of the target nucleic acid and binds a nickase having an active HNH domain and causes that nickase to cleave the strand that is complementary to that first gRNA, i.e., a first strand of the target nucleic acid; and a second gRNA is complementary with a second strand of the target nucleic acid and binds a nickase having an active HNH domain and causes that nickase to cleave the strand that is complementary to that second gRNA, i.e., the second strand of the target nucleic acid. In another arrangement, if one Cas9 molecule has an active RuvC-like domain and the other Cas9 molecule has an active HNH domain, the gRNAs for both Cas9 molecules can be complementary to the same strand of the target nucleic acid, so that the Cas9 molecule with the active RuvC-like domain will cleave the non-complementary strand and the Cas9 molecule with the HNH domain will cleave the complementary strand, resulting in a double stranded break.

### *8. 6 Homology arms of the donor template*

A homology arm should extend at least as far as the region in which end resection may occur, e.g., in order to allow the resected single stranded overhang to find a complementary region within the donor template. The overall length could be limited by parameters such as plasmid size or viral packaging limits. In certain embodiments, a homology arm does not extend into repeated elements, e.g., Alu repeats or LINE repeats.

Exemplary homology arm lengths include at least 50, 100, 250, 500, 750, 1000, 2000, 3000, 4000, or 5000 nucleotides. In certain embodiments, the homology arm length is 50-100, 100-250, 250-500, 500-750, 750-1000, 1000-2000, 2000-3000, 3000-4000, or 4000-5000 nucleotides.

A template nucleic acid, as that term is used herein, refers to a nucleic acid sequence which can be used in conjunction with a Cas9 molecule and a gRNA molecule to alter the structure of a DMD target position. In certain embodiments, the DMD target position can be a site between two nucleotides, e.g., adjacent nucleotides, on the target nucleic acid into which one or more nucleotides is added. Alternatively, the DMD target position may comprise one or more nucleotides that are altered by a template nucleic acid.

In certain embodiments, the target nucleic acid is modified to have some or all of the sequence of the template nucleic acid, typically at or near cleavage site(s). In certain embodiments, the template nucleic acid is single stranded. In certain embodiments, the template nucleic acid is double stranded. In certain embodiments, the template nucleic acid is DNA, e.g., double stranded DNA. In certain embodiments, the template nucleic acid is single stranded DNA. In certain embodiments, the template nucleic acid is encoded on the same vector backbone, e.g. AAV genome, plasmid DNA, as the Cas9 and gRNA. In certain embodiments, the template nucleic acid is excised from a vector backbone *in vivo*, e.g., it is flanked by gRNA recognition sequences. In certain embodiments, the template nucleic acid comprises endogenous genomic sequence.

In certain embodiments, the template nucleic acid alters the structure of the target position by participating in an HDR event. In certain embodiments, the template nucleic acid alters the sequence of the target position. In certain embodiments, the template nucleic acid results in the incorporation of a modified, or non-naturally occurring base into the target nucleic acid.

Typically, the template sequence undergoes a breakage mediated or catalyzed recombination with the target sequence. In certain embodiments, the template nucleic acid includes sequence that corresponds to a site on the target sequence that is cleaved by an eaCas9 mediated cleavage event. In certain embodiments, the template nucleic acid includes sequence that corresponds to both a first site on the target sequence that is cleaved in a first Cas9 mediated event, and a second site on the target sequence that is cleaved in a second Cas9 mediated event.

A template nucleic acid having homology with a DMD target position in a *DMD* gene regulatory region can be

used to alter the structure of the regulatory region.

A template nucleic acid typically comprises the following components:

[5' homology arm]-[replacement sequence]-[3' homology arm].

The homology arms provide for recombination into the chromosome, thus replacing the undesired element, e.g., a mutation or signature, with the replacement sequence. In certain embodiments, the homology arms flank the most distal cleavage sites.

In certain embodiments, the 3' end of the 5' homology arm is the position next to the 5' end of the replacement sequence. In certain embodiments, the 5' homology arm can extend at least 10, 20, 30, 40, 50, 100, 200, 300, 400, 500, 600, 700, 800, 900, 1000, 1500, 2000, 3000, 4000, or 5000 nucleotides 5' from the 5' end of the replacement sequence.

In certain embodiments, the 5' end of the 3' homology arm is the position next to the 3' end of the replacement sequence. In an embodiment, the 3' homology arm can extend at least 10, 20, 30, 40, 50, 100, 200, 300, 400, 500, 600, 700, 800, 900, 1000, 1500, 2000, 3000, 4000, or 5000 nucleotides 3' from the 3' end of the replacement sequence.

In certain embodiments, to alter one or more nucleotides at a DMD target position, the homology arms, e.g., the 5' and 3' homology arms, may each comprise about 1000 bp of sequence flanking the most distal gRNAs (e.g., 1000 bp of sequence on either side of the DMD target position).

In certain embodiments, one or both homology arms may be shortened to avoid including certain sequence repeat elements, e.g., Alu repeats or LINE elements. For example, a 5' homology arm may be shortened to avoid a sequence repeat element. In certain embodiments, a 3' homology arm may be shortened to avoid a sequence repeat element. In certain embodiments, both the 5' and the 3' homology arms may be shortened to avoid including certain sequence repeat elements.

In certain embodiments, template nucleic acids for altering the sequence of a DMD target position may be designed for use as a single-stranded oligonucleotide, e.g., a single-stranded oligodeoxynucleotide (ssODN). When using a ssODN, 5' and 3' homology arms may range up to about 200 bp in length, e.g., at least 25, 50, 75, 100, 125, 150, 175, or 200 bp in length. Longer homology arms are also contemplated for ssODNs as improvements in oligonucleotide synthesis continue to be made. In certain embodiments, a longer homology arm is made by a method other than chemical synthesis, e.g., by denaturing a long double stranded nucleic acid and purifying one of the strands, e.g., by affinity for a strand-specific sequence anchored to a solid substrate.

In certain embodiments, alt-HDR proceeds more efficiently when the template nucleic acid has extended homology 5' to the nick (i.e., in the 5' direction of the nicked strand). Accordingly, in certain embodiments, the template nucleic acid has a longer homology arm and a shorter homology arm, wherein the longer homology arm can anneal 5' of the nick. In certain embodiments, the arm that can anneal 5' to the nick is at least 25, 50, 75, 100, 125, 150, 175, or 200, 300, 400, 500, 600, 700, 800, 900, 1000, 1500, 2000, 3000, 4000, or 5000 nucleotides from the nick or the 5' or 3' end of the replacement sequence. In certain embodiments, the arm that can anneal 5' to the nick is at least about 10%, about 20%, about 30%, about 40%, or about 50% longer than the arm that can anneal 3' to the nick. In certain embodiments, the arm that can anneal 5' to the nick is at least 2x, 3x, 4x, or 5x longer than the arm that can anneal 3' to the nick. Depending on whether a ssDNA template can anneal to the intact strand or the nicked strand, the homology arm that anneals 5' to the nick may be at the 5' end of the ssDNA template or the 3' end of the ssDNA template, respectively.

Similarly, in certain embodiments, the template nucleic acid has a 5' homology arm, a replacement sequence, and a 3' homology arm, such that the template nucleic acid has extended homology to the 5' of the nick. For example, the 5' homology arm and 3' homology arm may be substantially the same length, but the replacement sequence may extend farther 5' of the nick than 3' of the nick. In certain embodiments, the replacement sequence extends at least about 10%, about 20%, about 30%, about 40%, about 50%, 2x, 3x, 4x, or 5x further to the 5' end of the nick than the 3' end of the nick.

In certain embodiments, alt-HDR proceeds more efficiently when the template nucleic acid is centered on the nick. Accordingly, in certain embodiments, the template nucleic acid has two homology arms that are essentially the same size. For instance, the first homology arm of a template nucleic acid may have a length that is within about 10%, about 9%, about 8%, about 7%, about 6%, about 5%, about 4%, about 3%, about 2%, or about 1% of the second homology arm of the template nucleic acid.

Similarly, in certain embodiments, the template nucleic acid has a 5' homology arm, a replacement sequence, and a 3' homology arm, such that the template nucleic acid extends substantially the same distance on either side of the nick. For example, the homology arms may have different lengths, but the replacement sequence may be selected to compensate for this. For example, the replacement sequence may extend further 5' from the nick than it does 3' of the nick, but the homology arm 5' of the nick is shorter than the homology arm 3' of the nick, to compensate. The converse is also possible, e.g., that the replacement sequence may extend further 3' from the nick than it does 5' of the nick, but the homology arm 3' of the nick is shorter than the homology arm 5' of the nick, to compensate.

### *8.7 Template Nucleic Acids*

In certain embodiments, the template nucleic acid is double stranded. In certain embodiments, the template nucleic acid is single stranded. In certain embodiments, the template nucleic acid comprises a single stranded portion and a double stranded portion. In certain embodiments, the template nucleic acid comprises about 50 to 100 bp, e.g., 55 to 95, 60 to 90, 65 to 85, or 70 to 80 bp, homology on either side of the nick and/or replacement sequence. In certain embodiments, the template nucleic acid comprises about 50, 55, 60, 65, 70, 75, 80, 85, 90, 95, or 100 bp homology 5' of the nick or replacement sequence, 3' of the nick or replacement sequence, or both 5' and 3' of the nick or replacement sequences.

In certain embodiments, the template nucleic acid comprises about 150 to 200 bp, e.g., 155 to 195, 160 to 190, 165 to 185, or 170 to 180 bp, homology 3' of the nick and/or replacement sequence. In certain embodiments, the template nucleic acid comprises about 150, 155, 160, 165, 170, 175, 180, 185, 190, 195, or 200 bp homology 3' of the nick or replacement sequence. In certain embodiments, the template nucleic acid comprises less than about 100, 90, 80, 70, 60, 50, 40, 30, 20, 15, or 10 bp homology 5' of the nick or replacement sequence.

In certain embodiment, the template nucleic acid comprises about 150 to 200 bp, e.g., 155 to 195, 160 to 190, 165 to 185, or 170 to 180 bp, homology 5' of the nick and/or replacement sequence. In certain embodiment, the template nucleic acid comprises about 150, 155, 160, 165, 170, 175, 180, 185, 190, 195, or 200 bp homology 5' of the nick or replacement sequence. In certain embodiments, the template nucleic acid comprises less than about 100, 90, 80, 70, 60, 50, 40, 30, 20, 15, or 10 bp homology 3' of the nick or replacement sequence.

In certain embodiments, the template nucleic acid comprises a nucleotide sequence, e.g., of one or more nucleotides, that will be added to or will template a change in the target nucleic acid. In other embodiments, the template nucleic acid comprises a nucleotide sequence that may be used to modify the target position.

The template nucleic acid may comprise a replacement sequence. In certain embodiments, the template nucleic acid comprises a 5' homology arm. In certain embodiments, the template nucleic acid comprises a 3' homology arm.

In certain embodiments, the template nucleic acid is linear double stranded DNA. The length may be, e.g., about 150-200 bp, e.g., about 150, 160, 170, 180, 190, or 200 bp. The length may be, e.g., at least 150, 160, 170, 180, 190, or 200 bp. In certain embodiments, the length is no greater than 150, 160, 170, 180, 190, or 200 bp. In certain embodiments, a double stranded template nucleic acid has a length of about 160 bp, e.g., about 155-165, 150-170, 140-180, 130-190, 120-200, 110-210, 100-220, 90-230, or 80-240 bp.

The template nucleic acid can be linear single stranded DNA. In certain embodiments, the template nucleic acid is (i) linear single stranded DNA that can anneal to the nicked strand of the target nucleic acid, (ii) linear single stranded DNA that can anneal to the intact strand of the target nucleic acid, (iii) linear single stranded DNA that can anneal to the plus strand of the target nucleic acid, (iv) linear single stranded DNA that can anneal to the minus strand of the target nucleic acid, or more than one of the preceding. The length may be, e.g., about 150-200 nucleotides, e.g., about 150, 160, 170, 180, 190, or 200 nucleotides. The length may be, e.g., at least 150, 160, 170, 180, 190, or 200 nucleotides. In certain embodiments, the length is no greater than 150, 160, 170, 180, 190, or 200 nucleotides. In certain embodiments, a single stranded template nucleic acid has a length of about 160 nucleotides, e.g., about 155-165, 150-170, 140-180, 130-190, 120-200, 110-210, 100-220, 90-230, or 80-240 nucleotides.

In certain embodiments, the template nucleic acid is circular double stranded DNA, e.g., a plasmid. In certain embodiments, the template nucleic acid comprises about 500 to 1000 bp of homology on either side of the replacement sequence and/or the nick. In certain embodiments, the template nucleic acid comprises about 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In certain embodiments, the template nucleic acid comprises at least 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In certain embodiments, the template nucleic acid comprises no more than 300, 400, 500, 600, 700, 800, 900, 1000, 1500, or 2000 bp of homology 5' of the nick or replacement sequence, 3' of the nick or replacement sequence, or both 5' and 3' of the nick or replacement sequence.

In certain embodiments, one or both homology arms may be shortened to avoid including certain sequence repeat elements, e.g., Alu repeats, LINE elements. For example, a 5' homology arm may be shortened to avoid a sequence repeat element, while a 3' homology arm may be shortened to avoid a sequence repeat element. In certain embodiments, both the 5' and the 3' homology arms may be shortened to avoid including certain sequence repeat elements.

In certain embodiments, the template nucleic acid is an adenovirus vector, e.g., an AAV vector, e.g., a ssDNA molecule of a length and sequence that allows it to be packaged in an AAV capsid. The vector may be, e.g., less than 5 kb and may contain an ITR sequence that promotes packaging into the capsid. The vector may be integration-deficient. In certain embodiments, the template nucleic acid comprises about 150 to 1000 nucleotides of homology on either side of the replacement sequence and/or the nick. In certain embodiments, the template nucleic acid comprises about 100,

150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In certain embodiments, the template nucleic acid comprises at least 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In certain embodiments, the template nucleic acid comprises at most 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence.

In certain embodiments, the template nucleic acid is a lentiviral vector, e.g., an IDLV (integration deficiency lentivirus). In certain embodiments, the template nucleic acid comprises about 500 to 1000 bp of homology on either side of the replacement sequence and/or the nick. In some embodiments, the template nucleic acid comprises about 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In certain embodiments, the template nucleic acid comprises at least 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In certain embodiments, the template nucleic acid comprises no more than 300, 400, 500, 600, 700, 800, 900, 1000, 1500, or 2000 bp of homology 5' of the nick or replacement sequence, 3' of the nick or replacement sequence, or both 5' and 3' of the nick or replacement sequence.

In certain embodiments, the template nucleic acid comprises one or more mutations, e.g., silent mutations, that prevent Cas9 from recognizing and cleaving the template nucleic acid. The template nucleic acid may comprise, e.g., at least 1, 2, 3, 4, 5, 10, 20, or 30 silent mutations relative to the corresponding sequence in the genome of the cell to be altered. In certain embodiments, the template nucleic acid comprises at most 2, 3, 4, 5, 10, 20, 30, or 50 silent mutations relative to the corresponding sequence in the genome of the cell to be altered. In certain embodiments, the cDNA comprises one or more mutations, e.g., silent mutations that prevent Cas9 from recognizing and cleaving the template nucleic acid. The template nucleic acid may comprise, e.g., at least 1, 2, 3, 4, 5, 10, 20, or 30 silent mutations relative to the corresponding sequence in the genome of the cell to be altered. In certain embodiments, the template nucleic acid comprises at most 2, 3, 4, 5, 10, 20, 30, or 50 silent mutations relative to the corresponding sequence in the genome of the cell to be altered.

In certain embodiments, the 5' and 3' homology arms each comprise a length of sequence flanking the nucleotides corresponding to the replacement sequence. In certain embodiments, a template nucleic acid comprises a replacement sequence flanked by a 5' homology arm and a 3' homology arm each independently comprising 10 or more, 20 or more, 50 or more, 100 or more, 150 or more, 200 or more, 250 or more, 300 or more, 350 or more, 400 or more, 450 or more, 500 or more, 550 or more, 600 or more, 650 or more, 700 or more, 750 or more, 800 or more, 850 or more, 900 or more, 1000 or more, 1100 or more, 1200 or more, 1300 or more, 1400 or more, 1500 or more, 1600 or more, 1700 or more, 1800 or more, 1900 or more, or 2000 or more nucleotides. In certain embodiments, a template nucleic acid comprises a replacement sequence flanked by a 5' homology arm and a 3' homology arm each independently comprising at least 50, 100, or 150 nucleotides, but not long enough to include a repeated element. In certain embodiments, a template nucleic acid comprises a replacement sequence flanked by a 5' homology arm and a 3' homology arm each independently comprising 5 to 100, 10 to 150, or 20 to 150 nucleotides. In certain embodiments, the replacement sequence optionally comprises a promoter and/or polyA signal.

### *8.8 Single-Strand Annealing*

Single strand annealing (SSA) is another DNA repair process that repairs a double-strand break between two repeat sequences present in a target nucleic acid. Repeat sequences utilized by the SSA pathway are generally greater than 30 nucleotides in length. Resection at the break ends occurs to reveal repeat sequences on both strands of the target nucleic acid. After resection, single strand overhangs containing the repeat sequences are coated with RPA protein to prevent the repeats sequences from inappropriate annealing, e.g., to themselves. RAD52 binds to and each of the repeat sequences on the overhangs and aligns the sequences to enable the annealing of the complementary repeat sequences. After annealing, the single-strand flaps of the overhangs are cleaved. New DNA synthesis fills in any gaps, and ligation restores the DNA duplex. As a result of the processing, the DNA sequence between the two repeats is deleted. The length of the deletion can depend on many factors including the location of the two repeats utilized, and the pathway or processivity of the resection.

In contrast to HDR pathways, SSA does not require a template nucleic acid to alter a target nucleic acid sequence. Instead, the complementary repeat sequence is utilized.

### *8.9 Other DNA Repair Pathways*

#### *8.9.1 SSBR (single strand break repair)*

Single-stranded breaks (SSB) in the genome are repaired by the SSBR pathway, which is a distinct mechanism from the DSB repair mechanisms discussed above. The SSBR pathway has four major stages: SSB detection, DNA end processing, DNA gap filling, and DNA ligation. A more detailed explanation is given in Caldecott 2008, and a summary is given here.

In the first stage, when a SSB forms, PARP1 and/or PARP2 recognize the break and recruit repair machinery. The binding and activity of PARP1 at DNA breaks is transient and it seems to accelerate SSBr by promoting the focal accumulation or stability of SSBr protein complexes at the lesion. Arguably the most important of these SSBr proteins is XRCC1, which functions as a molecular scaffold that interacts with, stabilizes, and stimulates multiple enzymatic components of the SSBr process including the protein responsible for cleaning the DNA 3' and 5' ends. For instance, XRCC1 interacts with several proteins (DNA polymerase beta, PNK, and three nucleases, APE1, APTX, and APLF) that promote end processing. APE1 has endonuclease activity. APLF exhibits endonuclease and 3' to 5' exonuclease activities. APTX has endonuclease and 3' to 5' exonuclease activity.

This end processing is an important stage of SSBR since the 3'- and/or 5'-termini of most, if not all, SSBs are 'damaged.' End processing generally involves restoring a damaged 3'-end to a hydroxylated state and and/or a damaged 5' end to a phosphate moiety, so that the ends become ligation-competent. Enzymes that can process damaged 3' termini include PNKP, APE1, and TDP1. Enzymes that can process damaged 5' termini include PNKP, DNA polymerase beta, and APTX. LIG3 (DNA ligase III) can also participate in end processing. Once the ends are cleaned, gap filling can occur.

At the DNA gap filling stage, the proteins typically present are PARP1, DNA polymerase beta, XRCC1, FEN1 (flap endonuclease 1), DNA polymerase delta/epsilon, PCNA, and LIG1. There are two ways of gap filling, the short patch repair and the long patch repair. Short patch repair involves the insertion of a single nucleotide that is missing. At some SSBs, "gap filling" might continue displacing two or more nucleotides (displacement of up to 12 bases have been reported). FEN1 is an endonuclease that removes the displaced 5'-residues. Multiple DNA polymerases, including Pol$\beta$, are involved in the repair of SSBs, with the choice of DNA polymerase influenced by the source and type of SSB.

In the fourth stage, a DNA ligase such as LIG1 (Ligase I) or LIG3 (Ligase III) catalyzes joining of the ends. Short patch repair uses Ligase III and long patch repair uses Ligase I.

Sometimes, SSBR is replication-coupled. This pathway can involve one or more of CtIP, MRN, ERCC1, and FEN1. Additional factors that may promote SSBR include: aPARP, PARP1, PARP2, PARG, XRCC1, DNA polymerase b, DNA polymerase d, DNA polymerase e, PCNA, LIG1, PNK, PNKP, APE1, APTX, APLF, TDP1, LIG3, FEN1, CtIP, MRN, and ERCC1.

#### *8.9.2 MMR (mismatch repair)*

Cells contain three excision repair pathways: MMR, BER, and NER. The excision repair pathways have a common feature in that they typically recognize a lesion on one strand of the DNA, then exo/endonucleases remove the lesion and leave a 1-30 nucleotide gap that is sub-sequentially filled in by DNA polymerase and finally sealed with ligase. A more complete picture is given in Li, Cell Research (2008) 18:85-98, and a summary is provided here.

Mismatch repair (MMR) operates on mispaired DNA bases.

The MSH2/6 or MSH2/3 complexes both have ATPases activity that plays an important role in mismatch recognition and the initiation of repair. MSH2/6 preferentially recognizes base-base mismatches and identifies mispairs of 1 or 2 nucleotides, while MSH2/3 preferentially recognizes larger ID mispairs.

hMLH1 heterodimerizes with hPMS2 to form hMutL $\alpha$ which possesses an ATPase activity and is important for multiple steps of MMR. It possesses a PCNA/replication factor C (RFC)-dependent endonuclease activity which plays an important role in 3' nick-directed MMR involving EXO1. (EXO1 is a participant in both HR and MMR.) It regulates termination of mismatch-provoked excision. Ligase I is the relevant ligase for this pathway. Additional factors that may promote MMR include: EXO1, MSH2, MSH3, MSH6, MLH1, PMS2, MLH3, DNA Pol d, RPA, HMGB1, RFC, and DNA ligase I.

#### *8.9.3 Base excision repair (BER)*

The base excision repair (BER) pathway is active throughout the cell cycle; it is responsible primarily for removing small, non-helix-distorting base lesions from the genome. In contrast, the related Nucleotide Excision Repair pathway (discussed in the next section) repairs bulky helix-distorting lesions. A more detailed explanation is given in Caldecott, Nature Reviews Genetics 9, 619-631 (August 2008), and a summary is given here.

Upon DNA base damage, base excision repair (BER) is initiated and the process can be simplified into five major steps: (a) removal of the damaged DNA base; (b) incision of the subsequent a basic site; (c) clean-up of the DNA ends; (d) insertion of the desired nucleotide into the repair gap; and (e) ligation of the remaining nick in the DNA backbone. These last steps are similar to the SSBR.

In the first step, a damage-specific DNA glycosylase excises the damaged base through cleavage of the N-glycosidic bond linking the base to the sugar phosphate backbone. Then AP endonuclease-1 (APE1) or bifunctional DNA glycosylases with an associated lyase activity incised the phosphodiester backbone to create a DNA single strand break (SSB). The third step of BER involves cleaning-up of the DNA ends. The fourth step in BER is conducted by Polβ that adds a new complementary nucleotide into the repair gap and in the final step XRCC1/Ligase III seals the remaining nick in the DNA backbone. This completes the short-patch BER pathway in which the majority (~80%) of damaged DNA bases are repaired. However, if the 5' ends in step 3 are resistant to end processing activity, following one nucleotide insertion by Pol β there is then a polymerase switch to the replicative DNA polymerases, Pol δ/ε, which then add ~2-8 more nucleotides into the DNA repair gap. This creates a 5' flap structure, which is recognized and excised by flap endonuclease-1 (FEN-1) in association with the processivity factor proliferating cell nuclear antigen (PCNA). DNA ligase I then seals the remaining nick in the DNA backbone and completes long-patch BER. Additional factors that may promote the BER pathway include: DNA glycosylase, APE1, Polb, Pold, Pole, XRCC1, Ligase III, FEN-1, PCNA, RECQL4, WRN, MYH, PNKP, and APTX.

*8.9.4 Nucleotide excision repair (NER)*

Nucleotide excision repair (NER) is an important excision mechanism that removes bulky helix-distorting lesions from DNA. Additional details about NER are given in Marteijn et al., Nature Reviews Molecular Cell Biology 15, 465-481 (2014), and a summary is given here. NER a broad pathway encompassing two smaller pathways: global genomic NER (GG-NER) and transcription coupled repair NER (TC-NER). GG-NER and TC-NER use different factors for recognizing DNA damage. However, they utilize the same machinery for lesion incision, repair, and ligation.

Once damage is recognized, the cell removes a short single-stranded DNA segment that contains the lesion. Endonucleases XPF/ERCC1 and XPG (encoded by ERCC5) remove the lesion by cutting the damaged strand on either side of the lesion, resulting in a single-strand gap of 22-30 nucleotides. Next, the cell performs DNA gap filling synthesis and ligation. Involved in this process are: PCNA, RFC, DNA Pol δ, DNA Pol ε or DNA Pol κ, and DNA ligase I or XRCC1/Ligase III. Replicating cells tend to use DNA pol ε and DNA ligase I, while non-replicating cells tend to use DNA Pol δ, DNA Pol κ, and the XRCC1/ Ligase III complex to perform the ligation step.

NER can involve the following factors: XPA-G, POLH, XPF, ERCC1, XPA-G, and LIG1. Transcription-coupled NER (TC-NER) can involve the following factors: CSA, CSB, XPB, XPD, XPG, ERCC1, and TTDA. Additional factors that may promote the NER repair pathway include XPA-G, POLH, XPF, ERCC1, XPA-G, LIG1, CSA, CSB, XPA, XPB, XPC, XPD, XPF, XPG, TTDA, UVSSA, USP7, CETN2, RAD23B, UV-DDB, CAK subcomplex, RPA, and PCNA.

*8.9.5 Interstrand Crosslink (ICL)*

A dedicated pathway called the ICL repair pathway repairs interstrand crosslinks. Interstrand crosslinks, or covalent crosslinks between bases in different DNA strand, can occur during replication or transcription. ICL repair involves the coordination of multiple repair processes, in particular, nucleolytic activity, translesion synthesis (TLS), and HDR. Nucleases are recruited to excise the ICL on either side of the crosslinked bases, while TLS and HDR are coordinated to repair the cut strands. ICL repair can involve the following factors: endonucleases, e.g., XPF and RAD51C, endonucleases such as RAD51, translesion polymerases, e.g., DNA polymerase zeta and Rev1), and the Fanconi anemia (FA) proteins, e.g., FancJ.

*8.9.6 Other pathways*

Several other DNA repair pathways exist in mammals.

Translesion synthesis (TLS) is a pathway for repairing a single stranded break left after a defective replication event and involves translesion polymerases, e.g., DNA polβ and Rev1.

Error-free postreplication repair (PRR) is another pathway for repairing a single stranded break left after a defective replication event.

***8.10 Examples of gRNAs in Genome Editing Methods***

gRNA molecules as described herein can be used with Cas9 molecules that generate a double strand break or a single strand break to alter the sequence of a target nucleic acid, e.g., a target position or target genetic signature.

gRNA molecules useful in these methods are described below.

In certain embodiments, the gRNA, e.g., a chimeric gRNA, is configured such that it comprises one or more of the following properties;

(a) it can position, e.g., when targeting a Cas9 molecule that makes double strand breaks, a double strand break (i) within 50, 100, 150, 200, 250, 300, 350, 400, 450, or 500 nucleotides of a target position, or (ii) sufficiently close that the target position is within the region of end resection;
(b) it has a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
(c)(i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes,* or *S. aureus* tail and proximal domain, or a sequence that differs by no more than 1, 2, 3, 4, 5; 6, 7, 8, 9 or 10 nucleotides therefrom;
(c)(ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes,* or *S. aureus* gRNA, or a sequence that differs by no more than 1, 2, 3, 4, 5; 6, 7, 8, 9 or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes*, or *S. aureus* gRNA, or a sequence that differs by no more than 1, 2, 3, 4, 5; 6, 7, 8, 9 or 10 nucleotides therefrom;
(c)(iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes,* or *S. aureus* tail domain, or a sequence that differs by no more than 1, 2, 3, 4, 5; 6, 7, 8, 9 or 10 nucleotides therefrom; or
(c)(v) the tail domain comprises 15, 20, 25, 30, 35, 40 nucleotides or all of the corresponding portions of a naturally occurring tail domain, e.g., a naturally occurring *S. pyogenes*, or *S. aureus* tail domain.

In certain embodiments, the gRNA is configured such that it comprises properties: a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(ii), and c(i); a(i), b(ii), and c(ii); a(i), b(iii), and c(i); a(i), b(iii), and c(ii); a(i), b(iv), and c(i); a(i), b(iv), and c(ii); a(i), b(v), and c(i); a(i), b(v), and c(ii); a(i), b(vi), and c(i); a(i), b(vi), and c(ii); a(i), b(vii), and c(i); a(i), b(vii), and c(ii); a(i), b(viii), and c(i); a(i), b(viii), and c(ii); a(i), b(ix), and c(i); a(i), b(ix), and c(ii); a(i), b(x), and c(i); a(i), b(x), and c(ii); a(i), b(xi), or c(i); a(i), b(xi), and c(ii).

In certain embodiments, the gRNA, e.g., a chimeric gRNA, is configured such that it comprises one or more of the following properties;

(a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;
(b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
(c)(i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes*, or *S. aureus* tail and proximal domain, or a sequence that differs by no more than 1, 2, 3, 4, 5, 6, 7, 8, 9 or 10 nucleotides therefrom;
(c)(ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes*, or *S. aureus* gRNA, or a sequence that differs by no more than 1, 2, 3, 4, 5; 6, 7, 8, 9 or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes*, or *S. aureus* gRNA, or a sequence that differs by no more than 1, 2, 3, 4, 5; 6, 7, 8, 9 or 10 nucleotides therefrom;
(c)(iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes*, or *S. aureus* tail domain, or a sequence that differs by no more than 1, 2, 3, 4, 5; 6, 7, 8, 9 or 10 nucleotides therefrom; or

(c)(v) the tail domain comprises 15, 20, 25, 30, 35, 40 nucleotides or all of the corresponding portions of a naturally occurring tail domain, e.g., a naturally occurring *S. pyogenes*, or *S. aureus* tail domain.

In certain embodiments, the gRNA is configured such that it comprises properties: a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(ii), and c(i); a(i), b(ii), and c(ii); a(i), b(iii), and c(i); a(i), b(iii), and c(ii); a(i), b(iv), and c(i); a(i), b(iv), and c(ii); a(i), b(v), and c(i); a(i), b(v), and c(ii); a(i), b(vi), and c(i); a(i), b(vi), and c(ii); a(i), b(vii), and c(i); a(i), b(vii), and c(ii); a(i), b(viii), and c(i); a(i), b(viii), and c(ii); a(i), b(ix), and c(i); a(i), b(ix), and c(ii); a(i), b(x), and c(i); a(i), b(x), and c(ii); a(i), b(xi), and c(i); a(i), b(xi), and c(ii)..

In certain embodiments, the gRNA is used with a Cas9 nickase molecule having HNH activity, e.g., a Cas9 molecule having the RuvC activity inactivated, e.g., a Cas9 molecule having a mutation at D10, e.g., the D10A mutation. In certain embodiments, the gRNA is used with a Cas9 nickase molecule having RuvC activity, e.g., a Cas9 molecule having the HNH activity inactivated, e.g., a Cas9 molecule having a mutation at 840, e.g., the H840A. In certain embodiments, the gRNAs are used with a Cas9 nickase molecule having RuvC activity, e.g., a Cas9 molecule having the HNH activity inactivated, e.g., a Cas9 molecule having a mutation at N863, e.g., the N863A mutation.

In certain embodiments, a pair of gRNAs, e.g., a pair of chimeric gRNAs, comprising a first and a second gRNA, is configured such that they comprises one or more of the following properties;

(a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;

(b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides;

(c) (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes*, or *S. aureus* tail and proximal domain, or a sequence that differs by no more than 1, 2, 3, 4, 5; 6, 7, 8, 9 or 10 nucleotides therefrom;

(c)(ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes*, or *S. aureus* gRNA, or a sequence that differs by no more than 1, 2, 3, 4, 5; 6, 7, 8, 9 or 10 nucleotides therefrom;

(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes*, or *S. aureus* gRNA, or a sequence that differs by no more than 1, 2, 3, 4, 5; 6, 7, 8, 9 or 10 nucleotides therefrom;

(c)(iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes*, or *S. aureus* tail domain; or, or a sequence that differs by no more than 1, 2, 3, 4, 5; 6, 7, 8, 9 or 10 nucleotides therefrom; or

(c)(v) the tail domain comprises 15, 20, 25, 30, 35, 40 nucleotides or all of the corresponding portions of a naturally occurring tail domain, e.g., a naturally occurring *S. pyogenes*, or *S. aureus* tail domain;

(d) the gRNAs are configured such that, when hybridized to target nucleic acid, they are separated by 0-50, 0-100, 0-200, at least 10, at least 20, at least 30 or at least 50 nucleotides;

(e) the breaks made by the first gRNA and second gRNA are on different strands; and

(f) the PAMs are facing outwards.

In certain embodiments, one or both of the gRNAs is configured such that it comprises properties: a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(i), c, and d; a(i), b(i), c, and e; a(i), b(i), c, d, and e; a(i), b(ii), and c(i); a(i), b(ii), and c(ii); a(i), b(ii), c, and d; a(i), b(ii), c, and e; a(i), b(ii), c, d, and e; a(i), b(iii), and c(i); a(i), b(iii), and c(ii); a(i), b(iii), c, and d; a(i), b(iii), c, and e; a(i), b(iii), c, d, and e; a(i), b(iv), and c(i); a(i), b(iv), and c(ii); a(i), b(iv), c, and d; a(i), b(iv), c, and e; a(i), b(iv), c, d, and e; a(i), b(v), and c(i); a(i), b(v), and c(ii); a(i), b(v), c, and d; a(i), b(v), c, and e; a(i), b(v), c, d, and e; a(i), b(vi), and c(i); a(i), b(vi), and c(ii); a(i), b(vi), c, and d; a(i), b(vi), c, and e; a(i), b(vi), c, d, and e; a(i), b(vii), and c(i); a(i), b(vii), and c(ii); a(i), b(vii), c, and d; a(i), b(vii), c, and e; a(i), b(vii), c, d, and e; a(i), b(viii), and c(i); a(i), b(viii), and c(ii); a(i), b(viii), c, and d; a(i), b(viii), c, and e; a(i), b(viii), c, d, and e; a(i), b(ix), and c(i); a(i), b(ix), and c(ii); a(i), b(ix), c, and d; a(i), b(ix), c, and e; a(i), b(ix), c, d, and e; a(i), b(x), and c(i); a(i), b(x), and c(ii); a(i), b(x), c, and d; a(i), b(x), c, and e; a(i), b(x), c, d, and e; a(i), b(xi), and c(i); a(i), b(xi), and c(ii); a(i), b(xi), c, and d; a(i), b(xi), c, and e; a(i), b(xi), c, d, and e.

In certain embodiments, the gRNAs are used with a Cas9 nickase molecule having HNH activity, e.g., a Cas9 molecule having the RuvC activity inactivated, e.g., a Cas9 molecule having a mutation at D10, e.g., the D10A mutation. In certain embodiments, the gRNAs are used with a Cas9 nickase molecule having RuvC activity, e.g., a Cas9 molecule having the HNH activity inactivated, e.g., a Cas9 molecule having a mutation at H840, e.g., the H840A mutation. In certain embodiments, the gRNAs are used with a Cas9 nickase molecule having RuvC activity, e.g., a Cas9 molecule having the HNH activity inactivated, e.g., a Cas9 molecule having a mutation at N863, e.g., the N863A mutation.

**9. Targets: Cells**

Cas9 molecules and gRNA molecules, e.g., a Cas9 molecule/gRNA molecule complex, can be used to manipulate a cell, e.g., to edit a target nucleic acid, in a wide variety of cells.

In certain embodiments, a cell is manipulated by altering one or more editing (e.g., introducing a mutation in) a *DMD* gene, as described herein. In certain embodiments, the expression of *DMD* gene is modulated, e.g., *in vivo.* In certain embodiments, the expression of the *DMD* gene is modulated, e.g., *ex vivo.*

The Cas9 and gRNA molecules described herein can be delivered to a target cell. In certain embodiments, the target cell is a skeletal muscle cell, e.g., a red (slow) skeletal muscle cell, a white (fast) skeletal muscle cell or an intermediate skeletal muscle cell. In certain embodiments, the target cell is a cardiac muscle cell, e.g., a cardiomyocyte or a nodal cardiac muscle cell. In certain embodiments, the target cell is a smooth muscle cell. In certain embodiments, the target cell is a muscle satellite cell or muscle stem cell.

In certain embodiments, the target cell is manipulated *ex vivo* by altering a DMD target position, then the target cell is administered to the subject. Sources of target cells for *ex vivo* manipulation may include, for example, the subject's blood, bone marrow, or cord blood. Other sources of target cells for *ex vivo* manipulation may include, for example, heterologous donor blood, cord blood, or bone marrow. In certain embodiments, a skeletal muscle cell, e.g., a red (slow) skeletal muscle cell, a white (fast) skeletal muscle cell or an intermediate skeletal muscle cell is removed from the subject, the gene altered *ex vivo,* manipulated *ex vivo* as described above, and the cell is returned to the subject. In certain embodiments, a cardiac muscle cell, e.g., a cardiomyocyte or a nodal cardiac muscle cell is removed from the subject, manipulated *ex vivo* as described above, and the cell is returned to the subject. In certain embodiments, a smooth muscle cell is removed from the subject, manipulated *ex vivo* as described above, and the cell is returned to the subject. In certain embodiments, a muscle satellite cell or muscle stem cell is removed from the subject, manipulated *ex vivo* as described above, and the cell is returned to the subject.

In certain embodiments, the cells are induced pluripotent stem cells (iPS) cells or cells derived from iPS cells, e.g., iPS cells from the subject, modified to alter the gene and differentiated into myoblasts, muscle progenitor cells, muscle satellite cells, muscle stem cells, skeletal muscle cells, cardiac muscle cells or smooth muscle cells and transplanted into the subject.

In certain embodiments, the cells are targeted *in vivo*, e.g., by delivery of the components, e.g., a Cas9 molecule and one or more gRNA molecules, to the target cells. In certain embodiments, the target cells are myoblasts, muscle progenitor cells, muscle satellite cells, muscle stem cells, skeletal muscle cells, cardiac muscle cells, smooth muscle cells, or a combination thereof. In certain embodiments, AAV is used to deliver the components, e.g., a Cas9 molecule and a gRNA molecule, e.g., by transducing the target cells.

**10. Delivery, Formulations and Routes of Administration**

The components, e.g., a Cas9 molecule, one or more gRNA molecules (e.g., a Cas9 molecule/gRNA molecule complex), and a donor template nucleic acid, or all three, can be delivered, formulated, or administered in a variety of forms, see, e.g., **Tables 3 and 4.** In certain embodiments, the Cas9 molecule, one or more gRNA molecules (e.g., two gRNA molecules) are present together in a genome-editing system. In certain embodiments, one Cas9 molecule and two or more (e.g., 2, 3, 4, or more) different gRNA molecules are delivered, e.g., by an AAV vector. In certain embodiments, the sequence encoding the Cas9 molecule and the sequence(s) encoding the two or more (e.g., 2, 3, 4, or more) different gRNA molecules are present on the same nucleic acid molecule, e.g., an AAV vector. When a Cas9 or gRNA component is delivered encoded in DNA the DNA will typically include a control region, e.g., comprising a promoter, to effect expression. Useful promoters for Cas9 molecule sequences include CMV, EFS, EF-1a, MSCV, PGK, CAG, the Skeletal Alpha Actin promoter, the Muscle Creatine Kinase promoter, the Dystrophin promoter, the Alpha Myosin Heavy Chain promoter, and the Smooth Muscle Actin promoter. In certain embodiments, the promoter is a constitutive promoter. In certain embodiments, the promoter is a tissue specific promoter. Useful promoters for gRNAs include T7.H1, EF-1a, 7SK, U6, U1 and tRNA promoters. Promoters with similar or dissimilar strengths can be selected to tune the expression of components. Sequences encoding a Cas9 molecule can comprise a nuclear localization signal (NLS), e.g., an SV40 NLS. In certain embodiments, the sequence encoding a Cas9 molecule comprises at least two nuclear localization signals. In certain embodiments a promoter for a Cas9 molecule or a gRNA molecule can be, independently, inducible,

tissue specific, or cell specific. **Table 3** provides examples of how the components can be formulated, delivered, or administered.

**Table 3**

| Elements | | | |
|---|---|---|---|
| **Cas9 Molecule (s)** | **gRNA Molecule (s)** | **Donor Template Nucleic Acid** | **Comments** |
| DNA | DNA | DNA | In certain embodiments, a Cas9 molecule, typically an eaCas9 molecule, and a gRNA are transcribed from DNA. In certain embodiments, they are encoded on separate molecules. In certain embodiments, the donor template is provided as a separate DNA molecule. |
| DNA | DNA | | In certain embodiments, a Cas9 molecule, typically an eaCas9 molecule, and a gRNA are transcribed from DNA. In this embodiment, they are encoded on separate molecules. In certain embodiments t, the donor template is provided on the same DNA molecule that encodes the gRNA. |
| DNA | | DNA | In certain embodiments, a Cas9 molecule, typically an eaCas9 molecule, and a gRNA are transcribed from DNA, here from a single molecule. In certain embodiments, the donor template is |
| | | | provided as a separate DNA molecule. |
| DNA | DNA | | In certain embodiments, a Cas9 molecule, typically an eaCas9 molecule, and a gRNA are transcribed from DNA. In certain embodiments, they are encoded on separate molecules. In certain embodiments, the donor template is provided on the same DNA molecule that encodes the Cas9. |
| DNA | RNA | DNA | In certain embodiments, a Cas9 molecule, typically an eaCas9 molecule, is transcribed from DNA, and a gRNA is provided as in vitro transcribed or synthesized RNA. In certain embodiments, the donor template is provided as a separate DNA molecule. |
| DNA | RNA | | In certain embodiments, a Cas9 molecule, typically an eaCas9 molecule, is transcribed from DNA, and a gRNA is provided as *in vitro* transcribed or synthesized RNA. In certain embodiments t, the donor template is provided on the same DNA molecule that encodes the Cas9. |
| mRNA | RNA | DNA | In certain embodiments, a Cas9 molecule, typically an eaCas9 molecule, is translated from in vitro transcribed mRNA, and a gRNA is provided as in vitro transcribed or synthesized RNA. In certain embodiments, the donor template is provided as a DNA molecule. |
| mRNA | DNA | DNA | In certain embodiments, a Cas9 molecule, typically an eaCas9 molecule, is translated from in vitro transcribed mRNA, and a gRNA is transcribed from DNA. In certain embodiments, the donor template is provided as a separate DNA molecule. |
| mRNA | DNA | | In certain embodiments, a Cas9 molecule, typically an eaCas9 molecule, is translated from in vitro transcribed mRNA, and a gRNA is transcribed from DNA. In certain embodiments, the donor template is provided on the same DNA molecule that encodes the gRNA. |
| Protein | DNA | DNA | In certain embodiments, a Cas9 molecule, typically an eaCas9 molecule, is provided as a protein, and a gRNA is transcribed from DNA. In certain |

(continued)

| Elements | | | |
|---|---|---|---|
| **Cas9 Molecule (s)** | **gRNA Molecule (s)** | **Donor Template Nucleic Acid** | **Comments** |
| | | | embodiments, the donor template is provided as a separate DNA molecule. |
| Protein | DNA | | In certain embodiments, a Cas9 molecule, typically an eaCas9 molecule, is provided as a protein, and a gRNA is transcribed from DNA. In certain embodiments, the donor template is provided on the same DNA molecule that encodes the gRNA. |
| Protein | RNA | DNA | In certain embodiments, an eaCas9 molecule is provided as a protein, and a gRNA is provided as transcribed or synthesized RNA. In certain embodiments, the donor template is provided as a DNA molecule. |

**Table 4** summarizes various delivery methods for the components of a Cas system, e.g., the Cas9 molecule component and the gRNA molecule component, as described herein.

**Table 4**

| **Delivery Vector/Mode** | | **Delivery into Non-Dividing Cells** | **Duration of Expression** | **Genome Integration** | **Type of Molecule Delivered** |
|---|---|---|---|---|---|
| Physical (e.g., electroporation, particle gun, Calcium Phosphate transfection, cell compression or squeezing) | | YES | Transient | NO | Nucleic Acids and Proteins |
| *Viral* | Retrovirus | NO | Stable | YES | RNA |
| | Lentivirus | YES | Stable | YES/NO with modifications | RNA |
| | Adenovirus | YES | Transient | NO | DNA |
| | Adeno-Associated Virus (AAV) | YES | Stable | NO | DNA |
| | Vaccinia Virus | YES | Very Transient | NO | DNA |
| | Herpes Simplex Virus | YES | Stable | NO | DNA |
| *Non-Viral* | Cationic Liposomes | YES | Transient | Depends on what is delivered | Nucleic Acids and Proteins |
| | Polymeric Nanoparticles | YES | Transient | Depends on what is delivered | Nucleic Acids and Proteins |
| *Biological Non-Viral Delivery Vehicles* | Attenuated Bacteria | YES | Transient | NO | Nucleic Acids |
| | Engineered Bacteriophages | YES | Transient | NO | Nucleic Acids |
| | Mammalian Virus-like Particles | YES | Transient | NO | Nucleic Acids |
| | Biological liposomes: Erythrocyte | YES | Transient | NO | Nucleic Acids |

(continued)

| Delivery Vector/Mode | | Delivery into Non-Dividing Cells | Duration of Expression | Genome Integration | Type of Molecule Delivered |
|---|---|---|---|---|---|
| | Ghosts and Exosomes | | | | |

### *10.1 DNA-based Delivery of a Cas9 molecule and/or one or more gRNA molecules*

Nucleic acid compositions encoding Cas9 molecules (e.g., eaCas9 molecules), gRNA molecules, a donor template nucleic acid, or any combination (e.g., two or all) thereof can be administered to subjects or delivered into cells by art-known methods or as described herein. For example, Cas9-encoding and/or gRNA-encoding DNA, as well as donor template nucleic acids can be delivered by, e.g., vectors (e.g., viral or non-viral vectors), non-vector based methods (e.g., using naked DNA or DNA complexes), or a combination thereof.

Nucleic acid compositions encoding Cas9 molecules (e.g., eaCas9 molecules) and/or gRNA molecules can be conjugated to molecules (e.g., N-acetylgalactosamine) promoting uptake by the target cells (e.g., hepatocytes). Donor template molecules can likewise be conjugated to molecules (e.g., N-acetylgalactosamine) promoting uptake by the target cells (e.g., hepatocytes).

In certain embodiments, the Cas9- and/or gRNA-encoding DNA is delivered by a vector (e.g., viral vector/virus or plasmid).

Vectors can comprise a sequence that encodes a Cas9 molecule and/or a gRNA molecule and/or a donor template with high homology to the region (e.g., target sequence) being targeted. In certain embodiments, the donor template comprises all or part of a target sequence. Exemplary donor templates are a repair template, e.g., a gene correction template, or a gene mutation template, e.g., point mutation (e.g., single nucleotide (nt) substitution) template). A vector can also comprise a sequence encoding a signal peptide (*e.g.,* for nuclear localization, nucleolar localization, or mitochondrial localization), fused, e.g., to a Cas9 molecule sequence. For example, the vectors can comprise a nuclear localization sequence (e.g., from SV40) fused to the sequence encoding the Cas9 molecule.

One or more regulatory/control elements, e.g., promoters, enhancers, introns, polyadenylation signals, a Kozak consensus sequences, internal ribosome entry sites (IRES), a 2A sequence, and splice acceptor or donor can be included in the vectors. In certain embodiments, the promoter is recognized by RNA polymerase II (e.g., a CMV promoter). In certain embodiments, the promoter is recognized by RNA polymerase III (e.g., a U6 promoter). In certain embodiments, the promoter is a regulated promoter (e.g., inducible promoter). In certain embodiments, the promoter is a constitutive promoter. In certain embodiments, the promoter is a tissue specific promoter. In certain embodiments, the promoter is a viral promoter. In other embodiments, the promoter is a non-viral promoter.

In certain embodiments, the vector or delivery vehicle is a viral vector (e.g., for generation of recombinant viruses). In certain embodiments, the virus is a DNA virus (e.g., dsDNA or ssDNA virus). In certain embodiments, the virus is an RNA virus (e.g., an ssRNA virus). In certain embodiments, the virus infects dividing cells. In other embodiments, the virus infects non-dividing cells. Exemplary viral vectors/viruses include, e.g., retroviruses, lentiviruses, adenovirus, adeno-associated virus (AAV), vaccinia viruses, poxviruses, and herpes simplex viruses.

In certain embodiments, the virus infects dividing cells. In certain embodiments, the virus infects non-dividing cells. In certain embodiments, the virus infects both dividing and non-dividing cells. In certain embodiments, the virus can integrate into the host genome. In certain embodiments, the virus is engineered to have reduced immunity, e.g., in human. In certain embodiments, the virus is replication-competent. In other embodiments, the virus is replication-defective, e.g., having one or more coding regions for the genes necessary for additional rounds of virion replication and/or packaging replaced with other genes or deleted. In certain embodiments, the virus causes transient expression of the Cas9 molecule and/or the gRNA molecule. In other embodiments, the virus causes long-lasting, e.g., at least 1 week, 2 weeks, 1 month, 2 months, 3 months, 6 months, 9 months, 1 year, 2 years, or permanent expression, of the Cas9 molecule and/or the gRNA molecule. The packaging capacity of the viruses may vary, e.g., from at least about 4 kb to at least about 30 kb, e.g., at least about 5 kb, 10 kb, 15 kb, 20 kb, 25 kb, 30 kb, 35 kb, 40 kb, 45 kb, or 50 kb.

In certain embodiments, the viral vector recognizes a specific cell type or tissue. For example, the viral vector can be pseudotyped with a different/alternative viral envelope glycoprotein; engineered with a cell type-specific receptor (e.g., genetic modification(s) of one or more viral envelope glycoproteins to incorporate a targeting ligand such as a peptide ligand, a single chain antibody, or a growth factor); and/or engineered to have a molecular bridge with dual specificities with one end recognizing a viral glycoprotein and the other end recognizing a moiety of the target cell surface (e.g., a ligand-receptor, monoclonal antibody, avidin-biotin and chemical conjugation).

Exemplary viral vectors/viruses include, e.g., retroviruses, lentiviruses, adenovirus, adeno-associated virus (AAV), vaccinia viruses, poxviruses, and herpes simplex viruses.

In certain embodiments, the Cas9- and/or gRNA-encoding sequence is delivered by a recombinant retrovirus. In certain embodiments, the retrovirus (e.g., Moloney murine leukemia virus) comprises a reverse transcriptase, e.g., that allows integration into the host genome. In certain embodiments, the retrovirus is replication-competent. In other embodiments, the retrovirus is replication-defective, e.g., having one of more coding regions for the genes necessary for additional rounds of virion replication and packaging replaced with other genes, or deleted.

In certain embodiments, the Cas9- and/or gRNA-encoding nucleic acid sequence is delivered by a recombinant lentivirus. In certain embodiments, the donor template nucleic acid is delivered by a recombinant retrovirus. For example, the lentivirus is replication-defective, e.g., does not comprise one or more genes required for viral replication.

In certain embodiments, the Cas9- and/or gRNA-encoding nucleic acid sequence is delivered by a recombinant lentivirus. In certain embodiments, the donor template nucleic acid is delivered by a recombinant lentivirus. For example, the lentivirus is replication-defective, e.g., does not comprise one or more genes required for viral replication.

In certain embodiments, the Cas9- and/or gRNA-encoding nucleic acid sequence is delivered by a recombinant adenovirus. In certain embodiments, the donor template nucleic acid is delivered by a recombinant adenovirus. In certain embodiments, the adenovirus is engineered to have reduced immunity in human.

In certain embodiments, the Cas9- and/or gRNA-encoding nucleic acid sequence is delivered by a recombinant AAV. In certain embodiments, the donor template nucleic acid is delivered by a recombinant AAV. In certain embodiments, the AAV does not incorporate its genome into that of a host cell, e.g., a target cell as describe herein. In certain embodiments, the AAV can incorporate at least part of its genome into that of a host cell, e.g., a target cell as described herein. In certain embodiments, the AAV is a self-complementary adeno-associated virus (scAAV), e.g., a scAAV that packages both strands which anneal together to form double stranded DNA. AAV serotypes that may be used in the disclosed methods, include AAV1, AAV2, modified AAV2 (e.g., modifications at Y444F, Y500F, Y730F and/or S662V), AAV3, modified AAV3 (e.g., modifications at Y705F, Y731F and/or T492V), AAV4, AAV5, AAV6, modified AAV6 (e.g., modifications at S663V and/or T492V), AAV8, AAV 8.2, AAV9, AAV rh10, and pseudotyped AAV, such as AAV2/8, AAV2/5 and AAV2/6 can also be used in the disclosed methods. In certain embodiments, an AAV capsid that can be used in the methods described herein is a capsid sequence from serotype AAV1, AAV2, AAV3, AAV4, AAV5, AAV6, AAV7, AAV8, AAV9, AAV.rh8, AAV.rh10, AAV.rh32/33, AAV.rh43, AAV.rh64R1, or AAV7m8.

In certain embodiments, the Cas9- and/or gRNA-encoding nucleic acid sequence is delivered in a re-engineered AAV capsid, e.g., with about 50% or greater, e.g., about 60% or greater, about 70% or greater, about 80% or greater, about 90% or greater, or about 95% or greater, sequence homology with a capsid sequence from serotypes AAV1, AAV2, AAV3, AAV4, AAV5, AAV6, AAV7, AAV8, AAV9, AAV.rh8, AAV.rh10, AAV.rh32/33, AAV.rh43, or AAV.rh64R1.

In certain embodiments, the Cas9- and/or gRNA-encoding nucleic acid sequence is delivered by a chimeric AAV capsid. In certain embodiments, the donor template nucleic acid is delivered by a chimeric AAV capsid. Exemplary chimeric AAV capsids include, but are not limited to, AAV9i1, AAV2i8, AAV-DJ, AAV2G9, AAV2i8G9, or AAV8G9.

In certain embodiments, the AAV is a self-complementary adeno-associated virus (scAAV), e.g., a scAAV that packages both strands which anneal together to form double stranded DNA.

In certain embodiments, the Cas9- and/or gRNA-encoding DNA is delivered by a hybrid virus, e.g., a hybrid of one or more of the viruses described herein. In certain embodiments, the hybrid virus is hybrid of an AAV (e.g., of any AAV serotype), with a Bocavirus, B19 virus, porcine AAV, goose AAV, feline AAV, canine AAV, or MVM

A packaging cell is used to form a virus particle that is capable of infecting a target cell. Exemplary packaging cells include 293 cells, which can package adenovirus, and ψ2 or PA317 cells, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed, e.g., Cas9. For example, an AAV vector used in gene therapy typically only possesses inverted terminal repeat (ITR) sequences from the AAV genome which are required for packaging and gene expression in the host or target cell. The missing viral functions can be supplied in *trans* by the packaging cell line and/or plasmid containing E2A, E4, and VA genes from adenovirus, and plasmid encoding *Rep* and *Cap* genes from AAV, as described in "Triple Transfection Protocol." Henceforth, the viral DNA is packaged in a cell line, which contains a helper plasmid encoding the other AAV genes, namely rep and cap, but lacking ITR sequences. In certain embodiments, the viral DNA is packaged in a producer cell line, which contains E1A and/or E1B genes from adenovirus. The cell line is also infected with adenovirus as a helper. The helper virus (e.g., adenovirus or HSV) or helper plasmid promotes replication of the AAV vector and expression of AAV genes from the helper plasmid with ITRs. The helper plasmid is not packaged in significant amounts due to a lack of ITR sequences. Contamination with adenovirus can be reduced by, e.g., heat treatment to which adenovirus is more sensitive than AAV.

In certain embodiments, the viral vector is capable of cell type and/or tissue type recognition. For example, the viral vector can be pseudotyped with a different/alternative viral envelope glycoprotein; engineered with a cell type-specific receptor (e.g., genetic modification of the viral envelope glycoproteins to incorporate targeting ligands such as peptide ligands, single chain antibodies, or growth factors); and/or engineered to have a molecular bridge with dual

specificities with one end recognizing a viral glycoprotein and the other end recognizing a moiety of the target cell surface (e.g., ligand-receptor, monoclonal antibody, avidin-biotin and chemical conjugation).

In certain embodiments, the viral vector achieves cell type specific expression. For example, a tissue-specific promoter can be constructed to restrict expression of the transgene (Cas9 and gRNA) to only the target cell. The specificity of the vector can also be mediated by microRNA-dependent control of transgene expression. In certain embodiments, the viral vector has increased efficiency of fusion of the viral vector and a target cell membrane. For example, a fusion protein such as fusion-competent hemagglutin (HA) can be incorporated to increase viral uptake into cells. In certain embodiments, the viral vector has the ability of nuclear localization. For example, a virus that requires the breakdown of the nuclear envelope (during cell division) and therefore will not infect a non-diving cell can be altered to incorporate a nuclear localization peptide in the matrix protein of the virus thereby enabling the transduction of non-proliferating cells.

In certain embodiments, the Cas9- and/or gRNA-encoding DNA is delivered by a non-vector based method (e.g., using naked DNA or DNA complexes). For example, the DNA can be delivered, e.g., by organically modified silica or silicate (Ormosil), electroporation, transient cell compression or squeezing (e.g., as described in Lee, et al., 2012, Nano Lett 12: 6322-27), gene gun, sonoporation, magnetofection, lipid-mediated transfection, dendrimers, inorganic nanoparticles, calcium phosphates, or a combination thereof.

In certain embodiments, delivery via electroporation comprises mixing the cells with the Cas9-and/or gRNA-encoding DNA in a cartridge, chamber or cuvette and applying one or more electrical impulses of defined duration and amplitude. In certain embodiments, delivery via electroporation is performed using a system in which cells are mixed with the Cas9-and/or gRNA-encoding DNA in a vessel connected to a device (e.g,, a pump) which feeds the mixture into a cartridge, chamber or cuvette wherein one or more electrical impulses of defined duration and amplitude are applied, after which the cells are delivered to a second vessel.

In certain embodiments, the Cas9- and/or gRNA-encoding DNA is delivered by a combination of a vector and a non-vector based method. In certain embodiments, the donor template nucleic acid is delivered by a combination of a vector and a non-vector based method. For example, virosomes combine liposomes with an inactivated virus (e.g., HIV or influenza virus), which can result in more efficient gene transfer, e.g., in respiratory epithelial cells than either viral or liposomal methods alone.

In certain embodiments, the delivery vehicle is a non-viral vector. In certain embodiments, the non-viral vector is an inorganic nanoparticle. Exemplary inorganic nanoparticles include, e.g., magnetic nanoparticles (e.g., $Fe_3MnO_2$) and silica. The outer surface of the nanoparticle can be conjugated with a positively charged polymer (e.g., polyethylenimine, polylysine, polyserine) which allows for attachment (e.g., conjugation or entrapment) of payload. In certain embodiments, the non-viral vector is an organic nanoparticle (e.g., entrapment of the payload inside the nanoparticle). Exemplary organic nanoparticles include, e.g., SNALP liposomes that contain cationic lipids together with neutral helper lipids which are coated with polyethylene glycol (PEG) and protamine and nucleic acid complex coated with lipid coating. Exemplary lipids for gene transfer are shown below in **Table 5.**

**Table 5**: Lipids Used for Gene Transfer

| Lipid | Abbreviation | Feature |
|---|---|---|
| 1,2- Dioleoyl-sn-glycero-3 -phosphatidylcholine | DOPC | Helper |
| 1,2-Dioleoyl-sn-glycero-3-phosphatidylethanolamine | DOPE | Helper |
| Cholesterol | | Helper |
| *N*-[1-(2,3-Dioleyloxy)propyl]*N*,*N*,*N*-trimethylammonium chloride | DOTMA | Cationic |
| 1,2-Dioleoyloxy-3-trimethylammonium-propane | DOTAP | Cationic |
| Dioctadecylamidoglycylspermine | DOGS | Cationic |
| *N*-(3-Aminopropyl)-*N*,*N*-dimethyl-2,3-bis(dodecyloxy)-1-propanaminium bromide | GAP-DLRIE | Cationic |
| Cetyltrimethylammonium bromide | CTAB | Cationic |
| 6-Lauroxyhexyl ornithinate | LHON | Cationic |
| 1-(2,3-Dioleoyloxypropyl)-2,4,6-trimethylpyridinium | 2Oc | Cationic |
| 2,3-Dioleyloxy-*N*-[2(sperminecarboxamido-ethyl]-*N*,*N*-dimethyl-1-propanaminium trifluoroacetate | DOSPA | Cationic |
| 1,2-Dioleyl-3-trimethylammonium-propane | DOPA | Cationic |
| *N*-(2-Hydroxyethyl)-*N*,*N*-dimethyl-2,3-bis(tetradecyloxy)-1-propanaminium bromide | MDRIE | Cationic |

(continued)

| Lipid | Abbreviation | Feature |
|---|---|---|
| Dimyristooxypropyl dimethyl hydroxyethyl ammonium bromide | DMRI | Cationic |
| 3β-[*N*-(*N*',*N*'-Dimethylaminoethane)-carbamoyl]cholesterol | DC-Chol | Cationic |
| Bis-guanidium-tren-cholesterol | BGTC | Cationic |
| 1,3-Diodeoxy-2-(6-carboxy-spermyl)-propylamide | DOSPER | Cationic |
| Dimethyloctadecylammonium bromide | DDAB | Cationic |
| Dioctadecylamidoglicylspermidin | DSL | Cationic |
| rac-[(2,3-Dioctadecyloxypropyl)(2-hydroxyethyl)]-dimethylammonium chloride | CLIP-1 | Cationic |
| rac-[2(2,3-Dihexadecyloxypropyloxymethyloxy)ethyl]trimethylammonium bromide | CLIP-6 | Cationic |
| Ethyldimyristoylphosphatidylcholine | EDMPC | Cationic |
| 1,2-Distearyloxy-*N*,*N*-dimethyl-3-aminopropane | DSDMA | Cationic |
| 1,2-Dimyristoyl-trimethylammonium propane | DMTAP | Cationic |
| *O*,*O*'-Dimyristyl-N-lysyl aspartate | DMKE | Cationic |
| 1,2-Distearoyl-sn-glycero-3-ethylphosphocholine | DSEPC | Cationic |
| *N*-Palmitoyl D-erythro-sphingosyl carbamoylspermine | CCS | Cationic |
| *N*-*t*-Butyl-*N*0-tetradecyl-3-tetradecylaminopropionamidine | diC 14-amidine | Cationic |
| Octadecenolyoxy[ethyl-2-heptadecenyl-3 hydroxyethyl] imidazolinium chloride | DOTIM | Cationic |
| *N*1-Cholesteryloxycarbonyl-3,7-diazanonane-1,9-diamine | CDAN | Cationic |
| 2-(3-[Bis(3-amino-propyl)-amino]propylamino)-*N*-ditetradecylcarbamoylme-ethyl-acetamide | RPR209120 | Cationic |
| 1,2-dilinoleyloxy-3-dimethylaminopropane | DLinDMA | Cationic |
| 2,2-dilinoleyl-4-dimethylaminoethyl-[1,3]-dioxolane | DLin-KC2-DMA | Cationic |
| dilinoleyl-methyl-4-dimethylaminobutyrate | DLin-MC3-DMA | Cationic |

Exemplary polymers for gene transfer are shown below in **Table 6.**

**Table 6**: Polymers Used for Gene Transfer

| Polymer | Abbreviation |
|---|---|
| Poly(ethylene)glycol | PEG |
| Polyethylenimine | PEI |
| Dithiobis(succinimidylpropionate) | DSP |
| Dimethyl-3,3'-dithiobispropionimidate | DTBP |
| Poly(ethylene imine)biscarbamate | PEIC |
| Poly(L-lysine) | PLL |
| Histidine modified PLL | |
| Poly(*N*-vinylpyrrolidone) | PVP |
| Poly(propylenimine) | PPI |
| Poly(amidoamine) | PAMAM |
| Poly(amido ethylenimine) | SS-PAEI |

(continued)

| Polymer | Abbreviation |
|---|---|
| Triethylenetetramine | TETA |
| Poly(β-aminoester) | |
| Poly(4-hydroxy-L-proline ester) | PHP |
| Poly(allylamine) | |
| Poly(α-[4-aminobutyl]-L-glycolic acid) | PAGA |
| Poly(D,L-lactic-co-glycolic acid) | PLGA |
| Poly(*N*-ethyl-4-vinylpyridinium bromide) | |
| Poly(phosphazene)s | PPZ |
| Poly(phosphoester)s | PPE |
| Poly(phosphoramidate)s | PPA |
| Poly(*N*-2-hydroxypropylmethacrylamide) | pHPMA |
| Poly (2-(dimethylamino)ethyl methacrylate) | pDMAEMA |
| Poly(2-aminoethyl propylene phosphate) | PPE-EA |
| Chitosan | |
| Galactosylated chitosan | |
| *N*-Dodacylated chitosan | |
| Histone | |
| Collagen | |
| Dextran-spermine | D-SPM |

In certain embodiments, the vehicle has targeting modifications to increase target cell update of nanoparticles and liposomes, e.g., cell specific antigens, monoclonal antibodies, single chain antibodies, aptamers, polymers, sugars (e.g., N-acetylgalactosamine (GalNAc)), and cell penetrating peptides. In certain embodiments, the vehicle uses fusogenic and endosome-destabilizing peptides/polymers. In certain embodiments, the vehicle undergoes acid-triggered conformational changes (e.g., to accelerate endosomal escape of the cargo). In certain embodiments, a stimuli-cleavable polymer is used, e.g., for release in a cellular compartment. For example, disulfide-based cationic polymers that are cleaved in the reducing cellular environment can be used.

In certain embodiments, the delivery vehicle is a biological non-viral delivery vehicle. In certain embodiments, the vehicle is an attenuated bacterium (e.g., naturally or artificially engineered to be invasive but attenuated to prevent pathogenesis and expressing the transgene (e.g., *Listeria monocytogenes*, certain *Salmonella strains*, *Bifidobacterium longum*, and modified *Escherichia coli*), bacteria having nutritional and tissue-specific tropism to target specific tissues, bacteria having modified surface proteins to alter target tissue specificity). In certain embodiments, the vehicle is a genetically modified bacteriophage (e.g., engineered phages having large packaging capacity, less immunogenic, containing mammalian plasmid maintenance sequences and having incorporated targeting ligands). In certain embodiments, the vehicle is a mammalian virus-like particle. For example, modified viral particles can be generated (e.g., by purification of the "empty" particles followed by *ex vivo* assembly of the virus with the desired cargo). The vehicle can also be engineered to incorporate targeting ligands to alter target tissue specificity. In certain embodiments, the vehicle is a biological liposome. For example, the biological liposome is a phospholipid-based particle derived from human cells (e.g., erythrocyte ghosts, which are red blood cells broken down into spherical structures derived from the subject (e.g., tissue targeting can be achieved by attachment of various tissue or cell-specific ligands), or secretory exosomes -subject (i.e., patient) derived membrane-bound nanovesicle (30 -100 nm) of endocytic origin (e.g., can be produced from various cell types and can therefore be taken up by cells without the need of for targeting ligands).

In certain embodiments, one or more nucleic acid molecules (e.g., DNA molecules) other than the components of a Cas system, e.g., the Cas9 molecule component and/or the gRNA molecule component described herein, are delivered. In certain embodiments, the nucleic acid molecule is delivered at the same time as one or more of the components of the Cas system are delivered. In certain embodiments, the nucleic acid molecule is delivered before or

after (e.g., less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. In certain embodiments, the nucleic acid molecule is delivered by a different means than one or more of the components of the Cas system, e.g., the Cas9 molecule component and/or the gRNA molecule component, are delivered. The nucleic acid molecule can be delivered by any of the delivery methods described herein. For example, the nucleic acid molecule can be delivered by a viral vector, e.g., an integration-deficient lentivirus, and the Cas9 molecule component and/or the gRNA molecule component can be delivered by electroporation, e.g., such that the toxicity caused by nucleic acids (e.g., DNAs) can be reduced. In certain embodiments, the nucleic acid molecule encodes a therapeutic protein, e.g., a protein described herein. In certain embodiments, the nucleic acid molecule encodes an RNA molecule, e.g., an RNA molecule described herein.

### *10.2 Delivery of RNA encoding a Cas9 molecule*

RNA encoding Cas9 molecules (e.g., eaCas9 molecules) and/or gRNA molecules, can be delivered into cells, e.g., target cells described herein, by art-known methods or as described herein. For example, Cas9-encoding and/or gRNA-encoding RNA can be delivered, e.g., by microinjection, electroporation, transient cell compression or squeezing (e.g., as described in Lee, et al., Nano Lett 12: 6322-27), lipid-mediated transfection, peptide-mediated delivery, or a combination thereof. Cas9-encoding and/or gRNA-encoding RNA can be conjugated to molecules promoting uptake by the target cells (e.g., target cells described herein).

In certain embodiments, delivery via electroporation comprises mixing the cells with the RNA encoding Cas9 molecules and/or gRNA molecules with or without donor template nucleic acid molecules, in a cartridge, chamber or cuvette and applying one or more electrical impulses of defined duration and amplitude. In certain embodiments, delivery via electroporation is performed using a system in which cells are mixed with the RNA encoding Cas9 molecules and/or gRNA molecules, with or without donor template nucleic acid molecules in a vessel connected to a device (e.g., a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (e.g., target cells described herein).

### *10.3 Delivery of Cas9*

Cas9 molecules (e.g., eaCas9 molecules) can be delivered into cells by art-known methods or as described herein. For example, Cas9 protein molecules can be delivered, e.g., by microinjection, electroporation, transient cell compression or squeezing (e.g., as described in Lee, et al., Nano Lett 12: 6322-27), lipid-mediated transfection, peptide-mediated delivery, or a combination thereof. Delivery can be accompanied by DNA encoding a gRNA or by a gRNA. Cas9 protein can be conjugated to molecules promoting uptake by the target cells (e.g., target cells described herein).

In certain embodiments, delivery via electroporation comprises mixing the cells with the Cas9 molecules and/or gRNA molecules, with or without donor nucleic acid, in a cartridge, chamber or cuvette and applying one or more electrical impulses of defined duration and amplitude. In certain embodiments, delivery via electroporation is performed using a system in which cells are mixed with the RNA encoding Cas9 molecules and/or gRNA molecules, in a vessel connected to a device (e.g., a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (e.g., target cells described herein).

### *10.4 Route of administration*

Systemic modes of administration include oral and parenteral routes. Parenteral routes include, by way of example, intravenous, intrarterial, intramuscular, intradermal, subcutaneous, intranasal, and intraperitoneal routes. Components administered systemically may be modified or formulated to target muscle cells, e.g., skeletal muscle cells, cardiac muscle cells or smooth muscle cells.

Local modes of administration include injection directly into one or more specific tissues, e.g., one or more muscles. In certain embodiments, local modes of administration include injection directly into one or more skeletal muscles. In certain embodiments, skeletal muscles include the following: abductor digiti minimi (foot), abductor digiti minimi (hand), abductor hallucis, abductor pollicis brevis, abductor pollicis longus, adductor brevis, adductor hallucis, adductor longus, adductor magnus, adductor pollicis, anconeus, articularis cubiti, articularis genu, aryepiglotticus, auricularis, biceps brachii, biceps femoris, brachialis, brachioradialis, buccinator, bulbospongiosus, constrictor of pharynx -inferior, constrictor of pharynx -middle, constrictor of pharynx -superior, coracobrachialis, corrugator supercilii, cremaster, cricothyroid, dartos, deep transverse perinei, deltoid, depressor anguli oris, depressor labii inferioris, diaphragm,

digastric, digastric (anterior view), erector spinae - spinalis, erector spinae - iliocostalis, erector spinae - longissimus, extensor carpi radialis brevis, extensor carpi radialis longus, extensor carpi ulnaris, extensor digiti minimi (hand), extensor digitorum (hand), extensor digitorum brevis (foot), extensor digitorum longus (foot), extensor hallucis brevis, extensor hallucis longus, extensor indicis, extensor pollicis brevis, extensor pollicis longus, external oblique abdominis, flexor carpi radialis, flexor carpi ulnaris, flexor digiti minimi brevis (foot), flexor digiti minimi brevis (hand), flexor digitorum brevis, flexor digitorum longus (foot), flexor digitorum profundus, flexor digitorum superficialis, flexor hallucis brevis, flexor hallucis longus, flexor pollicis brevis, flexor pollicis longus, frontalis, gastrocnemius, gemellus inferior, gemellus superior, genioglossus, geniohyoid, gluteus maximus, gluteus medius, gluteus minimus, gracilis, hyoglossus, iliacus, inferior oblique, inferior rectus, infraspinatus, intercostals external, intercostals innermost, intercostals internal, internal oblique abdominis, interossei - dorsal of hand, interossei -dorsal of foot, interossei- palmar of hand, interossei - plantar of foot, interspinales, intertransversarii, intrinsic muscles of tongue, ishiocavernosus, lateral cricoarytenoid, lateral pterygoid, lateral rectus, latissimus dorsi, levator anguli oris, levator ani-coccygeus, levator ani - iliococcygeus, levator ani-pubococcygeus, levator ani-puborectalis, levator ani-pubovaginalis, levator labii superioris, levator labii superioris, alaeque nasi, levator palpebrae superioris, levator scapulae, levator veli palatini, levatores costarum, longus capitis, longus colli, lumbricals of foot, lumbricals of hand, masseter, medial pterygoid, medial rectus, mentalis, m. uvulae, mylohyoid, nasalis, oblique arytenoid, obliquus capitis inferior, obliquus capitis superior, obturator externus, obturator internus (A), obturator internus (B), omohyoid, opponens digiti minimi (hand), opponens pollicis, orbicularis oculi, orbicularis oris, palatoglossus, palatopharyngeus, palmaris brevis, palmaris longus, pectineus, pectoralis major, pectoralis minor, peroneus brevis, peroneus longus, peroneus tertius, piriformis (A), piriformis (B), plantaris, platysma, popliteus, posterior cricoarytenoid, procerus, pronator quadratus, pronator teres, psoas major, psoas minor, pyramidalis, quadratus femoris, quadratus lumborum, quadratus plantae, rectus abdominis, rectus capitus anterior, rectus capitus lateralis, rectus capitus posterior major, rectus capitus posterior minor, rectus femoris, rhomboid major, rhomboid minor, risorius, salpingopharyngeus, sartorius, scalenus anterior, scalenus medius, scalenus minimus, scalenus posterior, semimembranosus, semitendinosus, serratus anterior, serratus posterior inferior, serratus posterior superior, soleus, sphincter ani, sphincter urethrae, splenius capitis, splenius cervicis, stapedius, sternocleidomastoid, sternohyoid, sternothyroid, styloglossus, stylohyoid, stylohyoid (anterior view), stylopharyngeus, subclavius, subcostalis, subscapularis, superficial transverse perinei, superior oblique, superior rectus, supinator, supraspinatus, temporalis, temporoparietalis, tensor fasciae lata, tensor tympani, tensor veli palatini, teres major, teres minor, thyro-arytenoid & vocalis, thyro-epiglotticus, thyrohyoid, tibialis anterior, tibialis posterior, transverse arytenoid, transversospinalis -multifidus, transversospinalis -rotatores, transversospinalis -semispinalis, transversus abdominis, transversus thoracis, trapezius, triceps, vastus intermedius, vastus lateralis, vastus medialis, zygomaticus major, or zygomaticus minor.

In certain embodiments, local modes of administration include injection directly into cardiac muscle or smooth muscle.

In certain embodiments, significantly smaller amounts of the components (compared with systemic approaches) may exert an effect when administered locally (for example, intravitreally) compared to when administered systemically (for example, intravenously). Local modes of administration can reduce or eliminate the incidence of potentially toxic side effects that may occur when therapeutically effective amounts of a component are administered systemically.

Administration may be provided as a periodic bolus (for example, intravenously) or as continuous infusion from an internal reservoir or from an external reservoir (for example, from an intravenous bag or implantable pump). Components may be administered locally, for example, by continuous release from a sustained release drug delivery device immobilized within a muscle.

In addition, components may be formulated to permit release over a prolonged period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon rate of release required by a particular application. Both non-degradable and degradable release systems can be used. Suitable release systems include polymers and polymeric matrices, non-polymeric matrices, or inorganic and organic excipients and diluents such as, but not limited to, calcium carbonate and sugar (for example, trehalose). Release systems may be natural or synthetic. However, synthetic release systems are preferred because generally they are more reliable, more reproducible and produce more defined release profiles. The release system material can be selected so that components having different molecular weights are released by diffusion through or degradation of the material.

Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as poly(ethylene oxide), poly(ethylene glycol), and poly(tetramethylene oxide); vinyl polymers-polyacrylates and polymethacrylates such as methyl, ethyl, other alkyl, hydroxyethyl methacrylate, acrylic and

methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof.

Poly(lactide-co-glycolide) microsphere can also be used for intraocular injection. Typically the microspheres are composed of a polymer of lactic acid and glycolic acid, which are structured to form hollow spheres. The spheres can be approximately 15-30 microns in diameter and can be loaded with components described herein.

### *10.5 Bi-Modal or Differential Delivery of Components*

Separate delivery of the components of a Cas system, e.g., the Cas9 molecule component and the gRNA molecule component, and more particularly, delivery of the components by differing modes, can enhance performance, e.g., by improving tissue specificity and safety.

In certain embodiments, the Cas9 molecule and the gRNA molecule are delivered by different modes, or as sometimes referred to herein as differential modes. Different or differential modes, as used herein, refer modes of delivery that confer different pharmacodynamic or pharmacokinetic properties on the subject component molecule, e.g., a Cas9 molecule, gRNA molecule, template nucleic acid, or payload. For example, the modes of delivery can result in different tissue distribution, different half-life, or different temporal distribution, e.g., in a selected compartment, tissue, or organ.

Some modes of delivery, e.g., delivery by a nucleic acid vector that persists in a cell, or in progeny of a cell, e.g., by autonomous replication or insertion into cellular nucleic acid, result in more persistent expression of and presence of a component. Examples include viral, e.g., AAV or lentivirus, delivery.

By way of example, the components, e.g., a Cas9 molecule and a gRNA molecule, can be delivered by modes that differ in terms of resulting half-life or persistent of the delivered component the body, or in a particular compartment, tissue or organ. In certain embodiments, a gRNA molecule can be delivered by such modes. The Cas9 molecule component can be delivered by a mode which results in less persistence or less exposure to the body or a particular compartment or tissue or organ.

More generally, in certain embodiments, a first mode of delivery is used to deliver a first component and a second mode of delivery is used to deliver a second component. The first mode of delivery confers a first pharmacodynamic or pharmacokinetic property. The first pharmacodynamic property can be, e.g., distribution, persistence, or exposure, of the component, or of a nucleic acid that encodes the component, in the body, a compartment, tissue or organ. The second mode of delivery confers a second pharmacodynamic or pharmacokinetic property. The second pharmacodynamic property can be, e.g., distribution, persistence, or exposure, of the component, or of a nucleic acid that encodes the component, in the body, a compartment, tissue or organ.

In certain embodiments, the first pharmacodynamic or pharmacokinetic property, e.g., distribution, persistence or exposure, is more limited than the second pharmacodynamic or pharmacokinetic property. In certain embodiments, the first mode of delivery is selected to optimize, e.g., minimize, a pharmacodynamic or pharmacokinetic property, e.g., distribution, persistence or exposure. In certain embodiments, the second mode of delivery is selected to optimize, e.g., maximize, a pharmacodynamic or pharmacokinetic property, e.g., distribution, persistence or exposure.

In certain embodiments, the first mode of delivery comprises the use of a relatively persistent element, e.g., a nucleic acid, e.g., a plasmid or viral vector, e.g., an AAV or lentivirus. As such vectors are relatively persistent product transcribed from them would be relatively persistent.

In certain embodiments, the second mode of delivery comprises a relatively transient element, e.g., an RNA or protein.

In certain embodiments, the first component comprises gRNA, and the delivery mode is relatively persistent, e.g., the gRNA is transcribed from a plasmid or viral vector, e.g., an AAV or lentivirus. Transcription of these genes would be of little physiological consequence because the genes do not encode for a protein product, and the gRNAs are incapable of acting in isolation. The second component, a Cas9 molecule, is delivered in a transient manner, for example as mRNA or as protein, ensuring that the full Cas9 molecule/gRNA molecule complex is only present and active for a short period of time.

Furthermore, the components can be delivered in different molecular form or with different delivery vectors that complement one another to enhance safety and tissue specificity.

Use of differential delivery modes can enhance performance, safety and efficacy. E.g., the likelihood of an eventual off-target modification can be reduced. Delivery of immunogenic components, e.g., Cas9 molecules, by less persistent modes can reduce immunogenicity, as peptides from the bacterially-derived Cas enzyme are displayed on the surface of the cell by MHC molecules. A two-part delivery system can alleviate these drawbacks.

Differential delivery modes can be used to deliver components to different, but overlapping target regions. The formation active complex is minimized outside the overlap of the target regions. Thus, in certain embodiments, a first

component, e.g., a gRNA molecule is delivered by a first delivery mode that results in a first spatial, e.g., tissue, distribution. A second component, e.g., a Cas9 molecule is delivered by a second delivery mode that results in a second spatial, e.g., tissue, distribution. In certain embodiments the first mode comprises a first element selected from a liposome, nanoparticle, e.g., polymeric nanoparticle, and a nucleic acid, e.g., viral vector. The second mode comprises a second element selected from the group. In certain embodiments, the first mode of delivery comprises a first targeting element, e.g., a cell specific receptor or an antibody, and the second mode of delivery does not include that element. In embodiment, the second mode of delivery comprises a second targeting element, e.g., a second cell specific receptor or second antibody.

When the Cas9 molecule is delivered in a virus delivery vector, a liposome, or polymeric nanoparticle, there is the potential for delivery to and therapeutic activity in multiple tissues, when it may be desirable to only target a single tissue. A two-part delivery system can resolve this challenge and enhance tissue specificity. If the gRNA molecule and the Cas9 molecule are packaged in separated delivery vehicles with distinct but overlapping tissue tropism, the fully functional complex is only be formed in the tissue that is targeted by both vectors.

### *10.6 Ex vivo* delivery

A presently disclosed genome-editing system can be implemented in a cell or in an *in vitro* contact. In certain embodiments, each component of the genome-editing system described in **Table 3** are introduced into a cell which is then introduced into a subject. Methods of introducing the components can include, e.g., any of the delivery methods described in **Table 4.**

## 11. Modified Nucleosides, Nucleotides, and Nucleic Acids

Modified nucleosides and modified nucleotides can be present in nucleic acids, e.g., particularly gRNA, but also other forms of RNA, e.g., mRNA, RNAi, or siRNA. As described herein, "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:

(i) alteration, e.g., replacement, of one or both of the non-linking phosphate oxygens and/or of one or more of the linking phosphate oxygens in the phosphodiester backbone linkage;
(ii) alteration, e.g., replacement, of a constituent of the ribose sugar, e.g., of the 2' hydroxyl on the ribose sugar;
(iii) wholesale replacement of the phosphate moiety with "dephospho" linkers;
(iv) modification or replacement of a naturally occurring nucleobase;
(v) replacement or modification of the ribose-phosphate backbone;
(vi) modification of the 3' end or 5' end of the oligonucleotide, e.g., removal, modification or replacement of a terminal phosphate group or conjugation of a moiety; and
(vii) modification of the sugar.

The modifications listed above can be combined to provide modified nucleosides and nucleotides that can have two, three, four, or more modifications. For example, a modified nucleoside or nucleotide can have a modified sugar and a modified nucleobase. In certain embodiments, every base of a gRNA is modified, e.g., all bases have a modified phosphate group, e.g., all are phosphorothioate groups. In certain embodiments, all, or substantially all, of the phosphate groups of a unimolecular or modular gRNA molecule are replaced with phosphorothioate groups.

In certain embodiments, modified nucleotides, e.g., nucleotides having modifications as described herein, can be incorporated into a nucleic acid, e.g., a "modified nucleic acid." In certain embodiments, the modified nucleic acids comprise one, two, three or more modified nucleotides. In certain embodiments, at least 5% (e.g., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid are a modified nucleotides.

Unmodified nucleic acids can be prone to degradation by, e.g., cellular nucleases. For example, nucleases can hydrolyze nucleic acid phosphodiester bonds. Accordingly, in certain embodiments, the modified nucleic acids described herein can contain one or more modified nucleosides or nucleotides, e.g., to introduce stability toward nucleases.

In certain embodiments, the modified nucleosides, modified nucleotides, and modified nucleic acids described herein can exhibit a reduced innate immune response when introduced into a population of cells, both *in vivo* and *ex vivo.* The term "innate immune response" includes a cellular response to exogenous nucleic acids, including single

stranded nucleic acids, generally of viral or bacterial origin, which involves the induction of cytokine expression and release, particularly the interferons, and cell death. In certain embodiments, the modified nucleosides, modified nucleotides, and modified nucleic acids described herein can disrupt binding of a major groove interacting partner with the nucleic acid. In certain embodiments, the modified nucleosides, modified nucleotides, and modified nucleic acids described herein can exhibit a reduced innate immune response when introduced into a population of cells, both *in vivo* and *ex vivo,* and also disrupt binding of a major groove interacting partner with the nucleic acid.

### *11.1 Definitions of Chemical Groups*

As used herein, "alkyl" is meant to refer to a saturated hydrocarbon group which is straight-chained or branched. Example alkyl groups include methyl (Me), ethyl (Et), propyl (*e.g.*, n-propyl and isopropyl), butyl (*e.g.*, n-butyl, isobutyl, t-butyl), pentyl (*e.g.*, n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (*e.g.*, having 2, 3 or 4 fused rings) aromatic hydrocarbons such as, for example, phenyl, naphthyl, anthracenyl, phenanthrenyl, indanyl, indenyl, and the like. In certain embodiments, aryl groups have from 6 to about 20 carbon atoms.

As used herein, "alkenyl" refers to an aliphatic group containing at least one double bond.

As used herein, "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic non-aromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl.

### *11.2 Phosphate Backbone Modifications*

#### *11.2.1 The Phosphate Group*

In certain embodiments, the phosphate group of a modified nucleotide can be modified by replacing one or more of the oxygens with a different substituent. Further, the modified nucleotide, e.g., modified nucleotide present in a modified nucleic acid, can include the wholesale replacement of an unmodified phosphate moiety with a modified phosphate as described herein. In certain embodiments, the modification of the phosphate backbone can include alterations that result in either an uncharged linker or a charged linker with unsymmetrical charge distribution.

Examples of modified phosphate groups include phosphorothioate, phosphoroselenates, borano phosphates, borano phosphate esters, hydrogen phosphonates, phosphoroamidates, alkyl or aryl phosphonates and phosphotriesters. In certain embodiments, one of the non-bridging phosphate oxygen atoms in the phosphate backbone moiety can be replaced by any of the following groups: sulfur (S), selenium (Se), $BR_3$ (wherein R can be, e.g., hydrogen, alkyl, or aryl), C (e.g., an alkyl group, an aryl group, and the like), H, $NR_2$ (wherein R can be, e.g., hydrogen, alkyl, or aryl), or OR (wherein R can be, e.g., alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In certain embodiments, modifications to one or both non-bridging oxygens can also include the replacement of the non-bridging oxygens with a group independently selected from S, Se, B, C, H, N, and OR (R can be, e.g., alkyl or aryl).

The phosphate linker can also be modified by replacement of a bridging oxygen, (i.e., the oxygen that links the

phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

*11.2.2 Replacement of the Phosphate Group*

The phosphate group can be replaced by non-phosphorus containing connectors. In certain embodiments, the charge phosphate group can be replaced by a neutral moiety.

Examples of moieties which can replace the phosphate group can include, without limitation, e.g., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

*11.2.3 Replacement of the Ribophosphate Backbone*

Scaffolds that can mimic nucleic acids can also be constructed wherein the phosphate linker and ribose sugar are replaced by nuclease resistant nucleoside or nucleotide surrogates. In certain embodiments, the nucleobases can be tethered by a surrogate backbone. Examples can include, without limitation, the morpholino, cyclobutyl, pyrrolidine and peptide nucleic acid (PNA) nucleoside surrogates.

***11.3 Sugar Modifications***

The modified nucleosides and modified nucleotides can include one or more modifications to the sugar group. For example, the 2' hydroxyl group (OH) can be modified or replaced with a number of different "oxy" or "deoxy" substituents. In certain embodiments, modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, e.g., alkyl, cycloalkyl, aryl, aralkyl, heteroaryl or a sugar); polyethyleneglycols (PEG), $O(CH_2CH_2O)_nCH_2CH_2OR$ wherein R can be, e.g., H or optionally substituted alkyl, and n can be an integer from 0 to 20 (e.g., from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In certain embodiments, the "oxy"-2' hydroxyl group modification can include "locked" nucleic acids (LNA) in which the 2' hydroxyl can be connected, e.g., by a $C_{1-6}$ alkylene or $C_{1-6}$ heteroalkylene bridge, to the 4' carbon of the same ribose sugar, where exemplary bridges can include methylene, propylene, ether, or amino bridges; O-amino (wherein amino can be, e.g., $NH_2$; alkylamino, dialkylamino, heterocyclyl, arylamino, diarylamino, heteroarylamino, or diheteroarylamino, ethylenediamine, or polyamino) and aminoalkoxy, $O(CH_2)_n$-amino, (wherein amino can be, e.g., $NH_2$; alkylamino, dialkylamino, heterocyclyl, arylamino, diarylamino, heteroarylamino, or diheteroarylamino, ethylenediamine, or polyamino). In certain embodiments, the "oxy"-2' hydroxyl group modification can include the methoxyethyl group (MOE), ($OCH_2CH_2OCH_3$, e.g., a PEG derivative).

"Deoxy" modifications can include hydrogen (i.e. deoxyribose sugars, e.g., at the overhang portions of partially ds RNA); halo (e.g., bromo, chloro, fluoro, or iodo); amino (wherein amino can be, e.g., $NH_2$; alkylamino, dialkylamino, heterocyclyl, arylamino, diarylamino, heteroarylamino, diheteroarylamino, or amino acid); $NH(CH_2CH_2NH)_nCH_2CH_2$-amino (wherein amino can be, e.g., as described herein), - NHC(O)R (wherein R can be, e.g., alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino as described herein.

The sugar group can also contain one or more carbons that possess the opposite stereochemical configuration than that of the corresponding carbon in ribose. Thus, a modified nucleic acid can include nucleotides containing e.g., arabinose, as the sugar. The nucleotide "monomer" can have an alpha linkage at the 1' position on the sugar, e.g., alpha-nucleosides. The modified nucleic acids can also include "abasic" sugars, which lack a nucleobase at C-1'. These abasic sugars can also be further modified at one or more of the constituent sugar atoms. The modified nucleic acids can also include one or more sugars that are in the L form, e.g. L-nucleosides.

Generally, RNA includes the sugar group ribose, which is a 5-membered ring having an oxygen. Exemplary modified nucleosides and modified nucleotides can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). In certain embodiments, the modified nucleotides can include multicyclic forms

(e.g., tricyclo; and "unlocked" forms, such as glycol nucleic acid (GNA) (e.g., R-GNA or S-GNA, where ribose is replaced by glycol units attached to phosphodiester bonds), threose nucleic acid (TNA, where ribose is replaced with $\alpha$-L-threofuranosyl-(3'$\rightarrow$2')).

### *11.4 Modifications on the Nucleobase*

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. In certain embodiments, the nucleobase can include, for example, naturally-occurring and synthetic derivatives of a base.

*11.4.1 Uracil*

In certain embodiments, the modified nucleobase is a modified uracil. Exemplary nucleobases and nucleosides having a modified uracil include without limitation pseudouridine ($\psi$), pyridin-4-one ribonucleoside, 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho$^5$U), 5-aminoallyl-uridine, 5-halo-uridine (e.g., 5-iodo-uridine or 5-bromo-uridine), 3-methyl-uridine (m$^3$U), 5-methoxy-uridine (mo$^5$U), uridine 5-oxyacetic acid (cmo$^5$U), uridine 5-oxyacetic acid methyl ester (mcmo$^5$U), 5-carboxymethyl-uridine (cm$^5$U), 1-carboxymethyl-pseudouridine, 5-carboxyhydroxymethyl-uridine (chm$^5$U), 5-carboxyhydroxymethyl-uridine methyl ester (mchm$^5$U), 5-methoxycarbonylmethyl-uridine (mcm$^5$U), 5-methoxycarbonylmethyl-2-thio-uridine (mcm$^5$s2U), 5-aminomethyl-2-thio-uridine (nm$^5$s2U), 5-methylaminomethyl-uridine (mnm$^5$U), 5-methylaminomethyl-2-thio-uridine (mnm$^5$s2U), 5-methylaminomethyl-2-seleno-uridine (mnm$^5$se$^2$U), 5-carbamoylmethyl-uridine (ncm$^5$U), 5-carboxymethylaminomethyl-uridine (cmnm$^5$U), 5-carboxymethylaminomethyl-2-thio-uridine (cmnm$^5$s2U), 5-propynyl-uridine, 1-propynyl-pseudouridine, 5-taurinomethyl-uridine ($\tau$cm$^5$U), 1-taurinomethyl-pseudouridine, 5-taurinomethyl-2-thio-uridine($\tau$m$^5$s2U), 1-taurinomethyl-4-thio-pseudouridine, 5-methyl-uridine (m$^5$U, i.e., having the nucleobase deoxythymine), 1-methyl-pseudouridine (m$^1\psi$), 5-methyl-2-thio-uridine (m$^5$s2U), 1-methyl-4-thio-pseudouridine (m$^1$s$^4\psi$), 4-thio-1-methyl-pseudouridine, 3-methyl-pseudouridine (m$^3\psi$), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m$^5$D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp$^3$U), 1-methyl-3-(3-amino-3-carboxypropyl)pseudouridine (acp$^3\psi$), 5-(isopentenylaminomethyl)uridine (inm$^5$U), 5-(isopentenylaminomethyl)-2-thio-uridine (inm$^5$s2U), $\alpha$-thio-uridine, 2'-O-methyl-uridine (Um), 5,2'-O-dimethyl-uridine (m$^5$Um), 2'-O-methyl-pseudouridine ($\psi$m), 2-thio-2'-O-methyl-uridine (s2Um), 5-methoxycarbonylmethyl-2'-O-methyl-uridine (mcm$^5$Um), 5-carbamoylmethyl-2'-O-methyl-uridine (ncm$^5$Um), 5-carboxymethylaminomethyl-2'-O-methyl-uridine (cmnm$^5$Um), 3,2'-O-dimethyl-uridine (m$^3$Um), 5-(isopentenylaminomethyl)-2'-O-methyl-uridine (inm$^5$Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, pyrazolo[3,4-d]pyrimidines, xanthine, and hypoxanthine.

*11.4.2 Cytosine*

In certain embodiments, the modified nucleobase is a modified cytosine. Exemplary nucleobases and nucleosides having a modified cytosine include without limitation 5-aza-cytidine, 6-aza-cytidine, pseudoisocytidine, 3-methyl-cytidine (m$^3$C), N4-acetyl-cytidine (act), 5-formyl-cytidine (f$^5$C), N4-methyl-cytidine (m$^4$C), 5-methyl-cytidine (m$^5$C), 5-halo-cytidine (e.g., 5-iodo-cytidine), 5-hydroxymethyl-cytidine (hm$^5$C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k$^2$C), $\alpha$-thio-cytidine, 2'-O-methyl-cytidine (Cm), 5,2'-O-dimethyl-cytidine (m$^5$Cm), N4-acetyl-2'-O-methyl-cytidine (ac$^4$Cm), N4,2'-O-dimethyl-cytidine (m$^4$Cm), 5-formyl-2'-O-methyl-cytidine (f$^5$Cm), N4,N4,2'-O-trimethyl-cytidine (m$^4_2$Cm), 1-thio-cytidine, 2'-F-ara-cytidine, 2'-F-cytidine, and 2'-OH-ara-cytidine.

*11.4.3 Adenine*

In certain embodiments, the modified nucleobase is a modified adenine. Exemplary nucleobases and nucleosides having a modified adenine include without limitation 2-amino-purine, 2,6-diaminopurine, 2-amino-6-halo-purine

(e.g., 2-amino-6-chloro-purine), 6-halo-purine (e.g., 6-chloro-purine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine ($m^1A$), 2-methyl-adenine ($m^2A$), N6-methyl-adenosine ($m^6A$), 2-methylthio-N6-methyl-adenosine ($ms2m^6A$), N6-isopentenyl-adenosine ($i^6A$), 2-methylthio-N6-isopentenyl-adenosine ($ms^2i^6A$), N6-(cis-hydroxyisopentenyl)adenosine ($io^6A$), 2-methylthio-N6-(cis-hydroxyisopentenyl)adenosine ($ms2io^6A$), N6-glycinylcarbamoyl-adenosine ($g^6A$), N6-threonylcarbamoyl-adenosine ($t^6A$), N6-methyl-N6-threonylcarbamoyl-adenosine ($m^6t^6A$), 2-methylthio-N6-threonylcarbamoyl-adenosine ($ms^2g^6A$), N6,N6-dimethyl-adenosine ($m^6{}_2A$), N6-hydroxynorvalylcarbamoyl-adenosine ($hn^6A$), 2-methylthio-N6-hydroxynorvalylcarbamoyl-adenosine ($ms2hn^6A$), N6-acetyl-adenosine ($ac^6A$), 7-methyl-adenine, 2-methylthio-adenine, 2-methoxy-adenine, $\alpha$-thio-adenosine, 2'-O-methyl-adenosine (Am), $N^6$2'-O-dimethyl-adenosine ($m^6Am$), $N^6$-Methyl-2'-deoxyadenosine, N6,N6,2'-O-trimethyl-adenosine ($m^6{}_2Am$), 1,2'-O-dimethyl-adenosine ($m^1Am$), 2'-O-ribosyladenosine (phosphate) (Ar(p)), 2-amino-N6-methylpurine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

*11.4.4 Guanine*

In certain embodiments, the modified nucleobase is a modified guanine. Exemplary nucleobases and nucleosides having a modified guanine include without limitation inosine (I), 1-methyl-inosine ($m^1I$), wyosine (imG), methylwyosine (mimG), 4-demethyl-wyosine (imG-14), isowyosine (imG2), wybutosine (yW), peroxywybutosine ($o_2yW$), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine ($preQ_0$), 7-aminomethyl-7-deaza-guanosine ($preQ_1$), archaeosine ($G^+$), 7-deaza-8-aza-guanosine, 6-thio-guanosine, 6-thio-7-deaza-guanosine, 6-thio-7-deaza-8-aza-guanosine, 7-methyl-guanosine ($m^7G$), 6-thio-7-methyl-guanosine, 7-methyl-inosine, 6-methoxy-guanosine, 1-methyl-guanosine (m'G), N2-methyl-guanosine ($m^2G$), N2,N2-dimethyl-guanosine ($m^2{}_2G$), N2,7-dimethyl-guanosine ($m^2$,7G), N2, N2,7-dimethyl-guanosine ($m^2$,2,7G), 8-oxo-guanosine, 7-methyl-8-oxo-guanosine, 1-meth thio-guanosine, N2-methyl-6-thio-guanosine, N2,N2-dimethyl-6-thio-guanosine, $\alpha$-thio-guanosine, 2'-O-methyl-guanosine (Gm), N2-methyl-2'-O-methyl-guanosine ($m^2Gm$), N2,N2-dimethyl-2'-O-methyl-guanosine ($m^2{}_2Gm$), 1-methyl-2'-O-methyl-guanosine (m'Gm), N2,7-dimethyl-2'-O-methyl-guanosine ($m^2$,7Gm), 2'-O-methyl-inosine (Im), 1,2'-O-dimethyl-inosine (m'Im), $O^6$-phenyl-2'-deoxyinosine, 2'-O-ribosylguanosine (phosphate) (Gr(p)), 1-thio-guanosine, $O^6$-methyl-guanosine, $O^6$-Methyl-2'-deoxyguanosine, 2'-F-ara-guanosine, and 2'-F-guanosine.

***11.5 Exemplary Modified gRNAs***

In certain embodiments, the modified nucleic acids can be modified gRNAs. It is to be understood that any of the gRNAs described herein can be modified in accordance with this section, including any gRNA that comprises a targeting domain of the gRNAs of SEQ ID NOS: 206-826366.

As discussed above, transiently expressed or delivered nucleic acids can be prone to degradation by, e.g., cellular nucleases. Accordingly, in certain embodiments, the modified gRNAs described herein can contain one or more modified nucleosides or nucleotides which introduce stability toward nucleases. In certain embodiments, the modified gRNAs described herein can exhibit a reduced innate immune response when introduced into a population of cells, particularly the cells of the present invention. As noted above, the term "innate immune response" includes a cellular response to exogenous nucleic acids, including single stranded nucleic acids, generally of viral or bacterial origin, which involves the induction of cytokine expression and release, particularly the interferons, and cell death.

While some of the exemplary modification discussed in this section may be included at any position within the gRNA sequence, in certain embodiments, a gRNA comprises a modification at or near its 5' end (e.g., within 1-10, 1-5, or 1-2 nucleotides of its 5' end). In certain embodiments, a gRNA comprises a modification at or near its 3' end (e.g., within 1-10, 1-5, or 1-2 nucleotides of its 3' end). In certain embodiments, a gRNA comprises both a modification at or near its 5' end and a modification at or near its 3' end.

In certain embodiments, the 5' end of a gRNA is modified by the inclusion of a eukaryotic mRNA cap structure or cap analog (e.g., a *G(5')ppp(5')G* cap analog, a *m7G(5')ppp(5')G* cap analog, or a *3'-O-Me-m7G(5')ppp(5')G* anti reverse cap analog (ARCA)). The cap or cap analog can be included during either chemical synthesis or *in vitro* transcription of the gRNA. In certain embodiments, an *in vitro* transcribed gRNA is modified by treatment with a phosphatase (e.g., calf intestinal alkaline phosphatase) to remove the 5' triphosphate group.

In certain embodiments, the 3' end of a gRNA is modified by the addition of one or more (e.g., 25-200) adenine (A) residues. The polyA tract can be contained in the nucleic acid (e.g., plasmid, PCR product, viral genome) encoding the gRNA, or can be added to the gRNA during chemical synthesis, or following *in vitro* transcription using a polyadenosine polymerase (e.g., *E. coli* Poly(A)Polymerase).

In certain embodiments, *in vitro* transcribed gRNA contains both a 5' cap structure or cap analog and a 3' polyA

tract. In certain embodiments, an *in vitro* transcribed gRNA is modified by treatment with a phosphatase (e.g., calf intestinal alkaline phosphatase) to remove the 5' triphosphate group and comprises a 3' polyA tract.

In certain embodiments, gRNAs can be modified at a 3' terminal U ribose. For example, the two terminal hydroxyl groups of the U ribose can be oxidized to aldehyde groups and a concomitant opening of the ribose ring to afford a modified nucleoside as shown below:

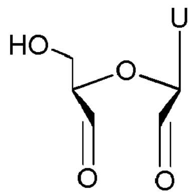

wherein "U" can be an unmodified or modified uridine.

In certain embodiments, the 3' terminal U can be modified with a 2'3' cyclic phosphate as shown below:

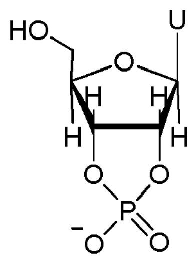

wherein "U" can be an unmodified or modified uridine.

In certain embodiments, the gRNA molecules may contain 3' nucleotides which can be stabilized against degradation, e.g., by incorporating one or more of the modified nucleotides described herein. In this embodiment, e.g., uridines can be replaced with modified uridines, e.g., 5-(2-amino)propyl uridine, and 5-bromo uridine, or with any of the modified uridines described herein; adenosines, and guanosines can be replaced with modified adenosines, and guanosines, e.g., with modifications at the 8-position, e.g., 8-bromo guanosine, or with any of the modified adenosines, or guanosines described herein.

In certain embodiments, sugar-modified ribonucleotides can be incorporated into the gRNA, e.g., wherein the 2' OH-group is replaced by a group selected from H, -OR, -R (wherein R can be, e.g., alkyl, cycloalkyl, aryl, aralkyl, heteroaryl or sugar), halo, -SH, -SR (wherein R can be, e.g., alkyl, cycloalkyl, aryl, aralkyl, heteroaryl or sugar), amino (wherein amino can be, e.g., $NH_2$; alkylamino, dialkylamino, heterocyclyl, arylamino, diarylamino, heteroarylamino, diheteroarylamino, or amino acid); or cyano (-CN). In certain embodiments, the phosphate backbone can be modified as described herein, e.g., with a phosphothioate group. In certain embodiments, one or more of the nucleotides of the gRNA can each independently be a modified or unmodified nucleotide including, but not limited to 2'-sugar modified, such as, 2'-O-methyl, 2'-O-methoxyethyl, or 2'-Fluoro modified including, e.g., 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aeo), 2'-O-methoxyethyl-5-methylcytidine (m5Ceo), and any combinations thereof.

In certain embodiments, a gRNA can include "locked" nucleic acids (LNA) in which the 2' OH-group can be connected, e.g., by a C1-6 alkylene or C1-6 heteroalkylene bridge, to the 4' carbon of the same ribose sugar, where exemplary bridges can include methylene, propylene, ether, or amino bridges; O-amino (wherein amino can be, e.g., $NH_2$; alkylamino, dialkylamino, heterocyclyl, arylamino, diarylamino, heteroarylamino, or diheteroarylamino, ethylenediamine, or polyamino) and aminoalkoxy or $O(CH_2)_n$-amino (wherein amino can be, e.g., $NH_2$; alkylamino, dialkylamino, heterocyclyl, arylamino, diarylamino, heteroarylamino, or diheteroarylamino, ethylenediamine, or polyamino).

In certain embodiments, a gRNA can include a modified nucleotide which is multicyclic (e.g., tricyclo; and "unlocked" forms, such as glycol nucleic acid (GNA) (e.g., R-GNA or S-GNA, where ribose is replaced by glycol units attached to phosphodiester bonds), or threose nucleic acid (TNA, where ribose is replaced with $\alpha$-L-threofuranosyl-(3'$\rightarrow$2')).

Generally, gRNA molecules include the sugar group ribose, which is a 5-membered ring having an oxygen. Exemplary modified gRNAs can include, without limitation, replacement of the oxygen in ribose (e.g., with sulfur (S), selenium (Se), or alkylene, such as, e.g., methylene or ethylene); addition of a double bond (e.g., to replace ribose with

cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). Although the majority of sugar analog alterations are localized to the 2' position, other sites are amenable to modification, including the 4' position. In certain embodiments, a gRNA comprises a 4'-S, 4'-Se or a 4'-C-aminomethyl-2'-O-Me modification.

In certain embodiments, deaza nucleotides, e.g., 7-deaza-adenosine, can be incorporated into the gRNA. In certain embodiments, O- and N-alkylated nucleotides, e.g., N6-methyl adenosine, can be incorporated into the gRNA. In certain embodiments, one or more or all of the nucleotides in a gRNA molecule are deoxynucleotides.

### *11.6 miRNA binding sites*

microRNAs (or miRNAs) are naturally occurring cellular 19-25 nucleotide long noncoding RNAs. They bind to nucleic acid molecules having an appropriate miRNA binding site, e.g., in the 3' UTR of an mRNA, and down-regulate gene expression. In certain embodiments, this down regulation occurs by either reducing nucleic acid molecule stability or inhibiting translation. An RNA species disclosed herein, e.g., an mRNA encoding Cas9, can comprise an miRNA binding site, e.g., in its 3'UTR. The miRNA binding site can be selected to promote down regulation of expression is a selected cell type.

## EXAMPLES

The following Examples are merely illustrative and are not intended to limit the scope or content of the invention in any way.

### Example 1: Cloning and Initial Screening of gRNAs

The suitability of candidate gRNAs can be evaluated as described in this example. Although described for a chimeric gRNA, the approach can also be used to evaluate modular gRNAs.

Cloning gRNAs into plasmid vector

For each gRNA, a pair of overlapping oligonucleotides is designed and obtained. Oligonucleotides are annealed and ligated into a digested vector backbone containing an upstream U6 promoter and the remaining sequence of a long chimeric gRNA. Plasmid is sequence-verified and prepped to generate sufficient amounts of transfection-quality DNA. Alternate promoters maybe used to drive in vivo transcription (e.g., H1 promoter) or for in vitro transcription (e.g., T7 promoter).

Cloning gRNAs in linear dsDNA molecule (STITCHR)

For each gRNA, a single oligonucleotide is designed and obtained. The U6 promoter and the gRNA scaffold (e.g. including everything except the targeting domain, e.g., including sequences derived from the crRNA and tracrRNA, e.g., including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule (STITCHR product) is purified for transfection. Alternate promoters may be used to drive in vivo transcription (e.g., H1 promoter) or for in vitro transcription (e.g., T7 promoter). Any gRNA scaffold may be used to create gRNAs compatible with Cas9s from any bacterial species.

Initial gRNA Screen

Each gRNA to be tested is transfected, along with a plasmid expressing Cas9 and a small amount of a GFP-expressing plasmid into human cells. In preliminary experiments, these cells can be immortalized human cell lines such as 293T, K562 or U2OS. Alternatively, primary human cells may be used. In this case, cells may be relevant to the eventual therapeutic cell target (for example, photoreceptor cells). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation. Following transfection, GFP expression can be determined either by fluorescence microscopy or by flow cytometry to

confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7E1-type assay or by sequencing. Alternatively, other mismatch-sensitive enzymes, such as Cell/Surveyor nuclease, may also be used.

For the T7E1 assay, PCR amplicons are approximately 500-700bp with the intended cut site placed asymmetrically in the amplicon. Following amplification, purification and size-verification of PCR products, DNA is denatured and re-hybridized by heating to 95°C and then slowly cooling. Hybridized PCR products are then digested with T7 Endonuclease I (or other mismatch-sensitive enzyme) which recognizes and cleaves non-perfectly matched DNA. If indels are present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = $(1-(1\text{-fraction cleaved})^{1/2})$. The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. For large sequencing numbers, Sanger sequencing may be used for determining the exact nature of indels after determining the NHEJ rate by T7E1.

Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low NHEJ rates.

### Example 2: Assessment of Gene Targeting by NHEJ

The gRNAs that induce the greatest levels of NHEJ in initial tests can be selected for further evaluation of gene targeting efficiency. For example, cells may be derived from disease subjects, relevant cell lines, and/or animal models and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency to generate the desired mutations (either knockout of a target gene or removal of a target sequence motif) may be determined by sequencing. For Sanger sequencing, PCR amplicons may be 500-700 bp long. For next generation sequencing, PCR amplicons may be 300-500 bp long. If the goal is to knockout gene function, sequencing may be used to assess what percent of alleles have undergone NHEJ-induced indels that result in a frameshift or large deletion or insertion that would be expected to destroy gene function. If the goal is to remove a specific sequence motif, sequencing may be used to assess what percent of alleles have undergone NHEJ-induced deletions that span this sequence.

### Example 3: Testing of gRNA Pairs Targeting the *DMD* Gene

Plasmid vectors encoding gRNAs targeting the *DMD* gene were transfected in pairs (125ng each), along with 750ng of a vector (pJDS246) encoding *S. pyogenes* Cas9 driven by a CMV promoter into 293 cells using Lipofectamine3000 (LifeTechnologies). Pairs of gRNAs are shown in **Table 7** along with the targeted deletion region (either exon 51, exons 51-55 or exons 45-55) and the targeted deletion size. Two days post-transfection, genomic DNA was isolated from transfected cells and PCR was performed with primers to amplify across the predicted deletion. Amplicons of the expected sizes (see **Table 7**) indicate that the predicted deletion had occurred and these PCR products were cloned into a plasmid vector using the Zero-Blunt TOPO kit and sequenced by Sanger sequencing. Sequencing results are shown in **Fig. 9.** Sequencing revealed the expected deletion events with the predominant result being a clean joining of the two cut sites and loss of all intervening sequence. Indels characteristic of NHEJ were also frequently observed in addition to the predicted targeted deletion.

**Table 7** shows pairs of gRNAs co-transfected with Cas9 into 293 cells. Left and Right gRNAs in the pair are indicated along with the region of deletion (exon 51, exons 51-55 or exons 45-55) and the predicted targeted deletion size. PCR primers used to assay the deletion are also shown and the expected PCR amplicon if the deletion has occurred is indicated.

Table 7

| Left gRNA | SEQ ID NO. of the targeting domain of Left gRNA | Right gRNA | SEQ ID NO. of targeting domain of Right gRNA | the Deletion | Deletion Size | PCR Primers | Expected amplicon size (bp) |
|---|---|---|---|---|---|---|---|
| DMD-5 | 692257 | DMD-9 | 692261 | exon 51 | 972bp | OME13+22 | 2709 |
| DMD-5 | 692257 | DMD-10 | 692262 | exon 51 | 1723bp | OME13+22 | 1958 |
| DMD-5 | 692257 | DMD-11 | 692263 | exon 51 | 893bp | OME13+22 | 2788 |
| DMD-5 | 692257 | DMD-12 | 692264 | exon 51 | 2665bp | OME13+22 | 1016 |
| DMD-6 | 692258 | DMD-9 | 692261 | exon 51 | 1326bp | OME13+22 | 2355 |
| DMD-6 | 692258 | DMD-10 | 692262 | exon 51 | 2077bp | OME13+22 | 1604 |
| DMD-6 | 692258 | DMD-11 | 692263 | exon 51 | 1247bp | OME13+22 | 2434 |
| DMD-6 | 692258 | DMD-12 | 692264 | exon 51 | 3019bp | OME13+22 | 662 |
| DMD-8 | 692260 | DMD-9 | 692261 | exon 51 | 1589bp | OME13+22 | 2092 |
| DMD-8 | 692260 | DMD-10 | 692262 | exon 51 | 2340bp | OME13+22 | 1341 |
| DMD-8 | 692260 | DMD-11 | 692263 | exon 51 | 1852bp | OME13+22 | 1829 |
| DMD-8 | 692260 | DMD-12 | 692264 | exon 51 | 3282bp | OME13+22 | 399 |
| DMD-5 | 692257 | DMD-18 | 692270 | exon 51-55 | 146.5kb | OME13+28 | 1462 |
| DMD-5 | 692257 | DMD-19 | 692271 | exon 51-55 | 146.5kb | OME13+28 | 1337 |
| DMD-5 | 692257 | DMD-20 | 692272 | exon 51-55 | 146.5kb | OME13+28 | 1056 |
| DMD-6 | 692258 | DMD-18 | 692270 | exon 51-55 | 146.5kb | OME13+28 | 1108 |
| DMD-6 | 692258 | DMD-19 | 692271 | exon 51-55 | 146.5kb | OME13+28 | 983 |
| DMD-6 | 692258 | DMD-20 | 692272 | exon 51-55 | 146.5kb | OME13+28 | 702 |
| DMD-8 | 692260 | DMD-18 | 692270 | exon 51-55 | 146.5kb | OME13+28 | 845 |
| DMD-8 | 692260 | DMD-19 | 692271 | exon 51-55 | 146.5kb | OME13+28 | 720 |
| DMD-8 | 692260 | DMD-20 | 692272 | exon 51-55 | 146.5kb | OME13+28 | 439 |

(continued)

| Left gRNA | SEQ ID NO. of the targeting domain of Left gRNA | Right gRNA | SEQ ID NO. of targeting domain of Right gRNA | the Deletion | Deletion Size | PCR Primers | Expected amplicon size (bp) |
|---|---|---|---|---|---|---|---|
| DMD-1 | 692253 | DMD-18 | 692270 | exon 45-55 | 341kb | OME9+28 | 721 |
| DMD-1 | 692253 | DMD-19 | 692271 | exon 45-55 | 341kb | OME9+28 | 596 |
| DMD-1 | 692253 | DMD-20 | 692272 | exon 45-55 | 341kb | OME9+28 | 315 |
| DMD-2 | 692254 | DMD-18 | 692270 | exon 45-55 | 341kb | OME9+28 | 652 |
| DMD-2 | 692254 | DMD-19 | 692271 | exon 45-55 | 341kb | OME9+28 | 527 |
| DMD-2 | 692254 | DMD-20 | 692272 | exon 45-55 | 341kb | OME9+28 | 246 |
| DMD-4 | 692256 | DMD-18 | 692270 | exon 45-55 | 341kb | OME9+28 | 1637 |
| DMD-4 | 692256 | DMD-19 | 692271 | exon 45-55 | 341kb | OME9+28 | 1512 |
| DMD-4 | 692256 | DMD-20 | 692272 | exon 45-55 | 341kb | OME9+28 | 1231 |

**Example 4: Dilution Cloning to Estimate Deletion Frequency**

Plasmid vectors encoding pairs of gRNAs targeting the *DMD* gene were co transfected with pJDS246 into 293 cells as described above. Two days post-transfection cells were plated into 96-well plates at a density of either 10 cells per well (for exon 51 deletions) or 100 cells per well (for exons 45-55 deletions). Once wells reached confluency, genomic DNA was isolated from cells and PCR was performed across the targeted deletion region. Generation of an amplicon of the predicted deletion size indicates that at least one cell in that well was carrying an allele with the targeted deletion. Total numbers of wells assays and wells that were positive by the PCR assay are shown in **Table 8.** The most conservative estimate of the number of modified alleles assumes that only one allele of one cell in the well has undergone the deletion event and divides this number by the total possible number of alleles present in the population, assuming 2 alleles per cell. A binomial distribution is then used to calculate a conservative estimate of the % modification of the population (the % of the population that has undergone the targeted deletion). This number was calculated for the exon51 deletion samples. Experiments with fewer cells per well can also been performed to calculate % modification for the exon45-55 deletion samples.

**Table 8** shows the results of dilution cloning of 293 cells transfected with pairs of gRNAs targerting the *DMD* gene and Cas9. The deletion region and deletion size are indicated for each gRNA pair. The # of cells per well and number of wells assayed are shown and the # positive wells indicates the number of wells that give a positive signal in the PCR assay. The "Conservative % modified alleles" indicates the most conservative estimate of the number of modified alleles.

Table 8

| Left gRNA Name | SEQ ID NO. of targeting domain of Left gRNA | Right gRNA Name | SEQ ID NO. of targeting domain of Right gRNA | Deletion | Deletion Size | # Cells/ Well | # Wells | # Pos. Wells | Conserv. % Modified Alleles | % Modified Based on Binomial Distr. |
|---|---|---|---|---|---|---|---|---|---|---|
| DMD-5 | 692257 | DMD-9 | 692261 | exon 51 | 972bp | 10 | 20 | 8 | 2.00 | 3.85 |
| DMD-5 | 692257 | DMD-10 | 692262 | exon 51 | 1723bp | 10 | 20 | 8 | 2.00 | 3.85 |
| DMD-6 | 692258 | DMD-9 | 692261 | exon 51 | 1326bp | 10 | 20 | 5 | 1.25 | 2.44 |
| DMD-6 | 692258 | DMD-10 | 692262 | exon 51 | 2077bp | 10 | 20 | 12 | 3.00 | 5.67 |
| DMD-8 | 692260 | DMD-11 | 692263 | exon 51 | 1852bp | 10 | 20 | 5 | 1.25 | 2.44 |
| DMD-8 | 692260 | DMD-12 | 692264 | exon 51 | 3282bp | 10 | 20 | 15 | 3.75 | 6.98 |
| DMD-1 | 692253 | DMD-18 | 692270 | 55 oxon 45-55 | 341kb | 100 | 30 | 30 | 0.50 | |
| DMD-2 | 692254 | DMD-18 | 692270 | exon 45-55 | 341kb | 100 | 30 | 28 | 0.47 | |
| DMD-4 | 692256 | DMD-19 | 692271 | oxon 45-55 | 341kb | 100 | 30 | 25 | 0.42 | |
| DMD-4 | 692256 | DMD-20 | 692272 | oxon 45-55 | 341kb | 100 | 26 | 22 | 0.42 | |

**Example 5: Quantification of Genomic Deletions by ddPCR**

Droplet digital PCR (ddPCR) is a method for performing digital PCR in which a single PCR reaction is fractionated into approximately 20,000 droplets in a water-oil emulsion and PCR amplification occurs separately in individual droplets. PCR conditions are optimized for a concentration of DNA template such that each droplet contains either one or no template molecules. Assays are designed to perform amplification using BioRad EvaGreen Supermix PCR system with all amplicons ranging in size from 250-350bp. Control assays are designed to amplify segments of the *DMD* gene at least 5kb away from the gRNA target sites. Assays to detect targeted genomic deletion are designed such that amplification of an allele that has undergone deletion will yield a PCR product in the size range of 250-350bp and amplification will not occur on a wildtype allele due to the increased distance between forward and reverse primers. PCR conditions are optimized on genomic DNA isolated from 293 cells that have been transfected with pairs of gRNAs and Cas9-expressing plasmid. Deletion assays are verified to generate no positive signal on genomic DNA isolated from unmodified cells. Assays may then be used to quantify genomic deletions on modified populations of the relevant cell type.

**Example 6 - Screening *S. Aureus* CRISPR-Cas9 paired-guide RNAs for efficient targeted deletion in DMD**

*1. Background*

Most DMD patients have exonic deletions in the *DMD* gene that result in a frameshift and nonfunctional protein. In contrast, BMD patients carry a range of exonic deletions in *DMD* that do not disrupt the reading frame, leading to a much milder disease phenotype. Thus, multiplex CRISPR/Cas9 targeted deletions that restore the reading frame can convert DMD genotypes into BMD-like genotypes and potentially treat this disease. Previously, this strategy has been demonstrated *in vitro* with zinc finger nucleases, TALENs, and *S. pyogenes* Cas9 leading to restoration of dystrophin expression in DMD patient myoblasts. However, these genome-editing enzymes are limited by the difficulty of delivering large transgenes with viral vectors *in vivo.* Alternatively, the smaller Cas9 ortholog from *S. aureus* (SaCas9) can be packaged with paired gRNAs in an all-in-one AAV vector for *in vivo* gene therapy. Recently, three groups have demonstrated that AAV-SaCas9 can mediate targeted deletions in *mdx* mice and restore dystrophin expression (C.E. Nelson et al., Science 10.1126/science.aad5143 (2015); M. Tabebordbar et al., Science 10.1126/science.aad5177 (2015); C. Long et al., Science 10.1126/science.aad5725 (2015)).

*2. Results*

An efficiency screen to identify highly active paired gRNAs for targeted deletion of exon 51 of the *DMD* gene was conducted as a first step towards developing a genome editing therapeutic for DMD. A sensitive, digital droplet PCR ("ddPCR") assay to quantify exon 51 deletion was validated with DMD patient samples (*see* Figs. 16A and 16B). The ddPCR assay is a medium-throughput assay that quantifies Exon 51 deletion regardless of which gRNA pair was used. Four sets of Guide (gRNA) pairs for Exon 51 deletion were screened.

Guide Set 1 (*see* Table 9) was an initial pilot study of 15 gRNAs. This initial pilot study identified a gRNA pair (1+9) that mediated 18% Exon 51 deletion in HEK293T cells three days after transfection of plasmids (*see* Fig. 17B). No increase in deletion efficiency was observed six days post-transfection compared to three days post-transfection (*see* Fig. 17B). gRNA pair (1+9) was selected as a positive control for the following screening tests.

**Table 9 - Guide Set 1 (8 paris)**

| gRNA No. | SEQ ID NO. of the nucleotide sequence comprised in the gRNA targeting domain | PAM | Strand | Location | Specificity Score | Target Position |
|---|---|---|---|---|---|---|
| 1 | 19199 (SEQ ID NO: 826369 for the corresponding DNA) | CTGGGT' | reverse | 29660464 | 36.36363636 | Intron 50 |
| 2 | 11347 (SEQ ID NO: 826370 for the corresponding DNA) | AGGGAT' | forward | 29660577 | 50 | Intron 50 |
| 3 | 19416 (SEQ ID NO: 826371 for the corresponding DNA) | GTGGGT' | reverse | 29660792 | 45.45454545 | Intron 50 |
| 4 | 134147 (SEQ ID NO: 826372 for the corresponding DNA) | CTGAAT' | reverse | 29660961 | 54.54545455 | Intron 50 |

(continued)

| gRNA No. | SEQ ID NO. of the nucleotide sequence comprised in the gRNA targeting domain | PAM | Strand | Location | Specificity Score | Target Position |
|---|---|---|---|---|---|---|
| 5 | 15089 (SEQ ID NO: 826373 for the corresponding DNA) | AGGAAT’ | reverse | 29665345 | 36.36363636 | Intron 50 |
| 6 | 11169 (SEQ ID NO: 826374 for the corresponding DNA) | GTGAAT’ | forward | 29666297 | 27.27272727 | Intron 50 |
| 7 | 1485 (SEQ ID NO: 826375 for the corresponding DNA) | AGGAGT’ | forward | 29678735 | 45.45454545 | Intron 50 |
| 8 | 9240 (SEQ ID NO: 826376 for the corresponding DNA) | TAGGAT’ | forward | 29681610 | 31.81818182 | Intron 50 |
| 9 | 18720 (SEQ ID NO: 826377 for the corresponding DNA) | GAGAGT’ | reverse | 29655049 | 50 | Intron 51 |
| 10 | 12290 (SEQ ID NO: 826378 for the corresponding DNA) | ATGGAT’ | reverse | 29651942 | 18.18181818 | Intron 51 |
| 11 | 11548 (SEQ ID NO: 826379 for the corresponding DNA) | AAGGGT’ | forward | 29651425 | 40.90909091 | Intron 51 |
| 12 | 364 (SEQ ID NO: 826380 for the corresponding DNA) | TAGAAT’ | forward | 29649354 | 36.36363636 | Intron 51 |
| 13 | 20079 (SEQ ID NO: 826381 for the corresponding DNA) | GTGAAT’ | reverse | 29649147 | 45.45454545 | Intron 51 |
| 14 | 21882 (SEQ ID NO: 826382 for the corresponding DNA) | GGGGAT’ | reverse | 29636772 | 36.36363636 | Intron 51 |
| 15 | 3452 (SEQ ID NO: 826383 for the corresponding DNA) | AGGAAT’ | forward | 29635249 | 50 | Intron 51 |

SaCas9 codon optimization and promoter alternatives were tested with the ddPCR assay and guide pairs known to mediate Exon 51 deletion (*see* Fig. 18B). The CMV and EFS promoters were tested with 3 codon optimization of SaCas9. As shown in Fig. 18A, no alternatives performed significantly better than pAF003 plasmid which expresses SaCas9 from a CMV promoter, using the SaCas9 codon optimization described in Ran et al., Nature (2015 Apr 9);520(7546):186-91.

Of note, this experiment also included transfections of 4 guide RNAs, as opposed to the usual 2 guide RNAs. It was hypothesized that more guide RNAs could increase deletion efficiency by increasing the likelihood of simultaneous cuts in both targeted introns. Generally, 4 guide RNAs were similarly efficient to 2 guide RNAs.

To determine whether adding a guanine (G) nucleotide to the 5’ of the gRNA can increase the SaCas9 deletion efficiency, the indel rate mediated by certain single gRNAs targeting Exon 51 with or without a 5’ G was measured by targeted deep sequencing. Primers were designed for a ~330 base pair amplicon of the Exon 51 genomic DNA. These PCR amplicons were sequenced on an Illumina MiSeq and 10,000 reads per sample (n=2) were analyzed for the presence or absence of indels within the amplicon.

As shown in Fig. 19, an added 5’ G increased SaCas9 deletion efficiency for most gRNAs. These single gRNAs (103-106, and 111) target within or very nearly in Exon 51 and were not used in guide pairs for targeted deletion of Exon 51 in the screen. Whether the added 5’ G was matched or mismatched in the genome did not have an apparent effect on the indel efficiency benefit. However, this may affect off-target editing considerations.

Guide Set 2 (*see* Table 10) was focused on human and non-human primate (NHP) cross-reactive gRNAs. From the set of 10,553 21-nt SaCas9 guides targeting human DMD introns 50 and 51, 53 gRNAs making 675 gRNAs pairs that met the following four filtering requirements were selected. The four filtering requirements were: 1) an endogenous 5’ G, 2) a 3’ T in the NNGRR(T) PAM, 3) cross-reactivity with the non-human primate (NHP) genome, and 4) no off-by-1 or off-by-2 mismatch sites in the human genome. These filters were selected to improve U6 promoter expression, improve SaCas9 cleavage/deletion efficiency, enable pre-clinical animal model studies, and minimize off-target editing concerns, respectively.

**Table 10 - Guide Set 2 (52 gRNAs, 675 pairs)**

| gRNA No. | SEQ ID NO. of the nucleotide sequence comprised in the gRNA targeting domain | SEQ ID NO. for guidePAM | Strand | type | human orthogonality | monkey orthogonality | Target Position |
|---|---|---|---|---|---|---|---|
| 16 | 10140 (SEQ ID NO: 826384 for the corresponding DNA) | 826385 | Sense | sa21 | 2489 | 2600 | Intron50 |
| 17 | 4072 (SEQ ID NO: 826386 for the corresponding DNA) | 826387 | Sense | sa21 | 12527 | 6364 | Intron50 |
| 18 | 13527 (SEQ ID NO: 826388 for the corresponding DNA) | 826389 | Antisense | sa21 | 5293 | 3997 | Intron50 |
| 19 | 1744 (SEQ ID NO: 826390 for the corresponding DNA) | 826391 | Sense | sa21 | 5082 | 107534 | Intron50 |
| 20 | 17253 (SEQ ID NO: 826392 for the corresponding DNA) | 826393 | Antisense | sa21 | 5007 | 2167 | Intron50 |
| 21 | 20924 (SEQ ID NO: 826394 for the corresponding DNA) | 826395 | Antisense | sa21 | 3319 | 1677 | Intron50 |
| 22 | 9496 (SEQ ID NO: 826396 for the corresponding DNA) | 826397 | Sense | sa21 | 11107 | 8145 | Intron50 |
| 23 | 14035 (SEQ ID NO: 826398 for the corresponding DNA) | 826399 | Antisense | sa21 | 11480 | 43955 | Intron50 |
| 24 | 13285 (SEQ ID NO: 826400 for the corresponding DNA) | 826401 | Antisense | sa21 | 14740 | 10181 | Intron50 |
| 25 | 1484 (SEQ ID NO: 826402 for the corresponding DNA) | 826403 | Sense | sa21 | 12720 | 5016402 | Intron50 |
| 26 | 7419 (SEQ ID NO: 826404 for the corresponding DNA) | 826405 | Sense | sa21 | 6569 | 6272 | Intron50 |

(continued)

| gRNA No. | SEQ ID NO. of the nucleotide sequence comprised in the gRNA targeting domain | SEQ ID NO. for guidePAM | Strand | type | human orthogonality | monkey orthogonality | Target Position |
|---|---|---|---|---|---|---|---|
| 27 | 9279 (SEQ ID NO: 826406 for the corresponding DNA) | 826407 | Sense | sa21 | 15927 | 10557 | Intron50 |
| 28 | 13228 (SEQ ID NO: 826408 for the corresponding DNA) | 826409 | Antisense | sa21 | 59359 | 46292 | Intron50 |
| 29 | 2769 (SEQ ID NO: 826410 for the corresponding DNA) | 826411 | Sense | sa21 | 6636 | 11045 | Intron50 |
| 30 | 5614 (SEQ ID NO: 826412 for the corresponding DNA) | 826413 | Sense | sa21 | 6388 | 5494 | Intron50 |
| 31 | 7252 (SEQ ID NO: 826414 for the corresponding DNA) | 826415 | Sense | sa21 | 9316 | 7635 | Intron50 |
| 32 | 21873 (SEQ ID NO: 826416 for the corresponding DNA) | 826417 | Antisense | sa21 | 5208 | 4030 | Intron50 |
| 33 | 20764 (SEQ ID NO: 826418 for the corresponding DNA) | 826419 | Antisense | sa21 | 25909 | 19154 | Intron50 |
| 34 | 14607 (SEQ ID NO: 826420 for the corresponding DNA) | 826421 | Antisense | sa21 | 5008 | 5985 | Intron50 |
| 35 | 13518 (SEQ ID NO: 826422 for the corresponding DNA) | 826423 | Antisense | sa21 | 39294 | 237187 | Intron50 |
| 36 | 18195 (SEQ ID NO: 826424 for the corresponding DNA) | 826425 | Antisense | sa21 | 11370 | 13731 | Intron50 |
| 37 | 16967 (SEQ ID NO: 826426 for the corresponding DNA) | 826427 | Antisense | sa21 | 5616 | 106854 | Intron50 |

(continued)

| gRNA No. | SEQ ID NO. of the nucleotide sequence comprised in the gRNA targeting domain | SEQ ID NO. for guidePAM | Strand | type | human orthogonality | monkey orthogonality | Target Position |
|---|---|---|---|---|---|---|---|
| 38 | 3536 (SEQ ID NO: 826428 for the corresponding DNA) | 826429 | Sense | sa21 | 6553 | 8785 | Intron50 |
| 39 | 9358 (SEQ ID NO: 826430 for the corresponding DNA) | 826431 | Sense | sa21 | 7828 | 6500 | Intron50 |
| 40 | 14298 (SEQ ID NO: 826432 for the corresponding DNA) | 826433 | Antisense | sa21 | 4384 | 5129 | Intron50 |
| 41 | 8569 (SEQ ID NO: 826434 for the corresponding DNA) | 826435 | Sense | sa21 | 22175 | 19606 | Intron50 |
| 42 | 14695 (SEQ ID NO: 826436 for the corresponding DNA) | 826437 | Antisense | sa21 | 6361 | 4578 | Intron50 |
| 43 | 15271 (SEQ ID NO: 826438 for the corresponding DNA) | 826439 | Antisense | sa21 | 12809 | 12288 | Intron51 |
| 44 | 18625 (SEQ ID NO: 826440 for the corresponding DNA) | 826441 | Antisense | sa21 | 7969 | 7488 | Intron51 |
| 45 | 4049 (SEQ ID NO: 826442 for the corresponding DNA) | 826443 | Sense | sa21 | 33948 | 134818 | Intron51 |
| 46 | 20527 (SEQ ID NO: 826444 for the corresponding DNA) | 826445 | Antisense | sa21 | 9951 | 11542 | Intron51 |
| 47 | 16235 (SEQ ID NO: 826446 for the corresponding DNA) | 826447 | Antisense | sa21 | 20349 | 119541 | Intron51 |
| 48 | 7649 (SEQ ID NO: 826448 for the corresponding DNA) | 826449 | Sense | sa21 | 2624 | 3675 | Intron51 |

(continued)

| gRNA No. | SEQ ID NO. of the nucleotide sequence comprised in the gRNA targeting domain | SEQ ID NO. for guidePAM | Strand | type | human orthogonality | monkey orthogonality | Target Position |
|---|---|---|---|---|---|---|---|
| 49 | 11675 (SEQ ID NO: 826450 for the corresponding DNA) | 826451 | Sense | sa21 | 35438 | 33867 | Intron51 |
| 50 | 9530 (SEQ ID NO: 826452 for the corresponding DNA) | 826453 | Sense | sa21 | 7779 | 5961 | Intron51 |
| 51 | 15622 (SEQ ID NO: 826454 for the corresponding DNA) | 826455 | Antisense | sa21 | 4358 | 6351 | Intron51 |
| 52 | 9102 (SEQ ID NO: 826456 for the corresponding DNA) | 826457 | Sense | sa21 | 83487 | 461195 | Intron51 |
| 53 | 20870 (SEQ ID NO: 826458 for the corresponding DNA) | 826459 | Antisense | sa21 | 7441 | 7169 | Intron51 |
| 54 | 19187 (SEQ ID NO: 826460 for the corresponding DNA) | 826461 | Antisense | sa21 | 3446 | 108272 | Intron51 |
| 55 | 18752 (SEQ ID NO: 826462 for the corresponding DNA) | 826463 | Antisense | sa21 | 10605 | 11171 | Intron51 |
| 56 | 9346 (SEQ ID NO: 826464 for the corresponding DNA) | 826465 | Sense | sa21 | 4093 | 2726 | Intron51 |
| 57 | 22573 (SEQ ID NO: 826466 for the corresponding DNA) | 826467 | Antisense | sa21 | 7273 | 9256 | Intron51 |
| 58 | 22786 (SEQ ID NO: 826468 for the corresponding DNA) | 826469 | Antisense | sa21 | 16194 | 11573 | Intron51 |
| 59 | 20145 (SEQ ID NO: 826470 for the corresponding DNA) | 826471 | Antisense | sa21 | 412 | 462 | Intron51 |

(continued)

| gRNA No. | SEQ ID NO. of the nucleotide sequence comprised in the gRNA targeting domain | SEQ ID NO. for guidePAM | Strand | type | human orthogonality | monkey orthogonality | Target Position |
|---|---|---|---|---|---|---|---|
| 60 | 21131 (SEQ ID NO: 826472 for the corresponding DNA) | 826473 | Antisense | sa21 | 15140 | 16468 | Intron51 |
| 61 | 6209 (SEQ ID NO: 826474 for the corresponding DNA) | 826475 | Sense | sa21 | 19629 | 116142 | Intron51 |
| 62 | 5869 (SEQ ID NO: 826476 for the corresponding DNA) | 826477 | Sense | sa21 | 15699 | 17636 | Intron51 |
| 63 | 4324 (SEQ ID NO: 826478 for the corresponding DNA) | 826479 | Sense | sa21 | 10940 | 10655 | Intron51 |
| 64 | 4165 (SEQ ID NO: 826480 for the corresponding DNA) | 826481 | Sense | sa21 | 8627 | 6012 | Intron51 |
| 65 | 23958 (SEQ ID NO: 826482 for the corresponding DNA) | 826483 | Antisense | sa21 | 21343 | 23731 | Intron51 |
| 66 | 18062 (SEQ ID NO: 826484 for the corresponding DNA) | 826485 | Antisense | sa21 | 5927 | 9192 | Intron51 |
| 67 | 4134 (SEQ ID NO: 826486 for the corresponding DNA) | 826487 | Sense | sa21 | 11601 | 8050 | Intron51 |

The Z score was calculated for deletion efficiencies of the gRNA pairs in Guide Set 2, and the results are shown in Fig. 20. Hits were defined as gRNA pairs with a Z score > 1.5 in at least one of two biological replicates, and are listed in Table 11.

**Table 11**

| Guide Set 2 | | |
|---|---|---|
| **Guide_R** | **Guide_L** | **Threshold Passed** |
| 43 | 35 | Z Score 2 > 1.5 |
| 47 | 16 | Avg Z score > 1.5 |
| 48 | 17 | Z Score 2 > 1.5 |

(continued)

| Guide Set 2 | | |
|---|---|---|
| Guide_R | Guide_L | Threshold Passed |
| 48 | 21 | Avg Z score > 1.5 |
| 48 | 22 | Z Score 2 > 1.5 |
| 48 | 25 | Avg Z score > 1.5 |
| 48 | 37 | Z Score 2 > 1.5 |
| 49 | 17 | Z score 1 > 1.5 |
| 49 | 25 | Z Score 2 > 1.5 |
| 49 | 29 | Z Score 2 > 1.5 |
| 50 | 38 | Z score 1 > 1.5 |
| 51 | 20 | Avg Z score > 1.5 |
| 53 | 31 | Z Score 2 > 1.5 |
| 53 | 37 | Z Score 2 > 1.5 |
| 53 | 38 | Z score 1 > 1.5 |
| 54 | 19 | Z score 1 > 1.5 |
| 54 | 20 | Z score 1 > 1.5 |
| 54 | 26 | Avg Z score > 1.5 |
| 54 | 31 | Z score 1 > 1.5 |
| 54 | 39 | Z Score 2 > 1.5 |
| 54 | 41 | Z Score 2 > 1.5 |
| 54 | 42 | Avg Z score > 1.5 |
| 55 | 19 | Z Score 2 > 1.5 |
| 55 | 20 | Z Score 2 > 1.5 |
| 55 | 26 | Z Score 2 > 1.5 |
| 55 | 34 | Avg Z score > 1.5 |
| 55 | 37 | Z Score 2 > 1.5 |
| 55 | 38 | Z Score 2 > 1.5 |
| 55 | 41 | Z score 1 > 1.5 |
| 56 | 26 | Z score 1 > 1.5 |
| 56 | 29 | Z score 1 > 1.5 |
| 56 | 33 | Z score 1 > 1.5 |
| 59 | 30 | Z Score 2 > 1.5 |
| 59 | 31 | Z Score 2 > 1.5 |
| 59 | 33 | Avg Z score > 1.5 |
| 59 | 34 | Z Score 2 > 1.5 |
| 59 | 38 | Avg Z score > 1.5 |
| 59 | 41 | Z score 1 > 1.5 |
| 60 | 18 | Z score 1 > 1.5 |
| 60 | 24 | Z Score 2 > 1.5 |

(continued)

| Guide Set 2 | | |
|---|---|---|
| Guide_R | Guide_L | Threshold Passed |
| 61 | 18 | Z Score 2 > 1.5 |
| 61 | 21 | Z Score 2 > 1.5 |
| 61 | 23 | Z Score 2 > 1.5 |
| 61 | 26 | Avg Z score > 1.5 |
| 61 | 32 | Z score 1 > 1.5 |
| 61 | 40 | Z score 1 > 1.5 |
| 62 | 17 | Z score 1 > 1.5 |
| 62 | 18 | Z score 1 > 1.5 |
| 62 | 25 | Z score 1 > 1.5 |
| 62 | 30 | Avg Z score > 1.5 |
| 62 | 38 | Avg Z score > 1.5 |
| 62 | 41 | Z Score 2 > 1.5 |
| 62 | 42 | Avg Z score > 1.5 |
| 64 | 25 | Z Score 2 > 1.5 |
| 64 | 27 | Z score 1 > 1.5 |
| 64 | 31 | Z Score 2 > 1.5 |
| 65 | 22 | Z Score 2 > 1.5 |
| 65 | 23 | Z Score 2 > 1.5 |
| 65 | 38 | Z Score 2 > 1.5 |
| 66 | 23 | Z Score 2 > 1.5 |
| 66 | 29 | Z score 1 > 1.5 |
| 66 | 38 | Avg Z score > 1.5 |
| 67 | 17 | Avg Z score > 1.5 |
| 67 | 21 | Z Score 2 > 1.5 |
| 67 | 38 | Z Score 2 > 1.5 |
| 67 | 41 | Z Score 2 > 1.5 |
| 86 | 68 | Z Score 2 > 1.5 |
| 82 | 68 | Avg Z score > 1.5 |
| 85 | 68 | Avg Z score > 1.5 |
| 92 | 68 | Avg Z score > 1.5 |
| 91 | 68 | Z score 1 > 1.5 |
| 88 | 68 | Avg Z score > 1.5 |
| 89 | 68 | Z score 1 > 1.5 |
| 83 | 68 | Z score 1 > 1.5 |
| 84 | 68 | Z score 1 > 1.5 |
| 87 | 68 | Z Score 2 > 1.5 |
| 85 | 70 | Z score 1 > 1.5 |

(continued)

| Guide Set 2 | | |
|---|---|---|
| Guide_R | Guide_L | Threshold Passed |
| 82 | 71 | Z score 1 > 1.5 |

Notably, the NHP cross-reactivity requirement skewed the distribution of possible targeted deletion lengths towards larger sizes, all greater than about 12.4 kb (*see* Fig. 21). However, it was hypothesized that smaller deletions would generally be more efficiently generated.

In order to test smaller deletions, an additional 174 pairs of human-only gRNAs (Guide Set 3) were designed for deletion lengths of about 0.8 to about 14 kb. Among the 174 gRNA pairs, 168 pairs (Guide Set 3; *see* Table 12) had a deletion length of less than 14 kb, and 6 pairs (Guide Set 4; *see* Table 13) had a deletion length of less than 1.3 kb. The deletion efficiency of the gRNA pairs in Guide Set 3 was measured and ranked (*see* Fig. 22). A heatmap representation of the gRNA pairs' deletion efficiencies in Guide Set 3 are shown in Fig. 23. There was not a strong trend for increased deletion efficiency with decreased deletion size. The Z score was calculated for the gRNA pairs in Guide Set 3, and the results are shown in Fig. 24 and Table 14. As shown in Fig. 26, the gRNAs pairs in Guide Sets 2 and 3 with passing Z scores (i.e., Z-score > 1.5) were included as hits for further validation, alongside the 6 pairs from Guide Set 4. In total, there were 85 pairs out of the original 857 pairs. The 85 pairs are shown in Table 15. The deletion efficiency of these 85 gRNA pairs was measured and ranked (*see* Fig. 25).

**Table 12 - Guide Set 3 (26 gRNAs, 168 pairs)**

| gRNA No. | SEQ ID NO. of the nucleotide sequence comprised in the gRNA targeting domain | SEQ ID NO. for guidePAM | Orientation | Type | Orthogonality | Target position | Position | Dist_ from Sprime |
|---|---|---|---|---|---|---|---|---|
| 68 | 2048 (SEQ ID NO: 826488 for the corresponding DNA) | 826489 | Sense | sa21 | 4372 | Intron50 | 31793286 | 1209 |
| 69 | 22095 (SEQ ID NO: 826490 for the corresponding DNA) | 826491 | Antisense | sa21 | 1396 | Intron50 | 31793773 | 1696 |
| 70 | 14296 (SEQ ID NO: 826492 for the corresponding DNA) | 826493 | Antisense | sa21 | 16654 | Intron50 | 31794247 | 2170 |
| 71 | 9765 (SEQ ID NO: 826494 for the corresponding DNA) | 826495 | Sense | sa21 | 1808 | Intron50 | 31794947 | 2870 |
| 72 | 21671 (SEQ ID NO: 826496 for the corresponding DNA) | 826497 | Antisense | sa21 | 4341 | Intron50 | 31795173 | 3096 |
| 73 | 21821 (SEQ ID NO: 826498 for the corresponding DNA) | 826499 | Antisense | sa21 | 32770 | Intron50 | 31795494 | 3417 |
| 74 | 7978 (SEQ ID NO: 826500 for the corresponding DNA) | 826501 | Sense | sa21 | 28751 | Intron50 | 31796044 | 3967 |
| 75 | 23363 (SEQ ID NO: 826502 for the corresponding DNA) | 826503 | Antisense | sa21 | 20749 | Intron50 | 31796289 | 4212 |
| 76 | 11281 (SEQ ID NO: 826504 for the corresponding DNA) | 826505 | Sense | sa21 | 11956 | Intron50 | 31796453 | 4376 |
| 77 | 16781 (SEQ ID NO: 826506 for the corresponding DNA) | 826507 | Antisense | sa21 | 1580 | Intron50 | 31797199 | 5122 |
| 78 | 17243 (SEQ ID NO: 826508 for the corresponding DNA) | 826509 | Antisense | sa21 | 8081 | Intron50 | 31797264 | 5187 |
| 79 | 2624 (SEQ ID NO: 826510 for the corresponding DNA) | 826511 | Sense | sa21 | 8398 | Intron50 | 31797427 | 5350 |
| 80 | 7228 (SEQ ID NO: 826512 for the corresponding DNA) | 826513 | Sense | sa21 | 7001 | Intron50 | 31797482 | 5405 |
| 81 | 6807 (SEQ ID NO: 826514 for the corresponding DNA) | 826515 | Sense | sa21 | 15793 | Intron50 | 31797946 | 5869 |

(continued)

| gRNA No. | SEQ ID NO. of the nucleotide sequence comprised in the gRNA targeting domain | SEQ ID NO. for guidePAM | Orientation | Type | Orthogonality | Target position | Position | Dist_ from Sprime |
|---|---|---|---|---|---|---|---|---|
| 82 | 18458 (SEQ ID NO: 826516 for the corresponding DNA) | 826517 | Antisense | sa21 | 26553 | Intron51 | 31791729 | 348 |
| 83 | 21570 (SEQ ID NO: 826518 for the corresponding DNA) | 826519 | Antisense | sa21 | 5075 | Intron51 | 31790998 | 1079 |
| 84 | 1977 (SEQ ID NO: 826520 for the corresponding DNA) | 826521 | Sense | sa21 | 5677 | Intron51 | 31790759 | 1318 |
| 85 | 1499 (SEQ ID NO: 826522 for the corresponding DNA) | 826523 | Sense | sa21 | 30791 | Intron51 | 31789043 | 3034 |
| 86 | 481 (SEQ ID NO: 826524 for the corresponding DNA) | 826525 | Sense | sa21 | 11009 | Intron51 | 31787981 | 4096 |
| 87 | 8709 (SEQ ID NO: 826526 for the corresponding DNA) | 826527 | Sense | sa21 | 8197 | Intron51 | 31787463 | 4614 |
| 88 | 16467 (SEQ ID NO: 826528 for the corresponding DNA) | 826529 | Antisense | sa21 | 2130 | Intron51 | 31787241 | 4836 |
| 89 | 23958 (SEQ ID NO: 826530 for the corresponding DNA) | 826531 | Antisense | sa21 | 21343 | Intron51 | 31787202 | 4875 |
| 90 | 819 (SEQ ID NO: 826532 for the corresponding DNA) | 826533 | Sense | sa21 | 13610 | Intron51 | 31785449 | 6628 |
| 91 | 9997 (SEQ ID NO: 826534 for the corresponding DNA) | 826535 | Sense | sa21 | 9860 | Intron51 | 31785417 | 6660 |
| 92 | 2121 (SEQ ID NO: 826536 for the corresponding DNA) | 826537 | Sense | sa21 | 10043 | Intron51 | 31785024 | 7053 |
| 93 | 21344 (SEQ ID NO: 826538 for the corresponding DNA) | 826539 | Antisene | sa21 | 7851 | Intron51 | 31784698 | 7379 |

Table 13 - Guide Set 4 (5 gRNAs, 6 pairs)

| gRNA No. | SEQ ID NO. of the nucleotide sequence comprised in the gRNA targeting domain | SEQ ID NO. for guidePAM | Strand | Specificity Score | Target Position | Dist from 5prime Exon51 | filtered for only off by 3 |
|---|---|---|---|---|---|---|---|
| 94 | 4709 (SEQ ID NO: 826540 for the corresponding DNA) | 826541 | 1 | 38.1778946 | Intron50 | 956 | Yes |
| 95 | 20303 (SEQ ID NO: 826542 for the corresponding DNA) | 826543 | -1 | 14.790082 | Intron50 | 815 | Yes |
| 96 | 18720 (SEQ ID NO: 826544 for the corresponding DNA) | 826545 | -1 | 44.9087042 | Intron51 | 1762 | Yes |
| 97 | 22349 (SEQ ID NO: 826546 for the corresponding DNA) | 826547 | -1 | 44.3933051 | Intron51 | 1823 | Yes |
| 98 | 10092 (SEQ ID NO: 826548 for the corresponding DNA) | 826549 | 1 | 34.9840006 | Intron51 | 2083 | Yes |

Table 14 Guide Set 3 Hits

| Guide Num_ R | Guide Num_ L | Deletion Efficiency_ BR1 | Deletion Efficiency_ BR2 | Z score_ 1_BR1 | Z Score_ BR2 | Threshold Passed |
|---|---|---|---|---|---|---|
| 86 | 68 | 15.12 | 23.48 | 1.30 | 2.34 | Z Score 2 > 1.5 |
| 82 | 68 | 17.31 | 19.06 | 1.52 | 1.57 | Avg Z score > 1.5 |
| 85 | 68 | 17.02 | 22.64 | 1.60 | 2.19 | Avg Z score > 1.5 |
| 82 | 71 | 14.65 | 18.17 | 1.60 | 1.42 | Avg Z score > 1.5 |
| 92 | 68 | 16.81 | 21.34 | 2.00 | 1.97 | Avg Z score > 1.5 |
| 91 | 68 | 20.36 | 15.30 | 2.58 | 0.92 | Avg Z score > 1.5 |
| 88 | 68 | 27.63 | 18.98 | 3.54 | 1.56 | Avg Z score > 1.5 |
| 89 | 68 | 17.69 | 14.45 | 2.00 | 0.78 | Z score 1 > 1.5 |
| 83 | 68 | 18.82 | 10.81 | 1.81 | 0.15 | Z score 1 > 1.5 |
| 84 | 68 | 18.39 | 16.61 | 1.79 | 1.15 | Z score 1 > 1.5 |
| 85 | 70 | 14.76 | 11.13 | 1.69 | 0.20 | Z score 1 > 1.5 |

(continued)

| Guide Num_ R | Guide Num_ L | Deletion Efficiency_ BR1 | Deletion Efficiency_ BR2 | Z score_ 1_BR1 | Z Score_ BR2 | Threshold Passed |
|---|---|---|---|---|---|---|
| 87 | 68 | 13.53 | 19.62 | 1.04 | 1.67 | Z Score 2 > 1.5 |

**Table 15**

| Guide Pair | gRNA No. | SEQ ID NO. of the nucleotide sequence comprised in the gRNA targeting domain |
|---|---|---|
| 84+68 | 84 | 1977 |
| | 68 | 2048 |
| 82+68 | 82 | 18458 |
| | 68 | 2048 |
| 1,9 | 1 | 19199 |
| | 9 | 18720 |
| 86+68 | 86 | 481 |
| | 68 | 2048 |
| 94+96 | 94 | 4709 |
| | 96 | 18720 |
| 91+68 | 91 | 9997 |
| | 68 | 2048 |
| 85+68 | 85 | 1499 |
| | 68 | 2048 |
| 92+68 | 92 | 2121 |
| | 68 | 2048 |
| 94+97 | 94 | 4709 |
| | 97 | 22349 |
| 87+68 | 87 | 8709 |
| | 68 | 2048 |
| 94+98 | 94 | 4709 |
| | 98 | 10092 |
| 83+68 | 83 | 21570 |
| | 68 | 2048 |
| 62+38 | 62 | 5869 |
| | 38 | 3536 |
| 82+71 | 82 | 18458 |
| | 71 | 9765 |
| 55+20 | 55 | 18752 |
| | 20 | 17253 |

(continued)

| Guide Pair | gRNA No. | SEQ ID NO. of the nucleotide sequence comprised in the gRNA targeting domain |
|---|---|---|
| 59+38 | 59 | 20145 |
| | 38 | 3536 |
| 54+31 | 54 | 19187 |
| | 31 | 7252 |
| 95+96 | 95 | 20303 |
| | 96 | 18720 |
| 55+38 | 55 | 18752 |
| | 38 | 3536 |
| 88+68 | 88 | 16467 |
| | 68 | 2048 |
| 55+34 | 55 | 18752 |
| | 34 | 14607 |
| 59+30 | 59 | 20145 |
| | 30 | 5614 |
| 95+98 | 95 | 20303 |
| | 98 | 10092 |
| 54+20 | 54 | 19187 |
| | 20 | 17253 |
| 85+70 | 85 | 1499 |
| | 70 | 14296 |
| 59+33 | 59 | 20145 |
| | 33 | 20764 |
| 59+34 | 59 | 20145 |
| | 34 | 14607 |
| 67+21 | 67 | 4134 |
| | 21 | 20924 |
| 55+37 | 55 | 18752 |
| | 37 | 16967 |
| 61+23 | 61 | 6209 |
| | 23 | 14035 |
| 61+18 | 61 | 6209 |
| | 18 | 13527 |
| 61+32 | 61 | 6209 |
| | 32 | 21873 |
| 56+26 | 56 | 9346 |
| | 26 | 7419 |
| 48+37 | 48 | 7649 |
| | 37 | 16967 |

(continued)

| Guide Pair | gRNA No. | SEQ ID NO. of the nucleotide sequence comprised in the gRNA targeting domain |
|---|---|---|
| 54+42 | 54 | 19187 |
| | 42 | 14695 |
| 55+26 | 55 | 18752 |
| | 26 | 7419 |
| 55+19 | 55 | 18752 |
| | 19 | 1744 |
| 56+33 | 56 | 9346 |
| | 33 | 20764 |
| 56+29 | 56 | 9346 |
| | 29 | 2769 |
| 62+30 | 62 | 5869 |
| | 30 | 5614 |
| 51+20 | 51 | 15622 |
| | 20 | 17253 |
| 54+26 | 54 | 19187 |
| | 26 | 7419 |
| 66+29 | 66 | 18062 |
| | 29 | 2769 |
| 53+38 | 53 | 20870 |
| | 38 | 3536 |
| 48+25 | 48 | 7649 |
| | 25 | 1484 |
| 89+68 | 89 | 23958 |
| | 68 | 2048 |
| 60+18 | 60 | 21131 |
| | 18 | 13527 |
| 54+19 | 54 | 19187 |
| | 19 | 1744 |
| 62+17 | 62 | 5869 |
| | 17 | 4072 |
| 59+41 | 59 | 20145 |
| | 41 | 8569 |
| 50+38 | 50 | 9530 |
| | 38 | 3536 |
| 66+38 | 66 | 18062 |
| | 38 | 3536 |
| 61+40 | 61 | 6209 |
| | 40 | 14298 |

(continued)

| Guide Pair | gRNA No. | SEQ ID NO. of the nucleotide sequence comprised in the gRNA targeting domain |
|---|---|---|
| 95+97 | 95 | 20303 |
| | 97 | 22349 |
| 62+41 | 62 | 5869 |
| | 41 | 8569 |
| 61+26 | 61 | 6209 |
| | 26 | 7419 |
| 54+41 | 54 | 19187 |
| | 41 | 8569 |
| 62+18 | 62 | 5869 |
| | 18 | 13527 |
| 59+31 | 59 | 20145 |
| | 31 | 7252 |
| 67+17 | 67 | 4134 |
| | 17 | 4072 |
| 64+31 | 64 | 4165 |
| | 31 | 7252 |
| 61+21 | 61 | 6209 |
| | 21 | 20924 |
| 53+37 | 53 | 20870 |
| | 37 | 16967 |
| 62+25 | 62 | 5869 |
| | 25 | 1484 |
| 64+25 | 64 | 4165 |
| | 25 | 1484 |
| 48+21 | 48 | 7649 |
| | 21 | 20924 |
| 53+31 | 53 | 20870 |
| | 31 | 7252 |
| 66+23 | 66 | 18062 |
| | 23 | 14035 |
| 67+38 | 67 | 4134 |
| | 38 | 3536 |
| 55+41 | 55 | 18752 |
| | 41 | 8569 |
| 67+41 | 67 | 4134 |
| | 41 | 8569 |
| 54+39 | 54 | 19187 |
| | 39 | 9358 |

(continued)

| Guide Pair | gRNA No. | SEQ ID NO. of the nucleotide sequence comprised in the gRNA targeting domain |
|---|---|---|
| 48+17 | 48 | 7649 |
| | 17 | 4072 |
| 62+42 | 62 | 5869 |
| | 42 | 14695 |
| 65+38 | 65 | 23958 |
| | 38 | 3536 |
| 47+16 | 47 | 16235 |
| | 16 | 10140 |
| 60+24 | 60 | 21131 |
| | 24 | 13285 |
| 43+35 | 43 | 15271 |
| | 35 | 13518 |
| 49+25 | 49 | 11675 |
| | 25 | 1484 |
| 49+29 | 49 | 11675 |
| | 29 | 2769 |
| 64+27 | 64 | 4165 |
| | 27 | 9279 |
| 48+22 | 48 | 7649 |
| | 22 | 9496 |
| 65+23 | 65 | 23958 |
| | 23 | 14035 |
| 49+17 | 49 | 11675 |
| | 17 | 4072 |
| 65+22 | 65 | 23958 |
| | 22 | 9496 |

Interestingly, a few individual gRNAs consistently appeared among the top performing pairs, regardless their partner gRNAs. The top hit (gRNA pair 68+84), a human-only gRNA pair with a deletion size of about 2.3 kb, demonstrated a reproducible deletion efficiency of about 32%. The 6 best human-only guide pairs and the 6 best NHP cross-reactive guide pairs are listed in Table 16.

**Table 16 - Hu/NHP DMD Exon 51 Deleting gRNA pairs**

| Guide Pair | gRNA No. | SEQ ID NO. of the nucleotide sequence comprised in the gRNA targeting domain | Length | Distance from 5' Exon 51 (bp) | Avg Del Effy (%) | Plate Normalized Avg Del Eff (a.u.) | Norm Stdev Del Eff | Deletion Size (bp) |
|---|---|---|---|---|---|---|---|---|
| 84+68 | 84 | 1977 | 21 | 1318 | 31.8 | 2.39 | 0.55 | 2527 |
| | 68 | 2048 | 21 | 1209 | | | | |

(continued)

| Guide Pair | gRNA No. | SEQ ID NO. of the nucleotide sequence comprised in the gRNA targeting domain | Length | Distance from 5' Exon 51 (bp) | Avg Del Effy (%) | Plate Normalized Avg Del Eff (a.u.) | Norm Stdev Del Eff | Deletion Size (bp) |
|---|---|---|---|---|---|---|---|---|
| 82+68 | 82 | 18458 | 21 | 348 | 28.92 | 2.09 | 0.5 | 1557 |
| | 68 | 2048 | 21 | 1209 | | | | |
| 1+9 | 1 | 19199 | 22 | 5104 | 27.87 | 2.04 | 0.31 | 5415 |
| | 9 | 18720 | 22 | 311 | | | | |
| 94+9 | 94 | 4709 | 22 | 495 | 26.66 | 2.01 | 0.56 | 806 |
| | 9 | 18720 | 22 | 311 | | | | |
| 86+68 | 86 | 481 | 21 | 4096 | 27.8 | 2 | 0.38 | 5305 |
| | 68 | 2048 | 21 | 1209 | | | | |
| 94+97 | 94 | 4709 | 22 | 495 | 25.4 | 1.85 | 0.52 | 867 |
| | 97 | 22349 | 22 | 372 | | | | |
| 62+38 | 62 | 5869 | 21 | 11283 | 22.23 | 1.64 | 0.28 | 20768 |
| | 38 | 3536 | 21 | 9485 | | | | |
| 55+20 | 55 | 18752 | 21 | 26857 | 21.02 | 1.56 | 0.33 | 44269 |
| | 20 | 17253 | 21 | 17421 | | | | |
| 59+38 | 59 | 20145 | 21 | 17913 | 20.15 | 1.51 | 0.37 | 27398 |
| | 38 | 3536 | 21 | 9485 | | | | |
| 54+31 | 54 | 19187 | 21 | 26522 | 19.83 | 1.43 | 0.48 | 71832 |
| | 31 | 7252 | 21 | 45310 | | | | |
| 55+38 | 55 | 18752 | 21 | 26857 | 18.44 | 1.32 | 0.32 | 36342 |
| | 38 | 3536 | 21 | 9485 | | | | |
| 54+26 | 54 | 19187 | 21 | 26522 | 13.37 | 0.95 | 0.11 | 60894 |
| | 26 | 7419 | 21 | 34372 | | | | |

*3. Methods and Materials*

ddPCR assay

ddPCR is a sensitive assay for measuring the absolute concentration of a specific gene within a larger, mixed DNA pool. The assay relied first on the careful partitioning of single DNA templates of interest into separate droplets. Once correctly partitioned, PCR-mediated amplification of the DNA sequence of interest releases a fluorophore into solution, thereby freeing the fluorophore from a quenching molecular interaction. The "freed' fluorophore was then able to fluoresce normally. The resultant fluorescent intensity, coupled with the careful partitioning of single DNA copies into separate droplets, allowed for the determination of the concentration of the original DNA template.

Multi-plexing the ddPCR reaction allowed for the simultaneous determination of the concentrations of two different regions of the DMD gene, Exon 51 (the target) and DMD Exon 59 (the reference). Because the genome editing strategy employed was designed to result in the deletion of Exon 51 while leaving Exon 59 unaffected, a comparison of the concentration of Exon 51 relative to the concentration of Exon 59 by ddPCR enabled the calculation of percentage of DMD loci in which Exon 51 was successfully excised, also know as the targeted deletion efficiency. The multiplexed FAM/VIC probe strategy for the ddPCR assay are illustrated in Figs. 14 and 15.

For a full list of components used in the ddPCR assay, *See* Table 17.

**Table 17**

| | | |
|---|---|---|
| Qx200 Droplet Generator | Bio-Rad | 1864002 |
| Qx200 Droplet Reader | Bio-Rad | 1864003 |
| ALPS 25 Manual Heat Sealer | Thermo Fisher | AB-0384/110 |
| Agencort DNAdvance | Beckman Coulter | A48705 |
| Droplet Generation Oil for Probes | Bio-Rad | 1863005 |
| DG8 Cartridges | Bio-Rad | 1864008 |
| DG8 Gasket | Bio-Rad | 1864007 |
| DG8 Cartridge Holder | Bio-Rad | 1863051 |
| Pierceable Foil Heat Seal | Bio-Rad | 1814040 |
| 2x ddPCR Supermix for Probves | Bio-Rad | 1863010 |
| 2x ddPCR Buffer Control for Probes | Bio-Rad | 1863502 |
| 96-Well twin.tec™ PCR Plates (Blue Skirted) | Fisher | 951020362 |

At Exon 59, a "pre-designed Gene Expression assay", i.e., Hs02563140_s1 with a VIC-MGB dye (Life Technologies), was used. At Exon 51, a Custom Taqman assay, *i.e.*, DMDEx51_CCCSVXJ with a FAM-MGB dye (sequences are provided below), was made.

DMDEx51_CCCSVXJ_F: CCTGCTCTGGCAGATTTCAAC [SEQ ID NO: 826550]

DMDEx51_CCCSVXJ_R: ACCCACCATCACCCTCTGT [SEQ ID NO: 826551]

DMDEx51_CCCSVXJ_M FAM: AAAGCCAGTCGGTAAGTTCTGTC [SEQ ID NO: 826552]

First, the multiplexed Taqman ddPCR reaction master-mix was set up (protocol modified from BioRad, *see* Table 18). The primer/probe sets were delivered at a 20x concentration, immediately diluted to be at 10x concentration upon arrival, and stored in aliquots. Samples were set up in a 96-well plate with 1.2x excess volume (for 24 μl per well) to ensure consistent volume transfer to the droplet generation cartridge. The reactions were set up on an ice pack plate holder and covered from light to minimize fluorophore degradation and to maintain droplet integrity. *See* Table 18 below.

**Table 18**

| COMPONENT | VOLUME PER RXN ul (1.2x) | FINAL CONCENTRATION |
|---|---|---|
| 2x ddPCR Supermix for Probes | 10 (12) | 1x |
| 10x target primers/probe (DMD EX51 VXJ FAM) | 2 (2.4) | 900 mM / 250 nM |
| 10x reference primers/probe (DMD EX59 VIC) | 2 (2.4) | 900 mM / 250 nM |
| Sample DNA | 30 ng (36 ng) by pico/nanodrop | |
| RNase-/DNase-free water | Variable | - |
| Total volume | 20 (24) | - |

6 wells of every 96-well ddPCR plate were used for a "standard curve". This "standard curve" was generated by mixing patient gDNA samples of known Exon51 deletion status at varying proportions. *See* Figs. 16A-16D. DMD patient samples were acquired from Coriell Institute for Medical Research. Mixtures with expected Exon 51 deletion rates of 0%, 5%, 10%, 15%, 20%, and 30% were generated by mixing DNA from a male patient carrying a homozygous deletion of Exon 51 with that from a female homozygous for the wild-type allele. During data analysis, a linear regression on the "standard curve" was used to correct "observed" deletion rates to "true" deletion rates, correcting for any efficiency biasing of the PCR reaction towards Exon 51 target probe or the Exon 59 reference probe. *See* Fig. 16C and 16D.

With the master mix transferred to a 96-well format, including the addition of relevant sample and control DNA, droplet generation began. DG8 cartridge was inserted into DG8 Cartridge Holders. 70 μl Droplet Generation Oil for Probes was added to the designated oil wells, followed by the addition of 20 μl of each sample to the adjacent sample wells. To empty sample wells was added 20 μl of ddPCR Buffer Control for Probes due to the fact that $H_2O$ or other low salt concentration buffers, *e.g.*, 0.1x EB buffer, could lead to failure in droplets generator. Samples wells were also

checked to ensure no bubbles were generated during sample transfer, as bubbles interfere with droplet generation. Any bubbles formed were removed by hand with a P10 pipet tip.

Fully-loaded 8-well DG8 cartridge were affixed with a gasket, then loaded into the QX200 droplet generator. Droplet generation took approximately 2 minutes per DG8 Cartridge, or about 25 minutes for a full 96-well plate. The Qx200 droplet generator generated a sample size of 40 μl, with 16,000 to 20,000 droplets per reaction. In a critical step,, the droplet mixture was transferred from the DG8 cartridge to a blue half-skirt 96-well PCR plate (Eppendorf), situated in the dark and on an ice pack plate holder, using slow and constant force. Careful aspiration and expulsion using a multi-channel P200 pipet ensured droplets remain intact. The above steps were repeated until all samples were partitioned into droplets and transferred to the blue half-skirt 96-well PCR plate. The plate was sealed at 180°C for 3 second using a pre-heated PCR plate-sealer and pierceable foil seals. Once sealed, the plate was kept in the dark and on ice until it was loaded into the PCR machine. The PCR conditions are summarized below.

- 95°C for 10 minutes; 95°C for 30 seconds, 60°C for 1 minute; 72°C for 15 seconds (45X); and 98°C for 10 minutes
- The cycling conditions was run at 50% ramp speed (2°C/s or slower)

After PCR cycling was complete, the droplets were read by a Qx200 Droplet reader (Bio-Rad). Raw data were first visually inspected to confirm the ddPCR assay cleanly separated "positive" droplets from "negative" droplets with positive droplets accounting for about 5-15% of the total droplets. Values above and below this range indicate under- and over-loading, respectively, of DNA into generated droplets, the consequence of which is inaccurate calculation of deletion efficiencies. Data points with positive droplet counts outside of this range, therefore, were discarded. Optimization was performed prior to the DMD screen, and a load of 30 ng of DNA per reaction was determined to result in the optimal positive droplet distribution.

Data was analyzed by first calculating deletion efficiencies for individual guide pairs:

$$\text{Deletion Efficiency (\%)} = 100 * (1-([\text{Exon51}]/[\text{Exon 59}]))$$

*[Exon51]* = experimentally determined concentration of Exon 51
*[Exon59]* = experimentally determined concentration of Exon 59

These "observed" deletion efficiencies were converted to "true" deletion efficiencies using the standard curve and linear regression as described above. "Observed" deletion efficiencies for positive (guides pair 1,9) and negative controls (backbone, pJT002, transfected alone) were also converted to "true" deletion efficiencies and subsequently cross-referenced with previous experiments to verify the quality of the assay for that particular plate. Generally, guide pairs were sorted by "true" deletion efficiency to determine top performers.

Given the size of Guide Set 2 (675 guide pairs), "true" deletion efficiencies were converted into z-scores *(See* below) in an attempt to normalize for variability across plates, transfections, and ddPCR runs. Guide pairs with at least a z-score greater than 1.5 in at least one of the two experimental biological replicates were selected for a Guide Hit Validation experiment, with the intent of carrying through top performers while recapitulating, and thereby confirming, previously observed positive results.

Z-score

Z score normalizes for variability across plates, transfections, ddPCR runs.

$$Z = \frac{X_i - \bar{X}}{S_x}$$

$X_i$ = deletion efficiency
$X$ = plate average
$S_x$ = *plate SD*

**Incorporation by Reference**

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

**Equivalents**

Those skilled in the art will recognize, or be able to ascertain using no more than routine experimentation, many equivalents to the specific embodiments of the invention described herein. Such equivalents are intended to be encompassed by the following claims.

## Claims

1. A composition comprising at least one of:

    (a)a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
    (b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
    (c) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
    (d) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
    (e) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
    (f) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
    (g) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
    (h) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
    (i) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
    (j) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and
    (k) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

2. The composition of claim 1, further comprising at least one Cas9 molecule, and optionally the at least one Cas9 molecule is an *S. aureus* Cas9 molecule or a mutant *S. aureus* Cas9 molecule.

3. The composition of claim 2, wherein the at least one Cas9 molecule recognizes a PAM sequence set forth in NNGRRT (SEQ ID NO: 204) or NNGRRV (SEQ ID NO:205).

4. A genome-editing system comprising:

(a) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(c) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;
(e) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;
(f) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;
(g) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;
(h) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;
(i) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;
(j) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; or
(k) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

**5.** The genome-editing system of claim 4, comprising at least one Cas9 molecule, and optionally the at least one Cas9 molecule is an *S. Aureus* Cas9 molecule.

**6.** The genome-editing system of claim 4 or 5, wherein the first gRNA molecule, the at least one Cas9 molecule and the second gRNA molecule are present in the system in a nanoparticle, a cationic liposome, and/or a biological non-viral delivery vehicle selected from the group consisting of: attenuated bacteria; engineered bacteriophages; mammalian virus-like particles; and biological liposome, and optionally the nanoparticle, cationic liposome, or biological non-viral delivery vehicle comprises a targeting modification capable of increasing target cell uptake of the system.

**7.** A vector comprising a polynucleotide encoding a first gRNA molecule and a second gRNA molecule, wherein the first gRNA molecule and the second gRNA molecule are selected from the group consisting of:

(a) a first gRNA molecule comprising a targeting domain that comprises a nucleotide sequence set forth in SEQ ID NO: 18458, and a second gRNA molecule comprising a targeting domain that comprises a nucleotide sequence set forth in SEQ ID NO: 2048;
(b) a first gRNA molecule comprising a targeting domain that comprises a nucleotide sequence set forth in SEQ ID NO: 19199, and a second gRNA molecule comprising a targeting domain that comprises a nucleotide sequence set forth in SEQ ID NO: 18720;
(c) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 18720;
(d) a first gRNA molecule comprising a targeting domain that comprises a nucleotide sequence set forth in SEQ ID NO: 481, and a second gRNA molecule comprising a targeting domain that comprises a nucleotide sequence set forth in SEQ ID NO: 2048;

(e) a first gRNA molecule comprising a targeting domain that comprises a nucleotide sequence set forth in SEQ ID NO: 4709, and a second gRNA molecule comprising a targeting domain that comprises a nucleotide sequence set forth in SEQ ID NO: 22349;

(f) a first gRNA molecule comprising a targeting domain that comprises a nucleotide sequence set forth in SEQ ID NO: 5869, and a second gRNA molecule comprising a targeting domain that comprises a nucleotide sequence set forth in SEQ ID NO: 3536;

(g) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 17253;

(h) a first gRNA molecule comprising a targeting domain that comprises a nucleotide sequence set forth in SEQ ID NO: 20145, and a second gRNA molecule comprising a targeting domain that comprises a nucleotide sequence set forth in SEQ ID NO: 3536;

(i) a first gRNA molecule comprising a targeting domain that comprises a nucleotide sequence set forth in SEQ ID NO: 19187, and a second gRNA molecule comprising a targeting domain that comprises a nucleotide sequence set forth in SEQ ID NO: 7252;

(j) a first gRNA molecule comprising a targeting domain that comprises a nucleotide sequence set forth in SEQ ID NO: 18752, and a second gRNA molecule comprising a targeting domain that comprises a nucleotide sequence set forth in SEQ ID NO: 3536; and

(k) a first gRNA molecule comprising a targeting domain that comprises a nucleotide sequence set forth in SEQ ID NO: 19187; and a second gRNA molecule comprising a targeting domain that comprises a nucleotide sequence set forth in SEQ ID NO: 7419.

**8.** The vector of claim 7, wherein the vector is a viral vector, and optionally the vector is an adeno-associated virus (AAV) vector.

**9.** The composition of any one of claims 1-3, the genome-editing system of any one of claims 4-6, or the vector of claim 7 or 8 for use in modifying a *DMD* gene in a cell.

**10.** The composition, genome-editing system or vector for use of claim 9, wherein the modification comprises deletion of exon 51 of the *DMD* gene, and/or restoration of correct reading frame of the *DMD* gene.

**11.** The composition, genome-editing system or vector for use of claim 9 or 10, wherein the cell is from a subject suffering from Duchenne muscular dystrophy.

**12.** The composition of any one of claims 1-3, the genome-editing system of any one of claims 4-6, or the vector of claim 7 or 8 for use in treating Duchenne muscular dystrophy in a subject.

**13.** The composition, genome-editing system or vector for use of claim 12, wherein the subject is suffering from Duchenne muscular dystrophy, Becker muscular dystrophy (BMD), or Dilated Cardiomyopathy (DCM) Type 3B.

**14.** A cell comprising the composition of any one of claims 1-3, the genome-editing system of any one of claims 4-6, or the vector of claim 7 or 8.

**Fig. 1A**

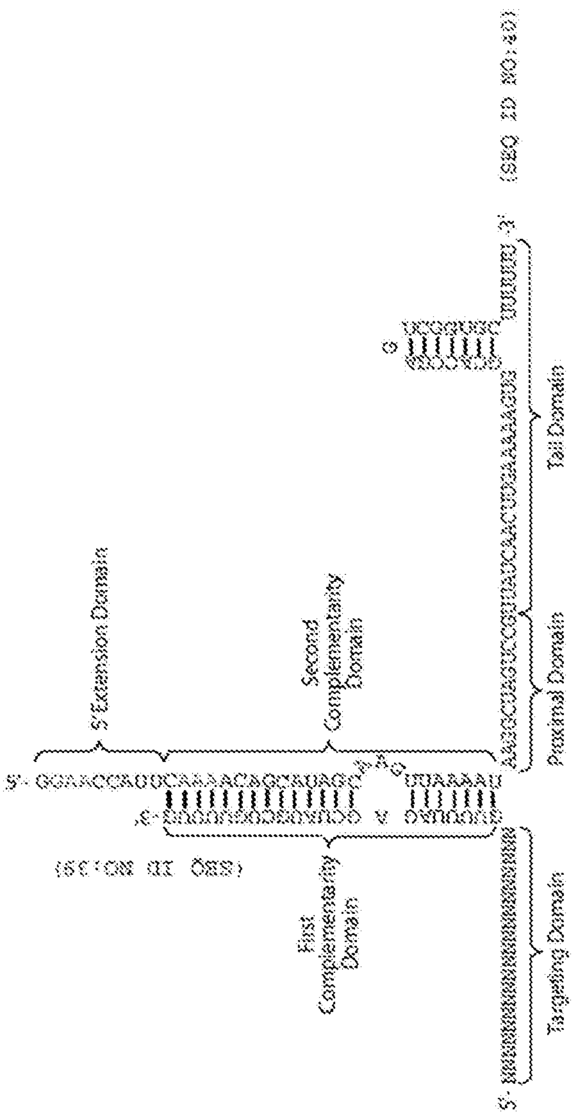
5' Extension Domain
Second Complementarity Domain
First Complementarity Domain
(SEQ ID NO:39)
Targeting Domain
Proximal Domain
Tail Domain
(SEQ ID NO:40)

Fig. 1B

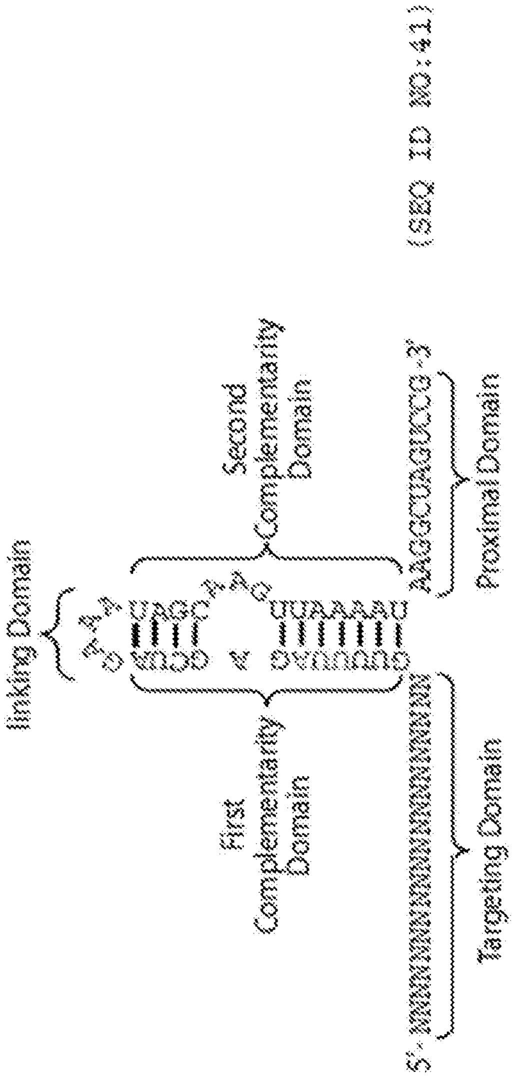
linking Domain
First Complementarity Domain
Second Complementarity Domain
AAGGCUAGUCCG-3'
Targeting Domain
Proximal Domain
(SEQ ID NO:41)

**Fig. 1C**

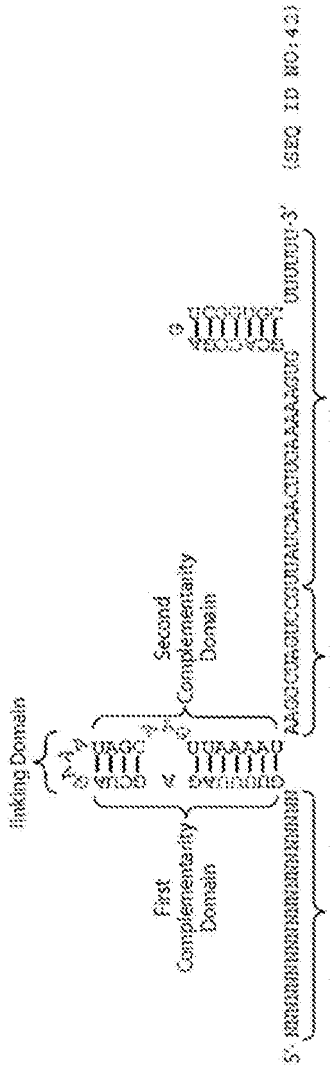
linking Domain
First Complementarity Domain
Second Complementarity Domain
Targeting Domain
Proximal Domain
Tail Domain
(SEQ ID NO: 43)

**Fig. 1D**

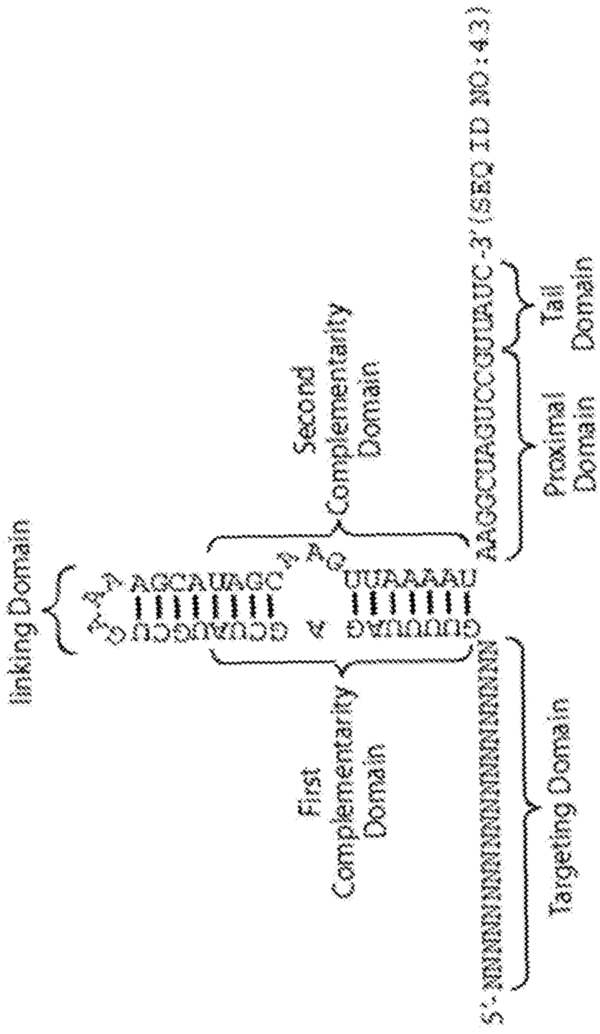
linking Domain
First Complementarity Domain
Second Complementarity Domain
AAGGCUAGUCCGUUAUC -3' (SEQ ID NO: 43)
Proximal Domain
Tail Domain
Targeting Domain

**Fig. 1E**

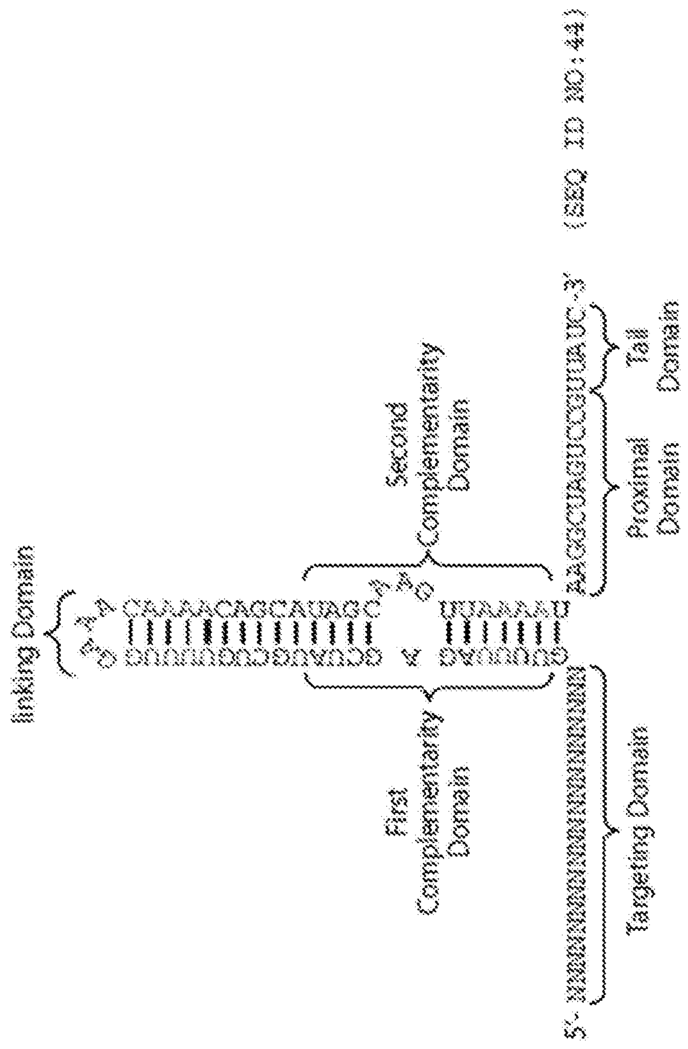
linking Domain
Second Complementarity Domain
First Complementarity Domain
Proximal Domain
Tail Domain
Targeting Domain
(SEQ ID NO:44)

Fig. 1F

***Streptococcus thermophilus***

**a) Structure** (See, e.g., Karvelis et al, RNA Biology, 2013; 10(5); 841-851)

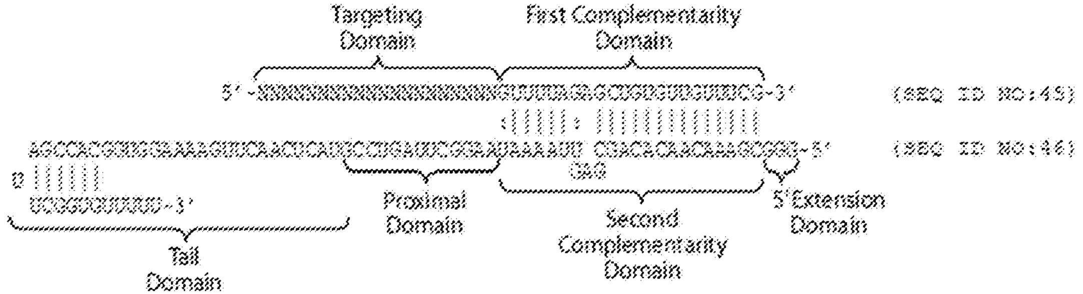

Fig. 1G

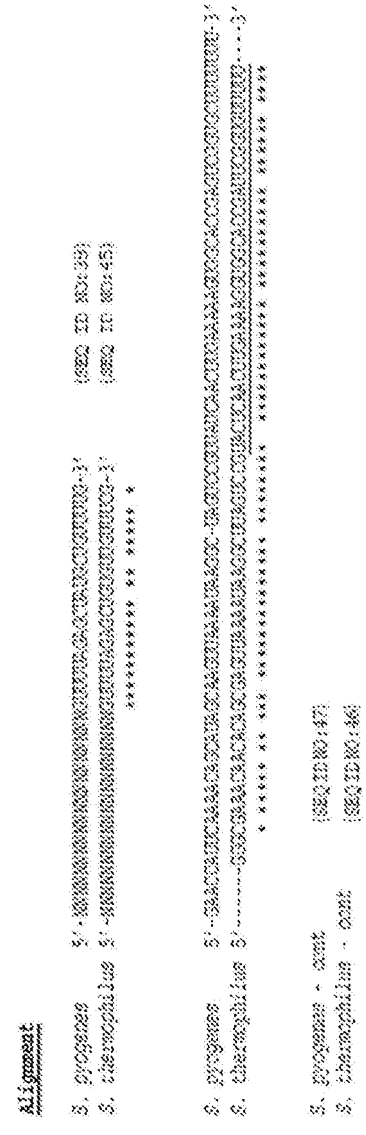

Fig. 1H

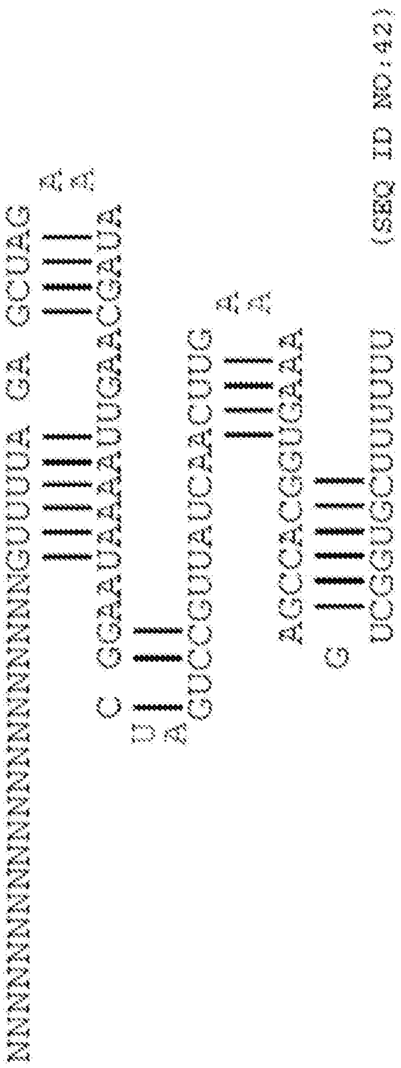

Fig. 1I

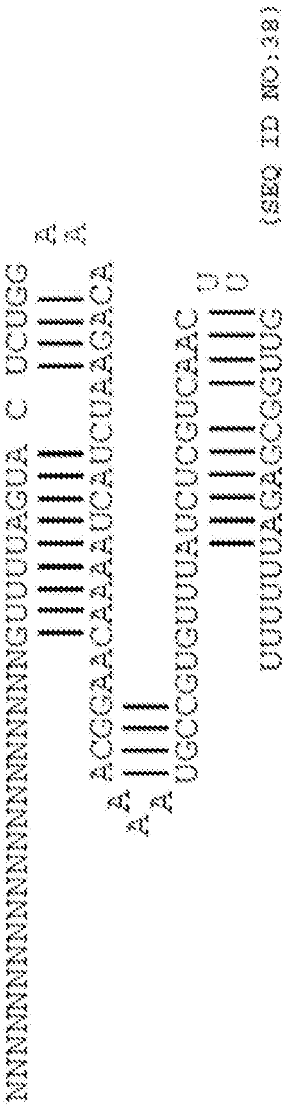

**Fig. 2A**

```
CLUSTAL format alignment by MAFFT (v7.058b)                [Y]

                   *
SM        MKKPYSIGLDIGTNSVGWAVVTDDYKVPAKKMKVLGNTDKSHIEKNLLGALLFDSGNTAED
SP        MDKKYSIGLDIGTNSVGWAVITDEYKVPSKKFKVLGNTDRHSIKKNLIGALLFDSGETAEA
ST        MTKPYSIGLDIGTNSVGWAVITDNYKVPSKKMKVLGNTSKKYIKKNLLGVLLFDSGITAEG
LI        MKKPYTIGLDIGTNSVGWAVLTDQYDLVKRKMKIAGDSEKKQIKKNFWGVRLFDEGQTAAD
            * *;***************** **;*.:   :*;*; *::.:   *;**; *, ***.* **
Motif:    M-K-Y*IGLDIGTNSVGWAV-TD*Y-*--*K*K*-G**-*--I*KN*-G--LFD-G-TA--

SM          RRLKRTARRRYTRRRNRILYLQEIFSEEMGKVDDSFFHRLEDSFLVTEDKRGERHPIFGN
SP          TRLKRTARRRYTRRKNRICYLQEIFSNEMAKVDDSFFHRLEESFLVEEDKKHERHPIFGN
ST          RRLKRTARRRYTRRRNRILYLQEIFSTEMATLDDAFFQRLDDSFLVPDDKRDSKYPIFGN
LI          RRMARTARRRIERRRNRISYLQGIFAEEMSKTDANFFCRLSDSFYVDNEKRNSRHPFFAT
             *; ******  **;*** *** **; **., *  ** **.;** * ;;*; .;;*;*..
Motif:      -R*-RTARRR--RR*NRI-YLQ-IF*-EM---D--FF-RL-*SF-V-**K*--**P*F--

SM          LEEEVKYHENFPTIYHLRQYLADNPEKVDLRLVYLALAHIIKFRGHFLIEGKFDTRNNDV
SP          IVDEVAYHEKYPTIYHLRKKLVDSTDKADLRLIYLALAHMIKFRGHFLIEGDLNPDNSDV
ST          LVEEKAYHDEFPTIYHLRKYLADSTKKADLRLVYLALAHMIKYRGHFLIEGEFNSKNNDI
LI          IEEEVEYHKNYPTIYHLREELVNSSEKADLRLVYLALAHIIKYRGNFLIEGALDTQNTSV
            : ;*  **,;;*******; *.:..,*,****;******;**;**;*****  ;:. *..;
Motif:      *-*E--YH-**PTIYHLR*-L-*---K-DLRL*YLALAH*IK*RGNFLIEG-**--N--*
```

Fig. 2B

```
SM        QRLFQEFLAVYDNTFERSS------LQEQRVQVEEILTDKISKSAKKDRVLKLFPNEKSN
SP        DKLFIQLVQTYNQLFEENP------INASGVDAKAILSARLSKSRRLENLIAQLPGEKKN
ST        QKIFQDFLDTYNAIFESDL------SLENSKQLEEIVKDKISKLEKKDRILKLFPGEKNS
LI        KGIYKQFIQTYNQVFASGIEDGSLKKLEDNKDVAKILVEKVTRKEKLERILKLYPGEKSA
          :  : ::: .*:  * ..              .. :   *:  :::: : :.::  *.**.
Motif:    *--*-***--Y*--f-----------------*---I*--****--*-*-**---P-EK--

SM        GRFAEFLKLIVGNQADFKKHFELEEKAPLQFSKDTYEEELEVLLAQIGDNYAELFLSAKK
SP        GLFGHLIALSLGLTPNFKSNFDLAEDAKLQLSKDTYDDDLDNLLAQIGDQYADLFLAAKN
ST        GIFSEFLKLIVGNQADFRKCFNLDEKASLHFSKESYDEDLETLLGYIGDDYSDVFLKAKK
LI        GMFAQFISLIVGSKGNFQKPFDLIEKSDIECAKDSYEEDLESLLALIGDEYAELFVAAKN
          * *.::: * :*   :*:. *:* *.: :. :*::*:::*: **. ***:*:::*: **:
Motif:    G-F-***-L-*G---*F*--F*L-E-*-*---*E**Y***L*-LL--IGD*Y***F*-AK*

SM        LYDSILLSGILTVTDVGTKAPLSASMIQRYNEHQMDLAQLKQFIRQKLSDKYNEVFSDVS
SP        LSDAILLSDILRVNTEITKAPLSASMIKRYDEHHQDLTLLKALVRQQLPEKYKEIFFDQS
ST        LYDAILLSGFLTVTDNETEAPLSSAMIKRYNEHKEDLALLKEYIRNISLKTYNEVFKDDT
LI        AYSAVVLSGIITVAETETNAKLSASMIERFDTHEEDLGELKAFIKLHLPKHYEEIFSNTE
             .:::**.:: *    *:* **::**:*:: *. **  **  ::     . *:*:* :
Motif:    ---***LS-**-V----T*A-LS**MI*R**-H--DL--LK--**-------Y*E*F-*--

SM        KDGYAGYIDGKTNQEAFYKYLKGLLNKIEGSGYFLDKIEREDFLRKQRTFDNGSIPHQIH
SP        KNGYAGYIDGGASQEEFYKFIKPILEKMDGTEELLVKLNREDLLRKQRTFDNGSIPHQIH
ST        KNGYAGYIDGKTNQEDFYVYLKKLLAEFEGADYFLEKIDREDFLRKQRTFDNGSIPYQIH
LI        KHGYAGYIDGKTKQADFYKYMKMTLENIEGADYFIAKIKREEFLRKQRTFDNGAIPHQLH
          *.******** :.*  ** ::*  * :::*:  :: *:::*::**********:**:*:*
Motif:    K-GYAGYIDG-*-Q--FY-**K--L-***G*---**-K***E**LRKQRTFDNG*IP*Q*H
```

**Fig. 2C**

```
SM       LQEMRAIIRRQAEFYPFLADNQDRIEKLLTFRIPYYVGPLARGKSDFAWLSRKSADKITP
SP       LGELHAILRRQEDFYPFLKDNREKIEKILTFRIPYYVGPLARGNSRFAWMTRKSEETITP
ST       LQEMRAILDKQAKFYPFLAKNKERIEKILTFRIPYYVGPLARGNSDFAWSIRKRNEKITP
LI       LEELEAILHQQAKYYPFLKENYDKIKSLVTFRIPYFVGPLANGQSEFAWLTRKADGEIRP
         * *:.**: :* .:**** .* ::*:.::******:*****.*:* ***  **    * *
Motif:   L-E*-AI*-*Q--*YPFL--N-**I*-**TFRIPY*VGPLA-G*S-FAW--RK----I-P

SM       WNFDEIVDKESSAEAFINRMTNYDLYLPNQKVLPKHSLLYEKFTVYNELTKVKYKTE-QG
SP       WNFEEVVDKGASAQSFIERMTNFDKNLPNEKVLPKHSLLYEYFTVYNELTKVKYVTEGMR
ST       WNFEDVIDKESSAEAFINRMTSFDLYLPEEKVLPKHSLLYETFNVYNELTKVRFIAESMR
LI       WNIEEKVDFGESAVDFIEKMTNEDTYLPKENVLPKHSLCYQKYLVYNELTKVRYIND-QG
         **::: :*    **  **::**. *  **:::******* *: : ********::   :
Motif:   WN***-*D---SA--FI**MT--D--LP***VLPKHSL-Y*-*-VYNELTKV**--*---

SM       KTAFFDANMKQEIFDGVFKVYRKVTKDKLMDFLEKEFDEFRIVDLTGLDKENKVFNASYG
SP       KPAFLSGEQKKAIVDLLFKTNRKVTVKQLKEDYFKKIECFDSVEISGVEDR---FNASLG
ST       DYQFLDSKQKKDIVRLYFKDKRKVTDKDIIEYL-HAIYGYDGIELKGIEKQ---FNSSLS
LI       ETSYFSGQEKEQIFNDLFKQKRKVKKKDLELFL-KNMSHVESPTIEGLEDS---FNSSYS
         .   ::..: *: *.    **  ***. ..:       : :        : *::.   **:* .
Motif:   ---**---*-K*-I----FK--RKV----*------*-*--------*-G**------FN*S--

SM       TYHDLCKIL-DKDFLDNSENEKILEDIVLTLTLFEDREMIRKRLENYSDLLTKEQVKKLE
SP       TYHDLLKIIKDKDFLDNEENEDILEDIVLTLTLFEDREMIEERLKTYAHLFDDKVMKQLK
ST       TYHDLLNIINDKEFLDDSSNEAIIEEIIHTLTIFEDREMIKQRLSKFENIFDKSVLKKLS
LI       TYHDLLKVGIKQEILDNPVNTEMLENIVKILTVFEDKRMIKEQLQQFSDVLDGVVLKELE
         *****  ::    .::**:  *   :*:*:  **:***:.**.::*. : .::     :*:*.
Motif:   TYHDL-**----***LD*--N--**E*I*--LT*FED*-MI-**L--*--**-----*E*L-
```

Fig. 2D

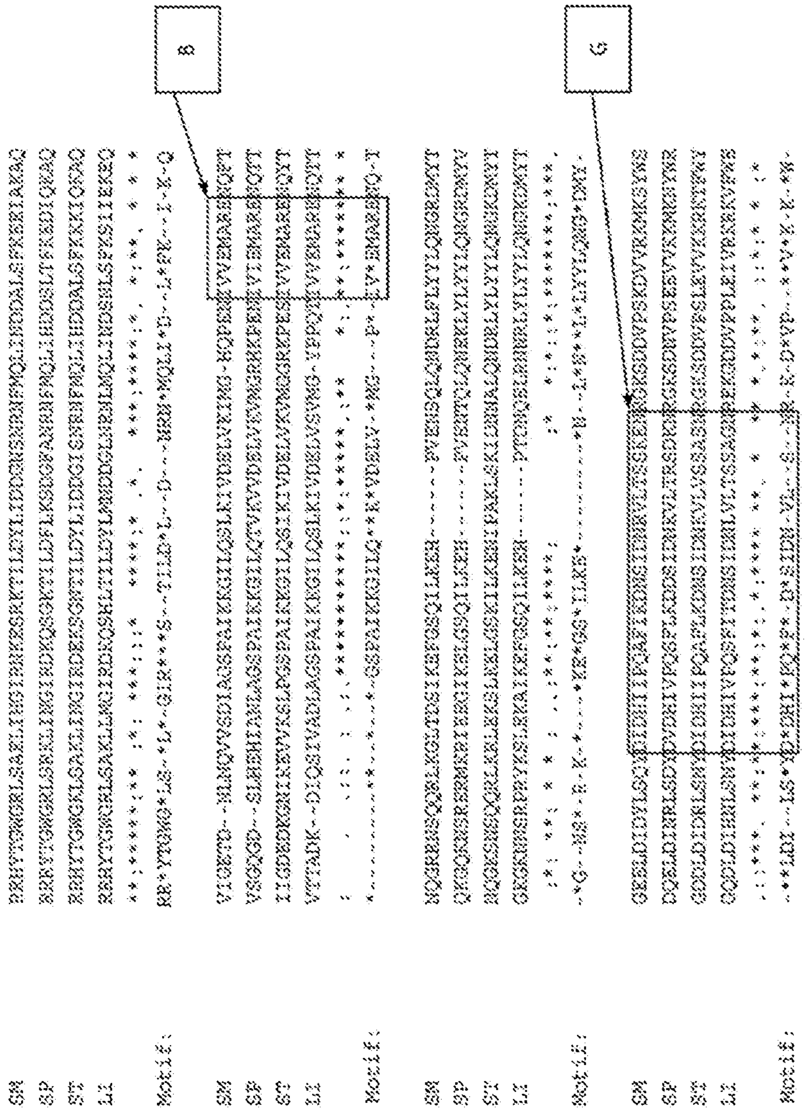

Fig. 2E

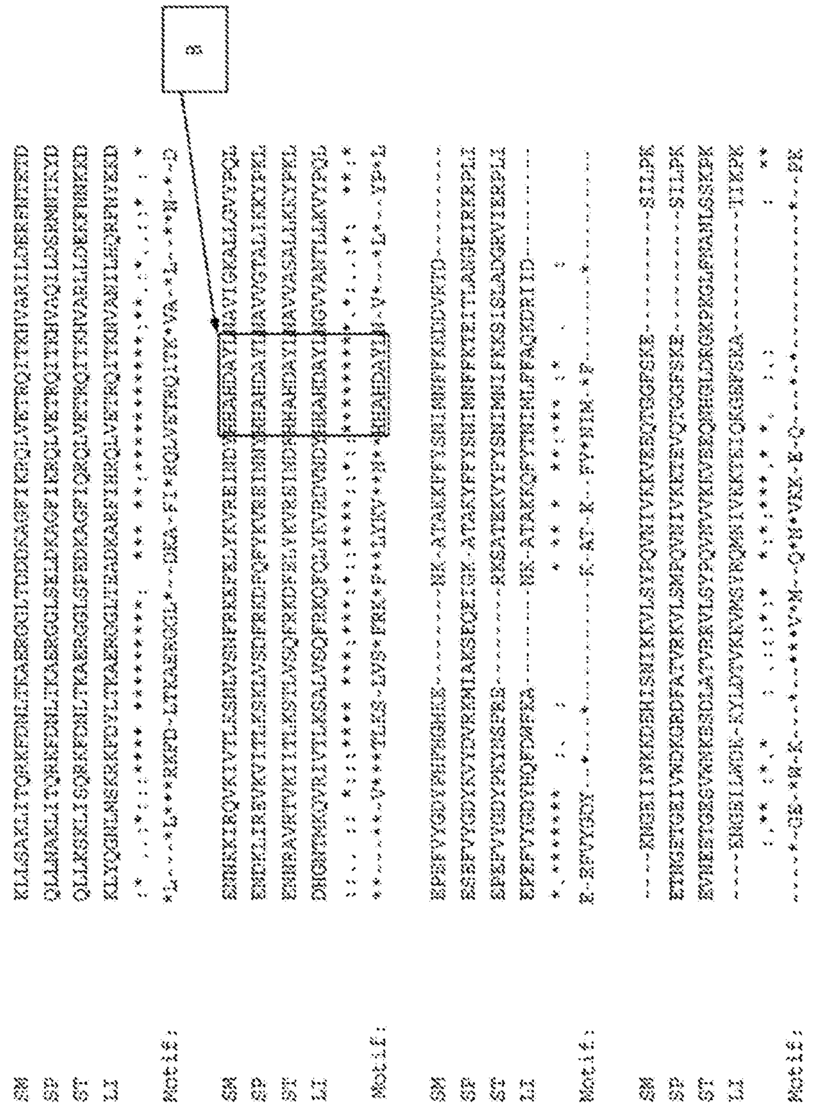

Fig. 2F

```
SM        GNSDK-LIPRKTKKFYWDTKKYGGFDSPIVAYSILVIADIEKGKSKKLKTVKALVGVTIM
SP        KNSDK-LIARKKD---WDPKKYGGFDSPTVAYSVLVVAKVEKGKSKKLKSVKELLGITIM
ST        PNSNKNLVGAKEY---LDPKKYGGYAGISNSFTVLVKGTIEKGAKKKITNVLEFQGISIL
LI        GNSSK-LIPRKTN---WDPMKYGGLDSPNMAYAVVI--EYAKGKN-ELVFEKKIIRVTIM
           **.: *:  *        *. ****  .    ::::::      ** . *:       :  ::*:
Motif:    ~NS~*~L*~~K~~~~~~D~~KYGG~~~~~~******~~~~~KG~~~K*~~~~~~*~~**I*

SM        KKMTFERDPVAFLERKGYRNVQEENIIKLPKYSLFKLENGRKRLLAS~~~~~~~ARELQK
SP        ERSSFEKNPIDFLEAKGYKEVEKDLIIKLPKYSLFELENGRKRMLAS~~~~~~~AGELQK
ST        DRINYRKDKLNFLLEKGYKDI--ELIIELPKYSLFELSDGSRRMLASILSTNNERGEIHK
LI        ERKAFEKDEKAFLEEQGYRQP--KVLAKLPKYTLYECEEGRRRMLAS~~~~~~~ANEAQK
          :: :.:: ** :**:: . : :****:*:: .:* :*:***         * :*
Motif:    **~~*~**~~~FL~~*GY**~~~~~~*~*LPKY*L**~~*G~*R*LAS~~~~~~~~~E~*K

SM        GNEIVLPNHLGTLLYHAKNIHKV~~~~~~DEPKHLDYVDKHKDEFKELLDVVSNFSKKYT
SP        GNELALPSKYVNFLYLASHYEKLKGSPEDNEQKQL~FVEQHKHYLDEIIEQISEFSKRVI
ST        GNQIFLSQKFVKLLYHAKRISNT~~~~~~INENHRKYVENHKKEFEELFYYILEFNENYV
LI        GNQQVLPNHLVTLLHHAANCEVS~~~~~~~DGKSLDYIESHREMFAELLAHVSEFAKRYT
          **:  *..:  .:*: * .               : :    ::.::. : *::  : :* :.
Motif:    GN*~~L~~*~~~*L*~A~~~~~~~~~~~~~~*~*~~~***~**~~*~E**~~*~*F~*~~~

SM        LAEGNLEKIKELYAQNNGEDLKELASSFI~~~~~~~~NLLTFTAIGAPATFKFFDKNIDR
SP        LADANLDKVLSAYNKHRDKPIREQAENII~~~~~~~~HLFTLTNLGAPAAFKYFDTTIDR
ST        GAKKNGKLLNSAFQSWQNHSIDELCSSFIGPTGSERKGLFELTSRGSAADFEFLGVKIPR
LI        LAEANLNKINQLFEQNKEGDIKAIAQSFV~~~~~~~~DLMAFNAMGAPASFKFFETTIER
           *. *  . : . : . .    :    ...::         *: :.  *:.* *:::  .* *
Motif:    ~A~~N~~~*~~~*~~~~~~~~*~~~~~~~**~~~~~~~~~L*~*~~~G*~A~F***~~~I~R
```

Fig. 2G

```
SM       KR-YTSTTEILNATLIHQSITGLYETRIDLNKLGGD    (SEQ ID NO:1)
SP       KR-YTSTKEVLDATLIHQSITGLYETRIDLSQLGGD    (SEQ ID NO:2)
ST       YRDYTPSSLLKDATLIHQSVTGLYETRIDLAKLGEG    (SEQ ID NO:4)
LI       KR-YNNLKELLMSTIIYQSITGLYESRKRLD----D    (SEQ ID NO:5)
          * *.  . : ::*:*:**:*****:*  *     .
Motif:   -R-Y-----*-**T*I*QS*TGLYE*R--L------    (SEQ ID NO:14)
```

Fig. 3A

Alignment of the N terminal RuvC-like Domains disclosed in Chylinski et al.
(excluding sequence outliers).
(CLUSTAL format alignment by MAFFT (v7.058b))

| | | |
|---|---|---|
| 1,12 | DIGTNSVGWAVT | (SEQ ID NO:120) |
| 3,20 | DVGTNSVGWAVT | (SEQ ID NO:121) |
| 15 | DMGTNSVGWAVT | (SEQ ID NO:122) |
| 4 | DVGTSSVGWAVT | (SEQ ID NO:123) |
| 7 | DIGTASVGWAVT | (SEQ ID NO:52) |
| 6 | DVGTGSVGWAVT | (SEQ ID NO:53) |
| 9 | DIGTNSVGWAVV | (SEQ ID NO:54) |
| 10 | DIGTNSVGWAVI | (SEQ ID NO:55) |
| 11 | DIGTNSVGWAVL | (SEQ ID NO:56) |
| 42 | DLGTNSIGWAVV | (SEQ ID NO:57) |
| 48 | DLGTNSIGWAI- | (SEQ ID NO:58) |
| 43 | DLGTNSIGWALV | (SEQ ID NO:59) |
| 2 | DIGTNSVGWCVT | (SEQ ID NO:60) |
| 14 | DIGTNSVGYAVT | (SEQ ID NO:61) |
| 5 | DMGTGSLGWAVT | (SEQ ID NO:62) |
| 16 | DIGTSSVGWAAI | (SEQ ID NO:63) |
| 8 | DLGTGSVGWAVV | (SEQ ID NO:64) |
| 22 | DLGVGSVGWAIV | (SEQ ID NO:65) |
| 23 | DLGIASIGWAII | (SEQ ID NO:66) |
| 24 | DLGIASVGWAIV | (SEQ ID NO:67) |
| 25 | DLGVASVGWSIV | (SEQ ID NO:68) |
| 26 | DIGIASVGWAIL | (SEQ ID NO:69) |
| 28 | DLGISSVGWSVI | (SEQ ID NO:70) |
| 32 | DIGIASVGWSVI | (SEQ ID NO:71) |
| 33 | DVGIGSIGWAVI | (SEQ ID NO:72) |
| 39 | DLGVGSIGFAIV | (SEQ ID NO:73) |
| 34 | DIGYASIGWAVI | (SEQ ID NO:74) |
| 47 | DTGTNSLGWAIV | (SEQ ID NO:75) |
| 50 | DLGTNSIGWCLL | (SEQ ID NO:76) |
| 49 | DIGTDSLGWAVF | (SEQ ID NO:77) |
| 18 | DIGSNSIGFAVV | (SEQ ID NO:78) |
| 41 | DLGVGSIGVAVA | (SEQ ID NO:79) |
| 45 | DLGIASCGWGVV | (SEQ ID NO:80) |

**Fig. 3B**

| | | |
|---|---|---|
| 21 | DLGIASVGWCLT | (SEQ ID NO:81) |
| 27 | DIGIGSVGVGIL | (SEQ ID NO:82) |
| 29 | DIGITSVGYGLI | (SEQ ID NO:83) |
| 30 | DIGITSVGFGII | (SEQ ID NO:84) |
| 31 | DVGITSTGYAVL | (SEQ ID NO:85) |
| 40 | DLGITSPGYAIL | (SEQ ID NO:86) |
| 17 | DIGNASVGWSAF | (SEQ ID NO:87) |
| 19 | DVGTNSCGWVAM | (SEQ ID NO:88) |
| 35 | DVGERSIGLAAV | (SEQ ID NO:89) |
| 36 | DVGLNSVGLAAV | (SEQ ID NO:90) |
| 37 | DVGLMSVGLAAI | (SEQ ID NO:91) |
| 38 | DVGTPSVGLAAI | (SEQ ID NO:92) |
| 13 | DIGTGSVGYACM | (SEQ ID NO:92) |
| 44 | DLGTTSIGPAHI | (SEQ ID NO:94) |
| 46 | DLGTNSIGSSVR | (SEQ ID NO:95) |
| | * * * * | |

**Fig. 4A**

Alignment of the N terminal RuvC-like Domains disclosed in Chylinski et al.
(CLUSTAL format alignment by MAFFT (v7.058b))

```
1,12          D----IGTNSVGWAVT          (SEQ ID NO:120)
3,20          D----VGTNSVGWAVT          (SEQ ID NO:131)
15            D----MGTNSVGWAVT          (SEQ ID NO:122)
4             D----VGTSSVGWAVT          (SEQ ID NO:133)
7             D----IGTASVGWAVT          (SEQ ID NO:52)
6             D----VGTGSVGWAVT          (SEQ ID NO:53)
9             D----IGTNSVGWAVV          (SEQ ID NO:54)
10            D----IGTNSVGWAVI          (SEQ ID NO:55)
52            D----IGTNSIGWAVI          (SEQ ID NO:96)
11            D----IGTNSVGWAVL          (SEQ ID NO:56)
42            D----LGTNSIGWAVV          (SEQ ID NO:57)
48            D----LGTNSIGWAI-          (SEQ ID NO:58)
43            D----LGTNSIGWALV          (SEQ ID NO:59)
3             D----IGTNSVGWCVT          (SEQ ID NO:60)
14            D----IGTNSVGYAVT          (SEQ ID NO:61)
5             D----MGTGSLGWAVT          (SEQ ID NO:62)
16            D----IGTSSVGWAAI          (SEQ ID NO:63)
9             D----LGTGSVGWAVV          (SEQ ID NO:64)
22            D----LGVGSVGWAIV          (SEQ ID NO:65)
23            D----LGIASIGWAII          (SEQ ID NO:66)
24            D----LGIASVGWAIV          (SEQ ID NO:67)
66            D----LGIASVGWAVV          (SEQ ID NO:97)
25            D----LGVASVGWSIV          (SEQ ID NO:68)
26            D----IGIASVGWAIL          (SEQ ID NO:69)
66            D----IGIASVGWAVL          (SEQ ID NO:98)
59            D----IGIASIGWAVI          (SEQ ID NO:99)
61            D----IGIASVGWAII          (SEQ ID NO:100)
64            D----VGIASVGWAVI          (SEQ ID NO:101)
62            D----IGIASVGWAL-          (SEQ ID NO:102)
67            D----IGIASVGWAMV          (SEQ ID NO:103)
32            D----IGIASVGWSVI          (SEQ ID NO:71)
28            D----LGISSVGWSVI          (SEQ ID NO:70)
63            D----IGITSVGWAVI          (SEQ ID NO:104)
```

**Fig. 4B**

| | |
|---|---|
| 33 | D----VGIGSIGWAVI |
| 57 | D----LGISSLGWAIV |
| 39 | D----LGVGSIGFAIV |
| 34 | D----IGYASIGWAVI |
| 50 | D----LGTNSIGWCLL |
| 54 | D----LGTNSIGWGLL |
| 47 | D----TGTNSLGWAIV |
| 49 | D----IGTDSLGWAVF |
| 51 | D----LGSTSLGWAIF |
| 58 | D----IGISSIGWAFS |
| 21 | D----LGIASVGWCLT |
| 45 | D----LGIASCGWGVV |
| 18 | D----IGSNSIGFAVV |
| 65 | D----IGTTSIGFSVI |
| 29 | D----IGITSVGYGLI |
| 30 | D----IGITSVGFGII |
| 44 | D----LGTTSIGFAHI |
| 27 | D----IGIGSVGVGIL |
| 41 | D----LGVGSIGVAVA |
| 31 | D----VGITSTGYAVL |
| 40 | D----LGITSFGYAIL |
| 53 | D----IGTSSIGWNLY |
| 55 | D----LGSNSLGWFVT |
| 56 | D----LGANSLGWFVV |
| 17 | D----IGNASVGWSAF |
| 19 | D----VGTNSCGWVAM |
| 35 | D----VGERSIGLAAV |
| 36 | D----VGLNSVGLAAV |
| 37 | D----VGLMSVGLAAI |
| 38 | D----VGTFSVGLAAI |
| 13 | D----IGTGSVGYACM |
| 46 | D----LGTNSIGSSVR |
| 60 | DIGLRIGITSCGWSI- |
| 69 | D----MGAKYTGVFYA |
| 73 | D----LGGKNTGFFSF |
| 74 | D----LGVKNTGVFSA |
| 70 | D----LGAKFTGVALY |
| 71 | D----LGGKFTGVCLS |
| 72 | D----LGGTYTGTFIT |

Fig. 5A

```
Alignment of the HNH-like Domains disclosed in Chylinski et al.
(CLUSTAL format alignment by MAFFT (v7.058b))

1              YDIDHIYPRS-LTKD------DSF-DNLVLCERTAN       (SEQ ID NO:196)
2              -DIDHIYPRSKVIKD------DSF-DNLVLVLKNEN       (SEQ ID NO:197)
3              -DRDHIYPQS-KIKD------DSI-DNLVLVNKTYN       (SEQ ID NO:198)
4              -DIDHIYPRS-KIKD------DSI-TNRVLVEKDIN       (SEQ ID NO:195)
6              -DIDHIYPQS-KIKD------DSI-SNRVLVCSSCN       (SEQ ID NO:124)
5              -DIDHIYPQS-KTMD------DSL-NNRVLVKKNYN       (SEQ ID NO:125)
7              -DQDHIYPKS-KIYD------DSL-ENRVLVKKNLN       (SEQ ID NO:126)
8              -QIDHIVPQS-LVKD------DSF-DNRVLVVPSEN       (SEQ ID NO:127)
9              -DIDHIIPQA-FIKD------NSI-DNRVLTSSKEN       (SEQ ID NO:128)
12             -DIDHIIPQA-FLKD------NSI-DNKVLVSSASN       (SEQ ID NO:129)
16             -DIDHIIPQA-YTKD------NSL-DNRVLVSNITN       (SEQ ID NO:130)
11             -DIDHIVPQS-FITD------NSI-DNLVLTSSAGN       (SEQ ID NO:131)
10             -DVDHIVPQS-FLKD------DSI-DNKVLTRSDKN       (SEQ ID NO:132)
14             -NIDHIYPQS-MVKD------DSL-DNKVLVQSEIN       (SEQ ID NO:133)
18             -DIDHILPQS-LIKD------DSL-DNRVLVNATIN       (SEQ ID NO:134)
19             -DIDHILPQS-FIKD------DSL-ENRVLVKKAVN       (SEQ ID NO:135)
13             -EVDHIFPRS-FIKD------DSI-DNKVLVIKKMN       (SEQ ID NO:136)
15             -EVDHIIPRS-YIKD------DSF-ENKVLVYREEN       (SEQ ID NO:137)
17             -DIDHIIPQA-VTQN------DSI-DNRVLVARAEN       (SEQ ID NO:138)
22             -EIDHIIPYS-ISFD------DSS-SNKLLVLAESN       (SEQ ID NO:139)
24             -EIDHIIPYS-LCFD------DSS-ANKVLVHKQSN       (SEQ ID NO:140)
32             -DIDHIIPYS-RSMD------DSY-SNKVLVLSGEN       (SEQ ID NO:141)
63             -DIDHIIPYS-KSMD------DSF-NNKVLCLAEEN       (SEQ ID NO:142)
59             -EIDHIYPYS-RSFD------DSY-MNKVLVFTKQN       (SEQ ID NO:143)
65             -QIDHIYPYS-RSMD------DSY-MNKVLVLTDEN       (SEQ ID NO:144)
64             -EIDHIIPFS-RSFD------DSL-SNKILVLGSEN       (SEQ ID NO:145)
68             -EIDHALPFS-RTWD------DSF-NNKVLVLGSEN       (SEQ ID NO:146)
69             -EIDHALPFS-RTWD------DSF-NNKVLVLASEN       (SEQ ID NO:147)
28             -EIDHIIPIS-ISLD------DSI-NNKVLVLSKAN       (SEQ ID NO:148)
30             -EVDHIIPIS-ISLD------DSI-TNKVLVTHREN       (SEQ ID NO:149)
62             -QVDHALPYS-RSYD------DSK-NNKVLVLTHEN       (SEQ ID NO:150)
27             -EVDHILPLS-ITFD------DSL-ANKVLVYATAN       (SEQ ID NO:151)
26             -EIDHIIPRS-ISFD------DAR-SNKVLVYRSEN       (SEQ ID NO:152)
```

**Fig. 5B**

| | | |
|---|---|---|
| 29 | -EVDHIIPRS-VSFD------NSY-HNKVLVKQSEN | (SEQ ID NO:153) |
| 67 | -DIDHILPYS-ITFD------DSF-RNKVLVTSQEN | (SEQ ID NO:154) |
| 58 | -EIDHILPRS-RSAD------DSF-ANKVLCLAKAN | (SEQ ID NO:155) |
| 51 | -EIEHLLPFS-LTLD------DSM-ANKTVCFRQAN | (SEQ ID NO:156) |
| 55 | -DIDHILPFS-VSLD------DSA-ANKVVCLREAN | (SEQ ID NO:157) |
| 57 | -DIDHLIPFS-ISWD------DSA-ANKVVCMRYAN | (SEQ ID NO:158) |
| 56 | -DIDHILPVA-MTLD------DSF-ANKIICMRYAN | (SEQ ID NO:159) |
| 54 | -DVDHILPYS-RTLD------DSF-PNRTLCLREAN | (SEQ ID NO:160) |
| 52 | -EIEHILPFS-RTLD------DSL-NNRTVAMRKAN | (SEQ ID NO:161) |
| 31 | -EVDHIIPYS-ISWD------DSY-TNKVLTSAKCN | (SEQ ID NO:162) |
| 45 | -QVDHILPWS-RFGD------DSY-LNKTLCTARSN | (SEQ ID NO:163) |
| 53 | -QVDHILPFS-KTLD------DSF-ANKVLAQHDAN | (SEQ ID NO:164) |
| 60 | -QIDHAFPLS-RSLD------DSQ-SNKVLCLTSSN | (SEQ ID NO:165) |
| 21 | -DIDHIVPRS-ISFD------DSF-SNLVIVNKLDN | (SEQ ID NO:166) |
| 23 | -EIEHIIPYS-MSYD------NSQ-ANKILTEKAEN | (SEQ ID NO:167) |
| 25 | -EIDHVIPYS-KSAD------DSW-FNKLLVKKSTN | (SEQ ID NO:168) |
| 49 | -EMDHILPYS-RSLD------NGW-HNKVLVHQKIN | (SEQ ID NO:169) |
| 33 | -EVDHIVPYS-LILD------NTI-NNKALVYAEEN | (SEQ ID NO:170) |
| 42 | -EIEHVIPQS-LYFD------DSF-SNKVICEAEVN | (SEQ ID NO:171) |
| 43 | -DIEHIIPQA-RLFD------DSF-SNKTLEARSVN | (SEQ ID NO:172) |
| 44 | -EIEHIVPKA-RVFD------DSF-SNKTLTFHEIN | (SEQ ID NO:173) |
| 20 | -DKDHIIPQS-MKKD------DSIINNLVLVNKNAN | (SEQ ID NO:174) |
| 66 | -EVEHIWPRS-RSFD------NSP-RNKTLCRKDVN | (SEQ ID NO:175) |
| 61 | -IVNHIIPYN-RSFD------DTY-HNKVLTLTETK | (SEQ ID NO:176) |
| 46 | -DMEHTIPKS-ISFD------NSD-QNLTLCSSYYN | (SEQ ID NO:177) |
| 47 | -DIEHTIPRS-AGGD------STK-MNLTLCSSRFN | (SEQ ID NO:178) |
| 48 | -DIEHTIPRS-ISQD------NSQ-MNKTLCSLKFN | (SEQ ID NO:179) |
| 50 | -DIDHVIPLA-RGGR------DSL-DNMVLCQSDAN | (SEQ ID NO:180) |
| 39 | -DIEHLPFIA-ESED------NGR-NNLVISHSACN | (SEQ ID NO:181) |
| 41 | -EVDHIFPRD-ETAD------NSY-GNKVVAHRQCN | (SEQ ID NO:182) |
| 40 | -DIEHIVPQS-LGGL------STD-YNTIVTLKSVN | (SEQ ID NO:183) |
| 35 | -ELDHIVPRT-DGGS------NRR-ENLAITCGACN | (SEQ ID NO:184) |
| 36 | -EMDHIVPRKGVGST------NTR-TNFAAVCAECN | (SEQ ID NO:185) |
| 37 | -EMDHIVPRKGVGST------NTR-VNLAAACAACN | (SEQ ID NO:186) |
| 38 | -EMDHIVPRAGQGST------NTR-ENLVAVCHRCN | (SEQ ID NO:187) |
| 70 | -EIDHILPRS-LIKDARGIVFNAE-PNLIYASSRGN | (SEQ ID NO:188) |
| 71 | -EIDHIIPRS-LTGRTKKTVFNSE-ANLIYCSSKGN | (SEQ ID NO:189) |
| 73 | -EIDHIIPRS-LTLKKSESIYNSE-VNLIFVSAQGN | (SEQ ID NO:190) |

**Fig. 5C**

```
72  -EIDHIYPRS-LSKKHFGVIFNSE-VNLIYCSSQGN    (SEQ ID NO:191)
74  -EIDHILPRS-HTLKIYGTVFNPE-GNLIYVHQKCN    (SEQ ID NO:192)
75  -ELDHIIPRS-HKKY---GTLNDE-ANLICVTRGDN    (SEQ ID NO:193)
34  -ELEHIVPHS-FRQS------NAL-SSLVLTWPGVN    (SEQ ID NO:194)
       :*  *             .    .        :
```

**Fig. 6A**

Alignment of the HNH-like Domains disclosed in Chylinski et al. (excluding sequence outliers). (CLUSTAL format alignment by MAFFT (v7.058b))

```
1     YDIDHIYPRS-LTKDDS-FDNLVLCERTAN     (SEQ ID NO:196)
2     -DIDHIYPRSKVIKDDS-FDNLVLVLKNEN     (SEQ ID NO:197)
3     -DRDHIYPQS-KIKDDS-IDNLVLVNKTYN     (SEQ ID NO:198)
4     -DIDHIYPRS-KIKDDS-ITNRVLVEKDIN     (SEQ ID NO:195)
6     -DIDHIYPQS-KIKDDS-ISNRVLVCSSCN     (SEQ ID NO:124)
5     -DIDHIYPQS-KTMDDS-LNNRVLVKKNYN     (SEQ ID NO:125)
7     -DQDHIYPKS-KIYDDS-LENRVLVKKNLN     (SEQ ID NO:126)
8     -QIDHIVPQS-LVKDDS-FDNRVLVVPSEN     (SEQ ID NO:127)
9     -DIDHIIPQA-FIKDNS-IDNRVLTSSKEN     (SEQ ID NO:128)
12    -DIDHIIPQA-FLKDNS-IDNKVLVSSASN     (SEQ ID NO:129)
16    -DIDHIIPQA-YTKDNS-LDNRVLVSRITN     (SEQ ID NO:130)
11    -DIDHIVPQS-FITDNS-IDNLVLTSSAGN     (SEQ ID NO:131)
10    -DVDHIVPQS-FLKDDS-IDNKVLTRSDKN     (SEQ ID NO:132)
14    -NIDHIYPQS-MVKDDS-LDNKVLVQSEIN     (SEQ ID NO:133)
18    -DIDHILPQS-LIKDDS-LDNRVLVNATIN     (SEQ ID NO:134)
19    -DIDHILPQS-FIKDDS-LENRVLVKKAVN     (SEQ ID NO:135)
13    -EVDHIFPRS-FIKDDS-IDNKVLVIKKMN     (SEQ ID NO:136)
15    -EVDHIIPRS-YIKDDS-FENEVLVYREEN     (SEQ ID NO:137)
17    -DIDHIIPQA-VTQNDS-IDNRVLVARAEN     (SEQ ID NO:138)
21    -DIDHIVPRS-ISFDDS-FSNLVIVNKLDN     (SEQ ID NO:166)
22    -EIDHIIPYS-ISFDDS-SSNKLLVLAESN     (SEQ ID NO:139)
24    -EIDHIIPYS-LCFDDS-SANKVLVHKQSN     (SEQ ID NO:140)
28    -EIDHIIPIS-ISLDDS-INNKVLVLSKAN     (SEQ ID NO:148)
30    -EVDHIIPIS-ISLDDS-ITNKVLVTHREN     (SEQ ID NO:149)
27    -EVDHILPLS-ITFDDS-LANKVLVYATAN     (SEQ ID NO:151)
26    -EIDHIIPRS-ISFDDA-RSNKVLVYRSEN     (SEQ ID NO:152)
29    -EVDHIIPRS-VSFDNS-YHNKVLVKQSEN     (SEQ ID NO:153)
31    -EVDHIIPYS-ISWDDS-YTNKVLTSAKCN     (SEQ ID NO:162)
32    -DIDHIIPYS-RSMDDS-YSNKVLVLSGEN     (SEQ ID NO:141)
23    -EIEHIIPYS-MSYDNS-QANKILTEKAEN     (SEQ ID NO:167)
33    -EVDHIVPYS-LILDNT-INNKALVYAEEN     (SEQ ID NO:170)
25    -EIDHVIPYS-KSADDS-WFNKLLVKKSTN     (SEQ ID NO:168)
49    -EMDHILPYS-RSLDNG-WHNKVLVHGKDN     (SEQ ID NO:169)
42    -EIEHVIPQS-LYFDDS-FSNKVICEAEVN     (SEQ ID NO:171)
43    -DIEHIIPQA-RLFDDS-FSNKTLEARSVN     (SEQ ID NO:172)
```

**Fig. 6B**

```
44   -EIEHIVPKA-RVFDDS-FSNKTLTFHRIN      (SEQ ID NO:173)
20   -DKDHIIPQS-MKKDDSIINNLVLVNKNAN      (SEQ ID NO:174)
45   -QVDHILPWS-RPGDDS-YLNKTLCTARSN      (SEQ ID NO:163)
50   -DIDHVIPLA-RGGRDS-LDNMVLCQSDAN      (SEQ ID NO:180)
46   -DMEHTIPKS-ISFDNS-DQNLTLCESYYN      (SEQ ID NO:177)
47   -DIEHTIPRS-AGGDST-KMNLTLCSSRFN      (SEQ ID NO:178)
48   -DIEHTIPRS-ISQDNS-QMNKTLCSLKFN      (SEQ ID NO:179)
39   -DIEHLFPIA-ESEDNG-RNNLVISHSACN      (SEQ ID NO:181)
41   -DVDHIFPRD-DTADNS-YGNKVVAHRQCN      (SEQ ID NO:182)
40   -DIEHIVPQS-LGGLST-DYNTIVTLKSVN      (SEQ ID NO:183)
35   -ELDHIVPRT-DGGSNR-HENLAITCGACN      (SEQ ID NO:184)
36   -EMDHIVPRKGVGSTNT-RTNFAAVCAECN      (SEQ ID NO:185)
37   -EMDHIVPRKGVGSTNT-RVNLAAACAACN      (SEQ ID NO:186)
38   -EMDHIVPRAGQGSTNT-RENLVAVCHRCN      (SEQ ID NO:187)
34   -ELEHIVPHS-FRQSNA-LSSLVLTWPGVN      (SEQ ID NO:194)
```

**Fig. 7**

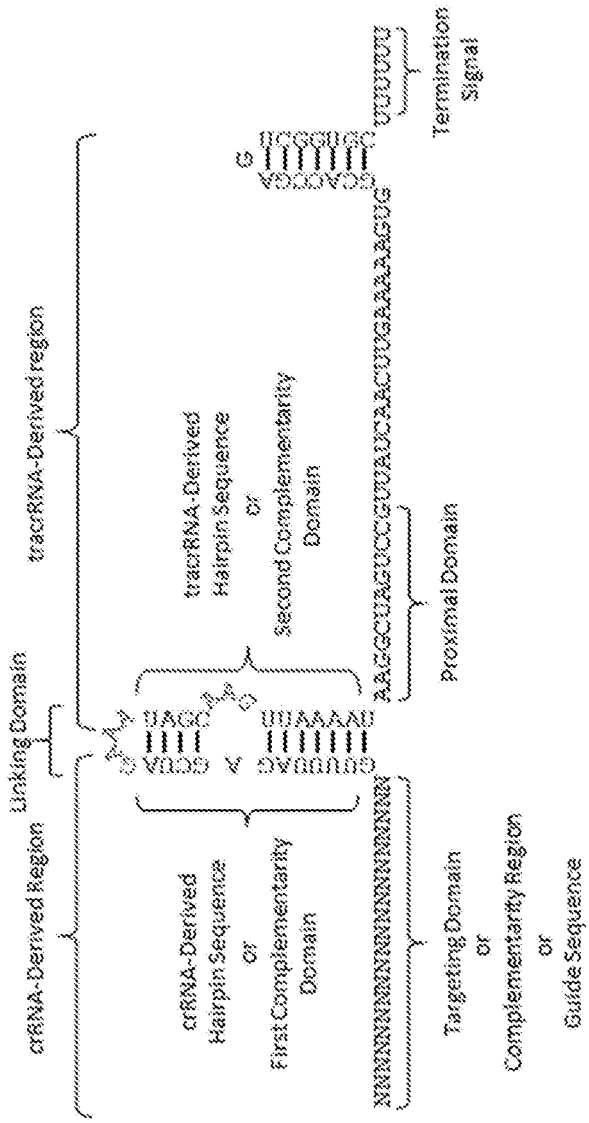
crRNA-Derived Region
Linking Domain
tracrRNA-Derived region
crRNA-Derived
Hairpin Sequence
or
First Complementarity
Domain
tracrRNA-Derived
Hairpin Sequence
or
Second Complementarity
Domain
Targeting Domain
or
Complementarity Region
or
Guide Sequence
Proximal Domain
Termination
Signal

**Fig. 8A**

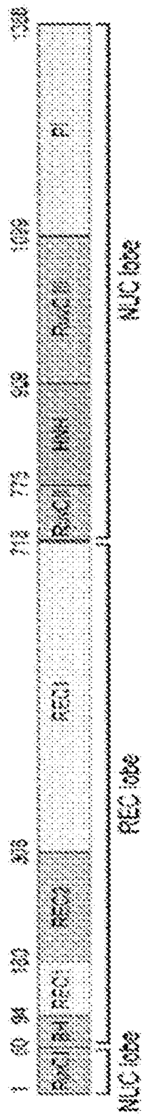

**Fig. 8B**

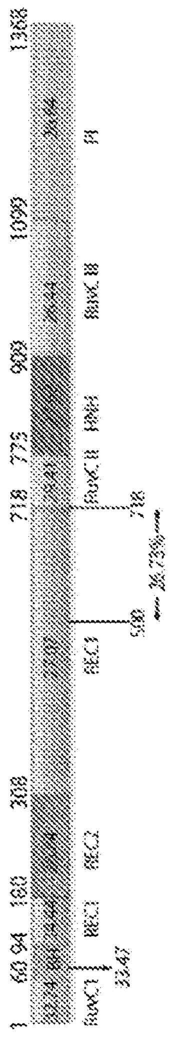
1
60 94
160
308
718
775
909
1099
1368
RuvC I
REC I
REC II
REC I
RuvC II
HNH
RuvC III
PI
718
26.73%

| SEQ ID NO | #2: gRNAs = DMD-5 and DMD-10 (20-mers), deletion = exon 51 (1723bp) |
|---|---|
| 826553 | CAAATTAATTTACTTCCTATTCAAGGGAATTTTAAATCAGAAA ... ATGATTGTGGTCAAGCCATCTCTGGATCTTCGTTTCCTATTC |
| 826554 | CAAATTAATTTACTTCCTAT----------------------- ... -----------------------------------CCTATTC Δ1740 |
| 826555 | CAAATTAATTTACTTCCTATTC--------------------- ... --------------------TCTGGATCTTCGTTTCCTATTC Δ1723 (x6) |
| 826556 | CAAATTAATTTACTTC--------------------------- ... ---------------------------TTCGTTTCCTATTC Δ1737 |
| 826557 | ------------------------------------------- ... ---------------------CTGGATCTTCGTTTCCTATTC 117bp inversion, Δ2306 |
| 826558 | CAAATTAATTTACTTCCTATT*A*--------------------- ... *GAAGGGAANGAAGGGAGGGANGAAGAAAANGGAAG* Δunknown +insertion |
| 826559 | CAAATTAATTTACTTCCTATT*T*C-------------------- ... --------------------TCTGGATCTTCGTTTCCTATTC Δ1723+1bp |

| SEQ ID NO | #5: gRNAs = DMD-6 and DMD-9 (20-mers), deletion = exon 51 (1326bp) |
|---|---|
| 826560 | TGTGGCTTTACCAAGGTCCCAGAGTTCCTAGGGCAGAGAACAGGA ... GTCTCAGTTAAATTTACTAAACAACCTGGTATTTTAAAAATCTA |
| 826561 | TGTGGCTTTACCAAGGTCCCAG----------------------- ... ---------------------------GGTATTTTAAAAATCTA Δ1333 |
| | ------------------------------------------- ... ------------------------------------------- Δ1935 |
| 826562 | TGTG---------------------------...CCTTACAAGAATAGTTGTCTCAGTTAAATTTACTAAA*TT*---TGGTATTTTAAAAATCTA Δ1240 (106bp of matching sequence 5′ of DMD-9 cut site) |
| 826563 | TGTGGCTTTACCAAGGTCCC------------------------- ... --------------------------TGGTATTTTAAAAATCTA Δ1334 |
| 826564 | TGTGGCTTTACCAAGG----------------------------- ... ------------------------------------AAAAATCTA Δ1347 |
| 826565 | TGTGGCTTTACCAAGGTCCCAGAG--------------------- ... -------------------------CTGGTATTTTAAAAATCTA Δ1328 |
| 826566 | TGTGGCTTTACCAAGGTCCCAGAG--------------------- ... ------------------------CCTGGTATTTTAAAAATCTA Δ1327 (x2) |
| 826567 | TGTGGCTTTACCAAGGTCCCAGAG--------------------- ... ------------------------------------------- Δ2089 |

Fig. 9A

| SEQ ID NO | | |
|---|---|---|
| | #6: gRNAs = DMD-6 and DMD-10 (20-mers), deletion = exon 51 (2077bp) | |
| 826568 | TGTGGCTTTACCAAGGTCCCAGAGTTCCTAGGGCAGAGAACAGGA ... ATGATTGTGGTCAAGCCATCTCTGGATCTTCGTTTCCTATTC | |
| 826569 | TGTGGCTTTACCAAGGTCCCAGAG--------------------- ... -------------------CTCTGGATCTTCGTTTCCTATTC | Δ2077 (x10) |
| 826570 | TGTGGCTTTACCAAGGTCCCAGAG--------------------- ... -----------------AACTCTGGATCTTCGTTTCCTATTC | Δ2075 |
| 826571 | TGTGGCTTTACCAAGGTC--------------------------- ... -------------------------------GTTTCCTATTC | Δ2095 |
| 826572 | GTAAAGCCACATAACCTCTCTAAG--------------------- ... --------------------TCTGGATCTTCGTTTCCTATTC | 33bp inversion Δ2092 |
| 826573 | TGTGGCTTTACCAAGGTCCCAGAGT-------------------- ... -------------------CTCTGGATCTTCGTTTCCTATTC | Δ2077 |
| 826574 | TGTGGCTTTACCAAGGTCCCAGAG--------------------- ... ------------------ACTCTGGATCTTCGTTTCCTATTC | Δ2076 |
| 826575 | TGTGGCTTTACCAAGGTCCCAGAGTTC------------------ ... ------------------TCTCTGGATCTTCGTTTCCTATTC | Δ2073 |
| 826576 | TGTGGCTTTACCAAGGTCCCAGAG--------------------- ... ------------------TCTCTGGATCTTCGTTTCCTATTC | Δ2077 (x2) |
| 826577 | TGTGGCTTTACCAAGGTCCCAGAG--------------------- ... ------------------------------------------ | Δ2104 |
| 826578 | TGTGGCTTTACCAAGGTCCCAGAGTTCCTAGGGCAGAGAACAGGA ... ---------------------- | Δ2110 spanning DMD-10 site but doesn't look like it cut at DMD-6 |
| | #11: gRNAs = DMD-8 and DMD-11 (17-mers), deletion = exon 51 (1852bp) | |
| 826579 | ACCTAACACTATTGATCACCTACTATAGTGTCAGGCGCTGTAATAA...AATAACAAAAGTAGCCATACATTAAAAAGGAAATATACAAAA | |
| 826580 | ACCTAACACTATTGATCACCTACTA---------------------...-------------------------AAAGGAAATATACAAAA | Δ1852 (x9) |
| 826581 | ACCTAACACTATTGATCACCTA------------------------...----------------------------GGAAATATACAAAA | Δ1858 (x3) |

Fig. 9B

SEQ ID NO

#12: gRNAs = DMD-8 and DMD-12 (17-mers), deletion = exon 51 (3282bp)

826582 TAACACTATTGATCACCTACTATAGTGTCAGGCGCTGTAATAA...TTCCTGCAGAAGTTACCAGATAATAGATTGGGAGAGAAAGTCCACA

826583 TAACACTATTGATCACCTACT----------------------...CTTCTCACTTGATTATTTCTAGAAATTTTAATAAAAATAGTCCACA Δ3294 +39bp insertion (chr2)

826584 TAACACTATTGATCACCTACT----------------------...------------------------AGATTGGGAGAGAAAGTCCACA Δ3281 (x7)

826585 TAACACTATTGATCACCTACT----------------------...-----------------------TAGATTGGGAGAGAAAGTCCACA Δ3280 (x5)

826586 TAACACTATTGATC-----------------------------...-----------------------TAGATTGGGAGAGAAAGTCCACA Δ3273

826587 TAACACTATTGATCACCTACT----------------------...-------------------------GATTGGGAGAGAAAGTCCACA Δ3282

826588 TAACACTATTGATCACCTACTA-----TATGTCAGAAGTTAGCTGGTTTGAAGTTTAAAAAGAG-----TAGATTGGGAGAGAAAGTCCACA Δ3279 +37bp insertion (chr5)

826589 TAACACTATTGATCACCTACTA---------------------...---------------------------------------GTCCACA Δ3296

826590 TAACACTATTGATCACCTACT----------------------...-----------------------------TGGGAGAGAAAGTCCACA Δ3275

#13: gRNAs = DMD-5 and DMD-18 (20-mers), deletion = exons 51-55 (147,634bp)

826591 CAAATTAATTTACTTCCTATTCAAGGGAATTTTAAATCAGAAA ... TTGGTGAGAATCATATTCTGTAGTACAAGGAGGCTCTTAAAGTTT

826592 CAAATTAATTTACTTCCTATT---------------------- ...-----------------------TACAAGGAGGCTCTTAAAGTTT Δ147,633 (x8)

826593 CAAATTAATTTACTTCCTATT---------------------- ...------------------------ACAAGGAGGCTCTTAAAGTTT Δ147,634

826594 CAAATTAATT--------------------------------- ...--------------------------------------AAAGTTT Δ147,659

826595 CAAATTAATTTACT----------------------------- ...--------------------------------------------- Δ147,848

826596 CAAATTAATTTACTTCCTATT---------------------- ...-----------------------------GAGGCTCTTAAAGTTT Δ147,649

Fig. 9C

```
SEQ ID
NO      #16: gRNAs = DMD-6 and DMD-18 (20-mers), deletion = exons 51-55 (147,998bp)
826597  GCTTTACCAAGGTCCCAGAGTTCCTAGGGCAGAGAACAGGAT... TTGGTGACAATCATATTCTGTAGTACAAGGAGGCTCTTAAAGTTT
826598  GCTTTACCAAGGTCCCAGAG----------------------... -----------------------TACAAGGAGGCTCTTAAAGTTT Δ147,987 (x13)
826599  GCTTTACCAAGGTCCCAGAG----------------------... ------------------------ACAAGGAGGCTCTTAAAGTTT Δ147,998 (x5)
826600  GCTCTACCAAGGTCCCAGAG----------------------... -----------------------TACAAGGAGGCTCTTAAAGTTT Δ147,987 +2bp
826601  GCTTTACCAAGGTCCC--------------------------... -----------------------TACAAGGAGGCTCTTAAAGTTT Δ147,983
        ------------------------------------------... ------------------------------------------TTT Δ148,210

        #20: gRNAs = DMD-8 and DMD-19 (17-mers), deletion = exons 51-55 (148,376bp)
826602  TAACACTATTGATCACCTACTATAGTGTCAGGCGCTGTAATAA... TTACTAGTCTCTCAAATACAATTTGGATATAAATCATTA
826603  TAACACTATTGATCACCTACTA---------------------... --------------------ATTTGGATATAAATCATTA      Δ148,375 (x14)
826604  TAACACTATTGATCACCTACT----------------------... --------------------ATTTGGATATAAATCATTA      Δ148,376 (x4)
826605  TAACACTATTGATCACCTA-TA---------------------... ---------TCTCAAA-----------TATAAATCATTA      Δ148,376
826606  TAACACTATTGATCACAT-------------------------... -----------------------TGGATATAAATCATTA      Δ148,370
826607  TAACACTATTGATCACCTACT---... ACCTCATGATCCGC...AGAAAGCCATTTTTTTT...---TTTGGATATAAATCATTA      Δ148,370 +89bp
                                                                                                   insertion (chr13)
```

Fig. 9D

| SEQ ID NO | | |
|---|---|---|
| | #21: gRNAs = DMD-8 and DMD-20 (17-mers), deletion = exons 51-55 (148,657) | |
| 826608 | TAACACTATTGATCACCTACTATAGTGTCAGGCGCTGTAATAA... GAGTACGTAACAGTGGCAATGTATGGCTCCTGAATTTATC | |
| 826609 | TAACACTATTGATCACCTA-----------------------... ----------------------TGGCTCCTGAATTTATC | Δ148,662 |
| 826610 | TAACACTATTGATCACCTACT---------------------... -----------------ATGTATGGCTCCTGAATTTATC | Δ148,655 (x3) |
| 826611 | TAACACTATTGATCACCTACT---------------------... ------------------TGTATGGCTCCTGAATTTATC | Δ148,656 (x7) |
| 826612 | TAACACTATTGATCACCTACTA--------------------... -------------------GTATGGCTCCTGAATTTATC | Δ148,656 |
| 826613 | TAACACTATTGATCACCTACT---------------------... ---------------------------CCTGAATTTATC | Δ148,665 |
| 826614 | TAACACTATTGATCACCTACT---...ACAGAGACTCTTAAAT...TCCTCCCATTTTTT...--TGTATGGCTCCTGAATTTATC | Δ148,656 +44bp insertion (chr6) |
| 826615 | TAACACTATTGATC----------------------------... ------------------TGTATGGCTCCTGAATTTATC | Δ148,663 |
| 826616 | TAACACTATTGATC----------------------------... ---------------------------CCTGAATTTATC | Δ148,672 |
| | #22: gRNAs = DMD-1 and DMD-18 (20-mers), deletion = exons 45-55 (342,830bp) | |
| 826617 | TCAAGAGAGTATGGCCAGGACAGCTTGTGAATAGTTCAGTA... GGTGAGAATCATATTCTGTAGTACAAGGAGGCTCTTAAAGTTT | |
| 826618 | TCAAGAGAGTATGGCCAGGA--------------------... --------------------TACAAGGAGGCTCTTAAAGTTT | Δ342,829 (x11) |
| 826619 | TCAAGAGAGTATG---------------------------... ---------------------ACAAGGAGGCTCTTAAAGTTT | Δ342,837 |
| 826620 | TCAAG-----------------------------------... ---------------------------GAGGCTCTTAAAGTTT | Δ342,850 |
| 826621 | TCAAGAGAGTATGGCC------------------------... --------------------TACAAGGAGGCTCTTAAAGTTT | Δ342,833 |
| 826622 | TCAAGAGAGTATGGCCA-----------...TTTATAATTCCTTCCTTCATATTC...--------ACAAGGAGGCTCTTAAAGTTT | Δ342,831 +24bp insertion (chr6?) |
| 826623 | TCAAGAGAGTATGGCCAGGA-..TCATGTTGCTGGTTGGGTGAGTCTTTTTACGTTACATGTT..-ACAAGGAGGCTCTTAAAGTTT | Δ342,827 +40bp insertion (chr2?) |
| 826624 | TCAAGA----------------------------------... --------------------TACAAGGAGGCTCTTAAAGTTT | Δ342,843 |
| 826625 | TCAAGAGAGTATGGCCAGGA--------------------... ------------------------------GGCTCTTAAAGTTT | Δ342,837 |

Fig. 9E

```
SEQ ID NO
        #25: gRNAs = DMD-2 and DMD-18 (20-mers), deletion = exons 45-55 (342,898bp)
826626  AATGGAGCAAAGCCAACTAGTTACAAGGAAATATCACATGT...GGTGAGAATCATATTCTGTAGTACAAGGAGGCTCTTAAAGTTT
826627  AATGGAGCAAAGCCAACTA----------------------...--------------------ATACAAGGAGGCTCTTAAAGTTT   Δ342,896 +1bp
826628  AATGGAGCAAAGCCAACTA----------------------...---------------------TACAAGGAGGCTCTTAAAGTTT   Δ342,896 (x3)
826629  AATGGAGCAA-------------------------------...--------------------------GGAGGCTCTTAAAGTTT   Δ342,911
826630  AATGGAGCAAAGCCAAC------------------------...------------------------AAGGAGGCTCTTAAAGTTT   Δ342,900
826631  AA---------------------------------------...--------------------------GGAGGCTCTTAAAGTTT   Δ342,919
826632  AATGGAGCAAAGCCAACT-----------------------...---------------------TACAAGGAGGCTCTTAAAGTTT   Δ342,897 (x6)
826633  AATGGAGCAAAGCCAACT-----------------------...-----------------GTA--ACAAGGAGGCTCTTAAAGTTT   Δ342,896 +3
826634  AATGGAGCAAAG-----------------------------...---------------------------GAGGCTCTTAAAGTTT   Δ342,909
826635  AATGGAGCAAAGCCAA-------------------------...---------------------------GAGGCTCTTAAAGTTT   Δ342,905
826636  AATGGAGCAAAGCCAACT-----------------------...------------------------AAGGAGGCTCTTAAAGTTT   Δ342,899

        #29: gRNAs = DMD-4 and DMD-19 (17-mers), deletion = exons 45-55 (342,038bp)
826637  TATGTTTGTTCTTTTTCACCTCTCGTATCCACGATCACTAAGA... TTACTAGTCTCTCAAATACAATTTGGATATAAATCATTA
826638  TATGTTTGTTCTTTTTCACCTCTCG-------------------... ---------------------ATTTGGATATAAATCATTA   Δ342,037 (x10)
826639  TATGTTTGTTCTTTTTCACCTCTC--------------------... ---------------TTTTTACGTGGATATAAATCATTA   Δ342,038 +8bp
826640  TATGTTTGTTCTTTTTCACCTCTC--------------------... --------------------AATTTGGATATAAATCATTA   Δ342,037 (x3)
826641  TATGTTTGTTCTTTTTCACCTCTC--------------------... --------------------------------ATCATTA   Δ342,049
826642  TATGTTTGTTCTTTTTCACCTCT---...TTTTTATATCATAT...GATATATCAAAATG...---ATTTGGATATAAATCATTA   Δ342,039 +61bp
                                                                                                   insertion (chr3?)
```

Fig. 9F

SEQ ID NO

#30: gRNAs = DMD-4 and DMD-20 (17-mers), deletion = exons 45-55 (342,319bp)

826643 TATGTTTGTTCTTTTTCACCTCTCGTATCCACGATCACTAAGA... GAGTACGTAACAGTGGCAATGTATGGCTCCTGAATTTATC

826644 TATGTTTGTTCTTTTTCACCTCTCG------------------... -------------------TGTATGGCTCCTGAATTTATC Δ342,317 (x14)

826645 TATGTTTGTTCTTTTTCACCTCT--------------------... ------------------------GGCTCCTGAATTTATC Δ342,324

826646 TATGTTTGTTCTTTTTCACCTCTC-------------------... -----------------------------CTGAATTTATC Δ342,328

826647 TATGTTTGTTCTTTTTCACCTCTCGT-----------------... ---------------------TATGGCTCCTGAATTTATC Δ342,318

826648 TATGTTTGTTCTTTTTCACCTCTC-------------------... -------------------TGTATGGCTCCTGAATTTATC Δ342,318

826649 TATGTTTGTTCTT------------------------------... ---------------------------------------- Δ342,356

Fig. 9G

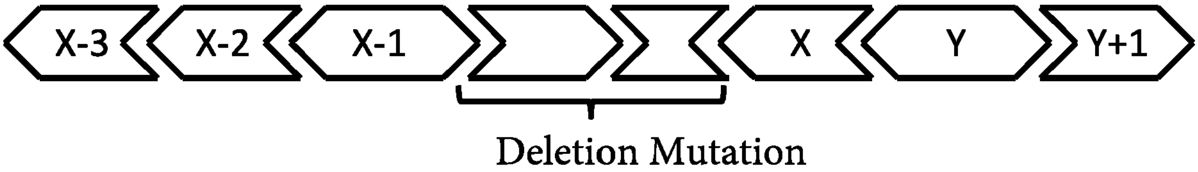


Fig. 10A

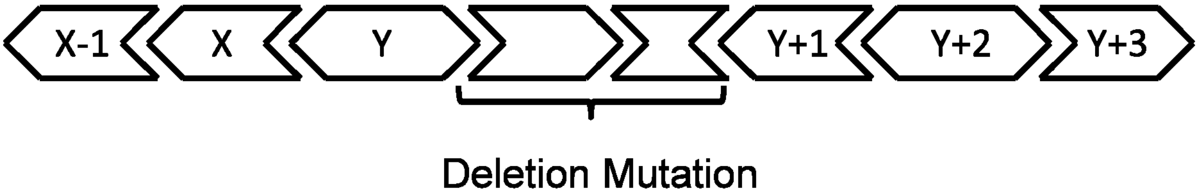


Fig. 10B

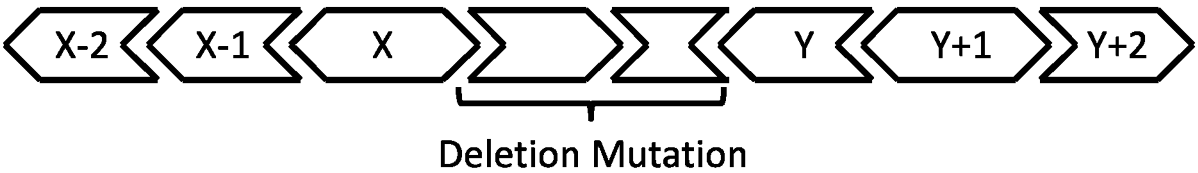


Fig. 10C

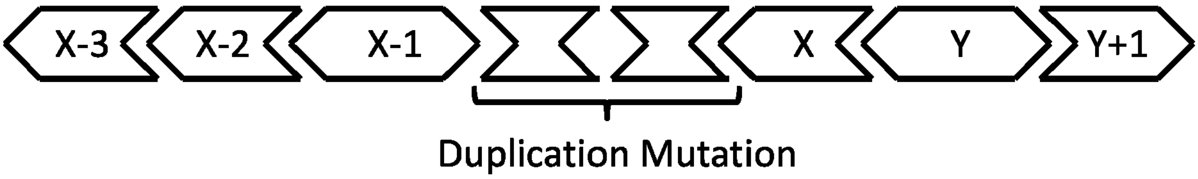


Fig. 11A

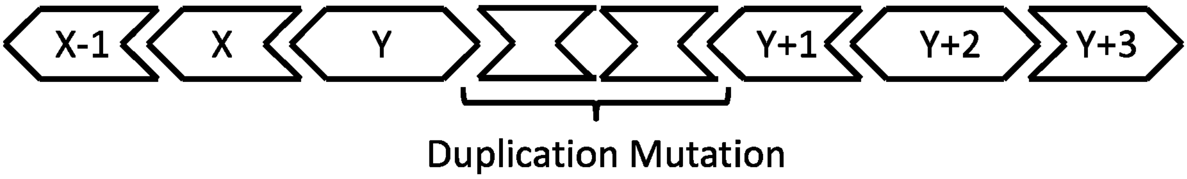


Fig. 11B

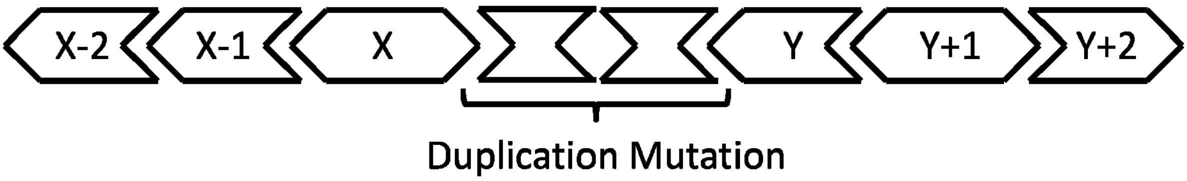


Fig. 11C

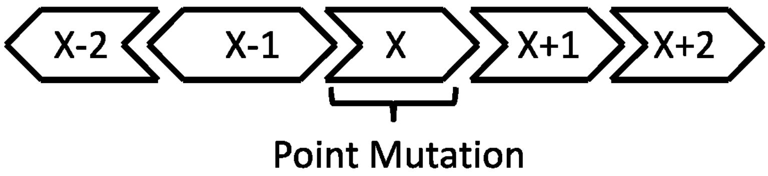


Fig. 12A

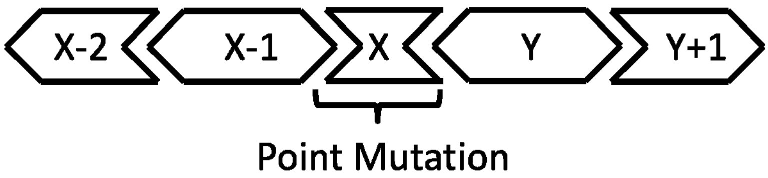


Fig. 12B

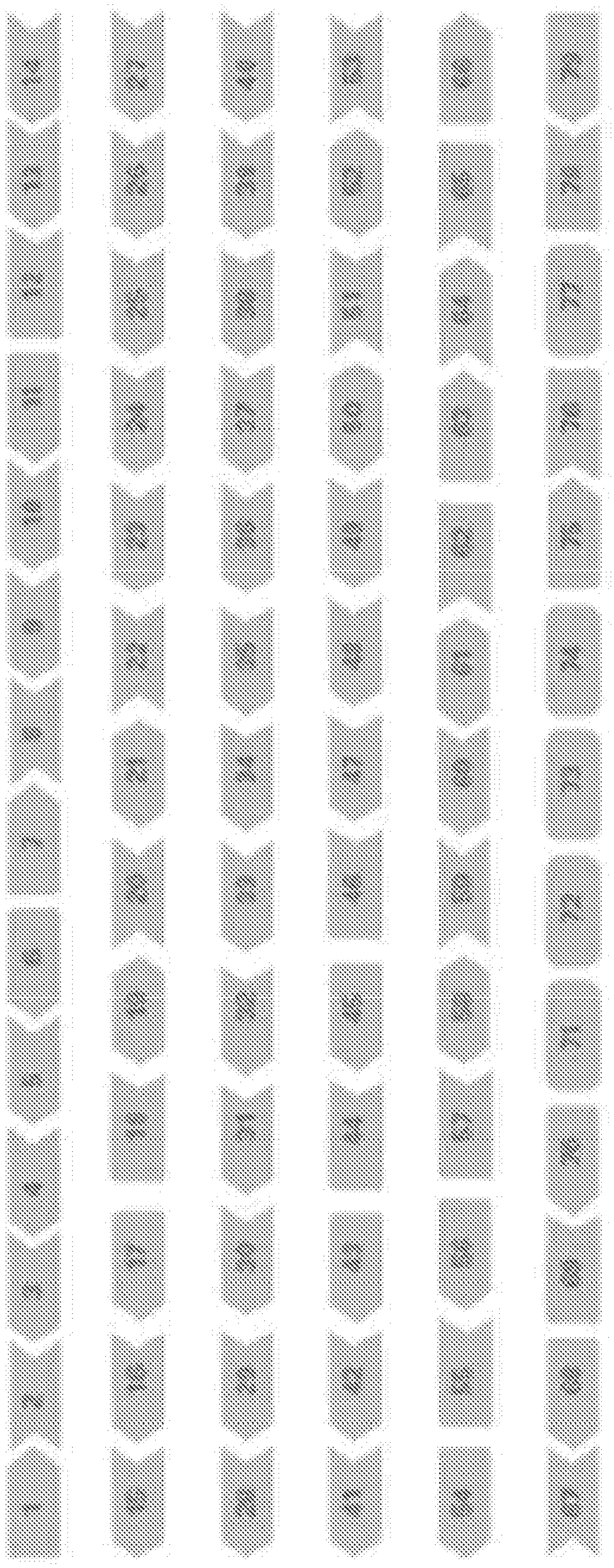

Fig. 13

Figure 14

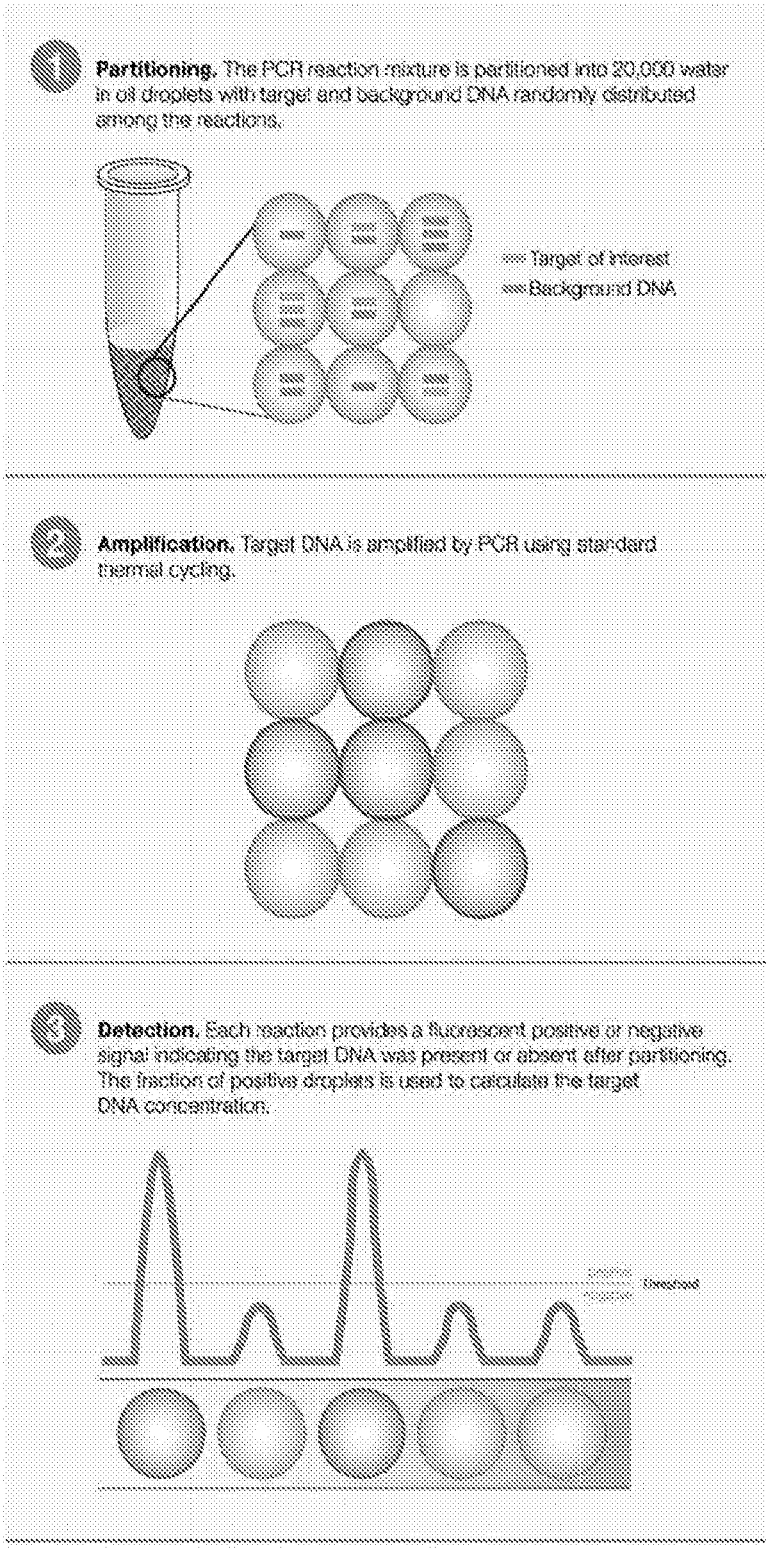
1
Partitioning. The PCR reaction mixture is partitioned into 20,000 water in oil droplets with target and background DNA randomly distributed among the reactions.
Target of interest
Background DNA
2
Amplification. Target DNA is amplified by PCR using standard thermal cycling.
3
Detection. Each reaction provides a fluorescent positive or negative signal indicating the target DNA was present or absent after partitioning. The fraction of positive droplets is used to calculate the target DNA concentration.

**Figure 15**

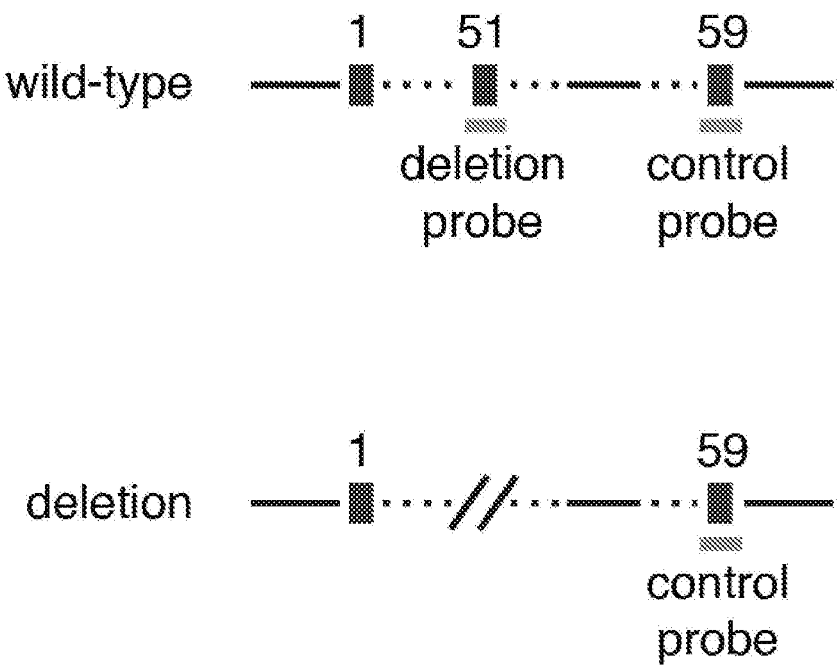
1
51
59
wild-type
deletion
probe
control
probe
1
59
deletion
control
probe

Figure 16A

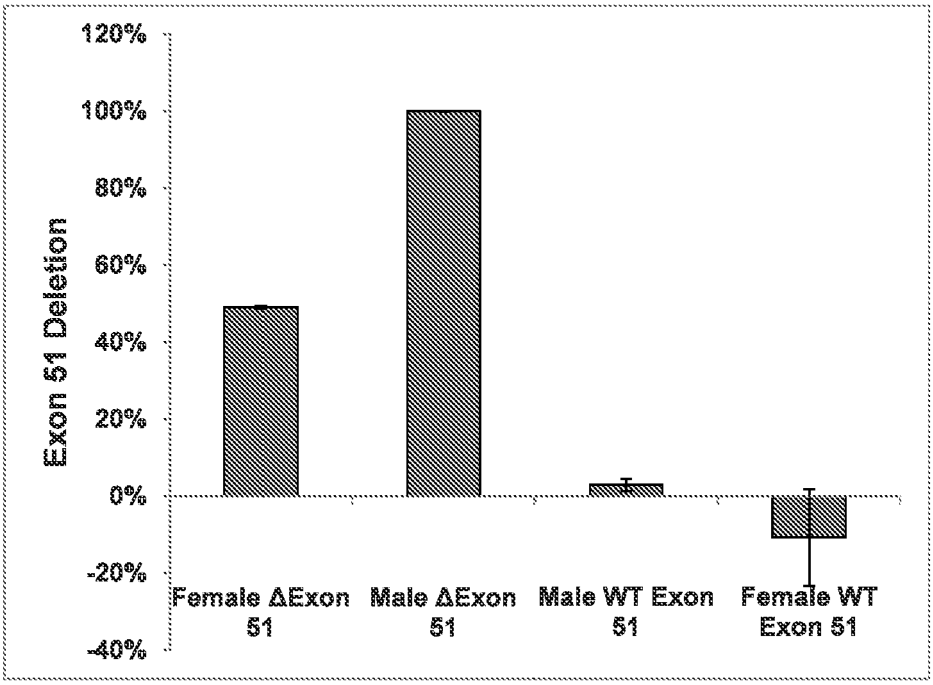


Figure 16B

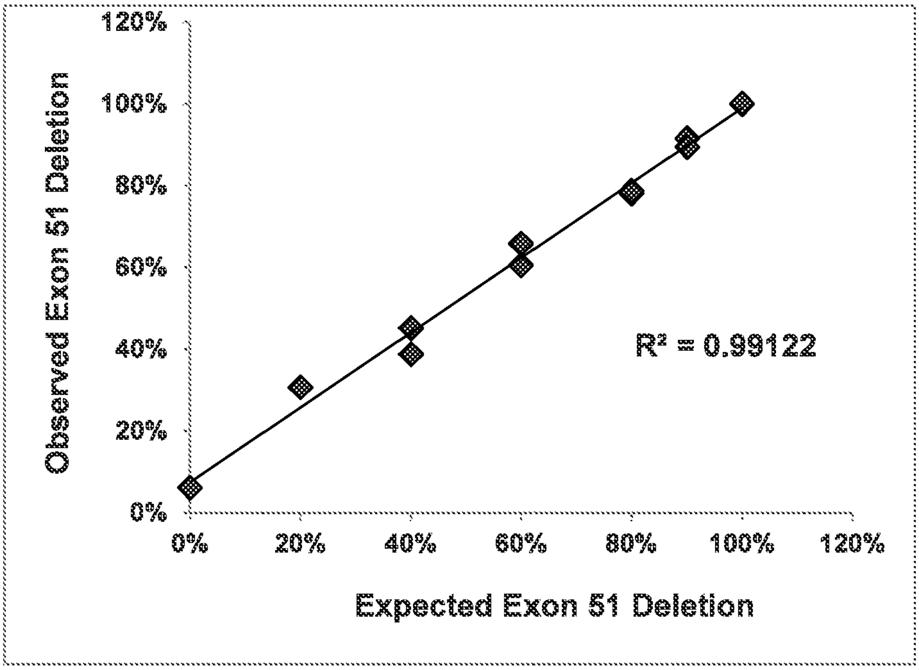

**Figure 16C**

| | 1 | 2 | 3 | 4 | 5 | 6 | 7 | 8 | 9 | 10 | 11 | 12 |
|---|---|---|---|---|---|---|---|---|---|---|---|---|
| A | | | | | | | | | | | | |
| B | | | | | Guide Pairs | 50 ng each | | | | | | |
| C | | | | | these are normalized to [50] | | | | | | | |
| D | | | | | transfect | | | | | | | |
| E | | | | | | | | | | | | |
| F | | | | | | | | | | | | |
| G | | | | | | | | | | | | |
| Row of Controls: H | Guide 1+88 (SUV) | Guide 1+88 (SUV) | Guide 1+9 (ddPCR Pos) | Guide 1+9 (ddPCR Pos) | pJT002 (88 only) | pJT002 (88 only) | 0% | 5% | 10% | 15% | 20% | 30% |

**Figure 16D**

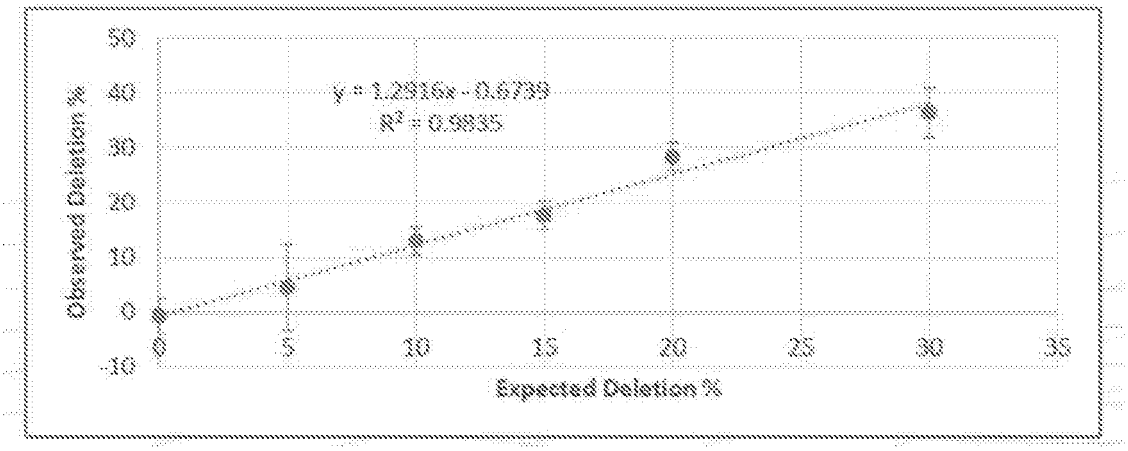

**Figure 17A**

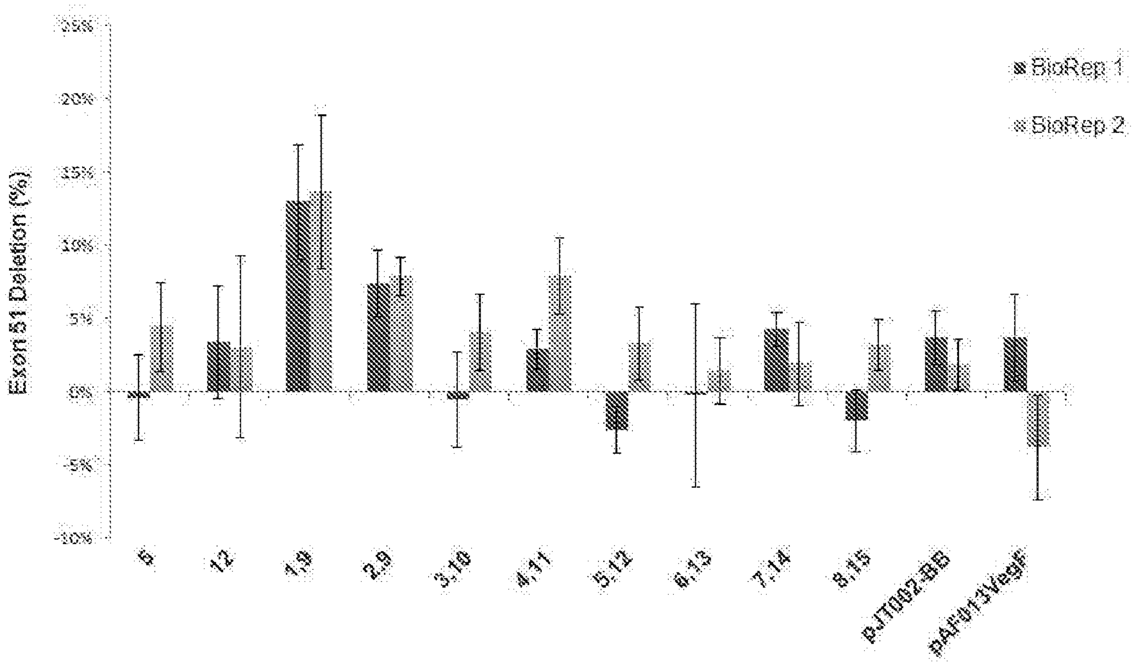


**Figure 17B**

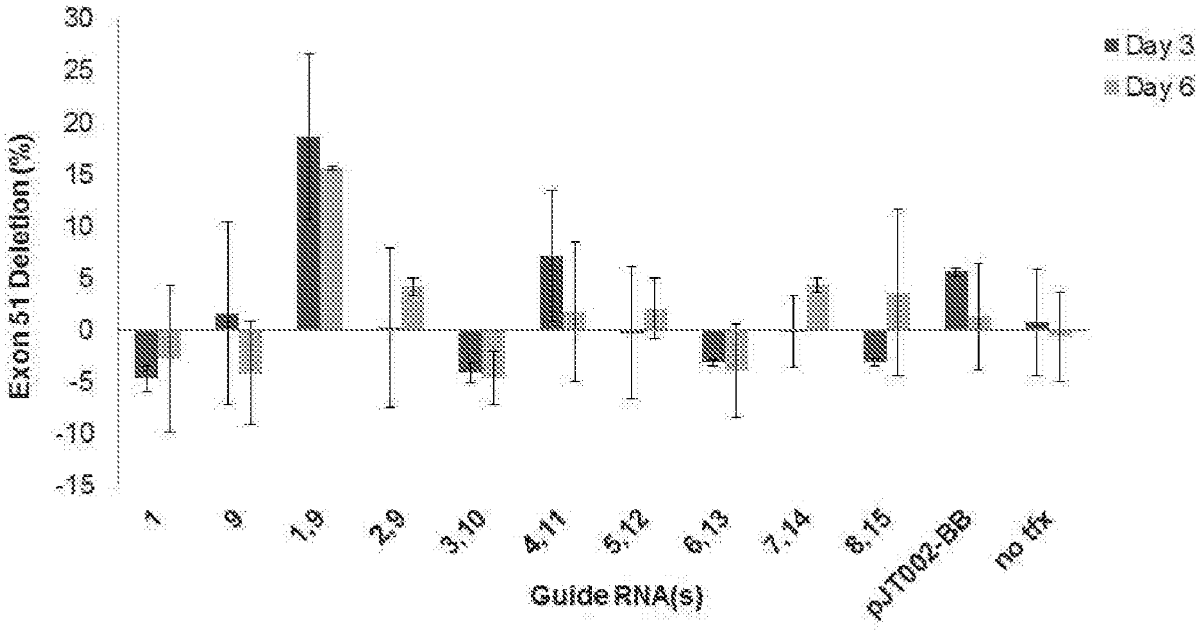

Figure 18A

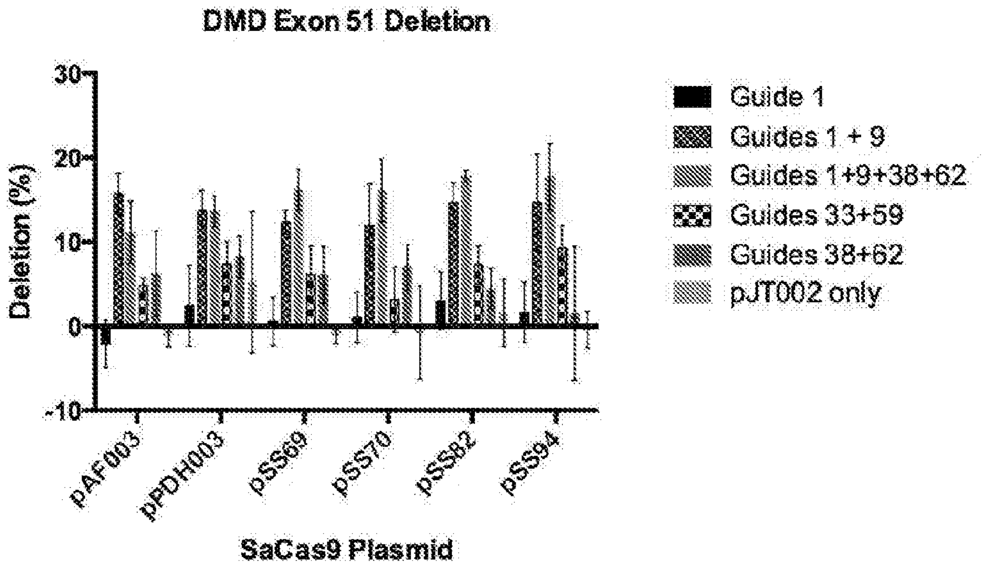


Figure 18B

| Plasmid Name | AAV ITR | Promoter | T7 Promoter | saCas9 CDS | Codon Optimization Version | polyA |
|---|---|---|---|---|---|---|
| pAF003 | - | CMV | + | NLS-saCas9v1-NLS-3xFlag | v1 | bgh pA |
| pPDH003 | - | EFS | - | NLS-saCas9v2-NLS | v2.1 | - |
| pSS70 | + | EFS | + | NLS-saCas9v2-NLS-Flagx3 | v2.2 | minpA |
| pSS69 | + | CMV | + | NLS-saCas9v2-NLS-Flagx3 | v2.2 | minpA |
| pSS82 | + | EFS | + | NLS-saCas9v3-NLS-3xFlag | v3 | minpA |
| pSS94 | + | CMV | + | NLS-saCas9v3-NLS-3xFlag | v3 | minpA |

**Figure 19**

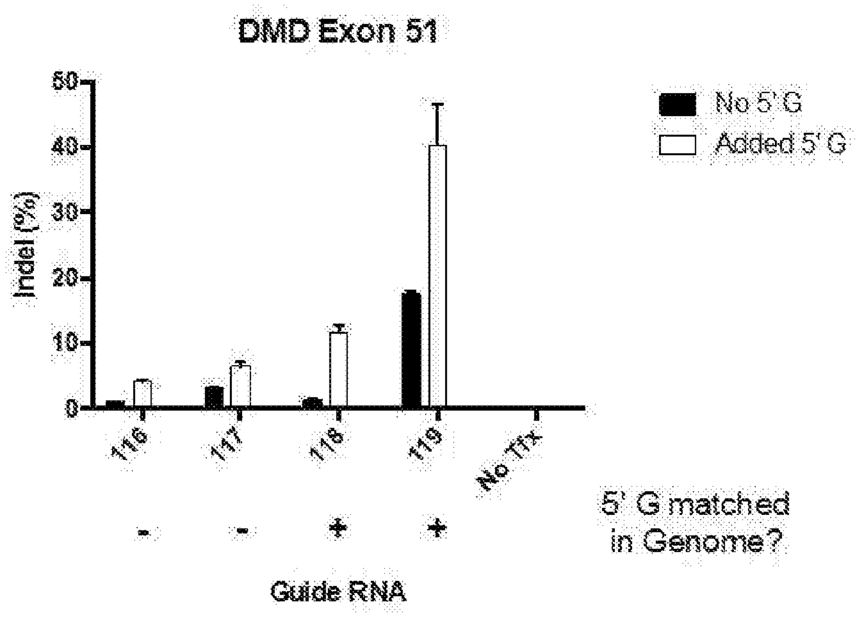
DMD Exon 51
50
40
30
20
10
0
Indel (%)
No 5' G
Added 5' G
116
117
118
119
No Tfx
-
-
+
+
5' G matched
in Genome?
Guide RNA

Figure 20

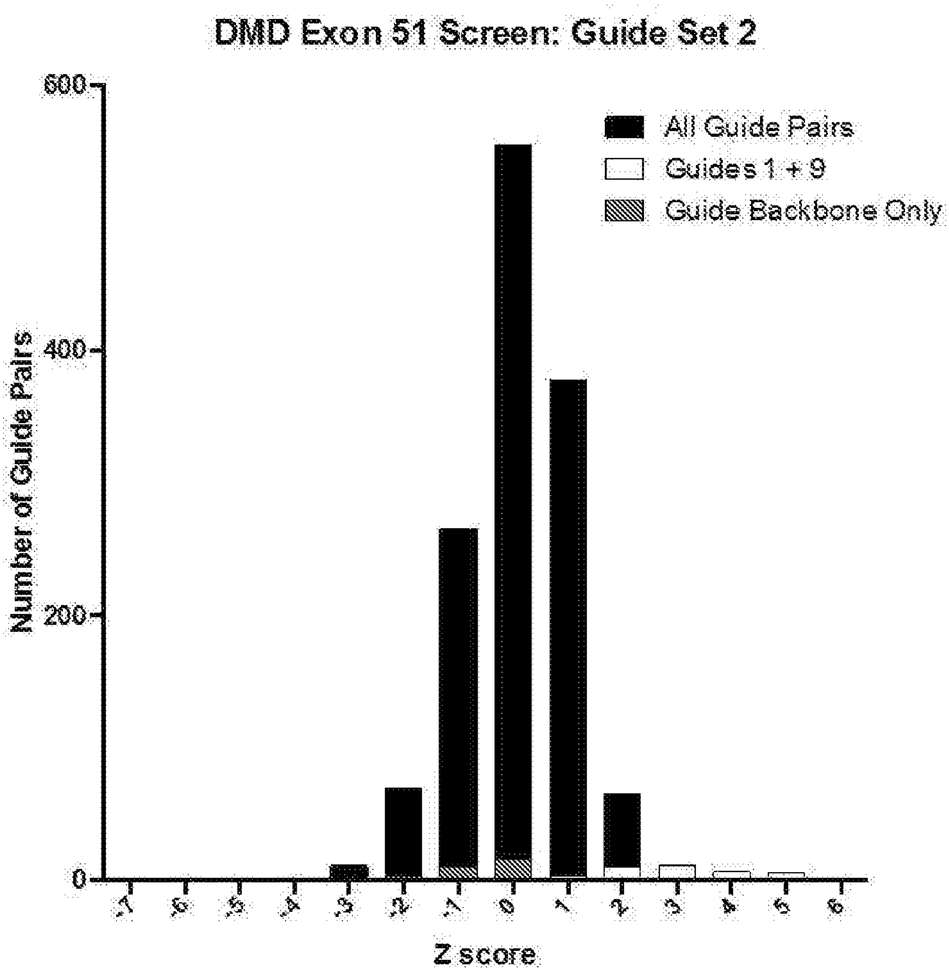
DMD Exon 51 Screen: Guide Set 2
All Guide Pairs
Guides 1 + 9
Guide Backbone Only
Number of Guide Pairs
600
400
200
0
-7
-6
-5
-4
-3
-2
-1
0
1
2
3
4
5
6
Z score

**Figure 21**

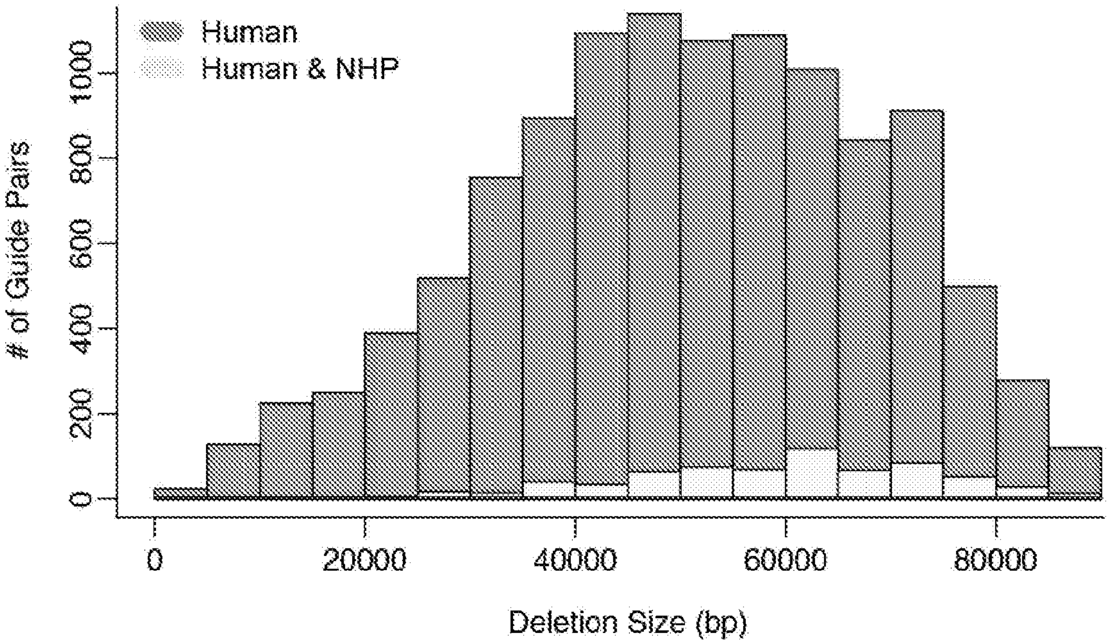
Human
Human & NHP
# of Guide Pairs
0
200
400
600
800
1000
0
20000
40000
60000
80000
Deletion Size (bp)

Figure 22

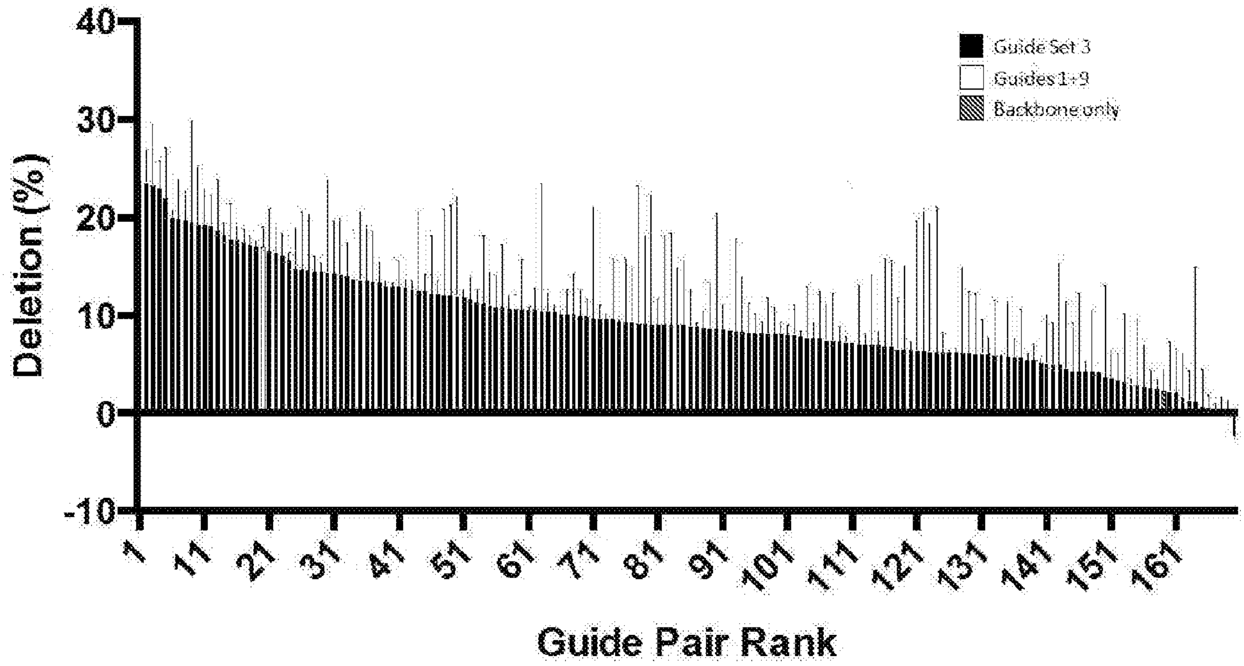
Guide Set 3
Guides 1+9
Backbone only
40
30
20
10
0
-10
Deletion (%)
1
11
21
31
41
51
61
71
81
91
101
111
121
131
141
151
161
Guide Pair Rank

**Figure 23**

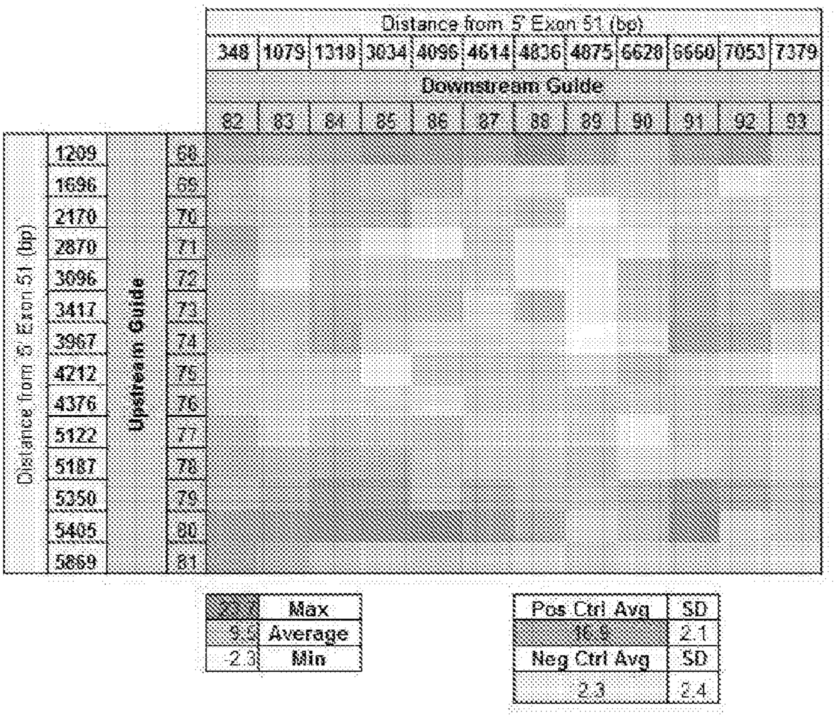


**Guide Design Criteria:**

1. Human-only
2. 3' T in PAM
3. 5' G
4. No off-by-2's
5. Length 21
6. Deletion size < 14 kb

Figure 24

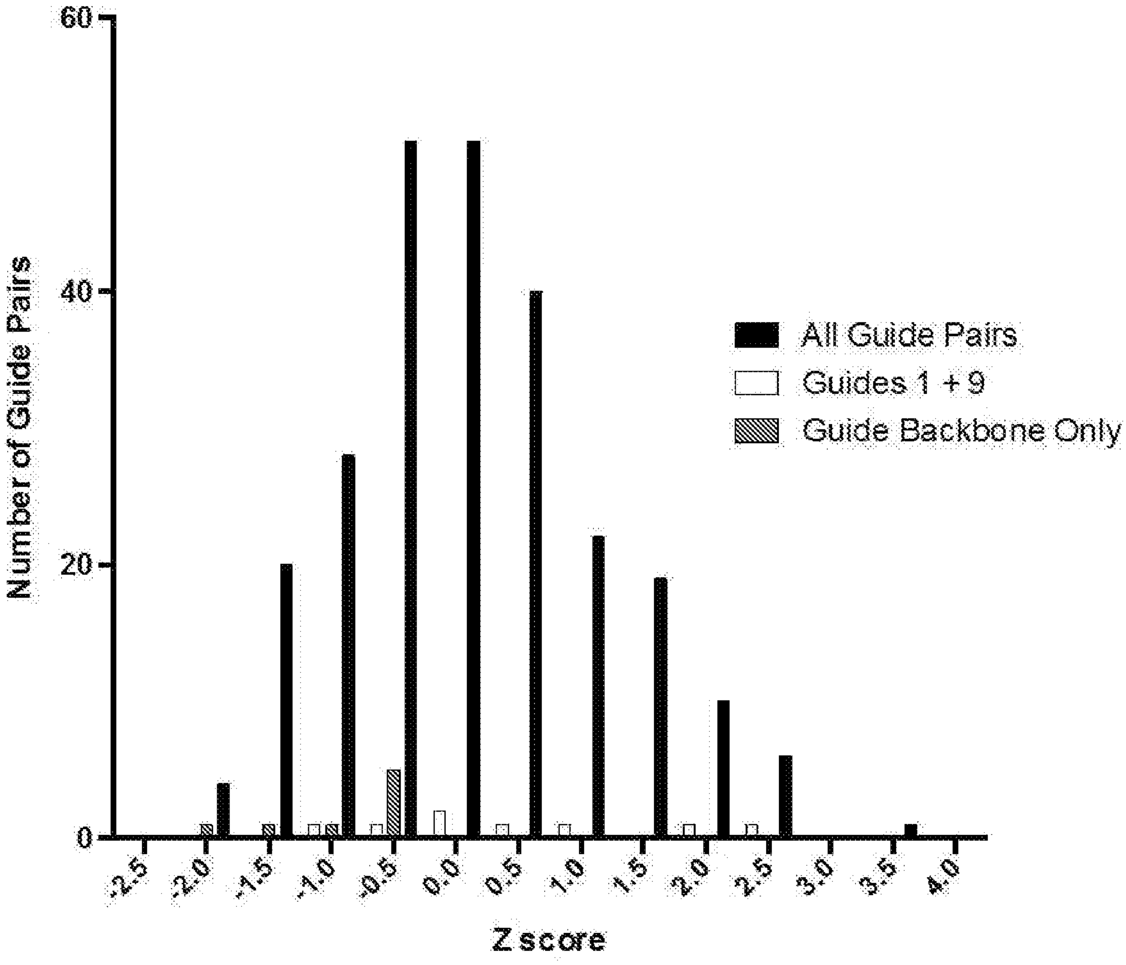
60
40
20
0
Number of Guide Pairs
All Guide Pairs
Guides 1 + 9
Guide Backbone Only
-2.5
-2.0
-1.5
-1.0
-0.5
0.0
0.5
1.0
1.5
2.0
2.5
3.0
3.5
4.0
Z score

Figure 25

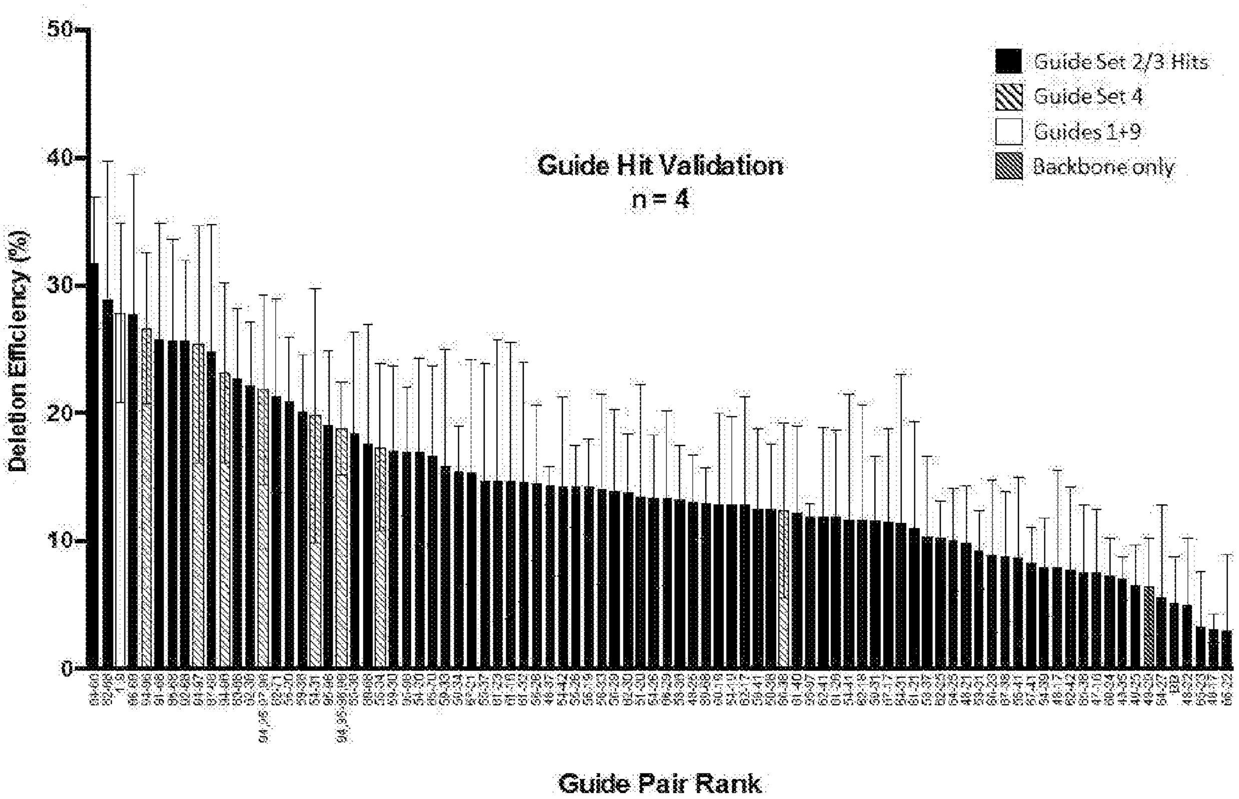
Guide Hit Validation
n = 4
Guide Set 2/3 Hits
Guide Set 4
Guides 1+9
Backbone only
Deletion Efficiency (%)
0
10
20
30
40
50
Guide Pair Rank

Fig. 26

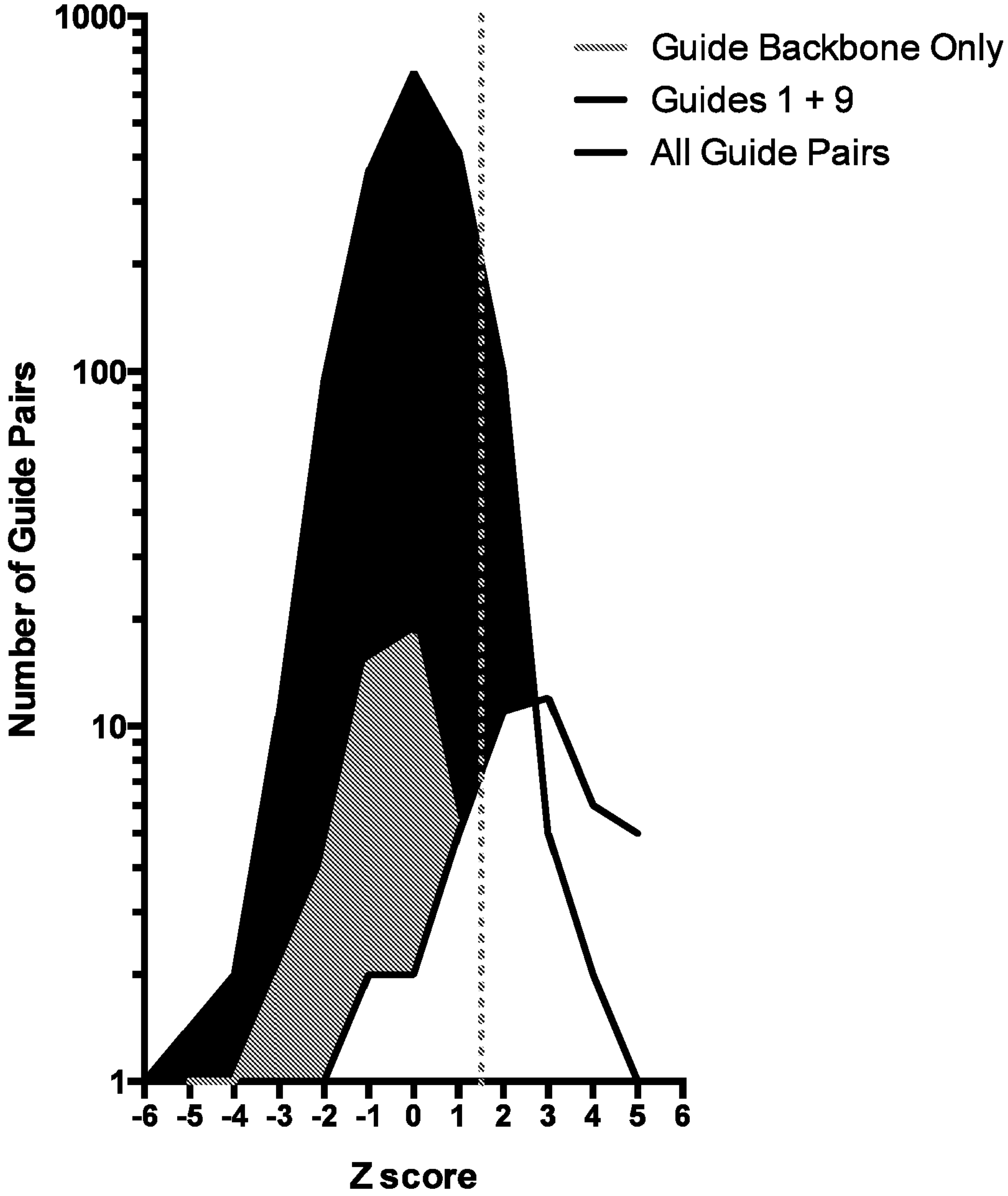
1000
100
10
1
Number of Guide Pairs
-6 -5 -4 -3 -2 -1 0 1 2 3 4 5 6
Z score
Guide Backbone Only
Guides 1 + 9
All Guide Pairs

Europäisches Patentamt
European Patent Office
Office européen des brevets

# EUROPEAN SEARCH REPORT

Application Number
EP 20 17 2019

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2014/197748 A2 (UNIV DUKE [US]) 11 December 2014 (2014-12-11) * the whole document * ----- | 1-14 | INV. C12N15/113 C12N15/10 C12N9/22 C12N9/52 |
| A | DAVID G. OUSTEROUT ET AL: "Multiplex CRISPR/Cas9-based genome editing for correction of dystrophin mutations that cause Duchenne muscular dystrophy", NATURE COMMUNICATIONS, vol. 6, 18 February 2015 (2015-02-18), page 6244, XP055196515, DOI: 10.1038/ncomms7244 * the whole document * ----- | 1-14 | |
| A | WO 2014/204728 A1 (BROAD INST INC [US]; MASSACHUSETTS INST TECHNOLOGY [US]; ZHANG FENG [U) 24 December 2014 (2014-12-24) * the whole document * ----- | 1-14 | |
| A | WO 2014/093635 A1 (BROAD INST INC [US]; MASSACHUSETTS INST TECHNOLOGY [US]; ZHANG FENG [U) 19 June 2014 (2014-06-19) * the whole document * ----- | 1-14 | TECHNICAL FIELDS SEARCHED (IPC) C12N |
| A | WO 2014/204726 A1 (BROAD INST INC [US]; MASSACHUSETTS INST TECHNOLOGY [US]; ZHANG FENG [U) 24 December 2014 (2014-12-24) * the whole document * ----- -/-- | 1-14 | |

The present search report has been drawn up for all claims

| Place of search | Date of completion of the search | Examiner |
|---|---|---|
| The Hague | 13 October 2020 | Spindler, Mark-Peter |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 20 17 2019

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | A E Friedland ET AL: "Staphyloccocus aureus Cas9: an alternative Cas9 for genome editing applications", , 12 January 2015 (2015-01-12), page 1, XP055292927, Retrieved from the Internet: URL:https://www.editasmedicine.com/wp-content/uploads/2019/10/AEF-Poster-Keystone-2015.pdf [retrieved on 2016-08-02] * the whole document * ----- | 1-14 | |
| | | | TECHNICAL FIELDS SEARCHED (IPC) |

The present search report has been drawn up for all claims

| Place of search | Date of completion of the search | Examiner |
|---|---|---|
| The Hague | 13 October 2020 | Spindler, Mark-Peter |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

page 2 of 2

Europäisches Patentamt
European Patent Office
Office européen des brevets

Application Number
EP 20 17 2019

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 20 17 2019

The Search Division considers that the present European patent application does not comply with the requirements of unity of invention and relates to several inventions or groups of inventions, namely:

1. claims: 1-14

CRISPR/Cas compositions/systems/vectors targeting exon 51 of the DMD gene

1.1. claims: 1-14(partially)

subject-matter relating to the combination of two gRNAs as defined by SEQ ID NO: 18458 and 2048

1.2. claims: 1-14(partially)

subject-matter relating to the combination of two gRNAs as defined by SEQ ID NO: 19199 and 18720

1.3. claims: 1-14(partially)

subject-matter relating to the combination of two gRNAs as defined by SEQ ID NO:4709 and 18720

1.4. claims: 1-14(partially)

subject-matter relating to the combination of two gRNAs as defined by SEQ ID NO:481 and 2048

1.5. claims: 1-14(partially)

subject-matter relating to the combination of two gRNAs as defined by SEQ ID NO: 4709 and 22349

1.6. claims: 1-14(partially)

subject-matter relating to the combination of two gRNAs as defined by SEQ ID NO: 5869 and 3536

1.7. claims: 1-14(partially)

subject-matter relating to the combination of two gRNAs as defined by SEQ ID NO: 18752 and 17253

1.8. claims: 1-14(partially)

subject-matter relating to the combination of two gRNAs as defined by SEQ ID NO: 20145 and 3536

1.9. claims: 1-14(partially)

subject-matter relating to the combination of two gRNAs as defined by SEQ ID NO: 19187 and 7252

1.10. claims: 1-14(partially)

Europäisches Patentamt
European Patent Office
Office européen des brevets

# LACK OF UNITY OF INVENTION
# SHEET B

Application Number
EP 20 17 2019

The Search Division considers that the present European patent application does not comply with the requirements of unity of invention and relates to several inventions or groups of inventions, namely:

subject-matter relating to the combination of two gRNAs as defined by SEQ ID NO: 18752 and 3536

1.11. claims: 1-14(partially)

subject-matter relating to the combination of two gRNAs as defined by SEQ ID NO: 19187 and 7419

---

Please note that all inventions mentioned under item 1, although not necessarily linked by a common inventive concept, could be searched without effort justifying an additional fee.

## ANNEX TO THE EUROPEAN SEARCH REPORT ON EUROPEAN PATENT APPLICATION NO.

EP 20 17 2019

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2020

| Patent document cited in search report | Publication date | Patent family member(s) | Publication date |
|---|---|---|---|
| WO 2014197748 A2 | 11-12-2014 | AU 2014274840 A1 | 21-01-2016 |
| | | AU 2020203924 A1 | 02-07-2020 |
| | | CA 2914519 A1 | 11-12-2014 |
| | | CN 105658805 A | 08-06-2016 |
| | | EP 3004370 A2 | 13-04-2016 |
| | | EP 3417880 A1 | 26-12-2018 |
| | | EP 3539573 A1 | 18-09-2019 |
| | | JP 2016521555 A | 25-07-2016 |
| | | JP 2020058349 A | 16-04-2020 |
| | | KR 20160023765 A | 03-03-2016 |
| | | US 2016201089 A1 | 14-07-2016 |
| | | US 2018320197 A1 | 08-11-2018 |
| | | WO 2014197748 A2 | 11-12-2014 |
| WO 2014204728 A1 | 24-12-2014 | AU 2014281030 B2 | 09-07-2020 |
| | | BR 112015031611 A2 | 12-12-2017 |
| | | CA 2915845 A1 | 24-12-2014 |
| | | CN 105683379 A | 15-06-2016 |
| | | DK 3011032 T3 | 20-01-2020 |
| | | EP 3011032 A1 | 27-04-2016 |
| | | EP 3620524 A1 | 11-03-2020 |
| | | ES 2767318 T3 | 17-06-2020 |
| | | JP 6738729 B2 | 12-08-2020 |
| | | JP 2016523082 A | 08-08-2016 |
| | | JP 2020063238 A | 23-04-2020 |
| | | KR 20160019553 A | 19-02-2016 |
| | | RU 2016101178 A | 19-11-2018 |
| | | SG 10201710488T A | 30-01-2018 |
| | | SG 11201510327T A | 28-01-2016 |
| | | US 2016153004 A1 | 02-06-2016 |
| | | WO 2014204728 A1 | 24-12-2014 |
| WO 2014093635 A1 | 19-06-2014 | AU 2013359212 A1 | 30-07-2015 |
| | | AU 2017202542 A1 | 11-05-2017 |
| | | AU 2019236591 A1 | 17-10-2019 |
| | | CA 2894684 A1 | 19-06-2014 |
| | | CN 105209621 A | 30-12-2015 |
| | | DK 2898075 T3 | 27-06-2016 |
| | | DK 3064585 T3 | 27-04-2020 |
| | | EP 2898075 A1 | 29-07-2015 |
| | | ES 2576126 T3 | 05-07-2016 |
| | | ES 2786193 T3 | 09-10-2020 |
| | | HK 1207119 A1 | 22-01-2016 |
| | | JP 6395765 B2 | 26-09-2018 |
| | | JP 6552965 B2 | 31-07-2019 |
| | | JP 6665230 B2 | 13-03-2020 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 17 2019

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2020

| Patent document cited in search report | Publication date | Patent family member(s) | Publication date |
|---|---|---|---|
| | | JP 2016182140 A | 20-10-2016 |
| | | JP 2016501532 A | 21-01-2016 |
| | | JP 2018143253 A | 20-09-2018 |
| | | JP 2020099333 A | 02-07-2020 |
| | | KR 20150105634 A | 17-09-2015 |
| | | PL 2898075 T3 | 30-09-2016 |
| | | PT 2898075 E | 16-06-2016 |
| | | SG 10201801969T A | 27-04-2018 |
| | | SG 10201912327S A | 27-02-2020 |
| | | SG 11201504519T A | 30-07-2015 |
| | | US 2014186919 A1 | 03-07-2014 |
| | | US 2014242700 A1 | 28-08-2014 |
| | | US 2014273234 A1 | 18-09-2014 |
| | | US 2014335620 A1 | 13-11-2014 |
| | | US 2017198269 A1 | 13-07-2017 |
| | | WO 2014093635 A1 | 19-06-2014 |
| WO 2014204726 A1 | 24-12-2014 | AU 2014281028 A1 | 28-01-2016 |
| | | CA 2915842 A1 | 24-12-2014 |
| | | CN 107995927 A | 04-05-2018 |
| | | EP 3011031 A1 | 27-04-2016 |
| | | JP 6738728 B2 | 19-08-2020 |
| | | JP 2016524472 A | 18-08-2016 |
| | | JP 2020026438 A | 20-02-2020 |
| | | KR 20160030187 A | 16-03-2016 |
| | | RU 2016101200 A | 28-03-2019 |
| | | SG 10201710486X A | 30-01-2018 |
| | | SG 11201510284X A | 28-01-2016 |
| | | US 2016153005 A1 | 02-06-2016 |
| | | WO 2014204726 A1 | 24-12-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62141833 B
- US 62310479 B
- US 2014057905 W


### Non-patent literature cited in the description


- **ROBERTS et al.** *Genomics,* 1993, vol. 16, 536-8
- **ERVASTI et al.** *Journal of Cell Biology,* 1993, vol. 122 (4), 809-823
- **DROUSIOTOU et al.** *Genetic Testing,* 1998, vol. 2, 55-60
- **EMERY.** *Neuromuscular Disorders,* 1991, vol. 1, 19-29
- **BRADLEY et al.** *Seminars in Neonatology,* 1998, vol. 3, 27-34
- **EMERY.** Duchenne Muscular Dystrophy. Oxford University Press, 1988
- **KUNKEL et al.** *Nature,* 1986, vol. 322, 73-75
- **WAPENNAR et al.** *Genomics,* 1998, vol. 2, 10-18
- **WHITE et al.** *American Journal of Human Genetics,* 2002, vol. 71, 365-374
- **BOLAND et al.** *Pediatric Neurology,* 1996, vol. 14 (1), 7-12
- **SUSSMAN et al.** *American Academy of Orthopedic Surgery,* 2002, vol. 10 (2), 138-151
- **BEGGS.** *Circulation,* 1997, vol. 95, 2344-2347
- **MANZUR et al.** *Cochrane Database of Systemic Reviews 2008*
- **MANZUR et al.** *Archives of Diseases of Childhood,* 2008, vol. 93 (11), 986-990
- **VILLANOVA et al.** *American Journal of Physical Medicine and Rehabilitation,* 2014, vol. 93 (7), 595-599
- **COLAN.** *Circulation,* 2005, vol. 112, 2756-2758
- **DUBOC et al.** *American Heart Journal,* 2007, vol. 154, 596-602
- **AARTSMA-RUS et al.** *Human Mutation,* 2009, vol. 30, 293-299
- **FLANIGAN et al.** *Human Mutation,* 2009, vol. 30, 1657-1666
- **BLADEN et al.** *Human Mutation,* 2015, vol. 36 (2
- **JINEK et al.** *SCIENCE,* 2012, vol. 337 (6096), 816-821
- **LI.** *Cell Research,* 2008, vol. 18, 85-98
- **CALDECOTT.** *Nature Reviews Genetics,* August 2008, vol. 9, 619-631
- **MARTEIJN et al.** *Nature Reviews Molecular Cell Biology,* 2014, vol. 15, 465-481
- **LEE et al.** *Nano Lett,* 2012, vol. 12, 6322-27
- **LEE et al.** *Nano Lett,* vol. 12, 6322-27
- **C.E. NELSON et al.** *Science,* 2015
- **M. TABEBORDBAR et al.** *Science,* 2015
- **C. LONG et al.** *Science,* 2015
- **RAN et al.** *Nature,* 09 April 2015, vol. 520 (7546), 186-91